# EUROPEAN PATENT SPECIFICATION

(11) **EP 1 280 904 B1**
(45) Date of publication and mention of the grant of the patent: **14.12.2005**
(21) Application number: 01933045.5
(22) Date of filing: 04.05.2001
(51) Int. Cl.: C12N 15/12, C07K 14/705, C12N 15/62, C12N 15/10, C12Q 1/68, C12N 5/10, G01N 33/68, C07K 16/28, A61P 25/04

(54) **PAIN SIGNALING MOLECULES**
SCHMERZ SIGNALISIERENDE MOLEKÜLE
MOLECULES DE SIGNALISATION DE LA DOULEUR

(30) Priority: 04.05.2000 US 202027 P; 01.08.2000 US 222344 P; 03.11.2000 US 704707; 19.04.2001 US 285493 P
(43) Date of publication of application: 05.02.2003
(73) Proprietor: CALIFORNIA INSTITUTE OF TECHNOLOGY, Pasadena, California 91125 (US)
(72) Inventor: ANDERSON, David, J., Altadena, CA 91001 (US); DONG, Xinzhong, Pasadena, CA 91106 (US); ZYLKA, Mark, Pasadena, CA 91106 (US); HAN, Sang-Kyou, Arcadia, CA 91006 (US); SIMON, Melvin, San Marino, CA 91108 (US)
(74) Representative: Becker Kurig Straus
(86) International application number: PCT/US2001/014519
(87) International publication number: WO 2001/083555

(56) References cited:
- WO-A-99/32519
- JP-A- 2000 023 676
- MA QIUFU ET AL: "NEUROGENIN1 and NEUROGENIN2 control two distinct waves of neurogenesis in developing dorsal root ganglia." GENES & DEVELOPMENT, vol. 13, no. 13, 1 July 1999 (1999-07-01), pages 1717-1728, XP002181516 ISSN: 0890-9369 cited in the application
- MARCHESE A ET AL: "Novel GPCRs and their endogenous ligands: expanding the boundaries of physiology and pharmacology" TRENDS IN PHARMACOLOGICAL SCIENCES, ELSEVIER TRENDS JOURNAL, CAMBRIDGE, GB, vol. 20, no. 9, 1 September 1999 (1999-09-01), pages 370-375, XP004178194 ISSN: 0165-6147
- KRESS M ET AL: "Capsaicin, protons and heat: new excitement about nociceptors" TRENDS IN PHARMACOLOGICAL SCIENCES, ELSEVIER TRENDS JOURNAL, CAMBRIDGE, GB, vol. 20, no. 3, 1 March 1999 (1999-03-01), pages 112-118, XP004165931 ISSN: 0165-6147

## Description

### Background of the Invention

### Field of the Invention

The invention relates generally to novel genes expressed in normal but not Neurogenin-1-deficient animals. The invention relates specifically to a novel family of G protein-coupled receptors and a novel family of two-transmembrane segment proteins that are expressed in dorsal root ganglia, and a method of screening for genes specifically expressed in nociceptive sensory neurons.

### Description of the Related Art

The treatment of acute and chronic intractable pain is a major target of drug development in the pharmaceutical industry. Pain sensation is mediated by primary sensory neurons in the dorsal root ganglia (DRG), which project peripherally to the skin and centrally to the spinal cord. These neurons express signaling molecules, such as receptors, ion channels and neuropeptides, which are involved in pain sensation. One example is the so-called Vanilloid Receptor-1 (VR-1), which is activated by capsaicin (chili pepper) as well as by heat and acid. Such pain signaling molecules may also influence pain sensation indirectly by acting as positive or negative modulators of the sensory pathway. Searching for drugs that interact with such signaling molecules, for example as receptor agonists or antagonists, is an important approach to the discovery of new therapeutics for the treatment of pain. New candidate signaling molecules expressed by pain-sensing ("nociceptive")sensory neurons are therefore highly desirable targets for new drug screening and drug discovery efforts. The present inventors have previously identified a novel family of basic helix-loop-helix (bHLH) transcription factors, called the Neurogenins (Ngns), which are essential for the development of sensory neurons in the DRG. Different Ngns are required for the development of different subsets of sensory neurons. In particular, Ngn1 is necessary for the development of most if not all nociceptive sensory neurons. In Ngn1^{-/-} mutant mice, although DRG are still present, they are reduced in size and the majority of nociceptive neurons, identified by expression of markers such as trkA and VR-1, are missing (Ma et al. Genes&Develop, 13: 1717-1728, (1999)). These results suggested that the isolation of genes expressed in wild-type (normal) but not Ngn1^{-/-} DRG might lead to the identification of novel drug target molecules expressed in differentiating or mature nociceptive sensory neurons.

While pain is usually a natural consequence of tissue injury, as the healing process commences the pain and tenderness associated with the injury resolve. However, some individuals experience pain without an obvious injury or suffer protracted pain after an initial insult. In addition, chronic or intractable pain may occur in association with certain illnesses, such as, for example, bone degenerative diseases, terminal cancer, AIDS, and Reflex sympathetic dystrophy (RSD). Such patients may be unable to receive relief with currently-available pain-relieving (anti-nociceptive) drugs, such as opioid compounds, e.g. morphine, due to problems such as dependence and tolerance. Therefore, there is a great need for novel therapeutic agents for the treatment of pain, in particular chronic pain.

JP2000023676 relates to the provision of a new seven-pass transmembrane receptor protein ERG 5, which is useful for the detection of tumor cells or the development of medicaments controlling the multiplication and the function of tumor cells. The protein is obtained by the screening a cDNA library arising from mouse lung by means of using cDNA of ERG family obtained from experimental allergic encephalomyelitis critical auto-reactive T cell 4R312 arising from mouse as a probe, integrating a gene coding the resultant ERG 5 into a vector to product the expression system and then introducing into a host cell for expression.

Marchese et al. (Novel GPCRs and their endogenous ligands: expanding the boundaries of physiology and pharmacology; TiPS; Sep.(1999), vol. 20, no. 9, pp. 370-75) relates to the GPCR family and wide spread occurrence of their members. In general, clues for the function of GPCRs or even their ligand identity may be obtained by testing GPCR knockout mice.

### Summary of the Invention

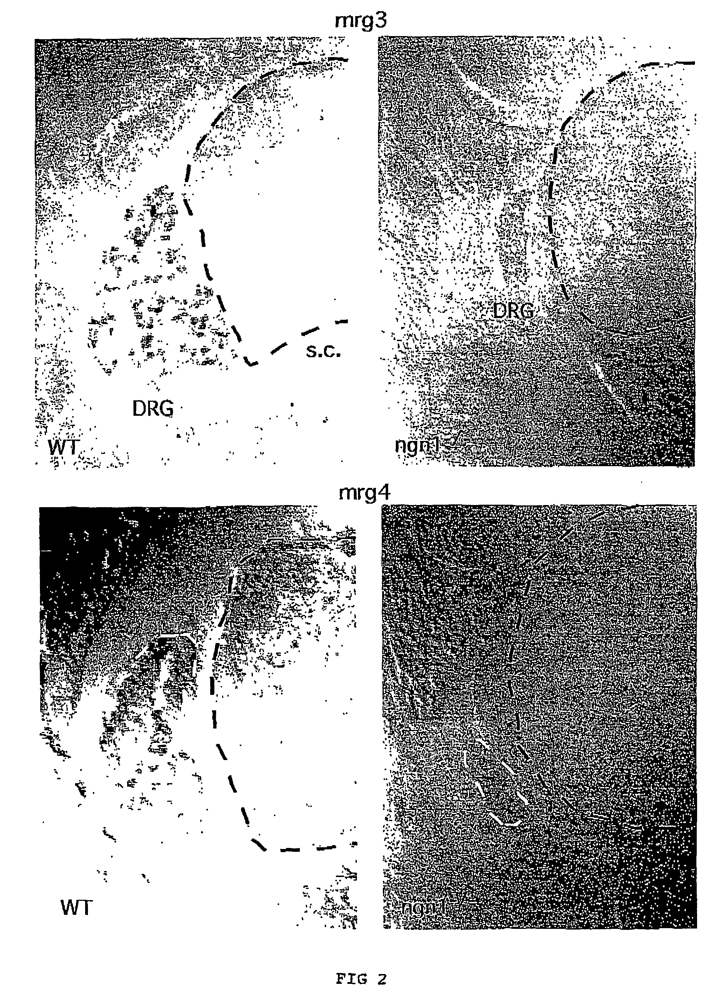

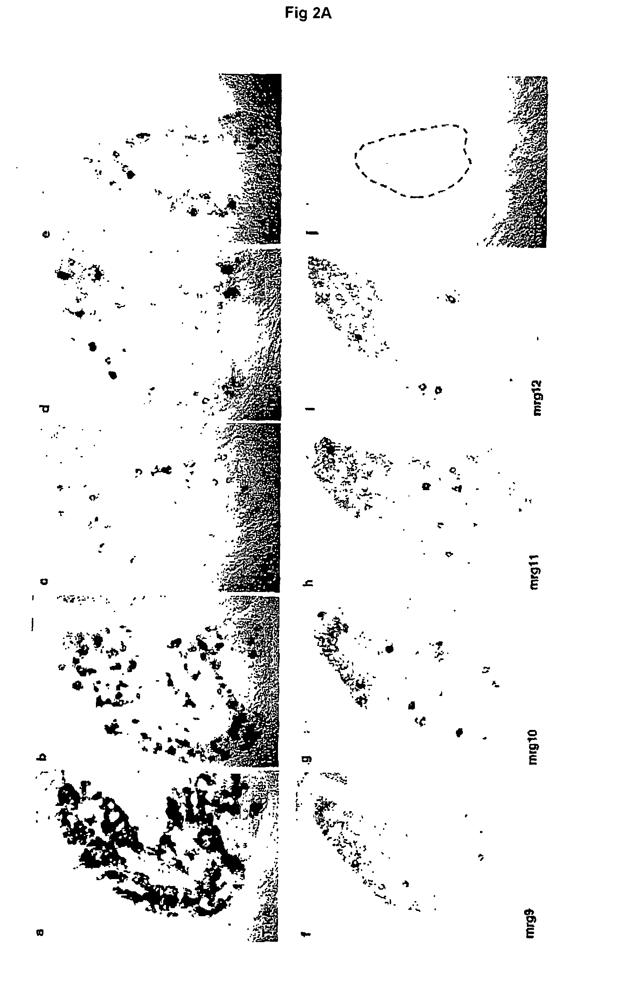

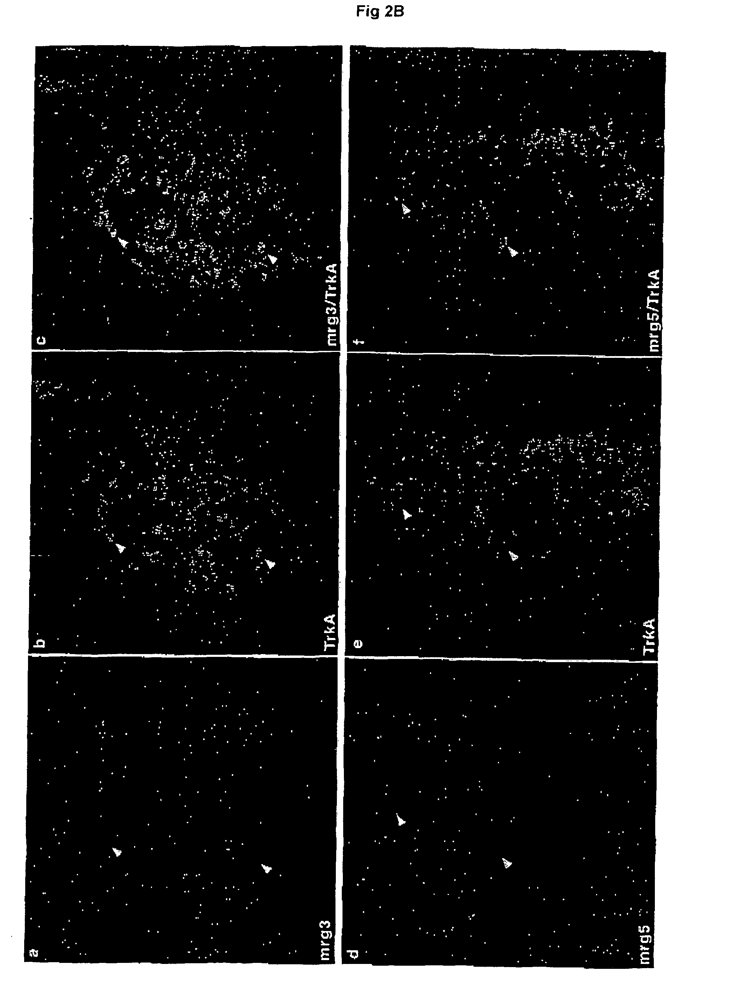

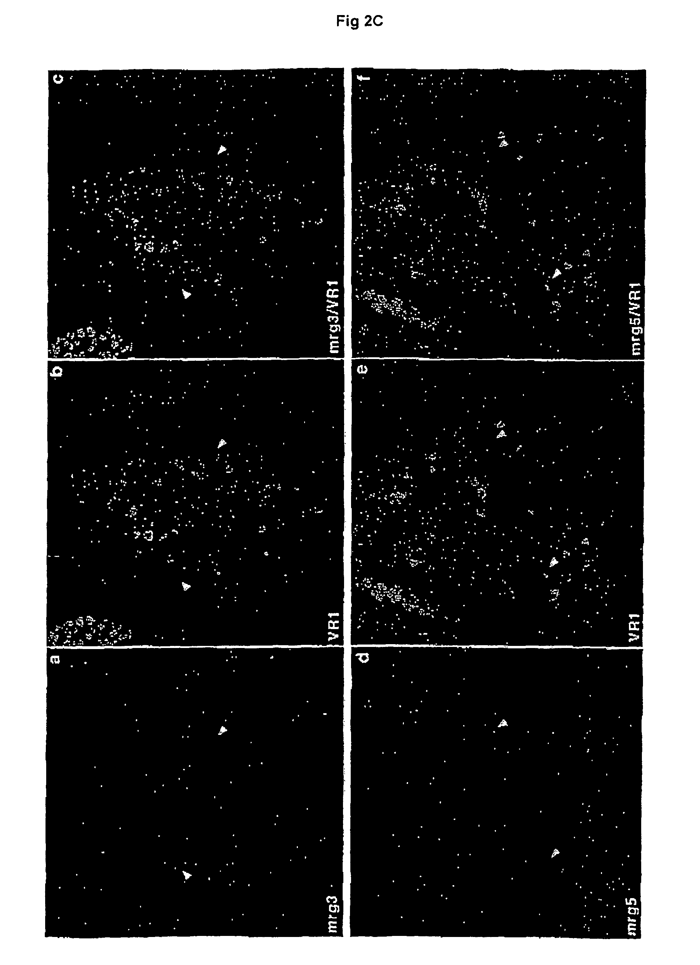

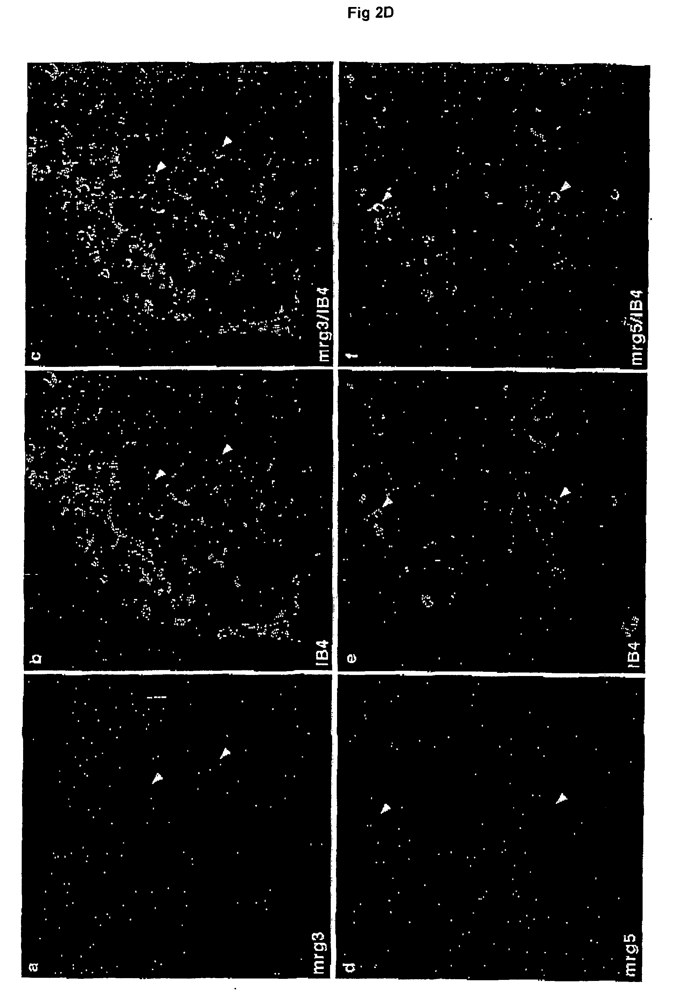

The present inventors have carried out a screen for genes expressed in wild-type but not Ngn1⁺ DRG using positive selection-based differential hybridization. This screen has identified both known signaling molecules involved in nociceptive neuron function, such as VR-1, and novel signaling molecules that are highly specifically expressed in nociceptive sensory neurons. The present invention therefore includes the discovery of new genes that are expressed in normal mice but not in Ngn1 null mutant mice. One family of novel genes isolated from the screen encodes a receptor protein with 7 transmembrane segments, mrg, a characteristic of G protein-coupled receptors. Subsequent staining experiments (see Fig. 2, 2A-D) confirmed that mrg genes were expressed specifically in subsets of nociceptive neurons in DRG.

In particular, the invention includes isolated nucleic acid molecules that encode a mrg protein selected from the group consisting of an isolated nucleic acid molecule that encodes the amino acid sequence of SEQ ID NOS: 2 and being characterized by the ability to be activated by an RF-amide-peptide an isolated nucleic acid molecule which hybridizes to the complement of a nucleic acid molecule comprising SEQ ID NOS: 1 or 11 and an isolated nucleic acid molecule which hybridizes to the complement of a nucleic acid molecule that encodes the amino acid sequence of SEQ ID NOS: 2 or 12. Nucleic acid molecules of the invention also include those that encode a protein that is expressed in dorsal root ganglia and have at least 80% sequence identity, and preferably at least about 90% sequence identity through the coding sequences of SEQ ID NOS: 1 or 11.

The present invention also includes the nucleic acid molecules described above operably linked to one or more expression control elements, including vectors comprising the isolated nucleic acid molecules. The invention further includes host cells transformed to contain the nucleic acid molecules of the invention and methods for producing a protein comprising the step of culturing a host cell transformed with a nucleic acid molecule of the invention under conditions in which the protein is expressed.

The invention further provides an isolated Mrg polypeptide selected from the group consisting of an isolated polypeptide comprising the amino acid sequence of SEQ ID NOS: 2 or 12 being characterized by the ability to be activated by an RF-amide peptide. Polypeptides of the invention also include polypeptides with an amino acid sequence having at least about 40%, 50%, 60%, 70% or 75% amino acid sequence identity with the sequence set forth in SEQ ID NO: 2 or 12 more preferably at least about 80%, even more preferably at least about 90%, and most preferably at least about 95% sequence identity with these sequences.

The invention further provides an isolated antibody that specifically binds to a polypeptide of the invention, including monoclonal and polyclonal antibodies.

The invention further provides in vitro methods of identifying an agent which modulates the expression of a nucleic acid encoding a protein of the invention, comprising the steps of: exposing cells which express the nucleic acid to the agent; and determining whether the agent modulates expression of such nucleic acid, thereby identifying an agent which modulates the expression of a nucleic acid encoding the protein.

The invention further provides in vitro methods of identifying an agent which modulates at least one activity of a protein of the invention, comprising the steps of: exposing cells which express the protein to the agent; and determining whether the agent modulates at least one activity of the protein, thereby identifying an agent which modulates at least one activity of the protein.

The invention further provides in vitro methods of identifying binding partners for a protein of the invention, comprising the steps of: exposing said protein to a potential binding partner; and determining if the potential binding partner binds to the protein, thereby identifying binding partners for the protein.

The present invention further provides in vitro methods of modulating the expression of a nucleic acid encoding a protein of the invention, comprising the step of: administering an effective amount of an agent which modulates the expression of a nucleic acid encoding the protein. The invention also provides methods of modulating at least one activity of a protein of the invention, comprising the step of: administering an effective amount of an agent which modulates at least one activity of the protein.

The present invention further includes non-human transgenic animals modified to contain the nucleic acid molecules of the invention or mutated nucleic acid molecules such that expression of the polypeptides of the invention is prevented.

The invention further the use of a composition for pain treatment.

### Brief Description of the Drawings

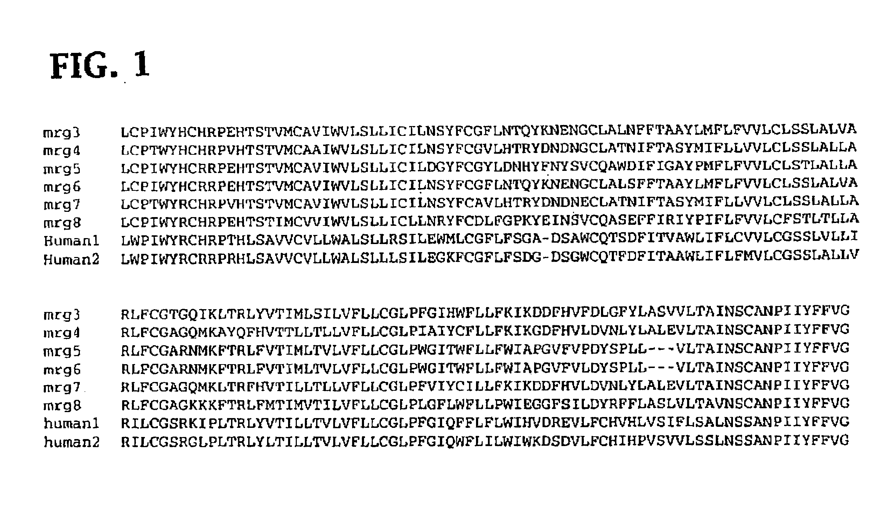

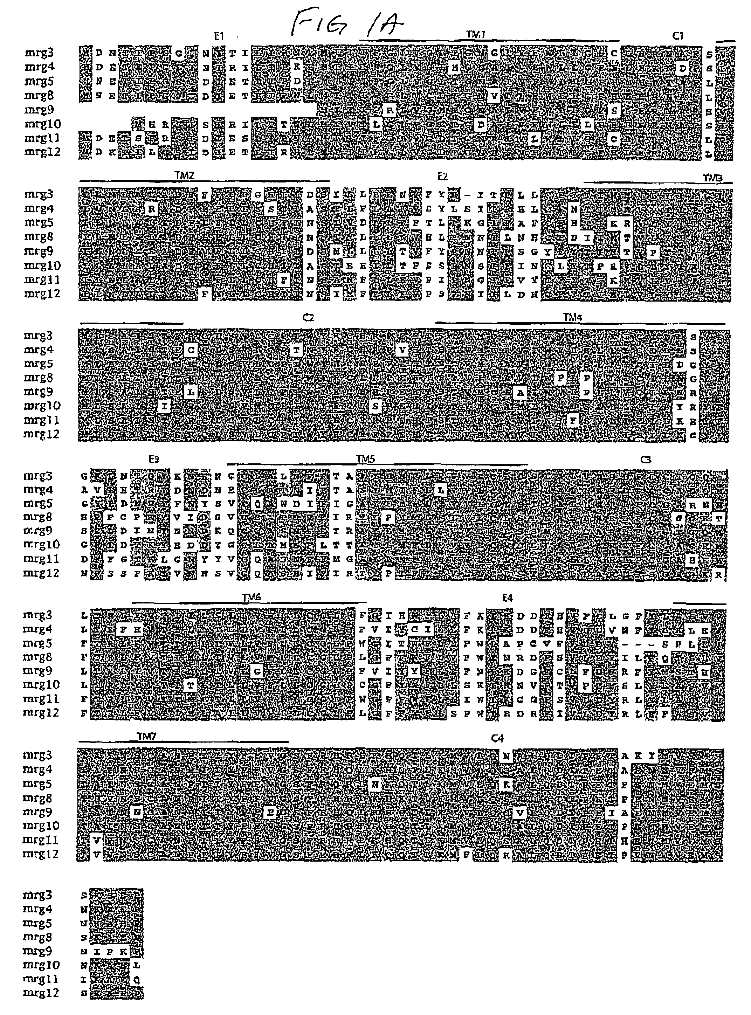

Figure 1 shows the alignment of a homologous region of the amino acid sequences of SEQ ID NO: 2, 4, 6, 8, 10 and 12, and also of two human members of the mrg family (SEQ ID NOS: 16 and 18), wherein Seq. ID NO: 2 and 12 form part of the present invention.

Figure 1A indicates that mrgs define a Novel G protein-couple receptor Gene Family. Amino acid sequences of eight mouse full-length mrg genes were aligned using ClustalW. Identical residues in >50% of the predicted proteins are darkly shaded; conservative substitutions are highlighted in light gray. The approximate locations of predicted transmembrane domain 1.7 are indicated on top of the sequences as TM1-TM7. The predicted extracellular and cytoplasmic domains are indicated as E1-E7 and C1-C7 respectively.

The microscopy images of in situ hybridization in Figure 2 show the localization of antisense staining against a nucleotide of SEQ ID NO: 2 ("mrg3") and of SEQ ID NO: 4 ("mrg4") which does not form part of the present invention in transverse sections of dorsal root ganglia (DRG) from newborn wild type (WT) and Neurogenin1 null mutant (Ngn1⁺) mice. White dashed lines outline the DRG and black dashed lines outline the spinal cord. Note that in the Ngn1⁺ mutant, the size of the DRG is severely reduced due to the loss of nociceptive sensory neurons, identified using three other independent markers (trkA; VR-1 and SNS-TTXi (Ma et al., (1999)). mrg3 is expressed in a subset of DRG in WT mice (A) but is absent in the Ngn1^{-/-} DRG (B). mrg4 is expressed in a smaller subset of DRG than that of mrg3 (C). It is also absent in the Ngn1^{-/-}DRG (D). The loss of mrg-expressing neurons in the Ngn1^{-/-} DRG indicates that these neurons are likely to be nociceptive.

Figure 2A shows expression of mrgs in subsets of dorsal root ganglia (DRG) neurons. Frozen transverse sections of DRG from wild-type (a-i) and ngn1^{-/-} (j) mutant new born mice were annealed with antisense digoxigenin RNA probes, and hybridization was visualized with an alkailine phosphatase-conjugated antibody. Positive signals are shown as dark purple stainings. TrkA is expressed in a large portion of wild-type DRG neurons (a) but absent in ngn1^{-/-} (data not shown). Each of the eight mrg genes (b-i) is expressed in a small subset of neurons in wild-type DRG in completely absent in ngn1^{-/-} DRG (j and data not shown). Black dash line outlines the ngn1^{-/-} mutant DRG.

Figure 2B shows that mrgs are expressed by TrkA⁺ nociceptive neurons. Double labeling technique was used to colocalize TrkA (b,e) and mrgs (a,d) in DRG neurons. During the double labeling experiments frozen sections of wild-type DRG were undergone in situ hybridizations with either mrg3 (a-c) or mrg5 (d-f) fluorescein-labeled antisense RNA probes followed by anti-TrkA antibody immunostaining. The same two frames (a and b, d and e) were digitally superimposed to reveal the extent of colocalization (c, f). The white arrowheads indicate examples of double positive cells.

Figure 2C shows that mrgs and VR1 define two different populations of nociceptive neurons in DRG. The combination of in situ hybridizations with either mrg3 or mrg5 fluorescein-labeled antisense RNA probes and anti-VR1 antibody immunostaining demonstrated that neither mrg3 (a-c) nor mrg5 (d-f) were expressed by VR1-positive neurons. In the merged images (c,f), there are no colocalizations of VR1 with either mrg3 or mrg5. The white arrowheads are pointed to mrgs-expressing but VR1-negative nociceptive neurons.

Figure 20 shows that mrgs are expressed by IB4⁺ nociceptive neurons. Double labeling technique was used to colocalize IB4 (b,e) and mrgs (a,d) in DRG neurons. The expressions of mrg3 and mrg5 were visualized by in situ hybridization as described before. The same DRG sections were subsequently undergone through FITC-conjugated lectin IB4 binding. In the merged images (c,f), there are extensive overlappings between mrgs and IB4 stainings (yellow neurons indicated by arrowheads).

Figure 3 compares the hydrophobicity plots predicting the transmembrane regions of the amino acid sequence of (A) mrg3 (SEQ ID NO: 2); (B) human1 gene (SEQ ID NO: 15); and (C) human2 gene (SEQ ID NO: 17). More positive values indicate hydrophobicity.

Figure 4 compares the hydrophobicity plots predicting the transmembrane regions of the amino acid sequence of (A) mouse drg12 (SEQ ID NO: 14); (B) human drg12 (SEQ ID NO: 19)

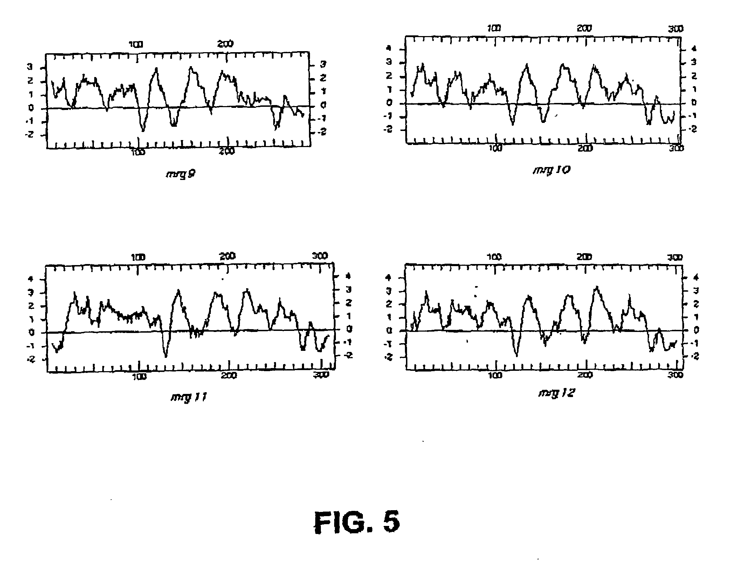

Figure 5 compares the hydrophobicity plots predicting the transmembrane regions of the amino acid sequence of mrg9 (SEQ ID NO: 21); mrg10 (SEQ ID NO: 23); mrg11 (SEQ ID NO: 25) and mrg12 (SEQ ID NO: 27).

Figure 6A is an alignment of the amino acid sequences of MRGA1-A8, deduced from nucleotide sequences of cDNA and BAC clones from strain C57BL/6J mice. The predicted locations of the transmembrane (TM1-TM7), extracellular (E1-E4), and cytoplasmic (C1-C4) domains are indicated above the aligned sequences.

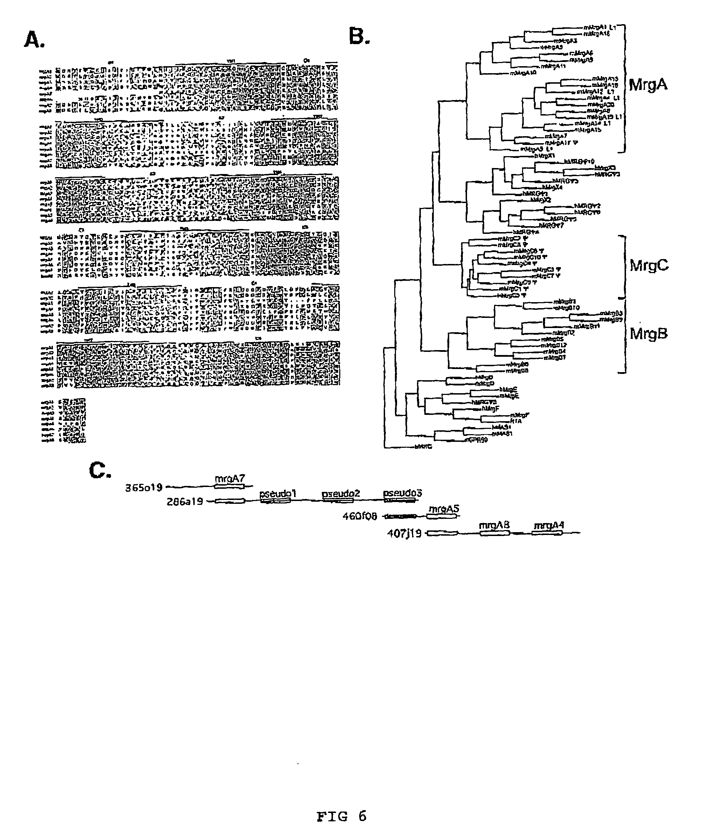

Figure 6B depicts a phylogenetic analysis of MRG family members identified from database searches. The protein sequences of all MRGs were aligned using CLUSTALW (Thompson et al. Nucleic Acids Res 22: 4673-80 (1994)). The dendrogram was generated with the PHYLUP software package using the Neighbor-Joining method and 1,000 bootstrap trials. The horizontal length of the branches is proportional to the number of amino acid changes. Vertical distances are arbitrary. Mouse (m)Mrg genes with retrotransposon sequences ^{~}650 nt 3' of their stop codon are highlighted (L1). All genes that are predicted to encode pseudogenes are indicated with the psi (Ψ) symbol.

Figure 6C shows the chromosomal organization of one mouse Mrg cluster deduced from analysis of overlapping BAC clones. The cluster contains four intact ORFs and three pseudogenes.

Figure 7A shows the distribution of nociceptive sensory neurons in a postnatal day 0 (PO) DRG as revealed by expression of the NGF receptor trkA. This population is selectively eliminated in Ngn1⁺ mutants (Ma et al. Genes & Dev. 13: 1717-1728 (1999)).

Figure 7B shows in situ hybridization with cRNA probes detecting MrgA1. MrgA1 is expressed in a pattern similar to that of trkA⁺ neurons on an adjacent section shown in Figure 7A.

Figure 7C shows in situ hybridization with cRNA probes detecting MrgA2-MrgA8.

Figure 7J shows that MrgA1 expression is eliminated in Ngn1^{-/-} mice, as is expression of other MrgA genes (not shown). Remaining DRG neurons are present in the area delimited by the dotted line, and can be visualized by expression of generic neuronal markers.

Figure 8 shows that expression of MrgAs is restricted to non-peptidergic nociceptors that project to inner lamina II. Shown are confocal microscopic images of in situ hybridizations using the Mrg probes indicated, combined with fluorescent antibody detection of trkA (A-D), substance P (I-L), CGRP (M-P), VR1 (Q-T) or staining with fluorescent isolectin IB4 (IB4; E-H). MrgA⁺ or MrgD⁺ cells co-express trkA and IB4 (A-H, arrowheads), but most do not express subP, CGRP or VR1 (I-T, arrowheads; arrows in I, M indicate a minor subset of MrgA1⁺ neurons that co-express SubP and CGRP).

Figure 9 is a schematic illustration of the restriction of MrgA (and MrgD) expression to non-peptidergic, IB4⁺, VR1 sensory neurons that project to lamina IIi (Snider and McMahon Neuron 20: 629-32 (1998)). Post-synaptic neurons in lamina IIi express PKC .

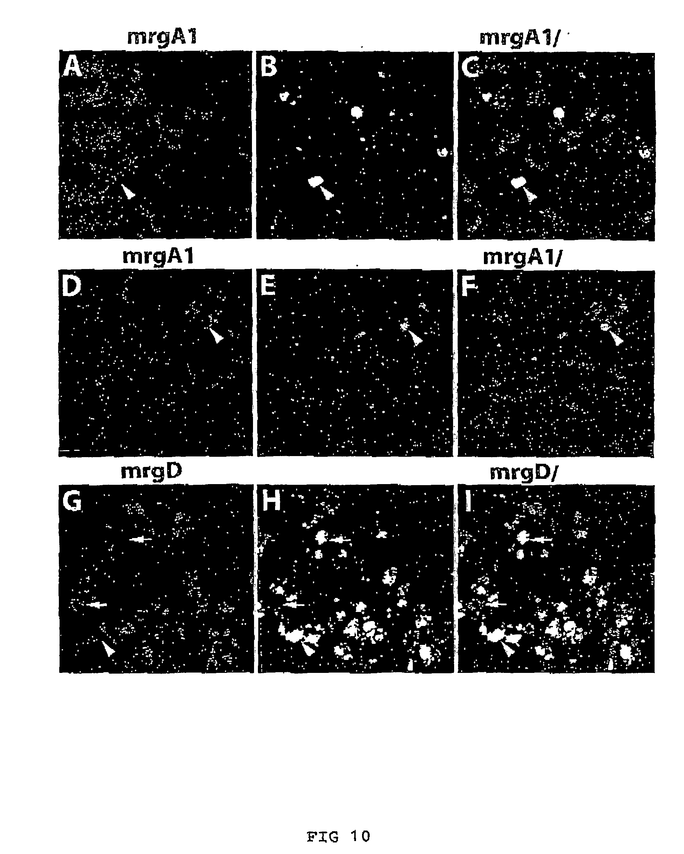

Figure 10 shows that individual sensory neurons co-express multiple MrgAs. (A-C) double label in situ hybridization with MrgA1 (A) and A3 (B). (D-F) double labeling with MrgA1 (D) and MrgA4 (E). In both cases, cells expressing MrgA3 or A4 are a subset of those expressing MrgA1 (C, F, arrowheads). Arrows in (F) indicate intranuclear dots of MrgA4 expression which may represent sites of transcription. (G-I) Double label in situ with MrgA1 and MrgD. Some overlap overlap between the two populations is seen (I, arrowhead), while most cells express one receptor but not the other (I, arrows). Approximately 15% of cells expressing either MrgA1 or MrgD co-express both genes. Vertical bars to the right of panels (C, F,l) represent a z-series viewed along the y-axis, horizontal bars below the panels a z-series viewed along the x-axis. (J, K) comparison of in situ hybridization signals obtained using a single MrgA probe (J) and a mixture of 7 MrgA probes (K). Approximately 1 % of neurons were labeled by the MrgA4 probe, while ^{~}4.5% were labeled by the mixed probe. The sum of the percentage of neurons labeled by the individual MrgA2-8 probes is ^{~}6.6%, suggesting that there is partial overlap within this population. (L) Venn diagram illustrating combinations of gene expression revealed by in situ analysis. The drawing is a conservative estimate of the number of subsets, since we do not yet know, for example, whether MrgAs2-8 partially overlap with MrgD. The sizes of the circles are not proportional.

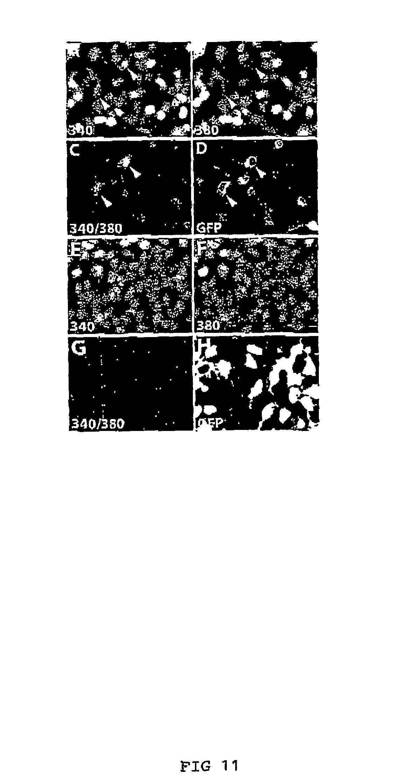

Figure 11 shows elevated intracellular free Ca⁺⁺ elicited by FLRF in HEK cells expressing MRGA1. (A, B) and (E, F) illustrate Fura-2 fluorescence at 340 nm (A, E) and 380 nm (B, F) in HEK-G ₁₅ cells expressing an MRGA1-GFP fusion protein (A-D) or GFP alone (E-H). The images were taken 2 minutes after the addition of 1 M of FLRFamide. The peri-nuclear, punctate distribution of MRGA1-GFP revealed by intrinsic GFP fluorescence (D, arrowheads) is characteristic of the ER-Golgi network, indicating membrane integration and intracellular transport of the receptors. In contrast, the control GFP protein is cytoplasmic (H). The intracellular Ca²⁺ ([Ca²⁺]ᵢ) release was determined from the FURA-2 340nM/380nM emission ratio (C, G). Note that MRGA1-expressing cells (but not surrounding untransfected cells) show an elevated ratio of Fura-2 fluorescence at 340/380 nm (C, arrowheads), indicating an increase in [Ca²⁺]ᵢ. In contrast, no such elevation is observed in control GFP-expressing cells (G). The elevated 340/380 fluorescence seen in MRGA1-expressing cells was dependent on the addition of ligand (not shown).

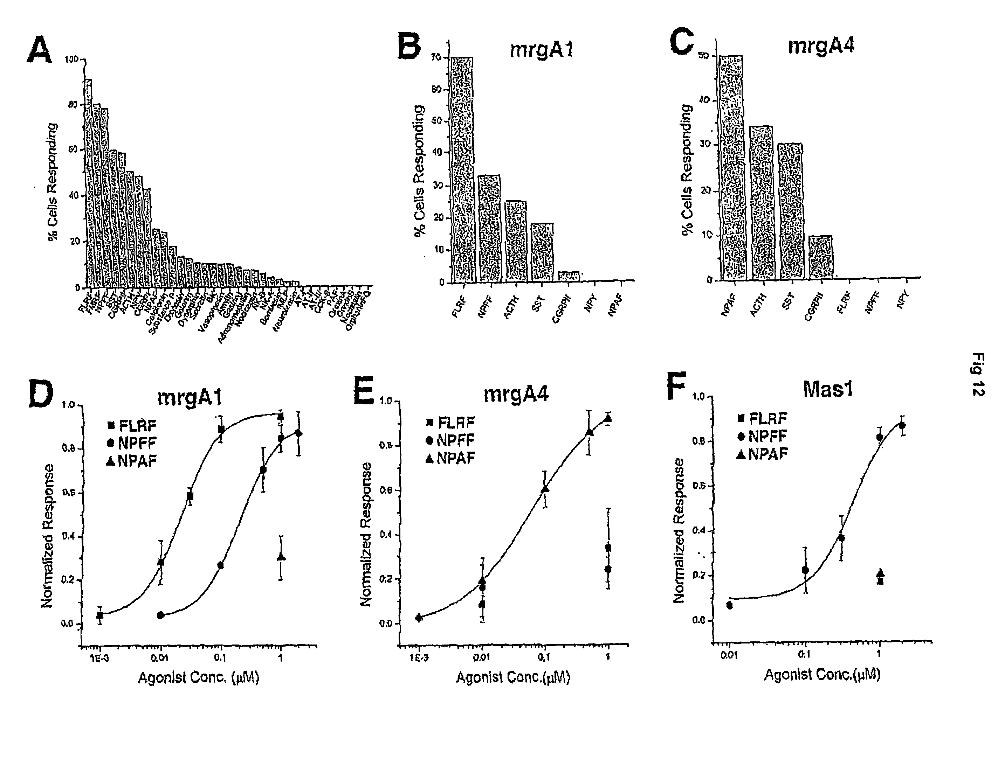

Figure 12A shows activation of MRGA receptors expressed in heterologous cells by neuropeptide ligands. HEK-G ₁₅ cells (Offermanns and Simon. J Biol Chem 270: 15175-80 (1995)) expressing MRGA1 were tested with the indicated ligands at a concentration of 1 µM. The data indicate the mean percentages of GFP-positive (i.e., transfected) cells showing calcium responses. None of the agonists indicated showed any responses through endogenous receptors in untransfected cells. Note that the RFamide neuropeptides FMRF, FLRF and NPFF, as well as NPY, ACTH, CGRP-1 and -II and somatostatin (SST) produced the strongest responses.

Figure 12B shows the ligand selectivity of MRGA1 expressed in HEK cells lacking G₁₅. The cells were exposed to ligands at a concentration of 1 µM as in (A).

Figure 12C shows the ligand selectivity of MRGA4. The data presented in Figures 12B and 12C indicate that the responses to the most effective ligands do not depend on the presence of G ₁₅. Note that MRGA1-expressing cells respond to FLRF and NPFF but not to NPAF, while conversely MRGA4-expressing cells respond to NPAF but not NPFF or FLRF

Figure 12D shows dose-response curves for MRGA1 expressed in HEK-G ₁₅ cells to selected RFamide neuropeptides. Each data point represents the mean ±S.E.M. of at least 3 independent determinations; at least 20 GFP⁺ cells were analyzed for each determination. Responses at each ligand concentration were normalized to the maximal response subsequently shown by the same cells to a 5 µM concentration of FLRF. MRGA1 (D) shows highest sensitivity to FLRF (squares, EC₅₀ 20 nM) and lower sensitivity to NPFF (circles, EC₅₀ 200 nM).

Figure 12E shows dose-response curves for MRGA4 expressed in HEK-G ₁₅ cells to selected RFamide neuropeptides. Each data point represents the mean ±S.E.M. of at least 3 independent determinations; at least 20 GFP⁺ cells were analyzed for each determination. Responses at each ligand concentration were normalized to the maximal response subsequently shown by the same cells to a 5 µM concentration of NPAF. MRGA4 is preferentially activated by NPAF (triangles, EC₅₀ 60 nM).

Figure 12F shows dose-response curves for MAS1 expressed in HEK-G ₁₅ cells to selected RFamide neuropeptides. Each data point represents the mean ±S.E.M. of at least 3 independent determinations; at least 20 GFP⁺ cells were analyzed for each determination. Responses at each ligand concentration were normalized to the maximal response subsequently shown by the same cells to a 5 µM concentration of NPFF. MAS1, like MRGA1, is activated by NPFF with similar efficacy (EC₅₀ 400 nM), but is not as well activated by FLRF (squares).

Figure 13 depicts the expression pattern of mMrgB1 in a sagital section of a newborn mouse. The staining pattern indicates that the mMrgB1 gene is expressed in the scattered cells in the epidermal layer of the skin, in the spleen and in the submandibular gland.

Figure 14 is a higher magnification of the mMrgB1 expression in the spleen and skin depicted in Figure 13.

Figure 15 shows the expression of mMrgD in adult dorsal root ganglia.

### Detailed Description of the Preferred Embodiment

### I. General Description

As described above, the present invention is based an the discovery of new genes that are expressed in the DRG of normal mice but not in Ngn1 null mutant mice. One of the novel gene families isolated from the screen encodes a receptor protein with 7 transmembrane segments, a characteristic of G protein-coupled receptors. This novel 7 transmembrane receptor is most closely related to the oncogene mas, and therefore was provisionally named mas-related gene-3 (mrg3). mrg3 is now known as MrgA1, and the terms are used interchangeably herein. The members of the Mas-related gene (Mrg) family identified are specifically expressed in non-peptidergic nociceptors. Large families of G protein-coupled receptors are also expressed in other classes of sensory neurons, such as olfactory and gustatory neurons.

The murine Mrg family of GPCRs contains three major subfamilies (MrgA, B and C), each consisting of more than 10 highly duplicated genes, as well as several single-copy genes such as Mas1, Rta, MrgD and MrgE (Figure 6B). The MrgA subfamily consists of at least twenty members in mice: MrgA1 (SEQ ID NO: 2); MrgA2 (SEQ ID NO: 4); MrgA3 (SEQ ID NO: 6); MrgA4 (SEQ ID NO: 11); MrgA5 (SEQ ID NO: 21); MrgA6 (SEQ ID NO: 23), MrgA7 (SEQ ID NO: 25); MrgA8 (SEQ ID NO: 27); MrgA9 (SEQ ID NO: 53); MrgA10 (SEQ ID NO: 55); MrgA11 (SEQ ID NO: 57); MrgA12 (SEQ ID NO: 59); MrgA13 (SEQ ID NO: 61); MrgA14 (SEQ ID NO: 63); MrgA15 (SEQ ID NO: 65); MrgA16 (SEQ ID NO: 67); MrgA17 (SEQ ID NO: 69); MrgA18 (SEQ ID NO: 71); MrgA19 (SEQ ID NO: 73); MrgA20 (SEQ ID NO: 75). Four human sequences that are most closes related to the MrgA subfamily have also been identified: MrgX1 (SEQ ID NO: 16); MrgX2 (SEQ ID NO: 18); MrgX3 (SEQ ID NO: 31); and MrgX4 (SEQ ID NO: 33).

The MrgB subfamily consists of at least twelve members in mice: MrgB1 (SEQ ID NO: 39); MrgB2 (SEQ ID NO: 41); MrgB3 (SEQ ID NO: 43); MrgB4 (SEQ ID NO: 45); MrgB5 (SEQ ID NO: 47); MrgB6 (SEQ ID NO: 77); MrgB7 (SEQ ID NO: 79); MrgB8 (SEQ ID NO: 81); MrgB9 (SEQ ID NO: 83); MrgB10 (SEQ ID NO: 85); MrgB11 (SED ID NO: 87); and MrgB12 (SEQ ID NO: 89).

Ten members of the MrgC subfamily have been identified in mice: MrgC1 (SEQ ID NO: 91); MrgC2 (SEQ ID NO: 93); MrgC3 (SEQ ID NO: 95); MrgC4 (SEQ ID NO: 97); MrgC5 (SEQ ID NO: 99); MrgC6 (SEQ ID NO: 101); MrgC7 (SEQ ID NO: 103); MrgC8 (SEQ ID NO: 105); MrgC9 (SEQ ID NO: 107); and MrgC10 (SEQ ID NO: 109).

A single member of the MrgD subfamily has been identified in mice, mMrgD (SEQ ID NO: 49) and its ortholog identified in humans, hMrgD (SEQ ID NO: 35). Similarly, a single member of the MrgE subfamily has been identified in mice, mMrgE (SEQ ID NO: 51) and humans, hMrgE (SEQ ID NO: 37).

As is the case in other GPCR subfamilies, a number of the Mrgs appear to be pseudogenes, including all members of the MrgC subfamily. The presence of L1 retrotransposon elements near several Mrg genes raises the possibility that pseudogene expansion may have been driven by L1-mediated transduction (Goodier et al. Hum Mol Genet 9: 653-7 (2000)).

In contrast to the murine MrgA and B subfamilies, which together contain almost 40 intact coding sequences, only four intact human MrgX sequences were identified. The remaining 10 human Mrg sequences appear to be pseudogenes. Inclusion of other related receptors such as hMrgD and hMas1 brings the total number of intact human coding sequences in this family to nine (Figure 6B).

Prior to the present invention, the primary nociceptive sensory neurons were thought not to specifically discriminate among different chemical stimuli, but rather to detect noxious stimuli of various modalities by virtue of broadly tuned receptors such as VR1 (Tominaga et al. Neuron 21: 531-43 (1998)). The expression of Mrgs reveals an unexpected degree of molecular diversification among nociceptive sensory neurons. Approximately 13-14% of sensory neurons express MrgA1, while 17-18% express MrgD and the overlap between these two populations is only 15%. The MrgA1⁺ population seems to include most or all neurons expressing MrgA2-8. However, these latter MrgA genes are not all expressed in the same neurons. Thus the 8 MrgA genes and MrgD define at least 6 different neuronal subpopulations, and the remaining 16 MrgA genes add even greater diversity.

It is striking that both MrgA and D are expressed in IB4⁺, VR1⁻ sensory neurons. IB4⁺ neurons are known to project to lamina Ili (Snider and McMahon Neuron 20: 629-32 (1998)), which has been implicated in chronic pain, such as that accompanying nerve injury (Malmberg et al. Science 278: 279-83 (1997)). VR1 is activated both by thermal stimuli and chemical stimuli such as capsaicin (Caterina et al. Nature 389: 816-824 (1997); Tominaga et al. Neuron 21: 531-43 (1998)), but VR1⁺ neurons are dispensable for the detection of noxious mechanical stimuli (Caterina et al. Science 288: 306-13 (2000)). This indicates that one of the functions of MrgA⁺ neurons is the detection of noxious mechanical stimuli accompanying neuropathic or inflammatory pain.

The existence of a family of putative G protein-coupled receptors specifically expressed in nociceptive sensory neurons suggests that these molecules are primary mediators or modulators of pain sensation. It is therefore of great interest to identify ligands, both endogenous and synthetic, that modulate the activity of these receptors, for the management of chronic intractable pain. Indeed, ligand screens in heterologous cell expression systems indicate that these receptors can interact with RF-amide neuropeptides of which the prototypic member is the molluscan cardioexcitatory peptide FMRF-amide (Price and Greenberg Science 197: 670-671 (1977)). Mammalian RF-amide peptides include NPFF and NPAF, which are derived from a common pro-peptide precursor expressed in neurons of laminae I and II of the dorsal spinal cord (Vilim et al. Mol Pharmacol 55: 804-11 (1999)). The expression of this neuropeptide FF precursor in the synaptic termination zone of Mrg-expressing sensory neurons, the ability of NPAF and NPFF to activate these receptors in functional assays, and the presence of binding sites for such peptides on primary sensory afferents in the dorsal horn (Gouarderes et al. Synapse 35: 45-52 (2000)), together indicate that these neuropeptides are ligands for Mrg receptors in vivo. As intrathecal injection of NPFF/NPAF peptides produces long-lasting antinociceptive effects in several chronic pain models (reviewed in Panula et al. Brain Res 848: 191-6 (1999)), including neuropathic pain (Xu et al. Peptides 20: 1071-7 (1999)), these data further indicate that Mrgs are directly involved in the modulation of pain.

One possibility for the extent of diversity among Mrgs expressed by murine nociceptors is that different Mrgs are expressed by sensory neurons that innervate different peripheral targets, such as gut, skin, hair follicles, blood vessels, bones and muscle. These targets may secrete different ligands for different Mrgs. Another possibility is that neurons expressing different Mrgs respond to a common modulator of peripheral nociceptor sensitivity, but with different affinities. Such a mechanism could, for example, provide a gradual restoration of normal sensitivities among the population of nociceptors during wound healing, as the concentration of such modulators gradually returned to baseline. Such a graded response might be coupled to, or even determine the activation thresholds of different subsets of nociceptors. Another novel gene family isolated in this screen, drg-12 encodes a protein with two putative transmembrane segments. Drg12 was identified from both mice (SEQ ID NO: 14) and in humans (SEQ ID NO: 29). In situ hybridization indicates that, like the mrg genes, this gene is also specifically expressed in a subset of DRG sensory neurons. As it is a membrane protein it may also be involved in signaling by these neurons. Although there are no obvious homologies between this protein and other known proteins, it is noteworthy that two purinergic receptors specifically expressed in nociceptive sensory neurons (P₂X₂ and P₂X₃) have a similar bipartite transmembrane topology. Therefore it is likely that the family drg-12 also encodes a receptor or ion channel involved in nociceptive sensory transduction or its modulation.

The proteins of the invention can serve as therapeutics and as a target for agents that modulate their expression or activity, for example in the treatment of chronic intractable pain and neuropathic pain. For example, agents may be identified which modulate biological processes associated with nociception such as the reception, transduction and transmission of pain signals.

### II. Specific Embodiments

### A. Definitions

Unless defined otherwise, technical and scientific terms used herein have the same meaning as commonly understood by one of ordinary skill in the art to which this invention belongs. See, e.g. Singleton et al., Dictionary of Microbiology and Molecular Biology 2nd ed., J. Wiley & Sons (New York, NY 1994); Sambrook et al., Molecular Cloning, A Laboratory Manual, Cold Springs Harbor Press (Cold Springs Harbor, NY 1989). For purposes of the present invention, the following terms are defined below.

As used herein, the "protein" or "polypeptide" refers, in part, to a protein that has the amino acid sequence depicted in SEQ ID NO: 2 and 12. The terms also refer to naturally occurring allelic variants and proteins that have a slightly different amino acid sequence than those specifically recited above. Allelic variants, though possessing a slightly different amino acid sequence than those recited above, will still have the same or similar biological functions associated with the protein.

Identity or homology with respect to amino acid sequences is defined herein as the percentage of amino acid residues in the candidate sequence that are identical with the known peptides, after aligning the sequences and introducing gaps, if necessary, to achieve the maximum percent homology, and not considering any conservative substitutions as part of the sequence identity (see section B for the relevant parameters). Fusion proteins, or N-terminal, C-terminal or internal extensions, deletions, or insertions into the peptide sequence shall not be construed as affecting homology.

Proteins can be aligned using CLUSTALW (Thompson at aL Nucleic Acids Res 22:4673-80 (1994)) and homology or identity at the nucleotide or amino acid sequence level may be determined by BLAST (Basic Local Alignment Search Tool) analysis using the algorithm employed by the programs blastp, blastn, blastx, tblastn and tblastx (Karlin , et al. Proc. Natl. Acad. Sci. USA 87: 2264-2268 (1990) and Altschul, S. F. J. Mol. Evol. 36: 290-300 (1993)) which are tailored for sequence similarity searching. The approach used by the BLAST program is to first consider similar segments between a query sequence and a database sequence, then to evaluate the statistical significance of all matches that are identified and finally to summarize only those matches which satisfy a preselected threshold of significance. For a discussion of basic issues in similarity searching of sequence databases, see Altschul et al. (Mature Genetics 6: 119-129 (1994)). The search parameters for histogram, descriptions, alignments, expect (i.e., the statistical significance threshold for reporting matches against database sequences), cutoff, matrix and filter are at the default settings. The default scoring matrix used by blastp, blastx, tblastn, and tblastx is the BLOSUM62 matrix (Henikoff, et al. Proc. Natl. Acad. Sci. USA 89:10915-10919 (1992). For blastn, the scoring matrix is set by the ratios of M (i.e., the reward score for a pair of matching residues) to N (i.e., the penalty score for mismatching residues), wherein the default values for M and N are 5 and -4, respectively. Four blastn parameters were adjusted as follows: Q=10 (gap creation penalty); R=10 (gap extension penalty); wink=1 (generates word hits at every winkth position along the query); and gapw=16 (sets the window width within which gapped alignments are generated). The equivalent Blastp parameter settings were Q=9; R=2; wink=1; and gapw=32. A Bestfit comparison between sequences, available in the GCG package version 10.0, uses DNA parameters GAP=50 (gap creation penalty) and LEN=3 (gap extension penalty) and the equivalent settings in protein comparisons are GAP=8 and LEN=2.

"Variants" are biologically active polypeptides having an amino acid sequence which differs from the sequence of a native sequence polypeptide of the present invention, such as that shown in FIG. 1 for mrg3 (SEQ ID NO: 2), by virtue of an insertion, deletion, modification and/or substitution of one or more amino acid residues within the native sequence. Variants include peptide fragments of at least 5 amino acids, preferably at least 10 amino acids, more preferably at least 15 amino acids, even more preferably at least 20 amino acids that retain a biological activity of the corresponding native sequence polypeptide. Variants also include polypeptides wherein one or more amino acid residues are added at the N- or C-terminus of, or within, a native sequence. Further, variants also include polypeptides where a number of amino acid residues are deleted and optionally substituted by one or more different amino acid residues.

As used herein, a "conservative variant" refers to alterations in the amino acid sequence that do not adversely affect the biological functions of the protein. A substitution, insertion or deletion is said to adversely affect the protein when the altered sequence prevents or disrupts a biological function associated with the protein. For example, the overall charge, structure or hydrophobiclhydrophilic properties of the protein can be altered without adversely affecting a biological activity. Accordingly, the amino acid sequence can be altered, for example to render the peptide more hydrophobic or hydrophilic, without adversely affecting the biological activities of the protein.

As used herein, the "family of proteins" related to the amino acid sequences of SEQ ID NO: 2 and 12 includes proteins that have been isolated from the dorsal root ganglia of organisms in addition to mice and humans. The methods used to identify and isolate other members of the family of proteins related to these proteins, such as the disclosed mouse and human proteins, are described below.

Unless indicated otherwise, the term "Mrg" when used herein refers to any one or more of the mammalian mas-related gene (Mrg) receptors (i.e. MrgA1-8, MrgB, MrgC, MrgD, MrgE, MrgX1-4 and any other members of the mas-related gene (Mrg) family now known or identified in the future), including native sequence mammalian, such as murine or human, Mrg receptors, Mrg receptor variants; Mrg receptor extracellular domain; and chimeric Mrg receptors (each of which is defined herein). The term specifically includes native sequence murine Mrg receptors of the MrgA family, such as SEQ ID NOs: 2, 4,6 12, 21, 23, 25, 27, 53, 55, 57, 59, 61, 63, 65, 67, 69, 71, 73, and 75; native sequence murine Mrg receptors of the MrgB family, such as SEQ ID NOs: 39, 41, 43, 45, 47, 77, 79, 81, 83, 85, 87, and 89; native sequence murine Mrg receptors of the MrgC family, such as SEQ ID NOs: 91, 93, 95, 97, 99, 101, 103, 105, 107 and 109; native sequence murine Mrg receptors of the MrgD family, such as SEQ ID NO: 49; native sequence murine Mrg receptors of the MrgE family, such as SEQ ID NO: 51; their human homologues, and the native sequence human Mrg receptors termed "MrgX" of SEQ ID NOs: 16, 18, 31 and 33.

The terms "mas-related gene", "mrg" and "Mrg" are used interchangeably herein. Further, the terms mrg3, MrgA1 and mMrgA1 are used interchangeably, as are the terms mrg4, MrgA2 and mMrgA2, the terms mrg5, MrgA3 and mMrgA3, the terms mrg8, MrgA4 and mMrgA4, the terms mrg9, MrgA5 and mMrgA5, the terms mrg10, MrgA6 and mMrgA6, the terms mrg11, MrgA7 and mMrgA7, the terms mrg12, MrgA8 and mMrgA8, the terms human1, MrgX1 and hMrgX1, the terms human2, MrgX2 and hMrgX2, the terms human 4, MrgX3 and hMrgX3, and the terms human5, MrgX4 and hMrgX4. These terms all refer to native sequence Mrg proteins as described herein as well as functional derivatives, including amino acid sequence variants thereof.

A "native" or "native sequence" Mrg receptor has the amino acid sequence of a naturally occurring Mrg receptor in any mammalian species (including humans), irrespective of its mode of preparation. Accordingly, a native or native sequence Mrg receptor may be isolated from nature, produced by techniques of recombinant DNA technology, chemically synthesized, or produced by any combinations of these or similar methods. Native Mrg and drg-12 receptors specifically include polypeptides having the amino acid sequence of naturally occurring allelic variants, isoforms or spliced variants of these receptors, known in the art or hereinafter discovered.

The "extracellular domain" (ECD) is a form of the Mrg receptor which is essentially free of the transmembrane and cytoplasmic domains, i.e., has less than 1% of such domains, preferably 0.5 to 0% of such domains, and more preferably 0.1 to 0% of such domains. Ordinarily, the ECD will have an amino acid sequence having at least about 60% amino acid sequence identity with the amino acid sequence of one or more of the ECDs of a native Mrg protein, for example as indicated in FIG. 1A for mrg3 (E1, E2 etc...), preferably at least about 65%, more preferably at least about 75%, even more preferably at least about 80%, even more preferably at least about 90%, with increasing preference of 95%, to at least 99% amino acid sequence identity, and finally to 100% identity, and thus includes polypeptide variants as defined below.

The first predicted extracellular domain (ECD1) comprises approximately amino acids 1 to 21 for MrgA1, 1 to 21 for MrgA2, 1 to 21 for MrgA3, 1 to 21 for MrgA4, 1 to 3 for MrgA5, 1 to 17 for MrgA6, 1 to 21 for MrgA7 and 1 to 21 for MrgA8. The second predicted extracellular domain (ECD2) comprises approximately amino acids 70 to 87 for MrgA1, 70 to 88 for MrgA2, 70 to 88 for MrgA3, 70 to 88 for MrgA4, 52 to 70 for MrgA5, 66 to 84 for MrgA6, 70 to 88 for MrgA7 and 70 to 88 for MrgA8. The third predicted extracellular domain (ECD3) comprises approximately amino acids 149 to 160 for MrgA1, 150 to 161 for MrgA2, 150 to 161 for MrgA3, 150 to 161 for MrgA4, 132 to 144 for MrgA5, 146 to 157 for MrgA6, 150 to 161 for MrgA7 and 150 to 161 for MrgA8. The fourth predicted extracellular domain (ECD4) comprises approximately amino acids 222 to 2244 for MrgA1, 223 to 245 for MrgA2, 223 to 242 for MrgA3, 223 to 245 for MrgA4, 205 to 225 for MrgA5, 219 to 241 for MrgA6, 223 to 245 for MrgA7 and 223 to 245 for MrgA8.

The term "drg-12" when used herein refers to any one or more of the mammalian drg-12 receptors now known or identified in the future, including native sequence mammalian, such as murine or human, drg-12 receptors, drg-12 receptor variants; drg-12 receptor extracellular domain; and chimeric drg-12 receptors (each of which is defined herein). The term specifically includes native sequence murine drg-12 receptor, such as SEQ ID NO: 14, and any human homologues, such as human drg-12 (SEQ ID NO:29), which do not form part of the present invention.

As used herein, "nucleic acid" is defined as RNA or DNA that encodes a protein or peptide as defined above, is complementary to a nucleic acid sequence encoding such peptides, hybridizes to such a nucleic acid and remains stably bound to it under appropriate stringency conditions, exhibits at least about 50%, 60%, 70%, 75%, 85%, 90% or 95% nucleotide sequence identity across the open reading frame, or encodes a polypeptide sharing at least about 50%, 60%, 70% or 75% sequence identity, preferably at least about 80%, and more preferably at least about 85%, and even more preferably at least about 90 or 95% or more identity with the peptide sequences. Specifically contemplated are genomic DNA, cDNA, mRNA and antisense molecules, as well as nucleic acids based on alternative backbones or including alternative bases whether derived from natural sources or synthesized. Such hybridizing or complementary nucleic acids, however, are defined further as being novel and unobvious over any prior art nucleic acid including that which encodes, hybridizes under appropriate stringency conditions, or is complementary to nucleic acid encoding a protein according to the present invention.

As used herein, the terms nucleic acid, polynucleotide and nucleotide are interchangeable and refer to any nucleic acid, whether composed of phosphodiester linkages or modified linkages such as phosphotriester, phosphoramidate, siloxane, carbonate, carboxymethylester, acetamidate, carbamate, thioether, bridged phosphoramidate, bridged methylene phosphonate, bridged phosphoramidate, bridged phosphoramidate, bridged methylene phosphonate, phosphorothioate, methylphosphonate, phosphorodithioate, bridged phosphorothioate or sultone linkages, and combinations of such linkages.

The terms nucleic acid, polynucleotide and nucleotide also specifically include nucleic acids composed of bases other than the five biologically occurring bases (adenine, guanine, thymine, cytosine and uracil). For example, a polynucleotide of the invention might contain at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyl-uracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5 -methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

Furthermore, a polynucleotide used in the invention may comprise at least one modified sugar moiety selected from the group including but not limited to arabinose, 2-fluoroarabinose, xylulose, and hexose.

"Stringent conditions'' are those that (1) employ low ionic strength and high temperature for washing, for example, 0.015 M NaCl/0.0015 M sodium citrate/0.1% SDS at 50°C., or (2) employ during hybridization a denaturing agent such as formamide, for example, 50% (vol/vol) formamide with 0.1% bovine serum albumin/0.1% Ficoll/0.1% polyvinylpyrrolidone/50 mM sodium phosphate buffer at pH 6.5 with 750 mM NaCl, 75 mM sodium citrate at 42°C. Another example is use of 50% formamide, 5 x SSC (0.75M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC and 0.1% SDS. A skilled artisan can readily determine and vary the stringency conditions appropriately to obtain a clear and detectable hybridization signal.

As used herein, a nucleic acid molecule is said to be "isolated" when the nucleic acid molecule is substantially separated from contaminant nucleic acid molecules encoding other polypeptides.

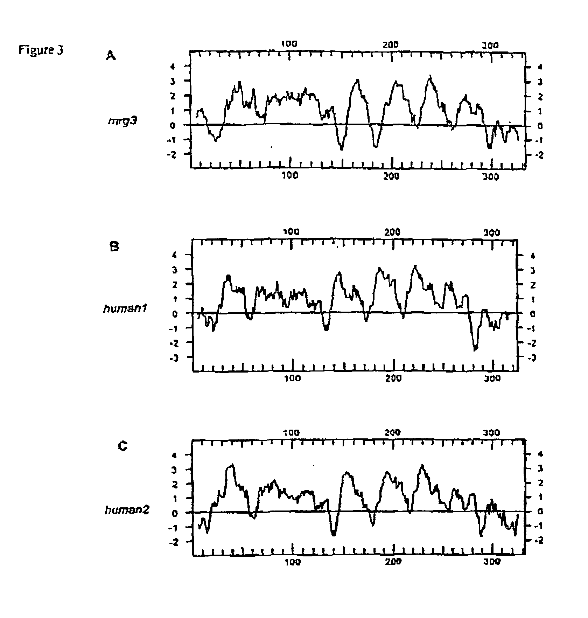

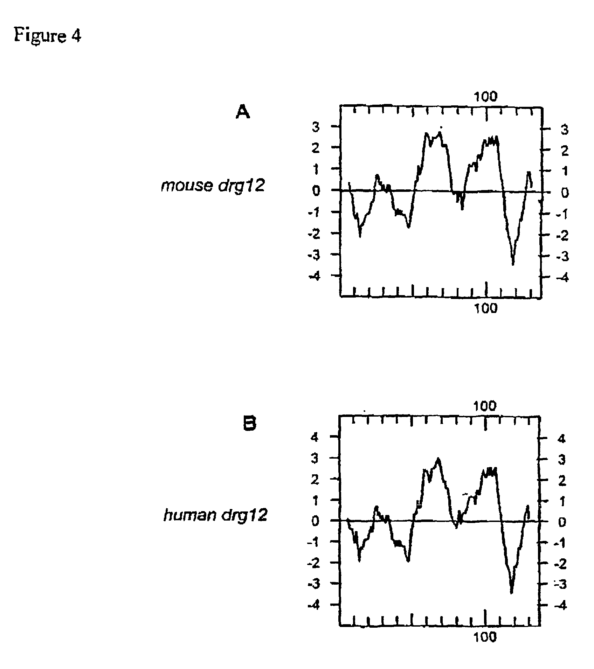

As used herein, a fragment of an encoding nucleic acid molecule refers to a small portion of the entire protein coding sequence. The size of the fragment will be determined by the intended use. For example, if the fragment is chosen so as to encode an active portion of the protein, the fragment will need to be large enough to encode the functional region(s) of the protein. For instance, fragments which encode peptides corresponding to predicted antigenic regions may be prepared (see Figures 3 and 4). If the fragment is to be used as a nucleic acid probe or PCR primer, then the fragment length is chosen so as to obtain a relatively small number of false positives during probing/priming (see the discussion in Section H).

Highly related gene homologs are polynucleotides encoding proteins that have at least about 60% amino acid sequence identity with the amino acid sequence of a naturally occurring native sequence polynucleotide of the invention, such as MrgA1 (SEQ ID NO: 2), preferably at least about 65%, 70%, 75%, 80%, with increasing preference of at least about 85% to at least about 99% amino acid sequence identity, in 1 % increments.

The term "mammal" is defined as an individual belonging to the class Mammalia and includes, without limitation, humans, domestic and farm animals, and zoo, sports, or pet animals, such as sheep, dogs, horses, cats or cows. Preferably, the mammal herein is human.

"Functional derivatives" include amino acid sequence variants, and covalent derivatives of the native polypeptides as long as they retain a qualitative biological activity of the corresponding native polypeptide.

By "Mrg ligand" is meant a molecule which specifically binds to and preferably activates an Mrg receptor. Examples of Mrg ligands include, but are not limited to RF-amide neuropeptides, such as FMRF, FLRF, NPAF, NPFF, and RFRP-1 for MrgA receptors, such as MrgA1. The ability of a molecule to bind to Mrg can be determined, for example, by the ability of the putative ligand to bind to membrane fractions prepared from cells expressing Mrg.

Similarly, a drg-12 ligand is a molecule which specifically binds to and preferably activates a drg-12 receptor.

A "chimeric" molecule is a polypeptide comprising a full-length polypeptide of the present invention, a variant, or one or more domains of a polypeptide of the present invention fused or bonded to a heterologous polypeptide. The chimeric molecule will generally share at least one biological property in common with a naturally occurring native sequence polypeptide. An example of a chimeric molecule is one that is epitope tagged for purification purposes. Another chimeric molecule is an immunoadhesin.

The term "epitope-tagged" when used herein refers to a chimeric polypeptide comprising Mrg fused to a "tag polypeptide". The tag polypeptide has enough residues to provide an epitope against which an antibody can be made, yet is short enough such that it does not interfere with the biological activity of the Mrg. The tag polypeptide preferably is fairly unique so that the antibody against it does not substantially cross-react with other epitopes. Suitable tag polypeptides generally have at least six amino acid residues and usually between about 8-50 amino acid residues (preferably between about 9-30 residues). Preferred are poly-histidine sequences, which bind nickel, allowing isolation of the tagged protein by Ni-NTA chromatography as described (See, e.g., Lindsay et al. Neuron 17:571-574(1996)).

"Agonists" are molecules or compounds that stimulate one or more of the biological properties of a polypeptide of the present invention. These may include, but are not limited to, small organic and inorganic molecules, peptides, peptide mimetics and agonist antibodies.

The term "antagonist" is used in the broadest sense and refers to any molecule or compound that blocks, inhibits or neutralizes, either partially or fully, a biological activity mediated by a receptor of the present invention by preventing the binding of an agonist. Antagonists may include, but are not limited to, small organic and inorganic molecules, peptides, peptide mimetics and neutralizing antibodies.

The proteins of the present invention are preferably in isolated form. As used herein, a protein is said to be isolated when physical, mechanical or chemical methods are employed to remove the protein from cellular constituents that are normally associated with the protein. A skilled artisan can readily employ standard purification methods to obtain an isolated protein. In some instances, isolated proteins of the invention will have been separated or purified from many cellular constituents, but will still be associated with cellular membrane fragments or membrane constituents.

Thus, "isolated Mrg" means Mrg polypeptide, respectively, that has been purified from a protein source or has been prepared by recombinant or synthetic methods and purified. Purified Mrg or drg-12 is substantially free of other polypeptides or peptides. "Substantially free' here means less than about 5%, preferably less than about 2%, more preferably less than about 1%, even more preferably less than about 0.5%, most preferably less than about 0.1% contamination with other source proteins.

"Essentially pure" protein means a composition comprising at least about 90% by weight of the protein, based on total weight of the composition, preferably at least about 95% by weight, more preferably at least about 90% by weight, even more preferably at least about 95% by weight. "Essentially homogeneous" protein means a composition comprising at least about 99% by weight of protein, based on total weight of the composition.

"Biological property" is a biological or immunological activity, where biological activity refer to a biological function (either inhibitory or stimulatory) caused by a native sequence or variant polypeptide molecule herein, other than the ability to induce the production of an antibody against an epitope within such polypeptide, where the latter property is referred to as immunological activity. Biological properties specifically include the ability to bind a naturally occurring ligand of the receptor molecules herein, preferably specific binding, and even more preferably specific binding with high affinity.

"Antibodies" (Abs) and "immunoglobulins" (lgs) are glycoproteins having the same structural characteristics. While antibodies exhibit binding specificity to a specific antigen, immunoglobulins include both antibodies and other antibody-like molecules that lack antigen specificity. Polypeptides of the latter kind are, for example, produced at low levels by the lymph system and at increased levels by myelomas.

"Native antibodies" and "native immunoglobulins" are usually heterotetrameric glycoproteins, composed of two identical light (L) chains and two identical heavy (H) chains. Each light chain is linked to a heavy chain by one covalent disulfide bond. while The number of disulfide linkages varies among the heavy chains of different immunoglobulin isotypes. Each heavy and light chain also has regularly spaced intra-chain disulfide bridges. Each heavy chain has at one end a variable domain (V_{H}) followed by a number of constant domains. Each light chain has a variable domain at one end (V_{L}) and a constant domain at its other end. The constant domain of the light chain is aligned with the first constant domain of the heavy chain, and the light- chain variable domain is aligned with the variable domain of the heavy chain. Particular amino acid residues are believed to form an interface between the light-and heavy-chain variable domains.

The term "antibody" herein is used in the broadest sense and specifically covers human, non-human (e.g. murine) and humanized monoclonal antibodies (including full length monoclonal antibodies), polyclonal antibodies, multi-specific antibodies (e.g., bispecific antibodies), and antibody fragments so long as they exhibit the desired biological activity.

"Antibody fragments" comprise a portion of a full-length antibody, generally the antigen binding or variable domain thereof. Examples of antibody fragments include Fab, Fab', F(ab')₂, and Fv fragments; diabodies; linear antibodies; single-chain antibody molecules; and multi-specific antibodies formed from antibody fragments.

The term "monoclonal antibody" as used herein refers to an antibody obtained from a population of antibodies wherein the individual antibodies comprising the population are identical except for possible naturally occurring mutations that may be present in minor amounts. Monoclonal antibodies are highly specific and are directed against a single antigenic site. In addition, monoclonal antibodies may be made by any method known in the art. For example, the monoclonal antibodies to be used in accordance with the present invention may be made by the hybridoma method first described by Kohler et al., Nature 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass. Fragments of chimeric antibodies are also included provided they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are antibodies that contain minimal sequence derived from non-human immunoglobulin. Humanized antibodies are generally human immunoglobulins in which hypervariable region residues are replaced by hypervariable region residues from a non-human species such as mouse, rat, rabbit or non-human primate having the desired specificity, affinity, and capacity. Framework region (FR) residues of the human immunoglobulin may be replaced by corresponding non-human residues. In addition, humanized antibodies may comprise residues that are not found in either the recipient antibody or in the donor antibody. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Reichmann et al., Nature 332:323-329 (1988); and Presta, Curr. Op. Struct. Biol. 2:593-596 (1992).

The term "epitope" is used to refer to binding sites for (monoclonal or polyclonal) antibodies on protein antigens.

By "agonist antibody" is meant an antibody which is a ligand for a receptor of the invention and thus, able to activate and/or stimulate one or more of the effector functions of native sequence Mrg or drg-12.

By "neutralizing antibody" is meant an antibody molecule as herein defined which is able to block or significantly reduce an effector function of a polypeptide of the invention. For example, a neutralizing antibody may inhibit or reduce Mrg or drg-12 activation by a known ligand.

The term "Mrg immunoadhesin" refers to a chimeric molecule that comprises at least a portion of an Mrg or drg-12 molecule (native or variant) and an immunoglobulin sequence. The immunoglobulin sequence preferably, but not necessarily, is an immunoglobulin constant domain. Immunoadhesins can possess many of the properties of human antibodies. Since immunoadhesins can be constructed from a human protein sequence with a desired specificity linked to an appropriate human immunoglobulin hinge and constant domain (Fc) sequence, the binding specificity of interest can be achieved using entirely human components. Such immunoadhesins are minimally immunogenic to the patient, and are safe for chronic or repeated use. If the two arms of the immunoadhesin structure have different specificities, the immunoadhesin is called a "bispecific immunoadhesin" by analogy to bispecific antibodies.

As used herein, "treatment" is a clinical intervention made in response to a disease, disorder or physiological condition manifested by a patient. The aim of treatment includes the alleviation or prevention of symptoms, slowing or stopping the progression or worsening of a disease, disorder, or condition and the remission of the disease, disorder or condition. "Treatment" refers to both therapeutic treatment and prophylactic or preventative measures. Those in need of treatment include those already affected by a disease or disorder or undesired physiological condition as well as those in which the disease or disorder or undesired physiological condition is to be prevented. Specifically, treatment may alleviate pain, including pain resulting from an existing condition or disorder, or to prevent pain in situations where pain is likely to be experienced.

In the methods of the present invention, the term "control" and grammatical variants thereof, are used to refer to the prevention, partial or complete inhibition, reduction, delay or slowing down of an unwanted event, such as the presence or onset of pain.

The term "effective amount" refers to an amount sufficient to effect beneficial or desirable clinical results. An effective amount of an agonist or antagonist is an amount that is effective to treat a disease, disorder or unwanted physiological condition.

"Pain" is a sensory experience perceived by nerve tissue distinct from sensations of touch, pressure, heat and cold. The range of pain sensations, as well as the variation of perception of pain by individuals, renders a precise definition of pain near impossible. In the context of the present invention, "pain" is used in the broadest possible sense and includes nociceptive pain, such as pain related to tissue damage and inflammation, pain related to noxious stimuli, acute pain, chronic pain, and neuropathic pain.

"Acute pain" is often short-lived with a specific cause and purpose; generally produces no persistent psychological reactions. Acute pain can occur during soft tissue injury, and with infection and inflammation. It can be modulated and removed by treating its cause and through combined strategies using analgesics to treat the pain and antibiotics to treat the infection.

"Chronic pain" is distinctly different from and more complex than acute pain. Chronic pain has no time limit, often has no apparent cause and serves no apparent biological purpose. Chronic pain can trigger multiple psychological problems that confound both patient and health care provider, leading to feelings of helplessness and hopelessness. The most common causes of chronic pain include low-back pain, headache, recurrent facial pain, pain associated with cancer and arthritis pain.

The pain is termed "neuropathic" when it is taken to represent neurologic dysfunction. "Neuropathic pain" has a complex and variable etiology. It is typically characterized by hyperalgesia (lowered pain threshold and enhanced pain perception) and by allodynia (pain from innocuous mechanical or thermal stimuli). Neuropathic pain is usually chronic and tends not to respond to the same drugs as "normal pain" (nociceptive pain), therefore, its treatment is much more difficult. Neuropathic pain may develop whenever nerves are damaged, by trauma, by disease such as diabetes, herpes zoster, or late-stage cancer, or by chemical injury (e.g., as an untoward consequence of agents including the false-nucleotide anti-HIV drugs). It may also develop after amputation (including mastectomy). Examples of neuropathic pain include monoradiculopathies, trigeminal neuralgia, postherpetic neuralgia, complex regional pain syndromes and the various peripheral neuropathies. This is in contrast with "normal pain" or "nociceptive pain," which includes normal post-operative pain, pain associated with trauma, and chronic pain of arthritis.

"Peripheral neuropathy" is a neurodegenerative disorder that affects the peripheral nerves, most often manifested as one or a combination of motor, sensory, sensorimotor, or autonomic dysfunction. Peripheral neuropathies may, for example, be characterized by the degeneration of peripheral sensory neurons, which may result from a disease or disorder such as diabetes (diabetic neuropathy), alcoholism and acquired immunodeficiency syndrome (AIDS), from therapy such as cytostatic drug therapy in cancer, or from genetic predisposition. Genetically acquired peripheral neuropathies include, for example, Krabbe's disease, Metachromatic leukodystrophy, and Charcot-Marie-Tooth (CMT) Disease. Peripheral neuropathies are often accompanied by pain.

"Pharmaceutically acceptable" carriers, excipients, or stabilizers are ones which are nontoxic to the cell or mammal being exposed thereto at the dosages and concentrations employed. Often the physiologically acceptable carrier is an aqueous pH buffered solution such as phosphate buffer or citrate buffer. The physiologically acceptable carrier may also comprise one or more of the following: antioxidants including ascorbic acid, low molecular weight (less than about 10 residues) polypeptides, proteins, such as serum albumin, gelatin, immunoglobulins; hydrophilic polymers such as polyvinylpyrrolidone, amino acids, carbohydrates including glucose, mannose, or dextrins, chelating agents such as EDTA, sugar alcohols such as mannitol or sorbitol, salt-forming counterions such as sodium, and nonionic surfactants such as Tween, polyethylene glycol (PEG), and Pluronics .

"Peptide mimetics" are molecules which serve as substitutes for peptides in interactions with the receptors of the present invention (Morgan et al., Ann. Reports Med. Cham. 24:243-252 (1999)). Peptide mimetics, as used herein, include synthetic structures that retain the structural and functional features of a peptide. Peptide mimetics may or may not contain amino acids and/or peptide bonds. The term, "peptide mimetics" also includes peptoids and oligopeptoids, which are peptides or oligomers of N-substituted amino acids (Simon et al., Proc. Natl. Acad. Sci. USA 89:9367-9371 (1972)). Further included as peptide mimetics are peptide libraries, which are collections of peptides designed to be of a given amino acid length and representing all conceivable sequences of amino acids corresponding thereto.

### A. Proteins Expressed in Primary Sensory Neurons of Dorsal Root Ganglia

The present invention provides isolated mrg proteins, allelic variants of the proteins, and conservative amino acid substitutions of the proteins. Polypeptide sequences of several Mrg proteins of the present invention are provided in SEQ ID NOs: 2 and 12 and are characterized by the ability to be activated by an RF -amide-peptide.

The proteins of the present invention further include insertion, deletion or conservative amino acid substitution variants of the sequences set forth in SEQ ID NOs: 2 and 12.

Ordinarily, the variants, allelic variants, the conservative substitution variants, and the members of the protein family, including corresponding homologues in other species, will have an amino acid sequence having at least 80% amino acid sequence identity with the sequences set forth in SEQ ID NOs: 2 or 12.

The proteins of the present invention include molecules having the amino acid sequence disclosed in SEQ ID NOs: 2 and 12 fragments thereof having a consecutive sequence of at least about 3, 4, 5, 6, 10, 15, 20, 25, 30, 35 or more amino acid residues of the protein; amino acid sequence variants wherein one or more amino acid residues has been inserted N. or C-terminal to, or within, the disdosed coding sequence; and amino acid sequence variants of the disclosed sequence, or their fragments as defined above, that have been substituted by another residue. Such fragments, also referred to as peptides or polypeptides, may contain antigenic regions, functional regions of the protein identified as regions of the amino acid sequence which correspond to known protein domains, as well as regions of pronounced hydrophilicity. The regions are all easily identifiable by using commonly available protein sequence analysis software such as MACVECTOR™ (Oxford Molecular).

Contemplated variants further include those containing predetermined mutations by, e.g., homologous recombination, site-directed or PCR mutagenesis, and the corresponding proteins of other animal species, including but not limited to rabbit, rat, porcine, bovine, ovine, equine, human and non-human primate species, and the alleles or other naturally occurring variants of the family of proteins; and derivatives wherein the protein has been covalently modified by substitution, chemical, enzymatic, or other appropriate means with a moiety other than a naturally occurring amino acid (for example a detectable moiety such as an enzyme or radioisotope).

Protein domains such as a ligand binding domain, an extracellular domain, a transmembrane domain (e.g. comprising seven membrane spanning segments and cytosolic loops or two membrane spanning domains and cytosolic loops), the transmembrane domain and a cytoplasmic domain and an active site may all be found in the proteins or polypeptides of the invention. Such domains are useful for making chimeric proteins and for in vitro assays of the invention.

Variations in native sequence proteins of the present invention or in various domains identified therein, can be made, for example, using any techniques known in the art. Variation can be achieved, for example, by substitution of at least one amino acid with any other amino acid in one or more of the domains of the protein. A change in the amino acid sequence of a protein of the invention as compared with a native sequence protein may be produced by a substitution, deletion or insertion of one or more codons encoding the protein. A comparison of the sequence of the Mrg polypeptide to be changed with that of homologous known protein molecules may provide guidance as to which amino acid residues may be inserted, substituted or deleted without affecting a desired biological activity. In particular, it may be beneficial to minimize the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

Polypeptide fragments are also useful in the methods of the present invention. Such fragments may be truncated at the N-terminus or C-terminus, or may lack internal residues, for example, when compared with a full-length native protein. Certain fragments lack amino acid residues that are not essential for a desired biological activity of the Mrg polypeptide.

Mrg fragments may be prepared by any of a number of conventional techniques. Desired peptide fragments may be chemically synthesized or generated by enzymatic digestion, such as by treating the protein with an enzyme known to cleave proteins at sites defined by particular amino acid residues. Alternatively, the DNA encoding the protein may be digested with suitable restriction enzymes and the desired fragment isolated. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, Mrg polypeptide fragments share at least one biological and/or immunological activity with a native Mrg polypeptide, respectively.

In making amino acid sequence variants that retain the required biological properties of the corresponding native sequences, the hydropathic index of amino acids may be considered. For example, it is known that certain amino acids may be substituted for other amino acids having a similar hydropathic index or score without significant change in biological activity. Thus, isoleucine, which has a hydropathic index of + 4.5, can generally be substituted for valine (+ 4.2) or leucine (+ 3.8), without significant impact on the biological activity of the polypeptide in which the substitution is made. Similarly, usually lysine (-3.9) can be substituted for arginine (-4.5), without the expectation of any significant change in the biological properties of the underlying polypeptide.Other considerations for choosing amino acid substitutions include the similarity of the side-chain substituents, for example, size, electrophilic character, charge in various amino acids. In general, alanine, glycine and serine; arginine and lysine; glutamate and aspartate; serine and threonine; and valine, leucine and isoleucine are interchangeable, without the expectation of any significant change in biological properties. Such substitutions are generally referred to as conservative amino acid substitutions, and are the preferred type of substitutions within the polypeptides of the present invention.

Non-conservative substitutions will entail exchanging a member of one class of amino acids for another class. Such substituted residues also may be introduced into the conservative substitution sites or, more preferably, into the remaining (non-conserved) sites.

The variations can be made using methods known in the art such as site-directed mutagenesis, alanine scanning mutagenesis, and PCR mutagenesis. Site-directed mutagenesis (Carter et al., Nucl. Acids Res., 13:4331 (1986); Zoller et al., Nucl. Acids Res., 10:6487 (1987)), cassette mutagenesis (Wells et al., Gene, 34:315 (1985)), restriction selection mutagenesis (Wells et a!., Philos. Trans. R. Soc. London SerA, 317:415 (1986)) or other known techniques can be performed on cloned DNA to produce the Mrg or drg-12 variant DNA.

Scanning amino acid analysis can be employed to identify one or more amino acids that can be replaced without a significant impact on biological activity. Among the preferred scanning amino acids are relatively small, neutral amino acids. Such amino acids include alanine, glycine, serine, and cysteine. Alanine is preferred because, in addition to being the most common amino acid, it eliminates the side-chain beyond the beta-carbon and is therefore less likely to alter the main-chain conformation of the variant (Cunningham and Wells, Science, 244: 1081-1085 (1989)). Further, alanine is frequently found in both buried and exposed positions (Creighton, The Proteins, (W.H. Freeman & Co., N.Y.); Chothia, J. Mol. Biol., 150:1 (1976)). If alanine substitution does not yield adequate amounts of variation, an isoteric amino acid can be used.

As described below, members of the family of proteins can be used in vitro. 1) to identify agents which modulate at least one activity of the protein; 2) to identify binding partnars for the protein, 3) as an antigen to raise polyclonal or monoclonal antibodies, 4) as a therapeutic target, 5) as diagnostic markers to specific populations of pain sensing neurons and 6) as targets for structure based ligand identification.

### B. Nucleic Acid Molecules

The present invention further provides nucleic acid molecules that encode the mrg proteins having SEQ ID NO: 2 or12, and being characterized by the ability to be activated by an RF-amide-peptide and the related polypeptides herein described, preferably in isolated form. cDNAs encoding full-length variants of Mrg receptors (mMrgA1-8) are provided in Figure 6A (SEQ ID NO: 1, 11).

Preferred molecules are those that hybridize under the above defined stringent conditions to the complement of SEQ ID NO: 1 or 11, and which encode a functional peptide. Preferred hybridizing molecules are those that hybridize under the above conditions to the complement strand of the open reading frame or coding sequences of SEQ ID NO: 1 or 11.

It is not intended that the methods of the present invention be limited by the source of the polynucleotide. The polynucleotide can be from a human or non-human mammal, derived from any recombinant source, synthesized in vitro or by chemical synthesis. The nucleotide may be DNA or RNA and may exist in a double-stranded, single-stranded or partially double-stranded form.

Nucleic acids useful in the present invention include, by way of example and not limitation, oligonucleotides such as antisense DNAs and/or RNAs; ribozymes; DNA for gene therapy; DNA and/or RNA chimeras; various structural forms of DNA including single-stranded DNA, double-stranded DNA, supercoiled DNA and/or triple-helix DNA; Z-DNA; and the like. The nucleic acids may be prepared by any conventional means typically used to prepare nucleic acids in large quantity. For example, DNAs and RNAs may be chemically synthesized using commercially available reagents and synthesizers by methods that are well-known in the art (see, e.g., Gait, 1985, Oligonucleotide Synthesis: A Practical Approach, IRL Press, Oxford, England).

Any mRNA transcript encoded by Mrg nucleic acid sequences may be used in the methods of the present invention, including in particular, mRNA transcripts resulting from alternative splicing or processing of mRNA precursors.

Nucleic acids having modified nucleoside linkages may also be used in the methods of the present invention. Modified nucleic acids may, for example, have greater resistance to degradation. Such nucleic acids may be synthesized using reagents and methods that are well known in the art. For example, methods for synthesizing nucleic acids containing phosphonate phosphorothioate, phosphorodithioate, phosphoramidate methoxyethyl phosphoramidate, formacetal, thioformacetal, diisopropylsilyl, acetamidate, carbamate, dimethylene-sulfide (-CH₂-S-CH₂), dimethylene-sulfoxide (-CH₂-SO-CH₂), dimethylene-sulfone (-CH₂-SO₂-CH₂), 2'-O-alkyl, and 2'-deoxy-2'-fluoro phosphorothioate internucleoside linkages are well known in the art.

In some embodiments of the present invention, the nucleotide used is an -anomeric nucleotide. An anameric nucleotide forms specific double-stranded hybrids with complementary RNA in which, contrary to the usual units, the strands run parallel to each other (Gautier et al., 1987, Nucl. Acids Res. 15:6625-6641). The nucleotide may be a 2'-O-methylribonucleotide (Inoue et al., 1987, Nucl. Acids Res. 15:6131-6148), or a chimeric RNA-DNA analogue (Inoue et al., 1987, FEBS Lett. 215:327-330).

Means for purifying the nucleic acids of the present invention are well known in the art and the skilled artisan will be able to choose the most appropriate method of purification for the particular circumstances. Such a choice may be made, in part, based on the size of the DNA, the amount to be purified and the desired purity. For example, the nucleic acids can be purified by reverse phase or ion exchange HPLC, size exclusion chromatography or gel electrophoresis.

Isolated or purified polynucleotides having at least 10 nucleotides (i.e., a hybridizable portion) of an Mrg coding sequence or its complement may also be used in the methods of the present invention. In other embodiments, the polynucleotides contain at least 25 (continuous) nucleotides, 50 nucleotides, 100 nucleotides, 150 nucleotides, or 200 nucleotides of an Mrg coding sequence, or a full-length Mrg coding sequence. Nucleic acids can be single or double stranded. Additionally, the invention relates to polynucleotides that selectively hybridize to a complement of the foregoing coding sequences. In preferred embodiments, the polynucleotides contain at least 10, 25,50,100,150 or 200 nucleotides or the entire length of an Mrg coding sequence.

Nucleotide sequences that encode a mutant of an Mrg protein, peptide fragments of Mrg, truncated forms of Mrg, and Mrg fusion proteins may also be useful in the methods of the present invention. Nucleotides encoding fusion proteins may include, but are not limited to, full length Mrg sequences, truncated forms of Mrg, or nucleotides encoding peptide fragments of Mrg fused to an unrelated protein or peptide, such as for example, a domain fused to an Ig Fc domain or fused to an enzyme such as a fluorescent protein or a luminescent protein which can be used as a marker.

Furthermore, polynucleotide variants that have been generated, at least in part, by some form of directed evolution, such as gene shuffling or recursive sequence recombination may be used in the methods of the present invention. For example, using such techniques novel sequences can be generated encoding proteins similar to Mrg but having altered functional or structural characteristics.

Highly related gene homologs of the Mrg encoding polynucleotide sequences described above may also be useful in the present invention. Highly related homologs can encode proteins sharing functional activities with Mrg proteins.

The present invention further provides fragments of the encoding nucleic acid molecule. Fragments of the encoding nucleic acid molecules of the present invention (i.e., synthetic oligonucleotides) that are used as probes or specific primers for the polymerase chain reaction (PCR), or to synthesize gene sequences encoding proteins of the invention, can easily be synthesized by chemical techniques, for example, the phosphotriester method of Matteucci, et al., (J. Am. Chem. Soc. 103:3185-3191, 1981) or using automated synthesis methods. In addition, larger DNA segments can readily be prepared by well known methods, such as synthesis of a group of oligonucleotides that define various modular segments of the gene, followed by ligation of oligonucleotides to build the complete modified gene.

The encoding nucleic acid molecules of the present invention may further be modified so as to contain a detectable label for diagnostic and probe purposes. A variety of such labels are known in the art and can readily be employed with the encoding molecules herein described. Suitable labels include, but are not limited to, biotin, radiolabeled nucleotides and the like. A skilled artisan can readily employ any such label to obtain labeled variants of the nucleic acid molecules of the invention.

Any nucleotide sequence which encodes the amino acid sequence of a protein of the invention can be used to generate recombinant molecules which direct the expression of the protein, as described in more detail below. In addition, the methods of the present invention may also utilize a fusion polynucleotide comprising an Mrg coding sequence and a second coding sequence for a heterologous protein.

### C. Isolation of Other Related Nucleic Acid Molecules

As described above, the identification and characterization of a nucleic acid molecule encoding an mrg protein allows a skilled artisan to isolate nucleic acid molecules that encode other members of the same protein family in addition to the sequences herein described

Essentially, a skilled artisan can readily use the amino acid sequence of SEQ ID NO: 2 or 12, to generate antibody probes to screen expression libraries prepared from appropriate cells. Typically, polyclonal antiserum from mammals such as rabbits immunized with the purified protein (as described below) or monoclonal antibodies can be used to probe a mammalian cDNA or genomic expression library, such as a lambda gtll library, to obtain the appropriate coding sequence for other members of the protein family. The cloned cDNA sequence can be expressed as a fusion protein, expressed directly using its own control sequences, or expressed by constructions using control sequences appropriate to the particular host used for expression of the protein.

Alternatively, a portion of the coding sequence herein described can be synthesized and used as a probe to retrieve DNA encoding a member of the Mrg protein family from cells derived from any mammalian organism, particularly cells believed to express Mrg proteins. Oligomers containing approximately 18-20 nucleotides (encoding about a 6-7 amino acid stretch) are prepared and used to screen genomic DNA or cDNA libraries to obtain hybridization under stringent conditions or conditions of sufficient stringency to eliminate an undue level of false positives. Oligonucleotides corresponding to either the 5' or 3' terminus of the coding sequence may be used to obtain longer nucleotide sequences.

It may be necessary to screen multiple cDNA libraries to obtain a full-length cDNA. In addition, it may be necessary to use a technique such as the RACE (Rapid Amplification of cDNA Ends) technique to obtain the complete 5' terminal coding region. RACE is a PCR-based strategy for amplifying the 5' end of incomplete cDNAs. To obtain the 5' end of the cDNA, PCR is carried out on 5'-RACE-Ready cDNA using an anchor primer and a 3' primer. A second PCR is then carried out using the anchored primer and a nested 3' primer. Once a full length cONA sequence is obtained, it may be translated into amino acid sequence and examined for identifiable regions such as a continuous open reading frame flanked by translation initiation and termination sites, a potential signal sequence and finally overall structural similarity to the protein sequences disclosed herein.

Related nucleic acid molecules may also be retrieved by using pairs of oligonucleotide primers in a polymarase chain reaction (PCR) to selectively clone an encoding nucleic acid molecule. The oligonucleotide primers may be degenerate oligonucleotide primer pools designed on the basis of the protein coding sequences disclosed herein. The template for the reaction may be cDNA obtained by reverse transcription (RT) of mRNA prepared from, for example, human or non-human cell lines or tissues known or suspected to express an Mrg gene allele, such as DRG tissue. A PCR denature/anneal/extend cycle for using such PCR primers is well known in the art and can readily be adapted for use in isolating other encoding nucleic acid molecules.

The PCR product may be subcloned and sequenced to ensure that the amplified sequences represent the sequences of an Mrg coding sequence. The PCR fragment may then be used to isolate a full-length cDNA clone by a variety of methods. For example, the amplified fragment may be labeled and used to screen a cDNA library. Alternatively, the labeled fragment may be used to isolate genomic clones via the screening of a genomic library.

PCR technology may also be utilized to isolate full-length cDNA sequences. RNA may be isolated, from an appropriate cellular or tissue source, such as dorsal root ganglion (ORG) and an RT reaction may be carried out using an oligonucleotide primer specific for the most 5' end of the amplified fragment to prime first strand synthesis. The resulting RNA/DNA hybrid may then be "tailed" with guanines in a terminal transferase reaction, the hybrid may be digested with RNAase H, and second strand synthesis may then be primed with a poly-C primer. This allows isolation of cDNA sequences upstream of the amplified fragment.

Nucleic acid molecules encoding other members of the mrg family may also be identified in existing genomic or other sequence information using any available computational method, including but not limited to: PSI-BLAST (Altschul, et al. (1997) Nucleic Acids Res. 25:3389-3402); PHI-BLAST (Zhang, et al. (1998), Nucleic Acids Res. 26:3986-3990), 30-PSSM (Kelly et al. J. Mol. Biol. 299(2): 499-520 (2000)); and other computational analysis methods (Shi et al. Biochem. Biophys. Res. Commun. 262(1):132-8 (1999) and Matsunami et. al. Nature 404(6778):601-4 (2000).

A cDNA clone of a mutant or alletic variant of an Mrg gene may also be isolated. A possible source of a mutant or variant protein is tissue known to express Mrg, such as DRG tissue, obtained from an individual putatively carrying a mutant or variant form of Mrg. Such an individual may be identified, for example, by a demonstration of increased or decreased responsiveness to painful stimuli. In one embodiment, a mutant or variant Mrg gene may be identified by PCR. The first cDNA strand may be synthesized by hybridizing an oligo-dT oligonucleotide to mRNA isolated from the tissue putatively carrying a variant and extending the new strand with reverse transcriptase. The second strand of the cDNA is then synthesized using an oligonucleotide that hybridizes specifically to the 5' end of the nomral gene. Using these two primers, the product is then amplified via PCR, cloned into a suitable vector, and subjected to DNA sequence analysis through methods well known to those of skill in the art. By comparing the DNA sequence of the mutant Mrg allele to that of the normal Mrg allele, the mutation(s) respansibla for any loss or alteration of function of the mutant Mrg gene product can be ascertained.

Alternatively, a genomic library can be constructed using DNA obtained from an individual suspected of or known to carry a mutant Mrg allele, or a cDNA library can be constructed using RNA from a tissue known, or suspected, to express a mutant Mrg allele. An unimpaired Mrg gene or any suitable fragment thereof may then be labeled and used as a probe to identify the corresponding mutant Mrg allele in such libraries. Clones containing the mutant Mrg gene sequences may then be purified and subjected to sequence analysis according to methods well known to those of skill in the art.

Additionally, an expression library can be constructed utilizing cDNA synthesized from, for example, RNA isolated from a tissue known, or suspected, to express a mutant Mrg allele in an individual suspected of carrying such a mutant allele. In this manner, gene products made by the putatively mutant tissue may be expressed and screened using standard antibody screening techniques in conjunction with antibodies raised against the normal Mrg gene product, as described, below.

### D. Recombinant DNA molecules containing a Nucleic Acid Molecule

The present invention further provides recombinant DNA molecules (rDNAs) that contain a coding sequence. As used herein, a rDNA molecule is a DNA molecule that has been subjected to molecular manipulation in situ. Methods for generating rDNA molecules are well known in the art, for example, see Sambrook et al., Molecular Cloning: A Laboratory Manual, 2nd edition, 1989; Cold Spring Harbor Laboratory Press, Cold Spring Harbor, NY. in the preferred rDNA molecules, a coding DNA sequence is operably linked to expression control sequences and/or vector sequences.

Thus the present invention also contemplates DNA vectors that contain any of the Mrg coding sequences and/or their complements, optionally associated with a regulatory element that directs the expression of the coding sequences. The choice of vector and/or expression control sequences to which one of the protein family encoding sequences of the present invention is operably linked depends directly, as is well known in the art, on the functional properties desired, e.g., protein expression, and the host call to be transformed. A vector contemplated by the present invention is at least capable of directing the replication or insertion into the host chromosome, and preferably also expression, of the structural gene included in the rDNA molecule.

Both cloning and expression vectors contain a nucleic acid sequence that enables the vector to replicate in one or more selected host cells. In cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2 plasmid origin is suitable for yeast, and various viral origins (SV40, polyoma, adenovirus, VSV or BPV) are useful for cloning vectors in mammalian cells.

In addition to being capable of replication in at least one class of organism most expression vectors can be transfected into another organism for expression. For example, a vector is replicated in E. coli and then the same vector is transfected into yeast or mammalian cells for expression.

DNA may also be amplified by insertion into the host genome. For example, transfection of Bacillus with a vector comprising a DNA sequence complementary to a Bacillus genomic sequence results in homologous recombination with the genome and insertion of the DNA from the vector. One disadvantage to this type of system is that the recovery of genomic DNA encoding the protein of interest is more complex than that of an exogenously replicated vector because restriction enzyme digestion is required to excise the DNA.

Expression control elements that are used for regulating the expression of an operably linked protein encoding sequence are known in the art and include, but are not limited to, inducible promoters, constitutive promoters, secretion signals, and other regulatory elements. Preferably, the inducible promoter is readily controlled, such as being responsive to a nutrient in the host cell's medium.

In one embodiment, the vector containing a coding nucleic acid molecule will include a prokaryotic replicon, i.e., a DNA sequence having the ability to direct autonomous replication and maintenance of the recombinant DNA molecule extrachromosomally in a prokaryotic host cell, such as a bacterial host cell, transformed therewith. Such replicons are well known in the art. In addition; vectors that include a prokaryotic replicon may also include a gene whose expression confers a detectable marker such as a drug resistance. Typical bacterial drug resistance genes are those that confer resistance to ampicillin or tetracycline.

Vectors that include a prokaryotic replicon can further include a prokaryotic or bacteriophage promoter capable of directing the expression (transcription and translation) of the coding gene sequences in a bacterial host cell, such as E. coli. A promoter is an expression control element formed by a DNA sequence that permits binding of RNA polymerase and transcription to occur. Promoter sequences that are compatible with bacterial hosts are typically provided in plasmid vectors containing convenient restriction sites for insertion of a DNA segment of the present invention. Typical of such vector plasmids are pUC8, pUC9, pBR322 and pBR329 available from BioRad Laboratories, (Richmond, CA), pPL and pKK223 available from Pharmacia (Piscataway, NJ).

Expression vectors compatible with eukaryotic cells, preferably those compatible with vertebrate cells, can also be used to form rDNA molecules that contain a coding sequence. Eukaryotic cell expression vectors are well known in the art and are available from several commercial sources. Typically, such vectors are provided containing convenient restriction sites for insertion of the desired DNA segment. Typical of such vectors are pSVL and pKSV-10 (Pharmacia), pBPV-1/pML2d (International Biotechnologies, Inc.), pTDT1 (ATCC, #31255), eukaryotic viral vectors such as adenoviral or retroviral vectors, and the like eukaryotic expression vectors.

Eukaryotic cell expression vectors used to construct the rDNA molecules of the present invention may further include a selectable marker that is effective in an eukaryotic cell, preferably a drug resistance selection marker. This gene encodes a factor necessary for the survival or growth of transformed host cells grown in a selective culture medium. Host cells not transformed with the vector containing the selection gene will not survive in the culture medium. Typical selection genes encode proteins that confer resistance to antibiotics or other toxins, e.g., ampicillin, neomycin, methotrexate, or tetracycline, complement auxotrophic deficiencies, or supply critical nutrients withheld from the media. A preferred drug resistance marker is the gene whose expression results in neomycin resistance, i.e., the neomycin phosphotransferase (neo) gene. (Southern et al., J. Mol. Anal. Genet. 1:327-341, 1982.) The selectable marker can optionally be present on a separate plasmid and introduced by co-transfection.

In one example of a selection system, mammalian cell transformants are placed under selection pressure such that only the transformants are able to survive by virtue of having taken up the vector(s). Selection pressure is imposed by progressively increasing the concentration of selection agent in the culture medium, thereby stimulating amplification of both the selection gene and the DNA that encodes the desired protein. Amplification is the process by which genes in greater demand for the production of a protein critical for growth are reiterated in tandem within the chromosomes of successive generations of recombinant cells. Increased quantities of the desired protein, such as Mrg, are synthesized from the amplified DNA. Examples of amplifiable genes include DHFR, thymidine kinase, metallothionein-I and -II, adenosine deaminase, and ornithine decarboxylase.

Thus in one embodiment Chinese hamster ovary (CHO) cells deficient in DHFR activity are prepared and propagated as described by Urlaub et al., Proc. Natl. Acad. Sci. USA, 77:4216 (1980). The CHO cells are then transformed with the DHFR selection gene and transformants are are identified by culturing in a culture medium that contains methotrexate (Mtx), a competitive antagonist of DHFR. The transformed cells are then exposed to increased levels of methotrexate. This leads to the synthesis of multiple copies of the DHFR gene, and, concomitantly, multiple copies of other DNA comprising the expression vectors, such as the DNA encoding the protein of interest, for example DNA encoding Mrg.

Alternatively, host cells can be transformed or co-transformed with DNA sequences encoding a protein of interest such as Mrg, wild-type DHFR protein, and another selectable marker such as aminoglycoside 3'-phosphotransferase (APH). The transformants can then be selected by growth in medium containing a selection agent for the selectable marker such as an aminoglycosidic antibiotic, e.g., kanamycin, neomycin, or G418.

As mentioned above, expression and cloning vectors usually contain a promoter that is recognized by the host organism and is operably linked to the nucleic acid encoding the protein of interest. Promoters are untranslated sequences located upstream (5') to the start codon of a structural gene (generally within about 100 to 1000 bp) and control the transcription and translation of the particular nucleic acid sequence, such as an Mrg nucleic acid sequence, to which they are operably linked. Promoters may be inducible or constitutive. Inducible promoters initiate increased levels of transcription from DNA under their control in response to some change in culture conditions, such as a change in temperature. Many different promoters are well known in the art, as are methods for operably linking the promoter to the DNA encoding the protein of interest. Both the native Mrg promoter sequence and many heterologous promoters may be used to direct amplification and/or expression of the Mrg DNA. However, heterologous promoters are preferred, as they generally permit greater transcription and higher yields of the desired protein as compared to the native promoter.

Promoters suitable for use with prokaryotic hosts include, for example, the lactamase and lactose promoter systems (Chang et al., Nature, 275:615 (1978); Goeddel et al., Nature, 281:544 (1979)). However, other bacterial promoters are well known in the art and are suitable. Promoters for use in bacterial systems also will contain a Shine-Delgarno (S.D.) sequence operably linked to the DNA encoding the protein of interest.

Promoter sequences that can be used in eukaryotic cells are also well known. Virtually all eukaryotic genes have an AT-rich region located approximately 25 to 30 bases upstream from the transcription initiation site. Another sequence found 70 to 80 bases upstream from the start of transcription of many genes is a CXCAAT region where X may be any nucleotide. At the 3' end of most eukaryotic genes is an AATAAA sequence that may be the signal for addition of the poly-A tail to the 3' end of the coding sequence. All of these sequences may be inserted into eukaryotic expression vectors.

Examples of suitable promoting sequences for use with yeast hosts include the promoters for 3-phosphoglycerate kinase (Hitzeman et al., J. Biol. Chem., 255:2073 (1980)) or other glycolytic enzymes (Hess et al., J. Adv. Enzyme Reg., 7:149 (1968); Holland, Biochemistry, 17:4900(1978)).

Inducible promoters for use with yeast are also well known and include the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Mrg transcription from vectors in mammalian host cells may also be controlled by promoters obtained from the genomes of viruses such as polyoma virus, fowlpox virus, adenovirus, bovine papilloma virus, avian sarcoma virus, cytomegalovirus, a retrovirus, hepatitis-B virus and most preferably Simian Virus 40 (SV40), from heterologous mammalian promoters, e.g., the actin promoter or an immunoglobulin promoter, from heat-shock promoters, and from the promoter normally associated with the native sequence, provided such promoters are compatible with the host cell systems.

Transcription may be increased by inserting an enhancer sequence into the vector. Enhancers are cis-acting elements of DNA, usually about 10 to 300 bp in length, that act on a promoter to increase its transcription. Many enhancer sequences are now known from mammalian genes (globin, elastase, albumin, fetoprotein, and insulin). Preferably an enhancer from a eukaryotic cell virus will be used. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5° or 3' to the protein-encoding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRMA. These sequences are often found in the 5' and, occasionally 3', untranslated regions of eukaryotic or viral DNAs or cDNAs and are well known in the art.

Plasmid vectors containing one or more of the Components described above are readily constructed using standard techniques well known in the art.

For analysis to confirm correct sequences in plasmids constructed, the plasmid may be replicated in E. coli, purified, and analyzed by restriction endonuclease digestion, and/or sequenced by conventional methods.

Particularly useful in the preparation of proteins of the present invention are expression vectors that provide for transient expression in mammalian cells of DNA encoding Mrg. Transient expression involves the use of an expression vector that is able to replicate efficiently in a host cell, such that the host cell accumulates many copies of the expression vector and, in turn, synthesizes high levels of a the polypeptide encoded by the expression vector. Sambrook et al., supra, pp. 16.17 · 16.22. Transient expression systems allow for the convenient positive identification of polypeptides encoded by cloned DNAs, as well as for the screening of such polypeptides for desired biological or physiological properties. Thus, transient expression systems are particularly useful in the invention for purposes of identifying biologically active analogs and variants of the polypeptides of the invention and for identifying agonists and antagonists thereof.

Other methods, vectors, and host cells suitable for adaptation to the synthesis of Mrg in recombinant vertebrate cell culture are well known in the art and are readily adapted to the specific circumstances. E. Host Cells Containing an Exogenously Supplied Coding Nucleic Acid Molecule

The present invention further provides host cells transformed with a nucleic acid molecule that encodes a protein of the present invention. The host cell can be either prokaryotic or eukaryotic but is preferably eukaryotic.

Eukaryotic cells useful for expression of a protein of the invention are not limited, so long as the cell line is compatible with cell culture methods and compatible with the propagation of the expression vector and expression of the gene product. Such host cells are capable of complex processing and glycosylation activities. In principle, any higher eukaryotic cell culture is workable, whether from vertebrate or invertebrate culture. Preferred eukaryotic host cells include, but are not limited to, yeast, insect and mammalian cells, preferably vertebrate cells such as those from a mouse, rat, monkey or human cell line. Preferred eukaryotic host cells include Chinese hamster ovary (CHO) cells available from the ATCC as CCL61, NIH Swiss mouse embryo cells (NIH/3T3) available from the ATCC as CRL 1658, baby hamster kidney cells (BHK), HEK293 cells and the like eukaryotic tissue culture cell lines.

Propagation of vertebrate cells in culture is a routine procedure. See, e.g., Tissue Culture, Academic Press, Kruse and Patterson, editors (1973). Additional examples of useful mammalian host cell lines that can be readily cultured are monkey kidney CV1 line transformed by SV40 (COS-7, ATCC CRL 1651); mouse sertoli cells (TM4, Mather, Biol., Reprod., 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); and mouse mammary tumor (MMT 060562, ATCC CCL51).

Xenopus oocytes may also be directly injected with RNA capable of expressing either the mrg or drg-12 proteins by standard procedures (sea Tominaga et aL Jpn J. Pharmacol. 83(1):20-4 (2000); Tominaga et al. Neuron 21(3):531-43 (1998) and Bisogno et al. Biochem, Biophys. Res. Commun. 262(1):275-84 (1999)).

Examples of invertebrate cells that can be used as hosts include plant and insect cells. Numerous baculoviral strains and variants and corresponding permissive insect host cells are known in the art and may be utilized in the methods of the present invention. In addition, plant cell cultures are known and may be transfected, for example, by incubation with Agrobacterium tumefaciens, which has been manipulated to contain Mrg encoding DNA.

Any prokaryotic host can be used to express a rDNA molecule encoding a protein or a protein fragment of the invention. Suitable prokaryotes include eubacteria, such as Gram-negative or Gram-positive organisms, for example, Enterobacteriaceae such as Escherichia, e.g., E. coli, Enterobacter, Erwinia, Klebsiella, Proteus, Salmonella, e.g., Salmonella typhimurium, Serratia, e.g., Serratia marcescans, and Shigella, as well as Bacilli such as B. subtilis and B. licheniformis (e.g., B. licheniformis 41 P disclosed in OD 266,710 published 12 April 1989), Pseudomonas such as P. aeruginosa, and Streptomyces. The preferred prokaryotic host is E. coli. In addition, it is preferably that the host cell secrete minimal amounts of proteolytic enzymes.

In addition to prokaryotes, eukaryotic microbes such as filamentous fungi or yeast are suitable cloning or expression hosts for Mrg encoding vectors. For example, Saccharomyces cerevisiae may be used. In addition a number of other genera, species, and strains are commonly available and useful herein, such as Schizosaccharomyces pombe (Beach et al. Nature, 290:140 (1981); EP 139,383); Kluyveromyces hosts (U.S. Patent No. 4,943,529; Fleer et al., supra) such as, e.g., K. lactis (MW98-8C, CBS683, CBS4574; Louvencourt et al., J. Bacteriol., 737 (1983)), K. fragilis (ATCC 12,424), K. bulgaricus (ATCC 16,045), K. wickeramii (ATCC 24,178), K. waltii (ATCC 56,500), K. drosophilarum (ATCC 36,906; Van den Berg et al., supra), K . thermotolerans, and K. marxianus; yarrowia (EP 402,226); Pichia pastoris (EP 183,070; Sreekrishna et al. J. Basic Microbiol., 28:265-278 (1988)); Candida; Trichoderma reesia (EP 244,234); Neurospora crassa (Case et al. Proc. Natl. Acad. Sci. USA, 76:5259-5263 (1979)); Schwanniomyces such as Schwanniomyces occidentalis (EP 394,538); and filamentous fungi such as, e.g., Neurospora, Penicillium, Tolypocladium (WO 91/00357), and Aspergillus hosts such as A. nidulans (Ballance et al. Biochem. Biophys. Res. Commun., 112:284-289 (1983); Tilburn et al., Gene, 26:205-221 (1983); Yelton et al. Proc. Natl. Acad. Sci. USA, 81:1470-1474 (1984)) and A. niger (Kelly et al. EMBO J., 4:475-479 (1985)).

Transformation of appropriate cell hosts with a rDNA molecule of the present invention is accomplished by well known methods that typically depend on the type of vector used and host system employed. With regard to transformation of prokaryotic host cells, electroporation and salt treatment methods are typically employed, see, for example, Cohen et al. Proc. Natl. Acad. Sci. USA 69:2110, (1972); and Maniatis et al., Molecular Cloning, A Laboratory Manual, Cold Spring Harbor Laboratory, Cold Spring Harbor, NY (1982). With regard to transformation of vertebrate cells with vectors containing rDNAs, electroporation, cationic lipid or salt treatment methods are typically employed, see, for example, Graham et al. Virol. 52:456, (1973); Wigler et al. Proc. Natl. Acad. Sci. USA 76:1373-76, (1979). The calcium phosphate precipitation method is preferred. However, other methods of for introducing DNA into cells may also be used, including nuclear microinjection and bacterial protoplast fusion.

For transient expression of recombinant channels, transformed host cells for the measurement of Na⁺ current or intracellular Na⁺ levels are typically prepared by co-transfecting constructs into cells such as HEK293 cells with a fluorescent reporter plasmid (such as pGreen Lantern-1, Life Technologies) using the calcium-phosphate precipitation technique (Ukomadu et al. Neuron 8, 663-676 (1992)). After forty-eight hours, cells with green fluorescence are selected for recording (Dib-Hajj et al. FEBS Lett. 416, 11-14 (1997)). Similarly, for transient expression of Mrg receptors and measurement of intracellular Ca²⁺ changes in response to receptor activation as described in Example 4, HEK cells can be co-transfected with Mrg expression constructs and a fluorescent reporter plasmid. HEK293 cells are typically grown in high glucose DMEM (Life Technologies) supplemented with 10% fetal calf serum (Life Technologies).

Prokaryotic cells used to produce polypeptides of this invention are cultured in suitable media as described generally in Sambrook et al., supra.

The mammalian host cells used to produce the polypeptides of this invention may be cultured in a variety of media, including but not limited to commercially available media such as Ham's F10 (Sigma), Minimal Essential Medium ((MEM), Sigma), RPMI-1640 (Sigma), and Dulbecco's Modified Eagle's Medium ((DMEM), Sigma). In addition, any of the media described in Ham et al. Meth. Enz., 58:44 (1979), Barnes et al. Anal. Biochem. 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics, trace elements, and glucose or an equivalent energy source. Any other necessary supplements may also be included at appropriate concentrations as determined by the skilled practitioner. The culture conditions are those previously used with the host cell selected for expression, and will be apparent to the skilled artisan.

The host cells referred to in this disclosure encompass cells in culture as well as cells that are within a host animal.

Successfully transformed cells, i.e., cells that contain a rDNA molecule of the present invention, can be identified by well known techniques including the selection for a selectable marker. For example, cells resulting from the introduction of an rDNA of the present invention can be cloned to produce single colonies. Cells from those colonies can be harvested, lysed and their DNA content examined for the presence of the rDNA using a method such as that described by Southern, J. Mol. Biol. 98:503, (1975), or Berent et al., Biotech. 3:208, (1985) or the proteins produced from the cell assayed via an immunological method as described below.

Gene amplification and/or expression may be measured by any technique known in the art, including Southern blotting, Northern blotting to quantitate the transcription of mRNA (Thomas, Proc. Natl. Acad. Sci. USA, 77:5201-5205 (1980)), dot blotting (DNA analysis), or in situ hybridization, using an appropriately labeled probe, based on the sequences provided herein. Various labels may be employed, most commonly radioisotopes, particularly ³²P.

Immunological methods for measuring gene expression include immunohistochemical staining of tissue sections or cells in culture, as well as assaying protein levels in culture medium or body fluids.. With immunohistochemical staining techniques, a cell sample is prepared by dehydration and fixation, followed by reaction with labeled antibodies specific for the gene product, where the labels are usually visually detectable, such as enzymatic labels, fluorescent labels, luminescent labels, and the like.

Antibodies useful for immunohistochemical staining and/or assay of sample fluids may be either monoclonal or polyclonal, and may be prepared as described herein.

### F. Production of Recombinant Proteins using an rDNA Molecule

The present invention further provides methods for producing a protein of the invention using nucleic acid molecules herein described. In general terms, the production of a recombinant form of a protein typically involves the following steps:

A nucleic acid molecule is first obtained that encodes a mrg protein of the invention, for example, nucleotides 115-1026 of SEQ ID NO: 1, nucleotides 115-1029 of SEQ ID NO: 1 nucleotides 1820-2734 of SEQ ID NO: 11. If the encoding sequence is uninterrupted by introns, as are these sequences, it is directly suitable for expression in any host.

The nucleic acid molecule is then preferably placed in operable linkage with suitable control sequences, as described above, to form an expression unit containing the protein open reading frame. The expression unit is used to transform a suitable host and the transformed host is cultured under conditions that allow the production of the recombinant protein. Optionally the recombinant protein is isolated from the medium or from the cells; recovery and purification of the protein may not be necessary in some instances where some impurities may be tolerated or when the recombinant cells are used, for instance, in high throughput assays.

Each of the foregoing steps can be done in a variety of ways. For example, the desired coding sequences may be obtained from genomic fragments and used directly in appropriate hosts. The construction of expression vectors that are operable in a variety of hosts is accomplished using appropriate replicons and control sequences, as set forth above. The control sequences, expression vectors, and transformation methods are dependent on the type of host cell used to express the gene and were discussed in detail earlier. Suitable restriction sites can, if not normally available, be added to the ends of the coding sequence so as to provide an excisable gene to insert into these vectors. A skilled artisan can readily adapt any host/expression system known in the art for use with the nucleic acid molecules of the invention to produce recombinant protein.

In one embodiment, Mrg may be produced by homologous recombination. Briefly, primary human cells containing an Mrg- encoding gene are transformed with a vector comprising an amplifiable gene (such as dihydrofolate reductase (DHFR)) and at least one flanking region of a length of at least about 150 bp that is homologous with a DNA sequence at the locus of the coding region of the Mrg gene. The amplifiable gene must be located such that it does not interfere with expression of the Mrg gene. Upon transformation the construct becomes homologously integrated into the genome of the primary cells to define an amplifiable region.

Transformed cells are then selected for by means of the amplifiable gene or another marker present in the construct. The presence of the marker gene establishes the presence and integration of the construct into the host genome. PCR, followed by sequencing or restriction fragment analysis may be used to confirm that homologous recombination occurred.

The entire amplifiable region is then isolated from the identified primary cells and transformed into host cells. Clones are then selected that contain the amplifiable region, which is then amplified by treatment with an amplifying agent. Finally, the host cells are grown so as to express the gene and produce the desired protein.

The proteins of this invention may be produced recombinantly not only directly, but also as a fusion polypeptide with a heterologous polypeptide. In one embodiment the heterologous polypeptide may be a signal sequence. In general, the signal sequence may be a component of the vector, or it may be a part of the Mrg DNA that is inserted into the vector. The heterologous signal sequence selected preferably is one that is recognized and processed (i.e., cleaved by a signal peptidase) by the host cell. For expression in prokaryotic host cells the signal sequence may be a prokaryotic signal sequence selected, for example, from the group consisting of the alkaline phosphatase, penicillinase, lpp, and heat-stable enterotoxin II leaders. For yeast secretion the native signal sequence may be substituted by, e.g., the yeast invertase leader, factor leader (including Saccharomyces and Kluyveromyces - factor leaders, or acid phosphatase leader and the C. albicans glucoamylase leader). In mammalian cell expression any native signal sequence is satisfactory. Alternatively it may be substituted with a signal sequence from related proteins, as well as viral secretory leaders, for example, the herpes simplex gD signal. The DNA for such precursor regions is ligated in reading frame to DNA encoding the mature protein or a soluble variant thereof.

The heterologous polypeptide may also be a marker polypeptide that can be used, for example, to identify the location of expression of the fusion protein. The marker polypeptide may be any known in the art, such as a fluorescent protein. A preffered marker protein is green fluorescent protein (GFP).

### G. Modifications of Mrg polypeptides

Covalent modifications of Mrg and their respective variants are included within the scope of this invention. In one embodiment, specific amino acid residues of a polypeptide of the invention are reacted with an organic derivatizing agent. Derivatization with bifunctional agents is useful, for instance, for crosslinking Mrg or Mrg fragments or derivatives to a water-insoluble support matrix or surface for use in methods for purifying anti-Mrg antibodies and identifying binding partners and ligands. In addition, Mrg or Mrg fragments may be crosslinked to each other to modulate binding specificity and effector function. Many crosslinking agents are known in the art and include, hut are not limited to, 1,1-bis(diazoacetyl)-2-phenylethane, glutaraldehyde, N-hydroxysuccinimide esters, bifunctional maleimides such as bis-N-maleimido-1,8-octane and agents such as methyl-3-[(p-azidophenyl)dithio]propioimidate.

Other contemplated modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains (T.E. Creighton, Proteins: Structure and Molecular Properties, W.H. Freeman & Co., San Francisco, pp. 79-86 (1983)), acetylation of the N-terminal amine, and amidation of any C-terminal carboxyl group.

Modification of the glycosylation patterns of the polypeptides of the invention are also contemplated. Methods for altering the glycosylation pattern of polypeptides are well known in the art. For example, one or more of the carbohydrate moities found in native sequence Mrg may be removed chemically, enzymatically or by modifying the glycosylation site. Alternatively, additional gycosylation can be added, such as by manipulating the composition of the carbohydrate moities directly or by adding glycosylation sites not present in the native sequence Mrg by altering the amino acid sequence.

Another type of covalent modification of the polypeptides of the invention comprises linking the polypeptide or a fragment or derivative thereof to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The polypeptides of the present invention may also be modified in a way to form a chimeric molecule comprising Mrg fused to another, heterologous polypeptide or amino acid sequence.

In one embodiment, such a chimeric molecule comprises a fusion of the Mrg with a tag polypeptide that provides an epitope to which an anti-tag antibody can selectively bind. The epitope tag is generally placed at the amino- or carboxyl- terminus of the polypeptide. The epitope tag allows for identification of the chimeric protein as well as purification of the chimeric protein by affinity purification using an anti-tag antibody or another type of affinity matrix that binds to the epitope tag. A number of tag polypeptides and their respective antibodies are well known in the art. Well known tags include poly-hislidine (poly-his) or poly-histidine-glycine (poly-his-gly) tags; the flue HA tag polypeptide (Field et al., Mol. Cell. Biol., 8:2159-2165 (1988)); the c-myc tag (Evan et al., Molecular and Cellular Biology, 5:3610-3616 (1985)); the Herpes Simplex virus glycoprotein D (gD) tag (Paborsky et al., Protein Engineering, 3(6):547-553 (1990)) and the Flag-peptide (Hopp et al., BioTechnology, 6:1204-1210 (1988)).

In another embodiment, the chimeric molecule comprises a fusion of Mrg with an immunoglobulin or a particular region of an immunoglobulin. To produce an immunoadhesin, the polypeptide of the invention or a fragment or specific domain(s) thereof could be fused to the Fc region of an IgG molecule. Typically the fusion is to an immunoglobulin heavy chain constant region sequence. Mrg- immunoglobulin chimeras for use in the present invention are normally prepared from nucleic acid encoding one or more extracellular domains, or fragments thereof, of an Mrg receptor fused C-terminally to nucleic acid encoding the N-terminus of an immunoglobulin constant domain sequence. N-terminal fusions are also possible.

While not required in the immunoadhesins of the present invention, an immunoglobulin light chain might be present either covalently linked to an Mrg- immunoglobulin heavy chain fusion polypeptide, or directly fused to Mrg. In order to obtain covalent association, DNA encoding an immunoglobulin light chain may be coexpressed with the DNA encoding the Mrg- immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs.

Bispecific immunoadhesins may also be made. Such immunoadhesins may combine an Mrg domain and a domain, such as the extracellular domain, from another receptor. Alternatively, the immunoadhesins herein might comprise portions of two different Mrg receptors, each fused to an immunoglobulin heavy chain constant domain sequence.

In yet another embodiment, the chimeric molecule of the present invention comprises a fusion of Mrg-or a fragment or domain(s) thereof, with a heterologous receptor or fragment or domain(s) thereof. The heterologous receptor may be a related Mrg family member, or may be completely unrelated. The heterologous protein fused to the Mrg protein may be chosen to obtain a fusion protein with a desired ligand specificity or a desired affinity for a particular ligand or to obtain a fusion protein with a desired effector function.

### H. Methods of Using mrgs or drgs as Molecular or Diagnostic Probes

The sequences and antibodies, proteins and peptides of the present invention may be used as molecular probes for the in vitro detection of cells or tissues related to or involved with sensory perception, especially perception of pain. Although many methods may be used to detect the nucleic acids or proteins of the invention in situ, preferred probes include antisense molecules and anti-mrg antibodies.

Probes for the detection of the nucleic acids or proteins of the invention may find use in the identification of the involvement of Mrg proteins in particular disease states, such as glaucoma or chronic pain, or in enhanced or inhibited sensory perception. In particular, probes of the present invention may be useful in determining if Mrg expression is increased or decreased in patients demonstrating changes in sensory perception, such as in patients with allodynia, hyperalgesia or chronic pain, or patients with a disease or disorder, such as glaucoma. A determination of decreased expression or overexpression of a polypeptide of the invention may be useful in identifying a therapeutic approach to treating the disorder, such as by administering Mrg agonists or antagonists.

Determination of changes in Mrg expression levels in animal models of disease states, particularly pain, may also be useful in identifying the types of disorders that might be effectively treated by compounds that modify expression or activity.

Further, the probes of the invention, including antisense molecules and antibodies, may be used to detect the expression of mutant or variant forms of Mrg variants. The ability to detect such variants may be useful in identifying the role that the variants play in particular disease states and in the symptoms experienced by particular patients. Identification of the involvement of a variant of Mrg in a disease or disorder may suggest a therapeutic approach for treatment of the disease or disorder, such as gene therapy or the administration of agonists or antagonists known to bind the receptor variant.

In addition, probes of the invention may be used to determine the exact expression patterns of the various Mrg family members, including the relationship of one to another. For example, the microscopy images of in situ hybridization in Figure 2 show the localization of antisense staining against a nucleotide of SEQ ID NO:2 ("mrg3") and of SEQ ID NO:4 ("mrg4") which does not form part of the present invention in transverse sections of dorsal root ganglia (DRG) from newborn wild type (WT) and Neurogenin1 null mutant (Ngn1⁺) mice. White dashed lines outline the DRG and black dashed lines outline the spinal cord. Note that in the Ngn1⁺ mutant, the size of the DRG is severely reduced due to the loss of nociceptive sensory neurons, identified using three other independent markers (trkA; VR-1 and SNS-TTXi (Ma et al., (1999)). mrg3 is expressed in a subset of DRG in WT mice (A) but is absent in the Ngn1⁺ DRG (B). mrg4 is expressed in a smaller subset of DRG than that of mrg3 (C). It is also absent in the Ngn1⁺ DRG (D). The loss of mrg-expressing neurons in the Ngn1⁺ DRG indicates that these neurons are likely to be nociceptive.

Expression of mrgs in subsets of dorsal root ganglia (DRG) neurons are shown in Figure 2A. Frozen transverse sections of DRG from wild-type (a-i) and ngn1⁺ (j) mutant new born mice were annealed with antisense digoxigenin RNA probes, and hybridization was visualized with an alkaline phosphatase-conjugated antibody. Positive signals are shown as dark purple stainings. TrkA is expressed in a large portion of wild-type DRG neurons (a) but absent in ngn1⁺ (data not shown). Each of the eight mrg genes (b-i) is expressed in a small subset of neurons in wild-type DRG in completely absent in ngn1⁺ DRG (j and data not shown). Black dash line outlines the ngn1⁺ mutant DRG.

In Figure 2B, mrgs are expressed by TrkA⁺ nociceptive neurons. Double labeling technique was used to colocalize TrkA (green; [b,e]) and mrgs (red; [a,d]) in DRG neurons. During the double labeling experiments frozen sections of wild-type DRG were undergone in situ hybridizations with either mrg3 (a-c) or mrg5 (d-f) fluoresceio-labeled antisense RNA probes followed by anti-TrkA antibody immunostaining. The same two frames (a and b, d and e) were digitally superimposed to reveal the extent of colocalization (c, f). The colocalizations of TrkA with either mrg3 or mrg5 appear yellow in merged images (c, f, respectively). The white arrowheads indicate examples of double positive cells.

In Figure 2C, mrgs and VR1 define two different populations of nociceptive neurons in DRG. The combination of in situ hybridizations (red) with either mrg3 or mrg5 fluorescein-labeled antisense RNA probes and anti-VR1 antibody immunostaining (green) demonstrated that neither mrg3 (a-c) nor mrg5 (d-f) were expressed by VR1-positive neurons. In the merged images (c,f), there are no colocalizations of VR1 with either mrg3 or mrg5. The white arrowheads are pointed to mrgs-expressing but VR1-negative nociceptive neurons.

In Figure 2D mrgs are shown to be expressed by IB4⁺ nociceptive neurons. Double labeling technique was used to colocalize IB4 (green; [b,e]) and mrgs (red; [a,d]) in DRG neurons. The expressions of mrg3 and mrg5 were visualized by in situ hybridization as described before. The same DRG sections were subsequently undergone through FITC-conjugated lectin IB4 binding. In the merged images (c,f), there are extensive overlappings between mrgs and IB4 stainings (yellow neurons indicated by arrowheads).

Information about the expression patterns of the receptors of the invention in normal tissue and tissue taken from animal models of disease or patients suffering from a disease or disorder will be useful in further defining the biological function of the receptors and in tailoring treatment regimens to the specific receptor or combination of receptors involved in a particular disease or disorder.

### I. Methods to Identify Binding Partners

As discussed in more detail below, several peptides have been putatively identified as endogenous ligands for Mrg receptors. In particular the RF-amide peptides, including NPAF and NPFF, have been shown to efficiently stimulate several of the Mrg receptors. In order to identify additional new ligands for the Mrg receptors it is first necessary to indentify compounds that bind to these receptors. Thus, another embodiment of the present invention provides methods of isolating and identifying binding partners or ligands of proteins of the invention. Macromolecules that interact with Mrg are referred to, for purposes of this discussion, as "binding partners."

Receptor binding can be tested using Mrg receptors isolated from their native source or synthesized directly. However, Mrg receptors obtained by the recombinant methods described above are preferred.

The compounds which may be screened in accordance with the invention include, but are not limited to polypeptides, peptides, including but not limited to members of random peptide libraries; (see, e.g., Lam, K.S. et al., 1991, Nature 354:82-84; Houghten, R. et al., 1991, Nature 354:84-86) and combinatorial chemistry-derived molecular libraries made of D. and/or L- configuration amino acids, phosphopeptides (including, but not limited to members of random or partially degenerate, directed phosphopeptide libraries; see, e.g., Songyang, Z. et al., 1993, Cell 72:767-778), peptide mimetics, antibodies (including, but not limited to, polyclonal, monoclonal, humanized, anti-idiotypic, chimeric or single chain antibodies, FAb, F(ab)₂ and FAb expression library fragments, and epitope-binding fragments thereof), and small organic or inorganic molecules.

The ability of candidate or test compounds to bind Mrg receptors can be measured directly or indirectly, such as in competitive binding assays. In competitive binding experiments, the concentration of the test compound necessary to displace 50% of another compound bound to the receptor (IC₅₀) is used as a measure of binding affinity. In these experiments the other compound is a ligand known to bind to the Mrg receptor with high affinity, such as an RF-amide peptide.

A variety of assay formats may be employed, including biochemical screening assays, immunoassays, cell-based assays and protein-protein binding assays, all of which are well characterized in the art. In one embodiment the assay involves anchoring the test compound onto a solid phase, adding the non-immobilized component comprising the Mrg receptor, and detecting Mrg/test compound complexes anchored on the solid phase at the end of the reaction. In an alternative embodiment, the Mrg may be anchored onto a solid surface, and the test compound, which is not anchored. In both situations either the test compound or the Mrg receptor is labeled, either directly or indirectly, to allow for identification of complexes. For example, an Mrg-Ig immunoadhesin may be anchored to a solid support and contacted with one or more test compounds.

Microtiter plates are preferably utilized as the solid phase and the anchored component may be immobilized by non-covalent or covalent attachments. Non-covalent attachment may be accomplished by simply coating the solid surface with a solution of the protein and drying. Alternatively, an immobilized antibody, preferably a monoclonal antibody, specific for the protein to be immobilized may be used to anchor the protein to the solid surface.

Alternatively, a reaction can be conducted in a liquid phase, the reaction products separated from unreacted components, and complexes detected; e.g., using an immobilized antibody specific for either Mrg polypeptide, peptide or fusion protein or the test compound to anchor any complexes formed in solution, and a labeled antibody specific for the other component of the possible complex to detect anchored complexes.

In one embodiment of these methods, a protein of the invention or a fragment of a protein of the invention , which are characterized by the ability to be activated by an RF-amide peptide, for instance, an extracellular domain fragment, is mixed with one or more potential binding partners, or an extract or fraction of a cell, under conditions that allow the association of potential binding partners with the protein of the invention. After mixing, peptides, polypeptides, proteins or other molecules that have become associated with a protein of the invention are separated from the mixture. The binding partner that bound to the protein of the invention can then be removed, identified and further analyzed. To identify and isolate a binding partner, the entire Mrg protein, for instance a protein comprising the entire amino acid sequence of SEQ ID NOs: 2 or 12 , which are characterized by the ability to be activated by an RF-amide peptide, can be used. Alternatively, a fragment of the Mrg polypeptide can be used.

As used herein, a cellular extract refers to a preparation or fraction which is made from a lysed or disrupted cell. The preferred source of cellular extracts will be cells derived from DRG. Alternatively, cellular extracts may be prepared from cells derived from any tissue, including normal human kidney tissue, or available cell lines, particularly kidney derived cell lines.

A variety of methods can be used to obtain an extract of a cell. Cells can be disrupted using either physical or chemical disruption methods. Examples of physical disruption methods include, but are not limited to, sonication and mechanical shearing. Examples of chemical lysis methods include, but are not limited to, detergent lysis and enzyme lysis. A skilled artisan can readily adapt methods for preparing cellular extracts in order to obtain extracts for use in the present methods.

Once an extract of a cell is prepared, the extract is mixed with the protein of the invention under conditions in which association of the protein with the binding partner can occur. Alternatively, one or more known compounds or molecules can be mixed with the protein of the invention. A variety of conditions can be used, the most preferred being conditions that closely resemble conditions found in the cytoplasm of a human cell. Features such as osmolarity, pH, temperature, and the concentration of cellular extract used, can be varied to optimize the association of the protein with the binding partner.

After mixing under appropriate conditions, the bound complex is separated from the mixture. A variety of techniques can be utilized to separate the mixture. For example, antibodies specific to a protein of the invention can be used to immunoprecipitate the binding partner complex. Alternatively, standard chemical separation techniques such as chromatography and density/sediment centrifugation can be used.

After removal of non-associated cellular constituents found in the extract, and/or unbound compounds or molecules, the binding partner can be dissociated from the complex using conventional methods. For example, dissociation can be accomplished by altering the salt concentration or pH of the mixture.

To aid in separating associated binding partner pairs from the mixed extract, the protein of the invention can be immobilized on a solid support. For example, the protein can be attached to a nitrocellulose matrix or acrylic beads. Attachment of the protein to a solid support aids in separating peptide/binding partner pairs from other constituents found in the extract. The identified binding partners can be either a single protein or a complex made up of two or more proteins or any other macromolecule.

Alternatively, binding partners may be identified using a Far-Western assay according to the procedures of Takayama et al. Methods Mol. Biol. 69:171-84 (1997) or Sauder et al. J Gen.Virol. 77(5): 991-6 or identified through the use of epitope tagged proteins or GST fusion proteins.

Binding partners may also be identified in whole cell binding assays that are well known in the art. In one embodiment, an Mrg receptor is expressed in cells in which it is not normally expressed, such as COS cells. The cells expressing Mrg are then contacted with a potential binding partner that has previously been labeled, preferably with radioactivity or a fluorescent marker. The cells are then washed to remove unbound material and the binding of the potential binding partner to the cells is assessed, for example by collecting the cells on a filter and counting radioactivity. The amount of binding of the potential binding partner to untransfected cells or mock transfected cells is subtracted as background.

This type of assay may be carried out in several alternative ways. For example, in one embodiment it is done using cell membrane fractions from cells transfected with an Mrg or known to express an Mrg, rather than whole cells. In another embodiment purified Mrg is refolded in lipids to produce membranes that are used in the assay.

Alternatively, the nucleic acid molecules of the invention can be used in cell based systems to detect protein-protein interactions (see WO99/55356). These systems have been used to identify other protein partner pairs and can readily he adapted to employ the nucleic acid molecules herein described.

Any in vitro method suitable for detecting protein-protein interactions may be employed for identifying proteins, including but not limited to soluble, transmembrane or intracellular proteins, that interact with Mrg receptors. Among the traditional methods which may be employed are co-immunoprecipitation, crosslinking and co-purification through gradients or chromatographic columns to identify proteins that interact with Mrg. For such assays, the Mrg component can be a full-length protein, a soluble derivative thereof, a peptide corresponding to a domain of interest, or a fusion protein containing some region of Mrg.

Methods may be employed which result in the simultaneous identification of genes that encode proteins capable of interacting with Mrg. These methods include, for example, probing expression libraries, using labeled Mrg or a variant thereof.

One method of detecting protein interactions in vivo that may be used to identify Mrg binding partners is the yeast two-hybrid system. This system is well known in the art and is commercially available from Clontech (Palo Alto, CA).

Briefly, two hybrid proteins are employed, one comprising the DNA-binding domain of a transcription activator protein fused to the Mrg receptor, or a polypeptide, peptide, or fusion protein therefrom, and the other comprising the transcription activator protein's activation domain fused to an unknown target protein. These proteins are expressed in a strain of the yeast Saccharomyces cerevisiae that contains a reporter gene (e.g., HBS or lacZ) whose regulatory region contains the transcription activator's binding site. While either hybrid protein alone cannot activate transcription of the reporter gene, interaction of the two hybrid proteins reconstitutes the functional activator protein and results in expression of the reporter gene, which is detected by an assay for the reporter gene product.

The target protein is preferably obtained from tissue or cells known to express the Mrg receptor, such as DRG cells. For example, a cDNA library prepared from DRG cells may be used.

Binding partners may also be identified by their ability to interfere with or disrupt the interaction of known ligands. Even if they do not activate Mrg receptors, binding partners that interfere with interactions with known ligands may be useful in regulating or augmenting Mrg activity in the body and/or controlling disorders associated with Mrg activity (or a deficiency thereof).

Compounds that interfere with the interaction between Mrg and a known ligand may be identified by preparing a reaction mixture containing Mrg, or some variant or fragment thereof, and a known binding partner, such as an RF-amide peptide, under conditions and for a time sufficient to allow the two to interact and bind, thus forming a complex. In order to test a compound for inhibitory activity, the reaction mixture is prepared in the presence and absence of the test compound. The test compound may be initially included in the reaction mixture, or may be added at a time subsequent to the addition of the Mrg and its binding partner. Control reaction mixtures are incubated without the test compound. The formation of any complexes between the Mrg and the binding partner is then detected. The formation of a complex in the control reaction, but not in the reaction mixture containing the test compound indicates that the compound interferes with the interaction of the Mrg and the known binding partner. Additionally, complex formation within reaction mixtures containing the test compound and normal Mrg protein may also be compared to complex formation within reaction mixtures containing the test compound and a mutant Mrg. This comparison may be important in those cases wherein it is desirable to identify compounds that specifically disrupt interactions of mutant, or mutated, Mrg but not the normal proteins.

The order of addition of reactants can be varied to obtain different information about the compounds being tested. For example, test compounds that interfere with the interaction by competition can be identified by conducting the binding reaction in the presence of the test substance. In this case the test compound is added to the reaction mixture prior to, or simultaneously with, Mrg and the known binding partner. Alternatively, test compounds that have the ability to disrupt preformed complexes can be identified by adding the test compound to the reaction mixture after complexes have been formed.

In an alternate embodiment of the invention, a preformed complex of Mrg and an interactive binding partner is prepared in which either the Mrg or its binding partners is labeled, but the signal generated by the label is quenched due to formation of the complex (see, e.g., U.S. Patent No. 4,109,496 to Rubenstein which utilizes this approach for immunoassays). The addition of a test compound that competes with and displaces one of the species from the preformed complex results in the generation of a signal above background. In this way, test substances which disrupt the interaction can be identified.

Whole cells expressing Mrg, membrane fractions prepared from cells expressing Mrg or membranes containing refolded Mrg may be used in the assays described above. However, these same asays can be employed using peptide fragments that correspond to the binding domains of Mrg and/or the interactive or binding partner (in cases where the binding partner is a protein), in place of one or both of the full length proteins. Any number of methods routinely practiced in the art can be used to identify and isolate the binding sites. These methods include, but are not limited to, mutagenesis of the gene encoding an Mrg protein and screening for disruption of binding of a known ligand.

The compounds identified can be useful, for example, in modulating the activity of wild type and/or mutant Mrg; can be useful in elaborating the biological function of Mrg receptors; can be utilized in screens for identifying compounds that disrupt normal Mrg receptor interactions or may themselves disrupt or activate such interactions; and can be useful therapeutically.

### J. In vitro Methods to Identify Agents that Modulate the Expression of a Nucleic Acid.

Another embodiment of the present invention provides in vitro methods for identifying agents that modulate the expression of a nucleic acid encoding a mrg protein of the invention or another protein involved in an mrg mediated pathway. These agents may be, but are not limited to, peptides, peptide mimetics, and small organic molecules that are able to gain entry into an appropriate cell (e.g., in the DRG) and affect the expression of a gene. Agents that modulate the expression of Mrg or a protein in an mrg mediated pathway may be useful therapeutically, for example to increase or decrease sensory perception, such as the perception of pain, to treat glaucoma, or to increase or decrease wound healing.

Such assays may utilize any available means of monitering for changes in the expression level of the nucleic acids of the invention. As used herein, an agent is said to modulate the expression of a nucleic acid of the invention, for instance a nucleic acid encoding the protein having the sequence of SEQ ID NOs: 2 or 12 which are characterised by the ability to be activated by an RF- amide - peptide if it is capable of up- or down-regulating expression of the gene or mRNA levels nucleic acid in a cell.

In one assay format, cell lines that contain reporter gene fusions between the open reading frames and/or the 5' or 3' regulatory sequences of a gene of the invention and any assayable fusion partner may be prepared. Numerous assayable fusion partners are known and readily available including the firefly luciferase gene and the gene encoding chloramphenicol acetyltransferase (Alam et al. Anal. Biochem. 188:245-254 (1990)). Cell lines containing the reporter gene fusions are then exposed to the agent to be tested under appropriate conditions and time. Differential expression of the reporter gene between samples exposed to the agent and control samples identifies agents which modulate the expression of a nucleic acid encoding a mrg protein.

Additional assay formats may be used to monitor the ability of the agent to modulate the expression of a nucleic acid encoding a mrg protein of the invention. For instance, mRNA expression may be monitored directly by hybridization to the nucleic acids of the invention. Cell lines are exposed to the agent to be tested under appropriate conditions and time and total RNA or mRNA is isolated by standard procedures such those disclosed in Sambrook et al. (Molecular Cloning: A Laboratory Manual, 2nd Ed. Cold Spring Harbor Laboratory Press, 1989).

Probes to detect differences in RNA expression levels between cells exposed to the agent and control cells may be prepared from the nucleic acids of the invention. It is preferable, but not necessary, to design probes which hybridize only with target nucleic acids under conditions of high stringency. Only highly complementary nucleic acid hybrids form under conditions of high stringency. Accordingly, the stringency of the assay conditions determines the amount of complementarity which should exist between two nucleic acid strands in order to form a hybrid. Stringency should be chosen to maximize the difference in stability between the probe:target hybrid and potential probe:non-target hybrids.

Probes may be designed from the nucleic acids of the invention through methods known in the art. For instance, the G+C content of the probe and the probe length can affect probe binding to its target sequence. Methods to optimize probe specificity are commonly available in Sambrook et al. (Molecular Cloning: A Laboratory Manual, 2nd Ed. Cold Spring Harbor Laboratory Press, NY, 1989) or Ausubel et al. (Current Protocols in Molecular Biology, Greene Publishing Co., NY, 1995).

Hybridization conditions are modified using known methods, such as those described by Sambrook et al. and Ausubel et al., as required for each probe. Hybridization of total cellular RNA or RNA enriched for polyA RNA can be accomplished in any available format. For instance, total cellular RNA or RNA enriched for polyA RNA can be affixed to a solid support and the solid support exposed to at least one probe comprising at least one, or part of one of the sequences of the invention under conditions in which the probe will specifically hybridize. Alternatively, nucleic acid fragments comprising at least one, or part of one of the sequences of the invention can be affixed to a solid support, such as a silicon chip or porous glass wafer. The wafer can then be exposed to total cellular RNA or polyA RNA from a sample under conditions in which the affixed sequences will specifically hybridize. Such wafers and hybridization methods are widely available, for example, those disclosed by Beattie (WO 95/11755). By examining for the ability of a given probe to specifically hybridize to an RNA sample from an untreated cell population and from a cell population exposed to the agent, agents which up or down regulate the expression of a nucleic acid encoding a mrg or drg-12 are identified.

Hybridization for qualitative and quantitative analysis of mRNAs may also be carried out by using a RNase Protection Assay (i.e., RPA, see Ma et al. Methods 10: 273-238 (1996)). Briefly, an expression vehicle comprising cDNA encoding the gene product and a phage specific DNA dependent RNA polymerase promoter (e.g., T7, T3 or SP6 RNA polymerase) is linearized at the 3' end of the cDNA molecule, downstream from the phage promoter, wherein such a linearized molecule is subsequently used as a template for synthesis of a labeled antisense transcript of the cDNA by in vitro transcription. The labeled transcript is then hybridized to a mixture of isolated RNA (i.e., total or fractionated mRNA) by incubation at 45°C overnight in a buffer comprising 80% formamide, 40 mM Pipes, pH 6.4, 0.4 M NaCl and 1 mM EDTA. The resulting hybrids are then digested in a buffer comprising 40 µg/ml ribonuclease A and 2 µg/ml ribonuclease. After deactivation and extraction of extraneous proteins, the samples are loaded onto urea/polyacrylamide gels for analysis.

In another assay format, products, cells or cell lines are first be identified which express mrg gene products physiologically. Cells and/or cell lines so identified would be expected to comprise the necessary cellular machinery such that the fidelity of modulation of the transcriptional apparatus is maintained with regard to exogenous contact of agent with appropriate surface transduction mechanisms and/or the cytosolic cascades. Such cells or cell lines are then transduced or transfected with an expression vehicle (e.g., a plasmid or viral vector) construct comprising an operable non-translated 5' or 3'-promoter containing end of the structural gene encoding the instant gene products fused to one or more antigenic fragments, which are peculiar to the instant gene products, wherein said fragments are under the transcriptional control of said promoter and are expressed as polypeptides whose molecular weight can be distinguished from the naturally occurring polypeptides or may further comprise an immunologically distinct tag. Such a process is well known in the art.

Cells or cell lines transduced or transfected as outlined above are then contacted with agents under appropriate conditions; for example, the agent comprises a pharmaceutically acceptable excipient and is contacted with cells comprised in an aqueous physiological buffer such as phosphate buffered saline (PBS) at physiological pH, Eagles balanced salt solution (BSS) at physiological pH, PBS or BSS comprising serum or conditioned media comprising PBS or BSS and/or serum incubated at 37° C. Said conditions may be modulated as deemed necessary by one of skill in the art. Subsequent to contacting the cells with the agent, said cells will be disrupted and the polypeptides of the lysate are fractionated such that a polypeptide fraction is pooled and contacted with an antibody to be further processed by immunological assay (e.g., ELISA, immunoprecipitation or Western blot). The pool of proteins isolated from the "agent-contacted" sample will be compared with a control sample where only the excipient is contacted with the cells and an increase or decrease in the immunologically generated signal from the "agent-contacted" sample compared to the control will be used to distinguish the effectiveness of the agent.

The probes described above for identifying differential expression of Mrg mRNA in response to applied agents can also be used to identify differential expression of Mrg mRNA in populations of mammals, for example populations with differing levels of sensory perception. Methods for identifying differential expression of genes are well known in the art. In one embodiment, mRNA is prepared from tissue or cells taken from patients exhibiting altered sensory perception, such as patients experiencing neuropathic pain, or suffering from a disease or disorder in which the Mrg receptor may play a role, such as glaucoma, and Mrg expression levels are quantified using the probes described above. The Mrg expression levels may then be compared to those in other populations to determine the role that Mrg expression is playing in the alteration of sensory perception and to determine whether treatment aimed at increasing or decreasing Mrg expression levels would be appropriate.

### K. In vitro Methods to Identify Agents that Modulate Protein Levels or at Least One Activity of the Proteins of DRG Primary Sensory Neurons.

Another embodiment of the present invention provides in vitro methods for identifying agents or conditions that modulate protein levels and/or at least one activity of a mrg protein of the invention, including agonists and antagonists. Such methods or assays may utilize any means of monitoring or detecting the desired activity.

In one format, the relative amounts of a protein of the invention between a cell population that has been exposed to the agent to be tested compared to an unexposed control cell population may be assayed. In this format, probes such as specific antibodies are used to monitor the differential expression of the protein in the different cell populations. Cell lines or populations are exposed to the agent to be tested under appropriate conditions and time. Cellular lysates may be prepared from the exposed cell line or population and a control, unexposed cell line or population. The cellular lysates are then analyzed with the probe.

In another embodiment, animals known to express Mrg receptors are subjected to a particular environmental stimulus and any change produced in Mrg protein expression by exposure to the stimulus is measured. Transgenic animals, such as transgenic mice, produced to express a particular Mrg in a particular location may be used. The environmental stimulus is not limited and may be, for example, exposure to stressful conditions, or exposure to noxious or painful stimuli. Differences in Mrg receptor expression levels in response to environmental stimuli may provide insight into the biological role of Mrgs and possible treatments for diseases or disorders related to the stimuli used.

Antibody probes are prepared by immunizing suitable mammalian hosts in appropriate immunization protocols using the peptides, polypeptides or proteins of the invention if they are of sufficient length, or, if desired, or if required to enhance immunogenicity, conjugated to suitable carriers. Methods for preparing immunogenic conjugates with carriers such as BSA, KLH, or other carrier proteins are well known in the art. In some circumstances, direct conjugation using, for example, carbodiimide reagents may be effective; in other instances linking reagents such as those supplied by Pierce Chemical Co. (Rockford, IL), may be desirable to provide accessibility to the hapten. The hapten peptides can be extended at either the amino or carboxy terminus with a cysteine residue or interspersed with cysteine residues, for example, to facilitate linking to a carrier. Administration of the immunogens is conducted generally by injection over a suitable time period and with use of suitable adjuvants, as is generally understood in the art. During the immunization schedule, titers of antibodies are taken to determine adequacy of antibody formation.

While the polyclonal antisera produced in this way may be satisfactory for some applications, for pharmaceutical compositions, use of monoclonal preparations is preferred. Immortalized cell lines which secrete the desired monoclonal antibodies may be prepared using the standard method of Kohler and Milstein Nature 256:495-497 (1975)) or modifications which effect immortalization of lymphocytes or spleen cells, as is generally known. The immortalized cell lines secreting the desired antibodies are screened by immunoassay in which the antigen is the peptide hapten, polypeptide or protein. When the appropriate immortalized cell culture secreting the desired antibody is identified, the cells can be cultured either in vitro or by production in ascites fluid.

The desired monoclonal antibodies are then recovered from the culture supernatant or from the ascites supernatant. Fragments of the monoclonals or the polyclonal antisera which contain the immunologically significant portion can be used as antagonists, as well as the intact antibodies. Use of immunologically reactive fragments, such as the Fab, Fab', of F(ab')₂ fragments is often preferable, especially in a therapeutic context, as these fragments are generally less immunogenic than the whole immunoglobulin.

The antibodies or fragments may also be produced, using current technology, by recombinant means. Antibody regions that bind specifically to the desired regions of the protein can also be produced in the context of chimeras with multiple species origin, such as humanized antibodies as discussed in more detail below.

### 1. Identification of Agonists and Antagonists

The present invention provides for in vitro assays to identify compounds that serve as agonists or antagonists of one or more of the biological properties of Mrg . Mrg agonists and antagonists may be useful in the prevention and treatment of problems associated with sensory perception, particularly nociception. For example, compounds identified as Mrg receptor agonists may be used to stimulate Mrg receptor activation and thus may be effective in treating pain. Compounds that are identified as Mrg receptor antagonists may be used, for example, to decrease the effector functions of Mrg receptors. This may be useful in cases where the Mrg receptors contain a mutation that produces increased responsiveness, or in cases of Mrg receptor overexpression. For instance, Mrg receptor antagonists may be useful in increasing the sensitivity of mammals to pain where appropriate, such as in diseases involving decreased sensory responsiveness, like some forms of diabetes.

Assays for identifying agonists or antagonsts may be done in vitro or in vivo, by monitoring the response of a cell following binding of the ligand to the receptor. An agonist will produce a cellular response, while an antagonist will have no affect on cellular response but will be capable of preventing cellular response to a known agonist.

### a. Small Molecules

Small molecules may have the ability to act as Mrg agonists or antagonists and thus may be screened in vitro for an effect on a biological activity of Mrg. Small molecules preferably have a molecular weight of less than 10 kD, more preferably less than 5 kD and even more preferably less than 2 kD. Such small molecules may include naturally occurring small molecules, synthetic organic or inorganic compounds, peptides and peptide mimetics. However, small molecules in the present invention are not limited to these forms. Extensive libraries of small molecules are commercially available and a wide variety of assays are well known in the art to screen these molecules for the desired activity.

Candidate Mrg agonist and antagonist small molecules are preferably first identified in an assay that allows for the rapid identification of potential agonists and antagonists. An example of such an assay is a binding assay wherein the ability of the candidate molecule to bind to the Mrg receptor is measured, such as those described above. In another example, the ability of candidate molecules to interfere with the binding of a known ligand, for example FMRFamide to MrgA1, is measured. Candidate molecules that are identified by their ability to bind to Mrg proteins or interfere with the binding of known ligands are then tested for their ability to stimulate one or more biological activities.

The activity of the proteins of the invention may be monitored in cells expressing the mrg proteins of the invention by assaying for physiological changes in the cells upon exposure to the agent or agents to be tested. Such physiological changes include but are not limited to the flow of current across the membrane of the cell.

In one embodiment the protein is expressed in a cell that is capable of producing a second messenger response and that does not normally express Mrg. The cell is then contacted with the compound of interest and changes in the second messenger response are measured. Methods to monitor or assay these changes are readily available. For instance, the mrg genes of the invention may be expressed in cells expressing G 15, a G protein subunit that links receptor activation to increases in intracellular calcium [Ca²⁺] which can be monitored at the single cell level using the FURA-2 calcium indicator dye as disclosed in Chandrashekar et al. Cell 100:703-711, (2000). This assay is described in more detail in Example 5.

Similar assays may also be used to identify inhibitors or antagonists of Mrg activation. For example, cells expressing Mrg and capable of producing a quantifiable response to receptor activation are contacted with a known Mrg activator and the compound to be tested. In one embodiment, HEK cells expressing G 15 and MrgA1 are contacted with FMRFamide and the compound to be tested. The cellular response is measured, in this case increase in [Ca²⁺]. A decreased response compared to the known activator by itself indicates that the compound acts as an inhibitor of activation.

While such assays may be formatted in any manner, particularly preferred formats are those that allow high - throughput screening (HTP). In HTP assays of the invention, it is possible to screen thousands of different modulators or ligands in a single day. For instance, each well of a microtiter plate can be used to run a separate assay, for instance an assay based on the ability of the test compounds to modulate receptor activation derived increases in intracellular calcium as described above.

Agents that are assayed in the above method can be randomly selected or rationally selected or designed. As used herein, an agent is said to be randomly selected when the agent is chosen randomly without considering the specific sequences involved in the association of the a protein of the invention alone or with its associated substrates, binding partners, etc. An example of randomly selected agents is the use a chemical library or a peptide combinatorial library, or a growth broth of an organism.

As used herein, an agent is said to be rationally selected or designed when the agent is chosen on a nonrandom basis which takes into account the sequence of the target site and/or its conformation in connection with the agent's action. Sites of interest might be peptides within the membrane spanning regions, cytoplasmic and extracellular peptide loops between these transmembrane regions, or selected sequences within the N-terminal extracellular domain or C-terminal intracellular domain. Agents can be rationally selected or rationally designed by utilizing the peptide sequences that make up these sites.

The agents of the present invention can be, as examples, peptides, small molecules, vitamin derivatives, as well as carbohydrates. Dominant negative proteins, DNAs encoding these proteins, antibodies to these proteins, peptide fragments of these proteins or mimics of these proteins may be introduced into cells to affect function. "Mimic" used herein refers to the modification of a region or several regions of a peptide molecule to provide a structure chemically different from the parent peptide but topographically and functionally similar to the parent peptide (see Grant GA. in: Meyers (ed.) Molecular Biology and Biotechnology (New York, VCH Publishers, 1995), pp. 659-664). A skilled artisan can readily recognize that there is no limit as to the structural nature of the agents of the present invention.

The peptide agents of the invention can be prepared using standard solid phase (or solution phase) peptide synthesis methods, as is known in the art. In addition, the DNA encoding these peptides may be synthesized using commercially available oligonucleotide synthesis instrumentation and produced recombinantly using standard recombinant production systems. The production using solid phase peptide synthesis is necessitated if non-gene-encoded amino acids are to be included.

### b. Antibodies

Another class of agents of the present invention are antibodies immunoreactive with critical positions of proteins of the invention. These antibodies may be human or non-human, polyclonal or monoclonal and may serve as agonist antibodies or neutralizing antibodies. They include amino acid sequence variants, glycosylation variants and fragments of antibodies. Antibody agents are obtained by immunization of suitable mammalian subjects with peptides, containing as antigenic regions, those portions of the protein intended to be targeted by the antibodies. General techniques for the production of such antibodies and the selection of agonist or neutralizing antibodies are well known in the art.

The antibodies of the present invention can be polyclonal antibodies, monoclonal antibodies, chimeric antibodies, humanized antibodies, human antibodies, heteroconjugate antibodies, or antibody fragments. In addition, the antibodies can be made by any method known in the art, including recombinant methods.

Mrg agonist and neutralizing antibodies may be preliminarily identified based on their ability to bind the Mrg receptor. For example, Western blot techniques well known in the art may be used to screen a variety of antibodies for their ability to bind Mrg. Mrg agonist and neutralizing antibodies are then identified from the group of candidate antibodies based on their biological activity. In one embodiment, Mrg agonist antibodies are identified by their ability to induce activation of a second messenger system in cells expressing the Mrg protein and comprising a second messenger system. For example, HEK cells overexpressing G 15 and transfected with mrg may be contacted with a potential Mrg agonist antibody. An increase in intracellular calcium, measured as described in Example 5, would indicate that the antibody is an agonist antibody.

Identification of a neutralizing antibody involves contacting a cell expressing Mrg with a known Mrg ligand, such as an RF-amide peptide, and the candidate antibody and observing the effect of the antibody on Mrg activation. In one embodiment, Mrg receptors expressed in HEK cells overexpressing G 15 are contacted with an Mrg ligand such as FMRFamide and the candidate neutralizing antibody. A decrease in responsiveness to the ligand, measured as described in Example 5, would indicate that the antibody is a neutralizing antibody.

### c. Other antagonists

The Mrg antagonists are not limited to Mrg ligands. Other antagonists include variants of a native Mrg receptor that retains the ability to bind an endogenous ligand but is not able to mediate a biological response. Soluble receptors and immunoadhesins that bind Mrg ligands may also be antagonists, as may antibodies that specifically bind a ligand near its binding site and prevent its interaction with the native receptor. These antagonists may be identified in the assays described above.

### d. Computer Modeling

Computer modeling and searching technologies permit identification of compounds, or the improvement of already identified compounds, that can modulate Mrg receptor expression or activity. Once an agonist or antagonist is identified, the active sites or regions, such as ligand binding sites, are determined. The active site can be identified using methods known in the art including, for example, by determing the effect of various amino acid substitutions or deletions on ligand binding or from study of complexes of the relevant compound or composition with its natural ligand, such as with X-ray crystallography.

Next, the three dimensional geometric structure of the active site is determined such as by X-ray crystallography, NMR, chemical crosslinking or other methods known in the art. Computer modeling can be utilized to make predictions about the structure where the experimental results are not clear. Examples of molecular modeling systems are the CHARMm and QUANTA programs (Polygen Corporation, Waltham, MA). Once a predicted structure is determined, candidate modulating compounds can be identified by searching databases containing compounds along with information on their molecular structure in an effort to find compuonds that have structures capable of interacting with the active site. The compounds found from this search are potential modulators of the activity of the proteins of the present invention and can be tested in the assays described above.

The agonistic or antagonistic activity of test compounds identified in cell based assays as described above can be further elucidated in assays using animals, for example transgenic animals that overexpress Mrg receptors as described in more detail below. In one embodiment, the effect of administration of potential Mrg antagonists or agonists on the responsiveness of such transgenic animals to sensory stimuli, such as noxious or painful stimuli, is measured. The therapeutic utility of such compounds may be confirmed by testing in these types of experiments or in animal models of particular disorders, for example animal models of neuropathic pain.

### L. Uses for Agents that modulate at Least One Activity of the Proteins.

As provided in the Examples, the mrg proteins and nucleic acids of the invention, are expressed in the primary nociceptive sensory neurons of ORG. In addition the Mrg receptors are expressed in specialized skin cells that play a role in wound repair. Further, proteins homologous to Mrg receptors are expressed in the trabecular meshwork of the eye and a role for them has been suggested in the regulation of pressure in the eye (Gonzalez et al. Invest. Ophth. Vis. Sci. 41: 3678-3693 (2000)). Thus, the present invention further provides compositions containing one or more agents that modulate expression or at least one activity of a protein of the invention. For example, the invention provides ligands that directly activate Mrg receptors.

Agents that modulate, up-or-down-regulate the expression of the protein or agents such as agonists or antagonists of at least one activity of the protein may be used to modulate biological and pathalagic processes associated with the protein's function and activity. Several agents that activate the Mrg receptors are identified in the examples, including the RF-amide peptides. Thus the present invention provides methods to treat pain, including neuropathic pain, as well as to promote wound healing, to restore normal sensitivity following injury and to treat ocular conditions, particularly those associated with pressure, such as glaucoma.

As described in the Figures and Examples, expression of a protein of the invention may be associated with biological processes of nociception, which may also be considered pathological processes. As used herein, an agent is said to modulate a biological or pathological process when the agent alters the degree, severity or nature of the process. For instance, the neuronal transmission of pain signals may be prevented or modulated by the administration of agents which up-regulate down-regulate or modulate in some way the expression or at least one activity of a protein of the invention.

The pain that may be treated by the proteins of the present invention and agonists and antagonists thereof, is not limited in any way and includes pain associated with a disease or disorder, pain associated with tissue damage, pain associated with inflammation, pain associated with noxious stimuli of any kind, and neuropathic pain, including pain associated with peripheral neuropathies, as well as pain without an identifiable source. The pain may be subjective and does not have to be associated with an objectively quantifiable behavior or response.

In addition to treating pain, the compounds and in vitro methods of the present invention may be useful for increasing or decreasing sensory responses. It may be useful to increase responsiveness to stimuli, including noxious stimuli and painful stimuli, in some disease states that are characterized by a decreased responsiveness to stimuli, for example in diabetes.

Certain conditions, such as chronic disease states associated with pain and peripheral neuropathies and particlularly conditions resulting from a defective Mrg gene, can benefit from an increase in the responsiveness to Mrg receptor ligands. Thus these condition may be treated by increasing the number of functional Mrg receptors in cells of patients suffering from such conditions. This could be increasing the expression of Mrg receptor in cells through gene therapy using Mrg-encoding nucleic acid. This includes both gene therapy where a lasting effect is achieved by a single treatment, and gene therapy where the increased expression is transient. Selective expression of Mrg in appropriate cells may be achieved by using Mrg genes controlled by tissue specific or inducible promoters or by producing localized infection with replication defective viruses carrying a recombinant Mrg gene, or by any other method known in the art.

In the therapeutic methods of the present invention the patient is administered an effective amount of a composition of the present invention, such as an Mrg protein, peptide fragment, Mrg variant, Mrg agonist, Mrg antagonist, or anti-Mrg antibody of the invention.

The agents of the present invention can be provided alone, or in combination with other agents that modulate a particular biological or pathological process. For example, an agent of the present invention can be administered in combination with other known drugs or may be combined with analgesic drugs or non-analgesic drugs used during the treatment of pain that occurs in the presence or absence of one or more other pathological processes. As used herein, two or more agents are said to be administered in combination when the two agents are administered simultaneously or are administered independently in a fashion such that the agents will act at the same time.

The agents of the present invention are administered to a mammal, preferably to a human patient, in accord with known methods. Thus the agents of the present invention can be administered via parenteral, subcutaneous, intravenous, intramuscular, intraperitoneal, intracerebrospinal, intra-articular, intrasynovial, intrathecal, transdermal, topical, inhalation or buccal routes. They may be administered continuously by infusion or by bolus injection. Generally, where the disorder permits the agents should be delivered in a site-specific manner. Alternatively, or concurrently, administration may be by the oral route. The dosage administered will be dependent upon the age, health, and weight of the recipient, kind of concurrent treatment, if any, frequency of treatment, and the nature of the effect desired.

The toxicity and therapeutic efficacy of agents of the present invention can be determined by standard pharmaceutical procedures in cell cultures or experimental animals. While agents that exhibit toxic side effects can be used, care should be taken to design a delivery system that targets such compounds to the desired site of action in order to reduce side effects.

While individual needs vary, determination of optimal ranges of effective amounts of each component is within the skill of the art. For the prevention or treatment of disease, the appropriate dosage of agent will depend on the type of disease to be treated, the severity and course of the disease, whether the agent is administered for preventive or therapeutic purposes, previous therapy, the patient's clinical history and response to the agent, and the discretion of the attending physician. Therapeutic agents are suitably administered to the patient at one time or over a series of treatments. Typical dosages comprise 0.1 to 100 µg/kg body wt. The preferred dosages comprise 0.1 to 10 µg/kg body wt. The most preferred dosages comprise 0.1 to 1 µg/kg body wt. For repeated administrations over several days or longer, depending on the condition, the treatment is sustained until a desired suppression of disease symptoms occurs. The progress of this therapy is easily monitored by conventional techniques and assays.

In addition to the pharmacologically active agent, the compositions of the present invention may contain suitable pharmaceutically acceptable carriers comprising excipients and auxiliaries that facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension include, for example, sodium carboxymethyl cellulose, sorbitol, and/or dextran. Optionally, the suspension may also contain stabilizers. Liposomes can also be used to encapsulate the agent for delivery into the cell. The agent can also be prepared as a sustained-release formulation, including semipermeable matrices of solid hydrophobic polymers containing the protein. The sustained release preparation may take the form of a gel, film or capsule.

The pharmaceutical formulation for systemic administration according to the invention may be formulated for enteral, parenteral or topical administration. Indeed, all three types of formulations may be used simultaneously to achieve systemic administration of the active ingredient.

Suitable formulations for oral administration include hard or soft gelatin capsules, pills, tablets, including coated tablets, elixirs, suspensions, syrups or inhalations and controlled release forms thereof.

In practicing the methods of this invention, the compounds of this invention may be used alone or in combination with other therapeutic or diagnostic agents. In certain preferred embodiments, the compounds of this invention may be co-administered along with other compounds typically prescribed for these conditions according to generally accepted medical practice. The compounds of this invention can be utilized in vivo, ordinarily in mammals, such as humans, sheep, horses, cattle, pigs, dogs, cats, rats and mice, or in vitro. When used in vivo, the compounds must be sterile. This is readily accomplished by filtration through sterile filtration membranes.

### a. Articles of Manufacture

In another embodiment of the invention, an article of manufacture containing materials useful for the treatment of the disorders described above is provided. The article of manufacture comprises a container and a label or package insert(s) on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is an Mrg agonist. The label or package insert indicates that the composition is used for treating the condition of choice, such as to reduce neuropathic pain. In one embodiment, the label or package inserts indicates that the composition comprising the Mrg agonist can be used to treat pain, glaucoma or to accelerate wound healing.

### M. Transgenic Non-Human Animals

Transgenic non-human animals containing mutant, knock-out or modified genes corresponding to the mrg sequences are also included in the invention. Transgenic non-human animals are genetically modified animals into which recombinant, exogenous or cloned genetic material has been experimentally transferred. Such genetic material is often referred to as a "transgene". The nucleic acid sequence of the transgene, in this case a form of SEG ID NOS: 1 or 11, may be integrated either at a locus of a genome where that particular nucleic acid sequence is not otherwise normally found or at the normal locus for the trans gene. The transgene may consist of nucleic acid sequences derived from the genome of the same species or of a different species than the species of the target animal.

The term "germ cell line transgenic animal" refers to a transgenic non-human animal in which the genetic alteration or genetic information was introduced into a germ line cell, thereby conferring the ability of the transgenic non-human animal to transfer the genetic information to offspring. If such offspring in fact possess some or all of that alteration or genetic information, then they too are transgenic animals.

The alteration or genetic information may be foreign to the species of non-human animal to which the recipient belongs, foreign only to the particular individual recipient, or may be genetic information already possessed by the recipient. In the last case, the altered or introduced gene may be expressed differently than the native gene.

Transgenic non-human animals can be produced by a variety of different methods including transfection, electroporation, microinjection, gene targeting in embryonic stem cells and recombinant viral and retroviral infection (see, e.g., U.S. Patent No. 4,736,866; U.S. Patent No. 5,602,307; Mullins et al. Hypertension 22(4):630-633 (1993); Brenin et aL Surg. Oncol. 6(2)99-110 (1997); Tuan (ed.), Recombinant Gene Expression Protocols, Methods in Molecular Biology No. 62, Humana Press (1997)).

A number of recombinant or transgenic mice have been produced, including those which express an activated oncogene sequence (U.S. Patent No. 4,736,866); express simian SV40 T-antigen (U.S. Patent No. 5,728,915); lack the expression of interferon regulatory factor 1 (IRF-1) (U.S. Patent No. 5,731,490); exhibit dopaminergic dysfunction (U.S. Patent No. 5,723,719); express at least one human gene which participates in blood pressure control (U.S. Patent No. 5,731,489); display greater similarity to the conditions existing in naturally occurring Alzheimer's disease (U.S. Patent No. 5,720,936); have a reduced capacity to mediate cellular adhesion (U.S. Patent No. 5,602,307); possess a bovine growth hormone gene (Clutter et al. Genetics 143(4):1753-1760 (1996)); or, are capable of generating a fully human antibody response (McCarthy The Lancet 349(9049):405 (1997)).

While mice and rats remain the animals of choice for most transgenic experimentation, in some instances it is preferable or even necessary to use alternative animal species. Transgenic procedures have been successfully utilized in a variety of non-murine animals, including sheep, goats, pigs, dogs, cats, monkeys, chimpanzees, hamsters, rabbits, cows and guinea pigs (see, e.g., Kim et al. Mol. Reprod. Dev. 46(4): 515-526 (1997); Houdebine Reprod. Nutr. Dev. 35(6):609-617 (1995); Patters Reprod. Fertil. Dev. 6(5):643-645 (1994); Schnieke et al. Science 278(5346):2130-2133 (1997); and Amoah J. Animal Science 75(2):578-585 (1997)).

The method of introduction of nucleic acid fragments into recombination competent mammalian cells can be by any method that favors co-transformation of multiple nucleic acid molecules. Detailed procedures for producing transgenic animals are readily available to one skilled in the art, including the disclosures in U.S. Patent No. 5,489,743 and U.S. Patent No. 5,602,307.

It is contemplated that mice lacking a particular Mrg gene, or in which expression of a particular Mrg has been increased or decreased will be used in an assay for determining how Mrgs influence behavior, including sensory responses, particularly responses to painful stimuli. In particular, transgenic mice will be used to determine if Mrg mediates the response to a particular type of noxious stimuli, such as mechanical, thermal or chemical. Thus in one embodiment transgenic mice lacking native Mrg receptors, or in which Mrg receptor expression levels have been modified, will be tested to determine their sensitivity to pressure, temperature, and other noxious stimuli. Assays for determining sensitivity to stimuli are well known in the art. These include, but are not limited to, assays that measure responsiveness to mechanical pain (von Frey hairs or tail pinch), thermal pain (latency to lick or jump in the hot plate assay), chemical pain (latency to lick when a noxious substance such as capsaicin or formalin is injected in the paw), visceral pain (abdominal stretching in response to intraperitoneal injection of acetic acid) and neuropathic pain. For example, mice in which one or more Mrgs have been deleted will be tested for their responsiveness to a variety of painful stimuli of varying intensity. By determining the sensory responses that are mediated by the Mrg receptors, therapeutic agents known to stimulate or inhibit Mrg receptors can be chosen for the treatment of disease states known to involve these types of responses. In addition, therapeutics specifically aimed at treating disorders involving these responses can be developed by targeting the Mrg receptors.

In one embodiment, transgenic mice expressing one or more human Mrg proteins are produced. The expression pattern of the human Mrg protein may then be determined and the effect of the expression of the human Mrg protein on various sensory modalities may be investigated. Further, the efficacy of potential therapeutic agents may be investigated in these mice.

In addition, the effects of changes in the expression levels of specific Mrg proteins can be investigated in animal models of disease states. By identifying the effect of increasing or decreasing Mrg receptor levels and activation, therapeutic regimens useful in treating the diseases can be developed. In one embodiment, mice in which Mrg receptor expression levels have been increased or decreased are tested in models of neuropathic pain.

Further, mice in which Mrg expression levels have been manipulated may be tested for their ability to respond to compounds known to modulate responsiveness to pain, such as analgesics. In this way the role of Mrg in the sensation of pain may be further elucidated. For example, a lack of response to a known analgesic in the transgenic mice lacking Mrg would indicate that the Mrg receptors play a role in mediating the action of the analgesic.

Another preferred transgenic mouse is one in which the Mrg gene is modified to express a marker or tracer such as green fluorescent protein (GFP). By examining the expression pattern of the marker or tracer, the exact location and projection of Mrg containing neurons and other cells can be mapped. This information will be compared to the location and projection of neurons and other cells whose involvment in specific disease states has previously been identified. In this way additional therapeutic uses for the compounds of the present invention may be realized.

### N. Diagnostic Methods

As described in the Examples, the genes and proteins of the invention may be used to diagnose or monitor the presence or absence of sensory neurons and of biological or pathological activity in sensory neurons in vitro. For instance, expression of the genes or proteins of the invention may be used to differentiate between normal and abnormal sensory neuronal activities associated with acute pain, chronic intractable pain, or allodynia. Expression levels can also be used to differentiate between various stages or the severity of neuronal abnormalities. One means of diagnosing pathological states of sensory neurons involved in pain transmission using the nucleic acid molecules or proteins of the invention involves obtaining tissue from living subjects. These subjects may be non-human animal models of pain.

The use of molecular biological tools has become routine in forensic technology. For example, nucleic acid probes may be used to determine the expression of a nucleic acid molecule comprising all or at least part of the sequences of the invention in forensic/pathology specimens. Further, nucleic acid assays may be carried out by any means of conducting a transcriptional profiling analysis. In addition to nucleic acid analysis, forensic methods of the invention may target the proteins of the invention to determine up or down regulation of the genes (Shiverick et al., Biochim Biophys Acta 393(1): 124-33 (1975)).

Methods of the invention may involve treatment of tissues with collagenases or other proteases to make the tissue amenable to cell lysis (Semenov et al., Biull Eksp Biol Med 104(7): 113-6 (1987)). Further, it is possible to obtain biopsy samples from different regions of the kidney or other tissues for analysis.

Assays to detect nucleic acid or protein molecules of the invention may be in any available format. Typical in vitro assays for nucleic acid molecules include hybridization or PCR based formats. Typical assays for the detection of proteins, polypeptides or peptides of the invention include the use of antibody probes in any available format such as in situ binding assays, etc. See Harlow et al., Antibodies: A Laboratory Manual, Cold Spring Harbor Laboratory, 1988 and Section G. In preferred embodiments, assays are carried-out with appropriate controls.

The above in vitro methods may also be used in other diagnostic protocols, including protocols and methods to detect disease states in other tissues or organs.

### 0. Methods of Identifying Other Genes Expressed in Primary Nociceptive Sensory Neurons.

As described in the Examples, the mrg genes of the invention have been identified RNA using a suppression-PCR-based method (Clontech) to enrich for genes expressed in the DRG of wild type but not Ngn1 mutant mice. This general method may be used to identify and isolate other DRG specific genes by producing transgenic mice that do not express other genes required for the development or presence of the nociceptive subset of DRG neurons. For instance, TrkA ⁺ mice may be used in the methods of the invention to isolate other genes associated with nociceptive DRG neurons (see Lindsay Philos. Trans R. Soc. Lond. B. Biol. Sci. 351(1338): 365-73 (1996) and Walsh et al. J. Neurosci. 19(10):4155-68).

Without further description, it is believed that one of ordinary skill in the art can, using the preceding description and the following illustrative examples, make and utilize the compounds of the present invention and practice the claimed methods. The following working examples therefore, specifically point out preferred embodiments of the present invention, and are not to be construed as limiting in any way the remainder of the disclosure.

### EXAMPLES

### Example 1: Positive selection-based differential hybridization between wild type and Ngn1^{-/-} DRG to identify candidate genes involved in nociception.

Previous studies have shown that Neurogenin1 (Ngn1), a bHLH transcription factor (Ma et al. Cell 87: 43-52 (1996)), is required for cell fate determination of nociceptive sensory neurons in dorsal root ganglia (DRG) (Ma et al. Genes & Dev. 13: 1717-1728 (1999)). In Ngn1^{-/-} mutant mouse embryos most if not all trkA⁺ neurons, which include the nociceptive subclass, fail to be generated. This mutant phenotype was exploited to isolate genes specifically expressed in such neurons, by subtracting cDNAs from neonatal wild-type and Ngn1^{-/-} DRG. Genes expressed in the former but not the latter cDNA population are specific to trkA⁺ nociceptive neurons.

Total RNA was isolated from the dorsal root ganglia (DRG) of newborn wild type or Ngn1^{-/-} mice (see Ma et al Genes Develop. 13:1717-1728 (1999), Fode et al. Neuron 20:483-494 (1998) and Ma et al. Neuron 20:469-482 (1998)). A suppression PCR based method (Clontech) was then used to enrich for genes expressed in wild type but not Ngn1 mutant DRG. Briefly, cDNA was synthesized from the RNA using Superscript reverse transcriptase (Gibco) with oligo dT primers, and was amplified with the Smart PCR Amplification Kit (Clontech). The amplified wild-type and Ngn1^{-/-} DRG cDNAs were used as tester and driver, respectively, in the PCR-Select subtractive hybridization protocol (Clontech). Differential screening by dot blot analysis identified several clones, which were enriched in cDNA from wild-type DRG compared to that from Ngn1^{-/-} DRG. These clones were analyzed further by nucleotide sequencing and in situ hybridization.

Approximately 1,600 positives were identified in the primary screen, and of these 142 were sequenced. Fifty of these represented known genes, and 92 represented new genes (see Table 2). Among the known genes were several signaling molecules specifically expressed in nociceptive sensory neurons. These included VR-1, calcitonin gene-related peptide (CGRP), the tetrodotoxin-insensitive sodium channel (SNS-TTXi) and diacylglycerol kinase. Among the new genes were several encoding proteins with structural features characteristic of ion channels or receptors, which were revealed by in situ hybridization to be specifically expressed in a subset of DRG sensory neurons. These molecules are described in more detail in Examples 2 and 3.

**Table 2.**

| Summary of results of the differential hybridization screening for genes involved in pain sensation. | |
|---|---|
| # of times isolated from the screen | Name |
| A. Known genes: | |
| 13 | NaN |
| 9 | Diacylglycerol kinase |
| 7 | Synaptophysin lia |
| 5 | Vanillinoid receptor1 |
| 3 | GluR5-2c |
| 2 | CGRP |
| 2 | CLIM1 |
| 1 | SNS-TTXi |
| 1 | Alpha N-catenin I |
| 1 | Brain Na channel III |
| 1 | NICA6 |
| 1 | Secretogranin |

| B. Novel genes: | |
|---|---|
| 2 | Mrg3 (a novel G-protein-coupled receptor) |
| 2 | DRG12 |
| Note: Previous studies have shown that the genes with bolded letters are expressed specifically in nociceptors. | |

### Example 2: A novel family of putative G protein-coupled receptors specifically expressed in nociceptive sensory neurons.

Among the novel genes isolated from the screen were two independent clones encoding a receptor protein with 7 transmembrane segments (SEQ ID NO: 1), a characteristic of G protein-coupled receptors. The novel 7 transmembrane receptor isolated is most closely related to the oncogene mas, and therefore has been named mas-related gene-3 (mrg3). mrg3 is also known as mas-related gene A1, or MrgA1. A complete coding sequence for mrg3 has been deduced from the genomic DNA sequence (Fig. 1A and SEQ ID NO: 2). MrgA1 shows significant homology (35% identity) to MAS1 (Young et al. Cell 45: 711-9 (1986)). It also shares significant homology (30-35% identity) with two other mammalian GPCRs, called Mas-related gene 1 (MRG1) (Monnot et al. Mol Endocrinol 5: 1477-87 (1991)) and rat thoracic aorta (RTA) (Ross et al. Proc Natl Acad Sci U S A 87: 3052-6 (1990)).

Such G protein-coupled receptors are expressed in other classes of sensory neurons, such as olfactory and gustatory neurons, but molecules in this class had not previously been described in DRG sensory neurons, with the exception of the Protease-Activated Receptors (PARs).

Further screening of mouse DRG cDNA library and mouse genomic library by using mrg3 DNA as a probe has identified nine additional closely related genes named mrg4 (MrgA2), mrg5 (MrgA3), mrg6, mrg7, mrg8 (MrgA4), mrg9 (MrgA5), mrg10 (MrgA6), mrg11 (MrgA7), and mrg12 (MrgA8). Among them, mrg4, 5 and mrg 8-12 contain full-length open reading frames (see Fig. 1). Two human homologues were found by searching databases using the blast program. The protein alignment of the eight mrg genes, mrg3-8 and human1-2, suggested that they define a novel G protein-coupled receptor gene family (Figure 1A).

In particular MrgA1-4 were isolated from a P0 mouse DRG cDNA library and clones containing the entire ORFs of MRGsA5-8 were isolated from a mouse genomic BAC library arrayed on filters (Incyte Genomics). Figure 6A shows an alignment of the polypeptide sequence of MrgA1-8 and indicates the transmembrane domains as well as the cytoplasmic and extracellular loops. In addition, other mouse MrgAs, as well as other human Mrg sequences, were identified by searching the Celera mouse and human (Venter et al. Science 291: 1304-51 (2001)) genomic databases, using the TBLASTN program with MrgA1 as the query. Table 3 shows that the MrgA genes are highly homologous to each other. This high degree of homology combined with the presence of certain characteristic conserved residues indicates that they define a novel subfamily of the MAS family of GPCRs.

To identify additional members of the mouse Mrg family, TBLASTN searches were run against the Celera mouse fragment database (indexed January 7, 2001; 18,251,375 fragments) using MRGA1 and MRGA4 protein sequences as queries. These searches identified 299 unique mouse genomic DNA fragments. The sequences of these fragments were downloaded and assembled into contigs with GELMERGE (GCG Wisconsin Package) under stringent conditions (90% identity, 20 nt minimum overlap). GELMERGE was run again (80% identity, 20 nt minimum overlap) to reduce the dataset further. The consensus nucleotide sequence from each contig was then queried against the Celera mouse fragment database with BLASTN to identify additional sequences for assembly (final n=536 fragments). The consensus sequences from the final assembly were placed into a FASTA formatted database. This database was then searched with TFASTY using MRGA1 as query to identify the potential coding regions from each consensus sequence, regardless of whether the error-prone genomic sequence introduced stop codons or frameshifts into the proteins (Pearson, W. R. (1999). Flexible similarity searching with the FASTA3 program package. In Bioinformatics Methods and Protocols, S. Misener and S. A. Krawetz, eds. (Totowa, NJ: Humana Press), pp. 185-219). The protein sequences from these searches were then combined into a single FASTA formatted file for phylogenetic analysis.

Using this analysis, 16 additional members of the murine MrgA subfamily were identified (Figure 6B). In addition to this subfamily, two closely related Mrg subfamilies called MrgB and MrgC, were also discovered (Figure 6B). To confirm the existence of an ORF in the mouse MrgB genes, high-fidelity PCR was used to amplify mMrgB1-5, mMrgD, and mMrgE from C57BI/6 mouse genomic DNA. Several independent clones were sequenced and confirmed the ORF predictions. The presence of numerous stop codons and frame shifts in the assembled Celera sequence indicated that the mMrgC genes are pseudogenes.

The MrgB subfamily contains 14 genes, whereas MrgC has 12 members. The percent sequence identity within each of these subfamilies is greater than 50% (Table 3). Strikingly, all 12 MrgC members appear to be pseudogenes (Fig. 1 B, " "), as they contain multiple premature stop codons, frameshift mutations or both. Together, therefore, the MrgA and MrgB subfamilies comprise 36 intact ORFs.

**Table 3.**

| Similarity and identity between murine MRG subfamilies | | | | | | | | | |
|---|---|---|---|---|---|---|---|---|---|
| | mMR GA1 | mMR GA2 | mMRG A3 | mMRG B1 | MMRG B2 | mMR GB3 | mMR GC1 | mMR GC2 | mMR GC3 |
| mMRGA1 | ..... | 77.9 | 73.1 | 48.1 | 46.3 | 43.6 | 44.9 | 46.7 | 47.8 |
| mMRGA2 | 87.5 | ..... | 71.8 | 42.4 | 45.4 | 42.7 | 41.5 | 44.5 | 43.5 |
| mMRGA3 | 85.1 | 83.1 | ..... | 47.9 | 46.8 | 44.2 | 46.0 | 49.8 | 46.6 |
| mMRGB1 | 72.1 | 66.8 | 70.2 | ..... | 57.6 | 50.0 | 42.9 | 47.1 | 45.3 |
| mMRGB2 | 68.7 | 67.7 | 69.4 | 72.7 | ..... | 53.5 | 41.8 | 44.4 | 43.1 |
| mMRGB3 | 65.2 | 65.7 | 64.6 | 69.5 | 73.5 | ..... | 37.0 | 38.8 | 36.4 |
| mMRGC1 | 69.5 | 65.2 | 70.9 | 64.4 | 67.0 | 63.3 | ..... | 76.0 | 79.1 |
| mMRGC2 | 69.8 | 72.5 | 74.2 | 69.4 | 70.8 | 65.7 | 81.4 | ..... | 78.8 |
| mMRGC3 | 70.9 | 67.2 | 71.0 | 66.2 | 69.5 | 64.6 | 86.1 | 86.3 | ..... |

Percent identity (top-right, bold) and percent similarity (bottom-left) between the protein sequences are indicated. "hMRG" indicates a human MRG amino acid sequence; "mMRG" indicates a murine MRG sequence. "hMRGX" is used to indicate a human homolog of mMRGA and mMRGB sequences (Fig. 1B). Values were derived from global alignments using the GAP program in the GCG package.

Searches of the Celera (Venter et al. Science 291: 1304-51 (2001)) and public (Consortium. Nature 409: 860-921 (2001)) genomic sequence databases, using both BLAST (Altschul et al. Journal of Molecular Biology 215: 403-410 (1990)) and Hidden Markov Models (HMMs (Eddy. Bioinformatics 14, 755-63 (1998)), revealed 4 closely related (~50% identity) full-length human genes, and at least 10 human pseudogenes. Briefly, TBLASTN searches were run against the Celera human genome database (Venter et al. Science 291: 1304-51 (2001)) using the mMrgA1 protein sequence as the query. The genomic sequences that were identified in this search were downloaded, placed into a FASTA formatted database and searched with TFASTY to identify a non-redundant set of proteins. With the exception of hMrgX3, hMrgE, and hMrgΨ8, all human Mrgs were independently identified from a similar analysis of the public human genome sequence (Consortium. Nature 409: 860-921 (2001)). Human MrgX1-4 sequences were independently verified from PCR-amplified products derived from human BAC clones containing the genes.

Although the human genes appear to be more similar to the murine MrgA subfamily than the MrgB subfamily in the phylogenetic tree (Fig. 6B, hMrgX1-4), in the absence of clear orthologous pairs we currently refer to them as hMrgX genes. In addition to the MrgA, B and C subfamilies, a number of additional Mas1-related orphan GPCRs were identified by this search, including those we refer to as Mrgs D-F (Fig. 6B). Several of these sequences, such as MrgD, have clear human orthologs (Fig. 6B, hMrgD and Table 4). All together, we identified almost 45 murine and 9 human intact coding sequences belonging to this family.

**Table 4.**

| Similarity and identity between human and murine MRGs | | | | | | | | |
|---|---|---|---|---|---|---|---|---|
| | hMRGX 2 | hMRGD | hMRGE | mMRG A1 | MMRG B4 | mMRG B1 | mMRG D | mMRG E |
| hMRGX2 | ----- | 39.3 | 40.2 | 55.6 | 50.1 | 53.4 | 40.5 | 38.8 |
| hMRGD | 65.4 | ----- | 34.4 | 37.6 | 35.4 | 33.8 | 55.8 | 35.9 |
| hMRGE | 62.8 | 54.6 | ----- | 36.6 | 32.8 | 32.8 | 33.9 | 76.5 |
| mMRGA1 | 74.8 | 63.4 | 57.7 | ----- | 48.1 | 48.1 | 37.1 | 39.7 |
| mMRGB4 | 71.0 | 64.0 | 58.0 | 70.4 | ----- | 54.5 | 34.8 | 36.6 |
| mMRGB1 | 73.5 | 58.6 | 60.5 | 72.1 | 74.1 | ----- | 36.5 | 33.8 |
| mMRGD | 61.1 | 72.6 | 57.6 | 59.5 | 64.2 | 61.3 | ----- | 35.1 |
| mMRGE | 59.0 | 59.5 | 84.0 | 62.5 | 63.7 | 59.1 | 59.3 | ----- |

Percent identity (top-right, bold) and percent similarity (bottom-left) between the protein sequences are indicated. "hMRG" indicates a human MRG amino acid sequence; "mMRG" indicates a murine MRG sequence. "hMRGX" is used to indicate a human homolog of mMRGA and mMRGB sequences (Fig. 1B). Values were derived from global alignments using the GAP program in the GCG package.

MRG receptors have short (3-21 amino acid) N-termini with no apparent signal peptide, which are predicted to be located extracellularly. The transmembrane domains and intracellular domains are highly conserved suggesting that the receptors have a shared function. The most divergent regions of MRGA-family receptors appear localized to the extracellular loops (Fig. 6A), suggesting that these receptors recognize different ligands, or the same ligand but with different affinities. Interestingly, we identified 12 single nucleotide polymorphisms in the MrgA1 coding sequence between murine strains C57BL/6J and 129SvJ. These 12 changes resulted in 6 amino acid substitutions, all of which were either conservative, or which substituted residues expressed at the same position by other family members;

A large mouse genomic contig was built by analyzing overlapping BAC clones containing MrgA sequences (Fig. 6C). There are 7 MrgA genes, including 3 pseudogenes, residing in this contig. Such clustering is a common feature of GPCR-encoding gene families (Xie et al. Mamm Genome 11: 1070-8 (2000)). Strikingly, all of the human Mrg genes (with the exception of Mas1 and Mrg1) are located on chromosome 11, which also contains 50% of all human olfactory receptors genes. All of the MrgA genes in the murine BAC contig (Fig. 6C) encode intact ORFs with N-terminal methionines, like many other GPCR-encoding genes. Using the Celera mouse genome database, sequences flanking each MrgA coding region were obtained and analyzed. This analysis revealed that at least six MrgA genes have L1 retrotransposon sequences located ^{~}650 bp downstream of their coding sequences (Fig. 6B, indicated by "L1").

All of the eight full-length mas-related genes, mrg3-5 and mrg8-12, are enriched in nociceptive sensory neurons as indicated by their expression in a subset of DRG sensory neurons which are eliminated in ngn1^{-/-} mutant DRG (Fig 2 and 2A).

### Example 3: A novel two-transmembrane segment protein specifically expressed in nociceptive sensory neurons.

Another novel gene isolated in this screen, drg12 (SEQ ID NO: 13), encodes a protein with two putative transmembrane segments (SEQ ID NO: 14). In situ hybridization indicates that, like the mrg genes, this gene is also specifically expressed in a subset of DRG sensory neurons. Although there are no obvious homologies between this protein and other sequences in the database, it is noteworthy that two purinergic receptors specifically expressed in nociceptive sensory neurons (P₂X₂ and P₂X₃) have a similar bipartite transmembrane topology. Therefore it is likely that drg12 also encodes a receptor or ion channel involved in nociceptive sensory transduction or its modulation. The hydrophobicity of a homologous region of a drg12 human sequence (SEQ ID NO: 19) is compared with the hydrophobicity of mouse drg 12 in Fig. 4.

### Example 4: mrg and drg-12 genes are specifically expressed in nociceptive sensory neurons.

The prediction of function for mrg-family and drg-12 genes is based on their structure and expression pattern, taken together with the identification of ligands as described below. To determine whether Mrg proteins are expressed in DRG neurons, in situ hybridization using dioxygenin-labeled riboprobes was performed. Briefly, tissue was obtained from P0 mouse pups and fixed in 4% paraformaldehyde overnight at 4°C, cryoprotected in 30% sucrose overnight and embedded in OCT. Tissue sections were cut transversely on a cryostat at 18 µm. Non-isotopic in situ hybridization on frozen sections was performed as previously described using cRNA probes (Ma et al. Cell 87: 43-52 (1996); Perez et al. Development 126: 1715-1728 (1999)). Eight MrgAs, 5 MrgBs and MrgD were used as probes. At least 10 DRGs were analyzed to count the number of neurons positive for each probe.

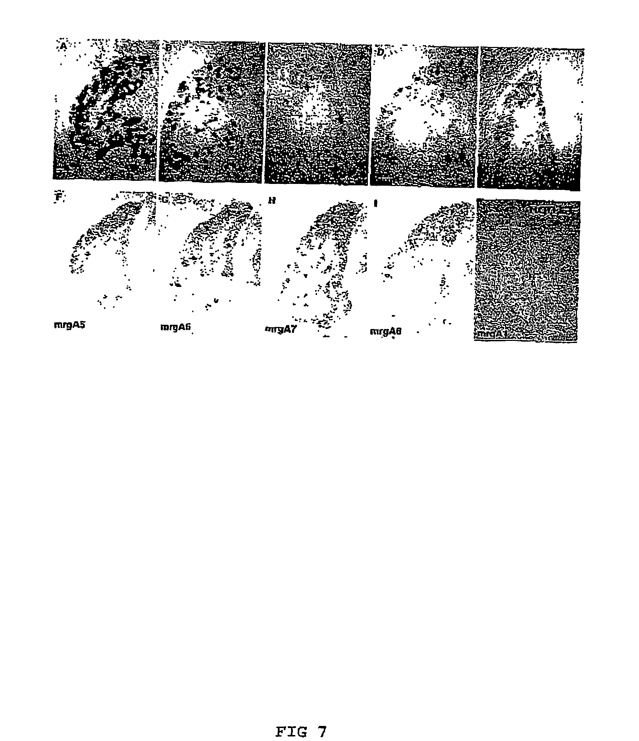

Mrg and drg12 genes, including all eight MrgAs (MrgA1-8), are expressed in subsets of small-diameter sensory neurons in the dorsal root ganglia (DRG) of the mouse (Fig. 7B-I). Importantly, the expression of all eight MrgAs was virtually absent in the DRGs of Ngn1^{-/-} animals (Figure 7J), consistent with the design of the substractive hybridization screen. Among the eight MrgA clones examined, MrgA1 has the widest expression within sensory neurons in DRGs (13.5%). Other MrgAs are only expressed in several cells per DRG section (ranging from 0.2-1.5% of DRG neurons). This differential abundance may explain why only MrgA1 was isolated in the original screen. No obvious differences in the expression patterns of MrgA1-8 were noticed in DRGs from different axial levels. This expression is highly specific, in that expression of these genes has thus far not been detected in any other tissue of the body or in any other region of the nervous system thus far examined.

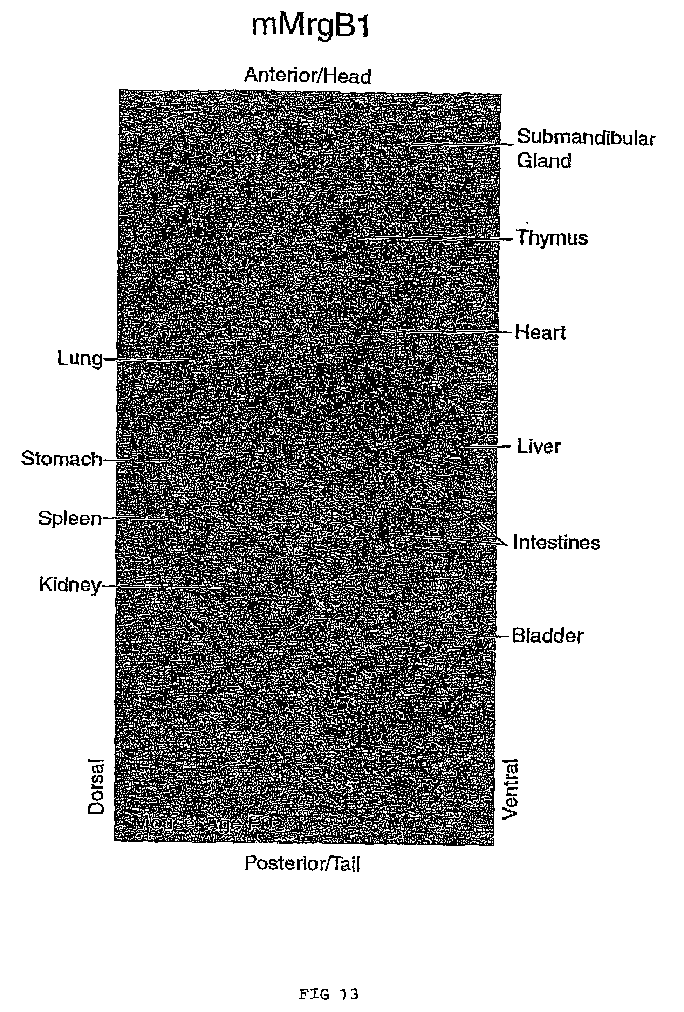

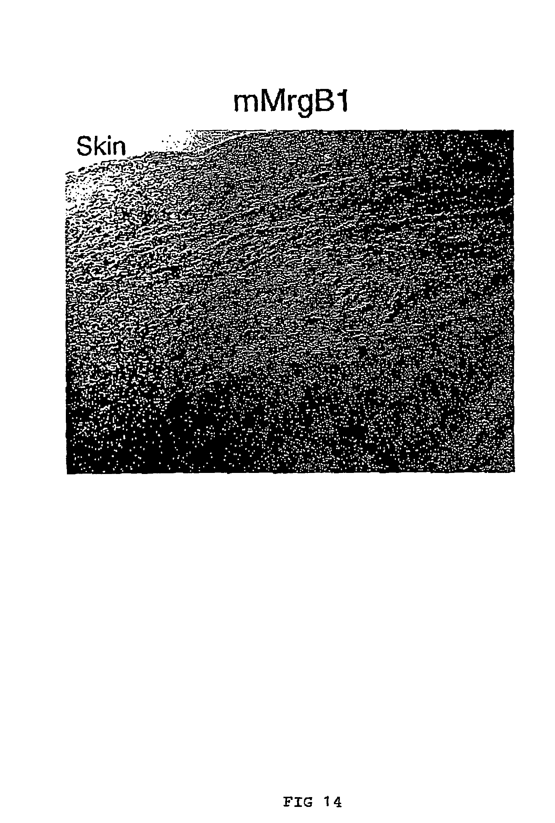

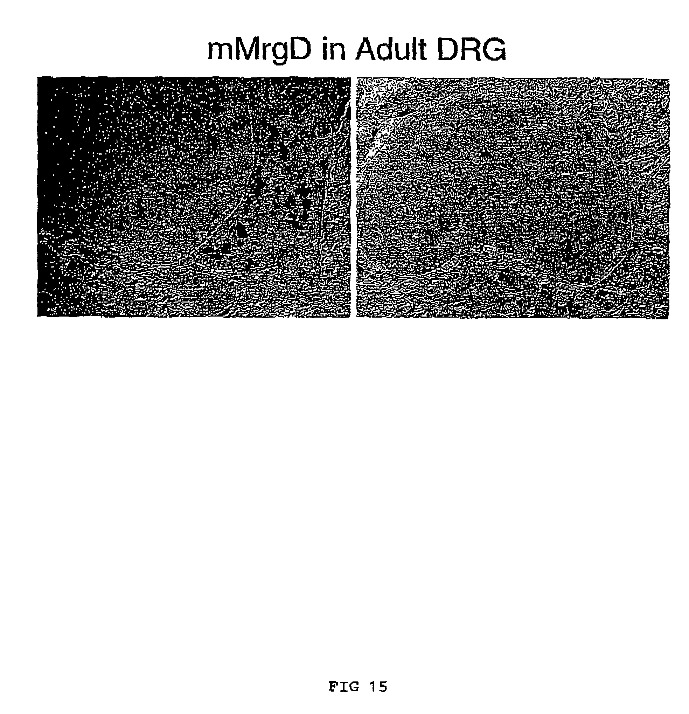

Like the MrgA genes, MrgD was also specifically expressed in a subset of DRG sensory neurons (see below, Figure 15). In contrast, MrgB1-5 were not detectably expressed in DRGs. However, mMrgB1 expression has been observed in scattered cells in the epidermal layer of skin in newborn mice, as well as in the spleen and the submandibular gland (Figures 13 and 14). These cells appear to be immune cells that play a role in wound repair. mMrgB2 also shows this expression pattern. In contrast, mMrgB3, mMrgB4 and mMrgB5 do not appear to be expressed in any of these tissues.

These results indicate that Mrg and drg12 genes are expressed in primary sensory neurons. However, DRG contain different classes of neurons subserving different types of sensation: e.g., heat, pain, touch and body position. Independent identification is provided by the fact that the neurons that express the mrg-family and drg12 genes are largely or completely eliminated in Ngn1^{-/-} DRG (Figure 2), because the Ngn1 mutation is independently known to largely or completely eliminate the nociceptive (noxious stimuli-sensing) subset of DRG neurons, identified by expression of the independent markers trkA, VR-1 and SNS-TTXi (Ma et. al. Genes & Dev. 13: 1717-1728 (1999)). The loss of mrg- and drg12- expressing neurons in Ngn1^{-/-} mutant DRG therefore indicates that these genes are very likely expressed in nociceptive sensory neurons. Although small numbers of sensory neurons of other classes (trkB⁺ and trkC⁺) are eliminated in the Ngn1^{-/-} mutant as well, mrg and drg12 genes are unlikely to be expressed in these classes of sensory neurons, because if they were then the majority of mrg- and drg12-expressing sensory neurons would be predicted to be spared in the Ngn1^{-/-} mutant, and that is not the case.

The lack of expression of MrgAs in DRGs from Ngn1^{-/-} mice is consistent with the idea that they are expressed in cutaneous sensory neurons. Furthermore, the distribution of MrgA1⁺ cells was similar to that of neurons expressing trkA, a marker of nociceptive sensory neurons (McMahon et al. Neuron 12: 1161-71 (1994); Snider and Silos-Santiago Philos Trans R Soc Lond B Biol Sci 351: 395-403 (1996)) (Fig. 7A, B). To directly determine whether MrgA genes are expressed in trkA⁺ cells, in situ hybridization was performed for MrgA1, A3 and A4 in conjunction with immunolabeling using anti-trkA antibodies, on neonatal DRG. Fluorescein-UTP-labeled cRNA probes were detected with alkaline phospatase- (AP-) conjugated anti-fluorescein antibody (1:2000, Roche) and developed with Fast Red (Roche) to generate a red fluorescent signal. After the fluorescent in situ hybridization was performed, sections were incubated in primary antibodies against TrkA (1:5000, gift from Dr. Louis Reichardt), VR1 (1:5000, gift from Dr. D. Julius), CGRP (1:500, Chemicon), or SubstanceP (1:1000, Diasorin). All antibodies were diluted in 1x PBS containing 1% normal goat serum and 0.1% TritonX-100. Primary antibody incubations were carried out overnight at 4 °C. Secondary antibodies used were goat-anti-rabbit-IgG conjugated to Alexa 488 (1:250, Molecular Probes). For double-labeling with Griffonia simplicifolia IB4 lectin, sections were incubated with 12.5 µg/ml FITC-conjugated IB4 lectin (Sigma) following in situ hybridization.

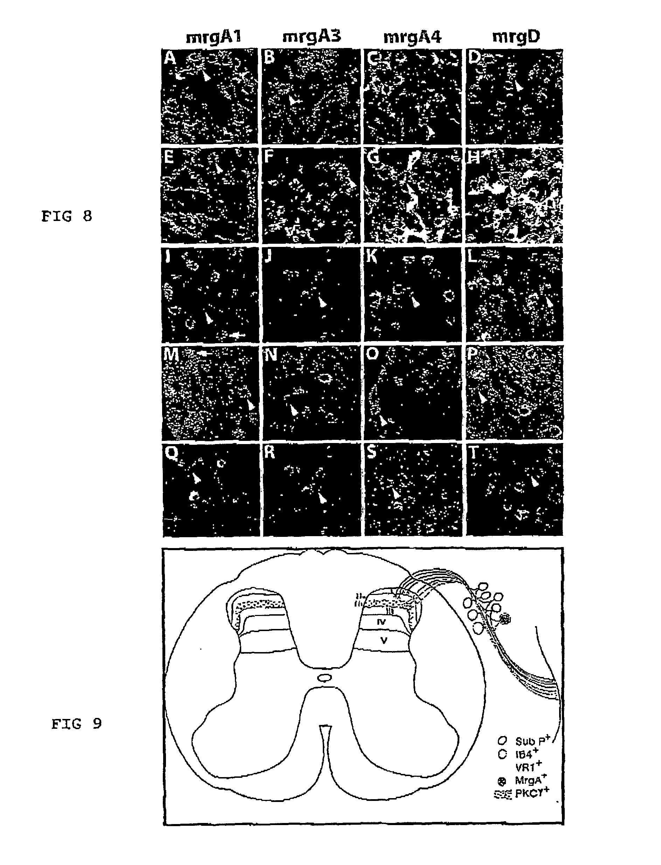

Double labeling experiment using mrgs antisense RNA probes with anti-trkA antibodies confirmed that mrgs, specifically MrgAs, are co-expressed by trkA+ nociceptive neurons in DRG (see Fig. 7B and Fig. 8A-C). Similar results were obtained for MrgD (Fig. 8D). Taken together, these data indicate that MrgAs and MrgD are specifically expressed by nociceptive sensory neurons in DRG.

Further experiments were carried out to determine whether Mrgs are expressed in particular subsets of nociceptors. Additional double labeling experiments using mrgs antisense RNA probes with anit-VR1 and isolectin B4 (IB4)-labeling, as described above, have shown that mrgs are preferentially expressed by IB4+ nociceptive neurons but not VR1-expressing nociceptive neurons (Fig. 2C and 2D). In particular, combined fluorescent labeling for IB4 together with in situ hybridization with MrgA1, A3, A4 and MrgD probes clearly showed that these receptors are expressed by IB4⁺ neurons (Fig. 8E-H), and may be restricted to this subset. This result indicates that these Mrgs are expressed by non-peptidergic nociceptive neurons that project to lamina IIi (Snider and McMahon Neuron 20: 629-32 (1998)). Consistent with this assignment, the majority (90%) of MrgA1⁺, and all MrgA3⁺, A4⁺ and MrgD⁺ cells, lack substance P expression (Fig. 8I-L). Similarly, the majority (70%) of MrgA1⁺, and all MrgA3⁺, A4⁺ and MrgD⁺ cells, do not express CGRP (Fig. 8M-P), another neuropeptide expressed by C-fiber nociceptors. Previous studies had shown that IB4+ nociceptive neurons were involved in neuropathic pain resulting from nerve injury (Malmberg, A. B. et al. Science 278: 279-83 (1997)). Neuropathic pain including postherpetic neuralgia, reflex sympathetic dystrophy, and phantom limb pain is the most difficult pain to be managed. Mrgs may play essential roles in mediating neuropathic pain and may provide alternative solutions to manage neuropathic pain.

Recent studies have provided evidence for the existence of two neurachemically and functionally distinct subpopulations of IB4⁺ nociceptors: those that express the vanilloid receptor VR1 (Caterina et al. Science 288: 306-13 (1997)), and those that do not (Michael and Priestley J Neurosci 19: 1844-54 (1999); Stucky and Lewin J Neurosci 19: 6497-505 (1999)). Strikingly, in situ hybridization with MrgA or D probes combined with anti-VR1 antibody immunostaining indicated that the MrgA1, A3, A4 and D-expressing cell population was mutually exclusive with VR1⁺ cells (Fig. 8Q-T). In summary, these expression data demonstrate that MrgA and D genes are expressed in the subclass of nonpeptidergic cutaneous sensory neurons that are IB4⁺ and VR1 (Fig. 9).

### MrgA1 is co-expressed with other MrgA genes

MrgA1 is more broadly expressed than are the other MrgA genes (Fig. 2), suggesting MrgA1 and MrgA2·8 are expressed by different or overlapping subsets of nociceptors. Double-label in situ hybridization studies using probes labeled with digoxigenin and fluorescein indicated that most or all neurons expressing MrgA3 or MrgA4 co-express MrgA1 (Fig. 10A-F). Interestingly, the fluorescent in situ hybridization signals for MrgA3 and A4 using tyramide amplification often appeared as dots within nuclei that were circumscribed by the cytoplasmic expression of MrgA1 mRNA, detected by Fast Red (Fig. 10F). Such dots were not observed using the less-sensitive Fast Red detection method, and were only observed in the nuclei of MrgA1⁺ cells. Similar intranuclear dots have previously been observed in studies of pheromone receptor gene expression, and have been suggested to represent sites of transcription (Pantages and Dulac Neuron 28: 835-845 (2000)). The results for MrgA1, 3 and 4 indicate that those neurons that express the rarer MrgA genes (MrgA2-8) are a subset of those that express MrgA1.

To address the question of whether MrgsA2-A8 are expressed in the same or in different neurons, the number of neurons labeled by single probes was compared to that labeled by a mixture of all 7 probes (Buck and Axel Cell 65: 175-187 (1991)). Approximately 3-fold more neurons (4.5% vs. 1%) were labeled by the mixed probe than by an individual probe to MrgA4 (Fig. 10J, K), indicating that these genes are not all co-expressed in the same population of neurons. However, the percentage of neurons labeled by the mixed probe (4.5%) was less than the sum of the percentage of neurons labeled by each of the 7 individual probes (6.6%), indicating that there is some overlap in the expression of MrgA2-A8. In additon, higher signal intensity was observed in individual neurons using the mixed probe, than using a single probe.

Double-labeling experiments with MrgA1 and MrgD probes were also performed. These proteins share only 60% sequence similarity, as shown in Fig. 6B and Table 3. The results of these experiments indicated only partial overlap between neurons expressing these two receptors (Fig. 10G-I). Approximately 15% (118/786) of neurons expressing either MrgA1 or MrgD co-expressed both genes. Thirty-four percent (118/344) of MrgA1⁺ cells co-expressed MrgD, while 26.7% (118/442) of MrgD⁺ cells co-expressed MrgA1.

Taken together, these data indicate the existence of at least three distinct subpopulations of IB4⁺, VR1⁻ sensory neurons: MrgA1⁺MrgD⁺; MrgA1⁺MrgD⁻ and MrgA1⁻MrgD⁺. The MrgA1⁺ subset is further subdivided into different subsets expressing one or more of the MrgsA2-A8.

### Mrg-family genes encode putative G-protein coupled receptors (GPCRs).

Hydrophobicity plots of the encoded amino acid sequences of the mrg-family genes predicts membrane proteins with 7 transmembrane segments. Such a structure is characteristic of receptors that signal through "G-proteins." G proteins are a family of cytoplasmic molecules that activate or inhibit enzymes involved in the generation or degradation of "second messenger" molecules, such as cyclic nucleotides (cAMP, cGMP), IP₃ and intracellular free calcium (Ca⁺⁺). Such second messenger molecules then activate or inhibit other molecules involved in intercellular signaling, such as ion channels and other receptors.

G protein-coupled receptors (GPCRs) constitute one of the largest super-families of membrane receptors, and contain many subfamilies of receptors specific for different ligands. These ligands include neurotransmitters and neuropeptides manufactured by the body (e.g., noradrenaline, adrenaline, dopamine; and substance P, somatostatin, respectively), as well as sensory molecules present in the external world (odorants, tastants).

Although the mrg-family genes are highly homologous, the most divergent regions were the extracellular domains (see Figure 6A). The variability of the extracellular domains of mrg family suggests that they may recognize different ligands.

The fact that the mrg-family genes encode GPCRs, and are specifically expressed in nociceptive sensory neurons, suggest that these receptors are involved, directly or indirectly, in the sensation or modulation of pain, heat or other noxious stimuli. Therefore the mrg-encoded receptors are useful as targets for identifying drugs that effect the sensation or modulation of pain, heat or other noxious stimuli. The nature of the most useful type of drug (agonistic or antagonistic) will reflect the nature of the normal influence of these receptors on the sensation of such noxious stimuli. For example, if mrg-encoded receptors normally act negatively, to inhibit or suppress pain, then agonistic drugs would provide useful therapeutics; conversely, if the receptors normally act positively, to promote or enhance pain, then antagonistic drugs would provide useful therapeutics. There might even be certain clinical settings in which it would be useful to enhance sensitivity to noxious stimuli, for example in peripheral sensory neuropathies associated with diabetes.

The nature of the influence of mrg-encoded GPCRs on pain sensation may be revealed by the phenotypic consequences of targeted mutation of these genes in mice. For example, if such mice displayed enhanced sensitivity to noxious stimuli, then it could be concluded that the receptors normally function to inhibit or suppress pain responses, and vice-versa. Alternatively, high-throughput screens may be used to identify small molecules that bind tightly to the mrg-encoded receptors. Such molecules would be expected to fall into two categories: agonists and antagonists. Agonists would be identified by their ability to activate intracellular second messenger pathways in a receptor-dependent manner, while antagonists would inhibit them. Testing of such drugs in animal models of pain sensitivity will then reveal further information concerning the function of the GPCRs: for example, if the molecules behave as receptor antagonists in vitro, and they suppress sensitivity or responsiveness to noxious stimuli in vivo, then it may be concluded that the receptor normally functions to promote or enhance pain sensation. Conversely, if receptor agonists suppress, while antagonists enhance, pain sensation in vivo, then it may be concluded that the receptor normally functions to suppress or inhibit pain sensation.

### drg12 encodes a putative transmembrane signaling molecule

Hydrophobicity plots of the encoded amino acid sequence of the drg12 gene predicts a membrane protein with 2 transmembrane segments. The membrane localization of this protein has been verified by immuno-staining of cultured cells transfected with an epitope-tagged version of the polypeptide. Although the DRG12 amino acid sequence has no homology to known families of proteins, its bipartite transmembrane structure strongly suggests that it is involved in some aspect of intercellular signaling, for example as a receptor, ion channel or modulator of another receptor or ion channel. This prediction is supported by the precedent that two known receptors with a similar bipartite transmembrane topology, the purinergic P₂X₂ and P₂X₃ receptors, are like DRG12, specifically expressed in nociceptive sensory neurons.

Based on this structural data, and its specific expression in nociceptive sensory neurons, it is probable that DRG12 is involved, directly or indirectly, in the sensation or modulation of noxious stimuli. Accordingly, the drg12-encoded protein is a useful target for the development of novel therapeutics for the treatment of pain.

### Example 5: Mrg proteins are receptors for neuropeptides.

As discussed above, the structure of the proteins encoded by Mrg genes indicates that they function as receptors. To identify ligands for the Mrg receptors, selected MrgA genes were tested in a calcium release assay. MrgA genes, including MrgA1 and MrgA4, were cloned into a eukaryotic expression vector and transfected into human embryonic kidney (HEK) 293 cells. HEK-293 cells were obtained from the ATCC and cultured in DMEM supplemented with 10% fetal bovine serum. An HEK293- Gα₁₅ cell line stably expressing Gα₁₅ was provided by Aurora Biosciences Corporation and grown on Matrigel™ (growth factor reduced Matrigel, Becton Dickinson, diluted 1 : 200 with serum-free DMEM)-coated flasks and maintained at 37 °C in DMEM (GibcoBRL) supplemented with 10% heat-inactivated fetal bovine serum, 2 mM L-glutamine, 0.1 mM non-essential amino acids, 1 mM sodium pyruvate ,25 mM HEPES and 3 µg/ml blastcidin-S. For transfection, cells were seeded on Matrigel-coated 35 mm glass-bottom dishes (Bioptech Inc., Butler, PA). After 16-24 hr, cells were transfected using FuGENE 6 (Roche). Transfection efficiencies were estimated by visualization of GFP fused to the C-terminus of MrgA1 and A4, and were typically > 60 %. Fusing GFP to the C-termini of the MrgA coding sequences additionally allowed for visual confirmation of the intracellular distribution of the receptors and their membrane integration in the transfected cells (Fig. 11D).

To increase the sensitivity of the calcium release assay, in some experiments the MRGA-GFP fusion proteins were expressed in HEK 293 cells modified to express G ₁₅, which couples GPCRs to a signal transduction pathway leading to the release of intracellular free Ca²⁺ (Offermann and Simon J Biol Chem 270: 15175-80 (1995)). This calcium release can be monitored ratiometrically using Fura-2 as a fluorescent indicator dye (Tsien et al. Cell Calcium 6: 145-57 (1985)) (Fig. 11A-C). This heterologous expression system has been previously used to identify ligands for taste receptors (Chandrashekar et al. Cell 100: 703-11 (2000)).

Because MRGAs exhibit the highest sequence similarity to peptide hormone receptors, approximately 45 candidate peptides were screened for their ability to activate MRGA1, using the intracellular Ca²⁺-release assay. Briefly, transfected cells were washed once in Hank's balanced salt solution with 11 mM D-glucose and 10 mM HEPES, pH 7.4 (assay buffer) and loaded with 2 µM Fura-2 AM (Molecular Probes) at room temperature for 90 min, with rotation. Loaded cells were washed twice with assay buffer and placed on a micro-perfusion chamber (Bioptech). The chamber was mounted on top of a Olympus IMT2 inverted microscope, and imaged with an Olympus DPlanApo 40X oil immersion objective lens. Samples were illuminated by a 75W xenon bulb, and a computer-controlled filter changer (Lambda-10; Shutter Instruments) was used to switch the excitation wavelength. A cooled CCD camera (Photometric) was used in detecting fluorescence. GFP-positive cells within a field were identified using an excitation wavelength of 400 nm, a dichroic 505 nm long-pass filter and an emitter bandpass of 535 nm (Chroma Technology). In the same field, calcium measurements were performed at an excitation wavelength of 340 nm and 380 nm, and an emission wavelength of 510 nm. Agonists were diluted in assay buffer and solution changes accomplished by micro-perfusion pump (Bioptech). Fura-2 fluorescence signals (340 nm, 380 nm and the 340/380 ratio) originating from GFP-positive cells were continuously monitored at 0.4- or 1-second intervals and collected using Axon Imaging Workbench 4.0 software (Axon). Instrument calibration was carried out with standard calcium solutions (Molecular probes) in glass bottom dishes (MatTek Corp.).

At a concentration of 1 µM, numerous neuropeptides produced some level of activation of MrgA1-expressing cells (Fig. 12A). These included ACTH, CGRP-I and -II, NPY and somatostatin (SST). Nevertheless, many other peptide hormones did not activate MRGA1, including angiotensins I-III and neurokinins A and B, alpha-MSH and gamma2-MSH (Fig. 12A and data not shown). MrgA1 was only very weakly activated by ecosanoid ligands such as Prostaglandin-E1 and Arachidonic Acid (data not shown).

The most efficient responses in MrgA1-expressing HEK cells were elicited by RFamide peptides, including FLRF and the molluscan cardioactive neuropeptide FMRFamide (Price and Greenberg Science 197: 670-671 (1977)) (Phe-Met-Arg-Phe-amide) (Fig. 11C, 12A). Two mammalian RFamide peptides, NPAF and NPFF, which are cleaved from a common pro-peptide precursor (Vilim et al. Mol Pharmacol 55: 804-11 (1999)) were then tested. The response of MrgA1-expressing cells to NPFF at 1 µM was similar to that seen with FMRFamide, while that to NPAF was significantly lower (Fig. 12A). MrgA1 was also weakly activated by two other RFamide ligands, ₁-MSH and schistoFLRF (data not shown).

In order to examine further the specificity of activation of MrgA1 and A4, the top candidate ligands emerging from the intial screen were tested on these same receptors expressed in HEK cells lacking G ₁₅. MrgA1 and A4 expressed in this system retained responses to RFamide peptides (Fig. 12B, C), demonstrating that the intracellular Ca²⁺ release responses seen in the initial screen are not dependent on the presence of exogenous G ₁₅. This indicates that MrgAs act in HEK cells via Gq or Gi. The response of MrgA1-expressing HEK cells to NPFF was lower than that to FLRF (Fig. 12B), and there was no response to NPAF. Conversely, MrgA4-expressing cells responded to NPAF, but not to NPFF or FLRF (Fig. 12C). In both cases, the response to NPY seen in G ₁₅-expressing cells (Fig. 11A) was lost completely, while those to CGRP-II and ACTH were considerably diminished.

In order to determine the lowest concentrations of RFamide ligands capable of activating MrgA1 and A4, dose-response experiments were carried out in HEK cells expressing G ₁₅, which afforded greater sensitivity (Fig. 12D, E). These experiments indicated that MrgA1 could be activated by FLRF at nanomolar concentrations (Fig. 12D; EC₅₀ 20 nM), and by NPFF at about an order of magnitude higher concentration (Fig. 12D; EC₅₀ 200 nM), whereas NPAF was much less effective. In contrast, MrgA4 was well activated by NPAF (Fig. 12E; EC₅₀ 60 nM), and much more weakly activated by FLRF and NPFF. Neither receptor showed strong activation in response to RFRP-1, -2 or -3, a series of RFamide ligands produced from a different precursor (Hinuma et al. Nat Cell Biol 2: 703-8 (2000)). These data confirm that MrgA1 and MrgA4 display different selectivities towards different RFamide ligands in this system. By contrast, these receptors responded similarly to ACTH (EC₅₀ ^{~}60- and 200 nM for MrgA1 and A4, respectively; data not shown).

Finally, given the sequence similarity between MRGA receptors and MAS1, the responsiveness of cells expressing.exogenous Mas1 to NPFF, NPAF and FLRF was tested. MAS1 showed a profile distinct from both MrgA1 and MrgA4 (Fig. 12F): like MrgA1, it was activated by NPFF at a similar concentration of the peptide (EC₅₀ 400 nM), but unlike MrgA1 it was poorly activated by FLRF. In contrast to MrgA4, MAS1 did not respond well to NPAF. No response was detected in MAS1-expressing cells upon exposure to Angiotensins I and II, ligands which have been previously reported to activate this receptor (Jackson, T. R., et al. Nature 335: 437-40 (1988)). Nor did MAS1 respond to ACTH. Thus, MAS1, MrgA1 and MrgA4 expressed in this heterologous system are all activated by RFamide family ligands, but with differing ligand-sensitivities and -selectivities (Table 4).

**Table 4.**

| Selectivity of activation of Mas-related GPCRs by RF-amide ligands in HEK cells | | | |
|---|---|---|---|
| | A. Ligand | | |
| receptor | FLRF | NPFF | NPAF |
| MRGA1 | +++ | ++ | +/- |
| MRGA4 | +/- | +/- | +++ |
| MAS1 | +/- | ++ | +/- |

Relative efficacy of activation of the indicated receptors by the indicated ligands is shown. For quantification, see Fig. 6. "+++" indicates 10 nM < EC₅₀ < 100 nM; "++" indicates 100 nM < EC₅₀ < 500 nM; "+/-" indicates weak response seen at 1 µM. For details see Fig. 6.

A novel family consisting of close to 50 MAS1 related g-protein coupled receptors has been identified. The specific expression of several classes of these receptors in a subset of nociceptive sensory neurons indicates that these receptors play a role in the sensation or modulation of pain. Consistently, these receptors have been shown to be activated by RFamide neuropeptides, which are known to mediate analgesia. As a result, these receptors provide a novel target for anti-nociceptive drugs.

## Claims

1. An isolated nucleic acid molecule, having at least 80 % sequence identity to
(a) a nucleic acid molecule that encodes an Mrg polypeptide comprising the amino acid sequence of SEQ ID No 2 or 12,
and being **characterized by** the ability to be activated by an RF-amide-peptide, or
(b) the complement of the nucleic acid of (a).

2. The isolated nucleic acid of claim 1, having an identity of at least 90 % to the molecules of (a) or (b).

3. The isolated nucleic acid of claim 1, having an identity of at least 95 % to the molecules of (a) or (b).

4. The isolated nucleic acid of claim 1, which is represented by nucleic acids 115 - 1026 of SEQ ID No 1.

5. The isolated nucleic acid of claim 1, which is represented by nucleic acids 1820 - 2734 of SEQ ID No 11.

6. The isolated nucleic acid molecule of claims 1 to 5, operably linked to an expression control element.

7. The isolated nucleic acid molecule of claim 6, operably linked to a promoter element.

8. A vector comprising an isolated nucleic acid molecule of claims 1 to 5.

9. A host cell comprising the vector of claim 8.

10. The host cell of claim 9, wherein said cell is a prokaryotic cell or an eukaryotic cell.

11. The host cell of claim 10, wherein said cell is an E. coli, a hamster embryonic kidney (HEK) cell, or a yeast cell.

12. A method for producing a polypeptide comprising culturing the host cell of claim 9 under conditions in which the protein encoded by said nucleic acid is expressed.

13. An isolated polypeptide produced by the method of claim 12.

14. An isolated polypeptide comprising an amino acid sequence comprising at least 40 % sequence identity to the amino acid sequence of SEQ ID No 2 or 12, and being **characterized by** the ability to be activated by an RF-amide peptide.

15. The isolated polypeptide according to claim 14, comprising at least 50 % sequence identity to the amino acid sequence of SEQ ID No 2 or 12, and being **characterized by** the ability to be activated by an RF-amide peptide.

16. The isolated polypeptide according to claim 14, comprising at least 60 % sequence identity to the amino acid sequence of SEQ ID No 2 or 12, and being **characterized by** the ability to be activated by an RF-amide peptide.

17. The isolated polypeptide according to claim 14, comprising at least 75 % sequence identity to the amino acid sequence of SEQ ID No 2 or 12, and being **characterized by** the ability to be activated by an RF-amide peptide.

18. The isolated polypeptide according to claim 14, comprising at least about 80 % sequence identity to the amino acid sequence of SEQ ID No 2 or 12, and being **characterized by** the ability to be activated by an RF-amide peptide.

19. The isolated polypeptide according to claim 14, which has the amino acid sequence of SEQ ID No 2.

20. The isolated polypeptide according to claim 14, which has the amino acid sequence of SEQ ID No 12.

21. A chimeric molecule comprising a Mrg polypeptide according to any of claims 14 to 20, fused to a heterologous amino acid sequence.

22. The chimeric molecule of claim 21, wherein said heterologous amino acid sequence is an epitope tag sequence or an immunoglobulin constant domain sequence.

23. An isolated antibody that specifically binds to an isolated Mrg polypeptide of any of claims 14 to 20.

24. The isolated antibody of claim 23, wherein said antibody is a monoclonal antibody, an antibody fragment, a humanized antibody, an agonist antibody or a neutralizing antibody.

25. A composition of matter comprising (a) an Mrg polypeptide according to any of claims 14 to 22 or (b) an anti-Mrg antibody according to any of claims 23 to 24 in admixture with a pharmaceutical acceptable carrier.

26. An article of manufacture comprising:
a container; a composition of matter of claim 25; and instructions for using the composition of matter to treat impaired sensory perception.

27. A method of identifying Mrg expression in a sample comprising contacting said sample with an anti Mrg antibody according to claim 23 or 24 and determining binding of said antibody to the sample.

28. An in vitro method of identifying a compound that binds to an Mrg polypeptide comprising the steps of:
1) contacting a test compound with at least a portion of an Mrg polypeptide according to any of claims 14 to 20; and
2) detecting Mrg/test compound complexes.

29. The method of claim 28, wherein at least one of the test compound or the Mrg polypeptide is attached to a solid support.

30. The method of claim 29, wherein said solid support is a microtiter plate.

31. The method of claim 28, wherein said Mrg polypeptide is present in a cell membrane.

32. The method of claim 31, wherein said Mrg polypeptide is present in a fraction of cell membrane prepared from cells expressing an Mrg polypeptide.

33. The method of claim 28, wherein said Mrg polypeptide is present in an immunoadhesin.

34. The method of claim 28, wherein said test compound is selected from the group consisting of peptides, peptide mimetics, antibodies, small organic molecules and small inorganic molecules.

35. The method of claim 34, wherein said peptide is anchored to a solid support by specifically binding an immobilized antibody.

36. The method of claim 28, wherein said Mrg polypeptide is labeled, and/or wherein said test compound is labeled.

37. The method of claim 28, wherein said test compound is contained in a cellular extract.

38. The method of claim 37, wherein said cellular extract is prepared from cells known to express an Mrg polypeptide.

39. The method of claim 38, wherein said cellular extract is prepared from dorsal root ganglion cells.

40. A method of identifying a molecule that binds to an Mrg polypeptide comprising the steps of:
1) contacting a host cell expressing an Mrg polypeptide according to any of the claims 14 to 20, with a test compound; and
2) determining binding of said test compound to said host cell.

41. The method of claim 40, wherein said test compound is labeled, e.g. radioactively labeled.

42. The method of claim 40, wherein said host cell is a eukaryotic cell, e.g. a COS cell.

43. An in vitro method of identifying a compound that binds an Mrg polypeptide comprising the steps of:
1) contacting an Mrg polypeptide according to any of claims 14 to 20, or a fragment thereof with a test compound and a known ligand under conditions where binding can occur; and
2) determining the ability of the test compound to interfere with binding of the known ligand.

44. The method of claim 43, wherein said Mrg polypeptide is contacted with the known ligand prior to being contacted with the test compound.

45. The method of claim 43, wherein said known ligand is an RFamide peptide.

46. An in vitro method for identifying a compound that modulates expression of a nucleic acid encoding an Mrg receptor comprising the steps of:
1) exposing a host cell transformed with a nucleic acid encoding a chimeric polypeptide comprising an Mrg polypeptide according to any of claims 14 to 20, and a reporter protein to a test compound; and
2) determining if there is differential expression of the reporter gene in cells exposed to the test compound compared to control cells that were not exposed to the test compound.

47. An in vitro method for identifying an Mrg polypeptide agonist comprising the steps of:
1) contacting a host cell known to be capable of producing a second messenger responses and expressing a Mrg polypeptide according to any of claims 14 to 20 with a potential agonist; and
2) measuring a second messenger response.

48. The method of claim 47, wherein said host cell is a eukaryotic cell, e.g. a hamster embryonic kidney (HEK) cell.

49. The method of claim 48, wherein said HEK cell expresses G 15.

50. The method of claim 47, wherein measuring a second messenger response comprises measuring a change in intercellular calcium concentration.

51. The method of claim 50, wherein said change in intercellular calcium concentration is measured with FURA-2 calcium indicator dye.

52. The method of claim 47, wherein measuring a second messenger response comprises measuring the flow of current across the membrane of the cell.

53. An in vitro method for identifying an Mrg polypeptide antagonist comprising the steps of:
1) contacting a host cell known to be capable of producing a second messenger response and expressing an Mrg polypeptide according to any of claims 14 to 20, with a known Mrg polypeptide agonist and a candidate antagonist;
2) measuring a second messenger response.

54. The method of claim 53, wherein said host cell is a eukaryotic cell, e.g. a hamster embryonic kidney (HEK) cell.

55. The method of claim 53, wherein said known Mrg polypeptide agonist is an RF-amide peptide.

56. The method of claim 53, wherein said second messenger response is a change in intercellular calcium concentration.

57. The method of claim 53, wherein said second messenger response is a change in the flow of current across the membrane of the cell.

58. An in vitro method of identifying an Mrg polypeptide agonist antibody comprising the steps of:
1) preparing a candidate agonist antibody that specifically binds to an Mrg polypeptide according to any of claims 14 to 20;
2) contacting a host cell known to be capable of producing a second messenger response and expressing the Mrg polypeptide with the candidate agonist antibody; and
3) measuring a second messenger response.

59. An in vitro method of identifying an Mrg polypeptide neutralizing antibody comprising the steps of:
1) preparing a candidate neutralizing antibody that specifically binds an Mrg polypeptide according to any of claims 14 to 20;
2) contacting a host cell known to be capable of producing a second messenger response and expressing the Mrg polypeptide with the candidate neutralizing antibody; and
3) measuring a second messenger response.

60. A transgenic non-human mammal with increased or decreased expression levels of an Mrg polypeptide, wherein said transgenic mammal has stably integrated into its genome a nucleic acid molecule encoding an Mrg polypeptide of any of claims 14 to 20.

61. Use of a composition of matter according to claim 25, in admixture with a pharmaceutical acceptable carrier, for the preparation of a medicament.

62. The use according to claim 61 for treating impaired sensory perception, in particular pain.

## Patentansprüche

1. Isoliertes Nukleinsäuremolekül, das aufweist, mindestens 80 % Sequenzidentität zu
(a) einem Nukleinsäuremolekül, das ein Mrg Polypeptid kodiert, das die Aminosäuresequenz von SEQ ID No 2 oder 12 umfasst,
und das **gekennzeichnet ist durch** die Fähigkeit **durch** ein RF-Amid-Peptid aktiviert zu werden, oder
(b) dem Komplement der Nukleinsäure von (a).

2. Isolierte Nukleinsäure nach Anspruch 1, die eine Identität von mindestens 90 % zu den Molekülen von (a) oder (b) aufweist.

3. Isolierte Nukleinsäure nach Anspruch 1, die eine Identität von mindestens 95 % zu den Molekülen von (a) oder (b) aufweist.

4. Isolierte Nukleinsäure nach Anspruch 1, die durch die Nukleinsäuren 115 - 1026 von SEQ ID No 1 dargestellt wird.

5. Isolierte Nukleinsäure nach Anspruch 1, die durch die Nukleinsäuren 1820 - 2734 von SEQ ID No 11 dargestellt wird.

6. Isoliertes Nukleinsäuremolekül nach Anspruch 1 bis 5, das in operabler Verknüpfung mit einem Expressionssteuerelement vorliegt.

7. Isoliertes Nukleinsäuremolekül nach Anspruch 6, das in operabler Verknüpfung mit einem Promoterelement vorliegt.

8. Vektor, der ein isoliertes Nukleinsäuremolekül nach Anspruch 1 bis 5 umfasst.

9. Wirtszelle, die den Vektor nach Anspruch 8 umfasst.

10. Wirtszelle nach Anspruch 9, wobei die Zelle eine prokaryotische Zelle oder eine eukaryotische Zelle ist.

11. Wirtszelle nach Anspruch 10, wobei die Zelle ein E. coli, eine embryonische Hamsternieren - (HEK, hamster embryonic kidney) Zelle oder eine Hefe-Zelle ist.

12. Verfahren zur Herstellung eines Polypeptids, welches umfasst, Kultivieren der Wirtszelle nach Anspruch 9 unter Bedingungen, bei denen das durch die Nukleinsäure kodierte Protein exprimiert wird.

13. Isoliertes Polypeptid, das durch das Verfahren nach Anspruch 12 hergestellt wird.

14. Isoliertes Polypeptid, das eine Aminosäuresequenz umfasst, die mindestens 40 % Sequenzidentität zu der Aminosäuresequenz von SEQ ID No 2 oder 12 umfasst, und durch die Fähigkeit durch ein RF-Amid-Peptid aktiviert zu werden **gekennzeichnet** ist.

15. Isoliertes Polypeptid nach Anspruch 14, das mindestens 50 % Sequenzidentität zu der Aminosäuresequenz von SEQ ID No 2 oder 12 umfasst und durch die Fähigkeit durch ein RF-Amid-Peptid aktiviert zu werden **gekennzeichnet** ist.

16. Isoliertes Polypeptid nach Anspruch 14, das mindestens 60 % Sequenzidentität zu der Aminosäuresequenz von SEQ ID No 2 oder 12 umfasst und durch die Fähigkeit durch ein RF-Amid-Peptid aktiviert zu werden **gekennzeichnet** ist.

17. Isoliertes Polypeptid nach Anspruch 14, das mindestens 75 % Sequenzidentität zu der Aminosäuresequenz von SEQ ID No 2 oder 12 umfasst und durch die Fähigkeit durch ein RF-Amid-Peptid aktiviert zu werden **gekennzeichnet** ist.

18. Isoliertes Polypeptid nach Anspruch 14, das mindestens etwa 80 % Sequenzidentität zu der Aminosäuresequenz von SEQ ID No 2 oder 12 umfasst und durch die Fähigkeit durch ein RF-Amid-Peptid aktiviert zu werden **gekennzeichnet** ist.

19. Isoliertes Polypeptid nach Anspruch 14, das die Aminosäuresequenz von SEQ ID No 2 aufweist.

20. Isoliertes Polypeptid nach Anspruch 14, das die Aminosäuresequenz von SEQ ID No 12 aufweist.

21. Chimäres Molekül, das ein Mrg Polypeptid nach einem der Ansprüche 14 bis 20 umfasst, das mit einer heterologen Aminosäuresequenz fusioniert ist.

22. Chimäres Molekül nach Anspruch 21, worin die heterologe Aminosäuresequenz eine Epitop-Tag-Sequenz oder eine Sequenz einer konstanten Immunoglobulin-Domäne ist.

23. Isolierter Antikörper, der spezifisch an ein isoliertes Mrg Polypeptid nach einem der Ansprüche 14 bis 20 bindet.

24. Isolierter Antikörper nach Anspruch 23, wobei der Antikörper ein monoklonaler Antikörper, ein Antikörperfragment, ein humanisierter Antikörper, ein Agonist-Antikörper oder ein neutralisierender Antikörper ist.

25. Stoffzusammensetzung, die umfasst, (a) ein Mrg Polypeptid nach einem der Ansprüche 14 bis 22 oder (b) einen anti-Mrg Antikörper nach einem der Ansprüche 23 bis 24 unter Beimengung eines pharmazeutisch akzeptablen Trägers.

26. Herstellungsgegenstand, der umfasst:
einen Behälter; eine Stoffzusammensetzung nach Anspruch 25; und Anweisungen zur Verwendung der Stoffzusammensetzung zur Behandlung von beeinträchtigter sensorischer Wahrnehmung.

27. Verfahren zum Identifizieren einer Mrg Expression in einer Probe, welches umfasst, in Kontakt bringen der Probe mit einem anti Mrg Antikörper nach Anspruch 23 oder 24 und Bestimmen eines Bindens des Antikörpers an die Probe.

28. In vitro Verfahren zum Identifizieren einer Verbindung, die an ein Mrg Polypeptid bindet, welches die Schritte umfasst:
1) In Kontakt bringen einer Test-Verbindung mit mindestens einem Abschnitt eines Mrg Polypeptids nach einem der Ansprüche 14 bis 20; und
2) Erfassen von Mrg/Test-Verbindungs-Komplexen.

29. Verfahren nach Anspruch 28, wobei mindestens eine von der Test-Verbindung oder dem Mrg Polypeptid an einem festen Träger angebracht ist.

30. Verfahren nach Anspruch 29, wobei der feste Träger eine Mikrotiterplatte ist.

31. Verfahren nach Anspruch 28, wobei das Mrg Polypeptid in einer Zellmembran vorliegt.

32. Verfahren nach Anspruch 31, wobei das Mrg Polypeptid in einer Zellmembranfraktion vorliegt, die aus Mrg Polypeptid exprimierenden Zellen hergestellt ist.

33. Verfahren nach Anspruch 28, wobei das Mrg Polypeptid in einem Immunoadhäsin vorliegt.

34. Verfahren nach Anspruch 28, wobei die Test-Verbindung ausgewählt ist aus der Gruppe, bestehend aus Peptiden, Peptidmimetika, Antikörpern, kleinen organischen Molekülen und kleinen anorganischen Molekülen.

35. Verfahren nach Anspruch 34, wobei das Peptid an einem festen Träger durch spezifisches Binden eines immobilisierten Antikörpers verankert ist.

36. Verfahren nach Anspruch 28, wobei das Mrg Polypeptid markiert ist und/oder wobei die Test-Verbindung markiert ist.

37. Verfahren nach Anspruch 28, wobei die Test-Verbindung in einem Zellextrakt enthalten ist.

38. Verfahren nach Anspruch 37, wobei der Zellextrakt aus Zellen hergestellt ist, von denen bekannt ist, dass sie ein Mrg Polypeptid exprimieren.

39. Verfahren nach Anspruch 38, wobei der Zellextrakt aus dorsalen Rückenmarkswurzelganglienzellen hergestellt ist.

40. Verfahren zum Identifizieren eines Moleküls, das an ein Mrg Polypeptid bindet, welches die Schritte umfasst:
1) In Kontakt bringen einer Wirtszelle, die ein Mrg Polypeptid nach einem der Ansprüche 14 bis 20 exprimiert, mit einer Test-Verbindung; und
2) Bestimmen eines Bindens der Test-Verbindung mit der Wirtszelle.

41. Verfahren nach Anspruch 40, wobei die Test-Verbindung markiert ist, beispielsweise radioaktiv markiert ist.

42. Verfahren nach Anspruch 40, wobei die Wirtszelle eine eukaryotische Zelle, beispielsweise eine COS Zelle ist.

43. In vitro Verfahren zum Identifizieren einer Verbindung, die an ein Mrg Polypeptid bindet, welches die Schritte umfasst:
1) In Kontakt bringen eines Mrg Polypeptids nach einem der Ansprüche 14 bis 20 oder eines Fragments davon mit einer Test-Verbindung und einem bekannten Ligand unter Bedingungen, bei denen ein Binden erfolgen kann; und
2) Bestimmen der Fähigkeit der Test-Verbindung bei einem Binden des bekannten Liganden zu interferieren.

44. Verfahren nach Anspruch 43, wobei das Mrg Polypeptid mit dem bekannten Ligand in Kontakt gebracht wird vor einem in Kontakt Bringen mit der Test-Verbindung.

45. Verfahren nach Anspruch 43, wobei der bekannte Ligand ein RF Amid Peptid ist.

46. In vitro Verfahren zum Identifizieren einer Verbindung, welche eine Expression einer Nukleinsäure moduliert, welche einen Mrg Rezeptor kodiert, welches die Schritte umfasst:
1) Aussetzen einer Wirtszelle, die mit einer Nukleinsäure transformiert ist, die ein chimäres Polypeptid kodiert, das umfasst, ein Mrg Polypeptid nach einem der Ansprüche 14 bis 20 und Reporter-Protein hinsichtlich einer Test-Verbindung; und
2) Bestimmen, ob eine unterschiedliche Expression des Reportergens in Zellen vorliegt, die der Test-Verbindung ausgesetzt sind, verglichen mit Kontroll-Zellen, die der Test-Verbindung nicht ausgesetzt waren.

47. In vitro Verfahren zum Identifizieren eines Mrg Polypeptid-Agonisten, das die Schritte umfasst:
1) In Kontakt bringen einer Wirtszelle, von der bekannt ist, dass sie fähig ist zum Herstellen von einer Second-Messenger-Antworten und zum Exprimieren eines Mrg Polypeptids nach einem der Ansprüche 14 bis 20 mit einem potentiellen Agonist; und
2) Erfassen einer Second-Messenger-Antwort.

48. Verfahren nach Anspruch 47, wobei die Wirtszelle eine eukaryotische Zelle, beispielsweise eine embryonische Hamsternieren - (HEK, hamster embryonic kidney) Zelle ist.

49. Verfahren nach Anspruch 48, wobei die HEK Zelle G 15 exprimiert.

50. Verfahren nach Anspruch 47, wobei ein Erfassen einer Second-Messenger-Antwort ein Erfassen einer Veränderung bei einer interzellulären Kalzium-Konzentration umfasst.

51. Verfahren nach Anspruch 50, wobei die Veränderung bei einer interzellulären Kalzium-Konzentration mit FURA-2 Kalzium-Indikatorfarbstoff erfasst wird.

52. Verfahren nach Anspruch 47, wobei ein Erfassen einer Second-Messenger-Antwort ein Erfassen des Stromflusses über die Membran der Zelle umfasst.

53. In vitro Verfahren zum Identifizieren eines Mrg Polypeptid-Antagonisten, welches die Schritte umfasst:
1) In Kontakt bringen einer Wirtszelle von der bekannt ist, dass sie fähig ist zum Herstellen einer Second-Messenger-Antwort und zum Exprimieren eines Mrg Polypeptids nach einem der Ansprüche 14 bis 20 mit einem bekannten Mrg Polypeptid-Agonist und einem Antagonist-Kandidaten; und
2) Erfassen einer Second-Messenger-Antwort.

54. Verfahren nach Anspruch 53, wobei die Wirtszelle eine eukaryotische Zelle ist, beispielsweise eine embryonische Hamsternieren - (HEK, hamster embryonic kidney) Zelle.

55. Verfahren nach Anspruch 53, wobei der bekannte Mrg Polypeptid-Agonist ein RF-Amid-Peptid ist.

56. Verfahren nach Anspruch 53, wobei die Second-Messenger-Antwort eine Veränderung bei einer interzellulären Kalzium-Konzentration ist.

57. Verfahren nach Anspruch 53, wobei die Second-Messenger-Antwort eine Veränderung in dem Stromfluss über die Membran der Zelle ist.

58. In vitro Verfahren zum Identifizieren eines Mrg Polypeptid-Agonist-Antikörpers, welches die Schritte umfasst:
1) Herstellen eines Agonist-Antikörper-Kandidats, der spezifisch ein Mrg Polypeptid nach einem der Ansprüche 14 bis 20 bindet;
2) In Kontakt bringen einer Wirtszelle von der bekannt ist, dass sie fähig ist zum Herstellen einer Second-Messenger-Antwort und zum Exprimieren des Mrg Polypeptids mit dem Agonist-Antikörper-Kandidat; und
3) Erfassen einer Second-Messenger-Antwort.

59. In vitro Verfahren zum Identifizieren eines neutralisierenden Mrg Polypeptid Antikörpers, welches die Schritte umfasst:
1) Herstellen eines neutralisierenden Antikörper-Kandidats, der spezifisch an ein Mrg Polypeptid nach einem der Ansprüche 14 bis 20 bindet;
2) In Kontakt bringen einer Wirtszelle von der bekannt ist, dass sie fähig ist zum Herstellen einer Second-Messenger-Antwort und zum Exprimieren des Mrg Polypeptids mit dem neutralisierenden Antikörper-Kandidaten; und
3) Erfassen einer Second-Messenger-Antwort.

60. Transgenes nicht-menschliches Säugetier mit erhöhten oder verringerten Expressionsniveaus an einem Mrg Polypeptid, wobei das transgene Säugetier ein in stabiler Weise in sein Genom integriertes Nukleinsäuremolekül aufweist, das ein Mrg Polypeptid nach einem der Ansprüche 14 bis 20 kodiert.

61. Verwendung einer Stoffzusammensetzung nach Anspruch 25, in Beimengung mit einem pharmazeutisch akzeptablen Träger fiir die Herstellung eines Medikaments.

62. Verwendung nach Anspruch 61 zur Behandlung beeinträchtigter sensorischer Wahrnehmung, insbesondere von Schmerz.

## Revendications

1. Une molécule d'acide nucléique isolé, ayant au moins 80 % d'identité de séquence à
(a) une molécule d'acide nucléique qui code un polypeptide Mrg comprenant la séquence d'acides aminés de SEQ ID No 2 ou 12,
et étant **caractérisé par** la capacité d'être activé par un amido-peptide RF, ou
(b) le complément de l'acide nucléique de (a).

2. L'acide nucléique isolé de la revendication 1, ayant une identité d'au moins 90 % aux molécules de (a) ou (b).

3. L'acide nucléique isolé de la revendication 1, ayant une identité d'au moins 95 % aux molécules de (a) ou (b).

4. L'acide nucléique isolé de la revendication 1, qui est représenté par les acides nucléiques 115 - 1026 de SEQ ID No 1.

5. L'acide nucléique isolé de la revendication 1, qui est représenté par les acides nucléiques 1820 - 2734 de SEQ ID No 11.

6. La molécule d'acide nucléique isolé des revendications 1 à 5, lié opérationnellement à un élément de contrôle d'expression.

7. La molécule d'acide nucléique isolé de la revendication 6, liée opérationnellement à un élément promoteur.

8. Un vecteur comprenant une molécule d'acide nucléique isolé des revendications 1 à 5.

9. Une cellule hôte comprenant le vecteur de la revendication 8.

10. La cellule hôte de la revendication 9, dans laquelle ladite cellule est une cellule procaryote ou une cellule eucaryote

11. La cellule hôte de la revendication 10, dans laquelle ladite cellule est un E. Coli, une cellule embryonnaire de rein de hamster (HEK) ou une cellule de levure.

12. Une méthode pour produire un polypeptide comprenant cultiver la cellule hôte de la revendication 9 sous des conditions dans lesquelles la protéine codée par ledit acide nucléique est exprimée.

13. Un polypeptide isolé produit par la méthode de la revendication 12.

14. Un polypeptide isolé comprenant une séquence d'acides aminés ayant au moins 40 % d'identité de séquence à la séquence d'acides aminés de SEQ ID No 2 ou 12, et étant **caractérisé par** la capacité d'être activé par un amido-peptide RF.

15. Un polypeptide isolé de la revendication 14, comprenant au moins 50 % d'identité de séquence à la séquence d'acides aminés de SEQ ID No 2 ou 12, et étant **caractérisé par** la capacité d'être activé par un amido-peptide RF.

16. Un polypeptide isolé de la revendication 14, comprenant au moins 60 % d'identité de séquence à la séquence d'acides aminés de SEQ ID No 2 ou 12, et étant **caractérisé par** la capacité d'être activé par un amido-peptide RF.

17. Un polypeptide isolé de la revendication 14, comprenant au moins 75 % d'identité de séquence à la séquence d'acides aminés de SEQ ID No 2 ou 12, et étant **caractérisé par** la capacité d'être activé par un amido-peptide RF.

18. Un polypeptide isolé de la revendication 14, comprenant au moins approximativement 80 % d'identité de séquence à la séquence d'acides aminés de SEQ ID No 2 ou 12, et étant **caractérisé par** la capacité d'être activé par un amido-peptide RF.

19. Le polypeptide isolé de la revendication 14, qui a la séquence d'acides aminés SEQ ID No 2.

20. Le polypeptide isolé de la revendication 14, qui a la séquence d'acides aminés SEQ ID No 12.

21. Une molécule chimère comprenant un polypeptide Mrg selon l'une quelconque des revendications 14 à 20, fusionné à une séquence d'acides aminés hétérologue.

22. La molécule chimère de la revendication 21, dans laquelle ladite séquence d'acides aminés hétérologue est une séquence d'épitope tag ou une séquence de domaine constant d'immunoglobuline.

23. Un anticorps isolé qui se lie spécifiquement à un polypeptide Mrg isolé de l'une quelconque des revendications 14 à 20.

24. L'anticorps isolé de la revendication 23, dans lequel ledit anticorps est un anticorps monoclonal, un fragment d'anticorps, un anticorps humanisé, un anticorps agoniste ou un anticorps neutralisant.

25. Une composition comprenant (a) un polypeptide Mrg selon l'une quelconque des revendications 14 à 22 ou (b) un anticorps anti-Mrg selon l'une quelconque des revendications 23 à 24, en mélange avec un support pharmaceutiquement acceptable.

26. Un article de manufacture comprenant :
un récipient ; une composition de la revendication 25 ; et des instructions pour utiliser la composition pour traiter la perception sensorielle déteriorée.

27. Une méthode pour identifier l'expression de Mrg dans un échantillon, comprenant contacter ledit échantillon avec un anticorps anti-Mrg selon la revendication 23 ou 24 et déterminer la liaison dudit anticorps à l'échantillon.

28. Une méthode in vitro pour identifier un composé qui se lie à un polypeptide Mrg comprenant les étapes de :
1) contacter un composé test avec au moins une portion d'un polypeptide Mrg selon l'une quelconque des revendications 14 à 20 ; et
2) détecter des complexes Mrg/composé test.

29. La méthode de la revendication 28, dans laquelle au moins un du composé test ou du polypeptide Mrg est attaché à un support solide.

30. La méthode de la revendication 29, dans laquelle ledit support solide est une plaque de micro-titration.

31. La méthode de la revendication 28, dans laquelle ledit polypeptide Mrg est présent dans une membrane de cellule.

32. La méthode de la revendication 31, dans laquelle ledit polypeptide Mrg est présent dans une fraction de membrane de cellule préparée à partir de cellules exprimant un polypeptide Mrg.

33. La méthode de la revendication 28, dans laquelle ledit polypeptide Mrg est présent dans une immunoadhésine.

34. La méthode de la revendication 28, dans laquelle ledit composé test est choisi parmi le groupe consistant en peptides, analogues de peptides, anticorps, petites molécules organiques et petites molécules inorganiques.

35. La méthode de la revendication 34, dans laquelle ledit peptide est ancré à un support solide se liant spécifiquement à un anticorps immobilisé.

36. La méthode de la revendication 28, dans laquelle ledit polypeptide Mrg est marqué et/ou dans laquelle le composé test est marqué.

37. La méthode de la revendication 28, dans laquelle ledit composé test est contenu dans un extrait cellulaire.

38. La méthode de la revendication 37, dans laquelle ledit extrait cellulaire est préparé à partir de cellules connues pour exprimer un polypeptide Mrg.

39. La méthode de la revendication 38, dans laquelle ledit extrait cellulaire est préparé à partir de cellules de ganglions radicalaires dorsaux.

40. Une méthode pour identifier une molécule qui se lie à un polypeptide Mrg comprenant les étapes de :
1) contacter une cellule hôte exprimant un polypeptide Mrg selon l'une quelconque des revendications 14 à 20, avec un composé test ; et
2) déterminer la liaison dudit composé test à ladite cellule hôte.

41. La méthode de la revendication 40, dans laquelle ledit composé test est marqué, par exemple marqué radioactivement.

42. La méthode de la revendication 40, dans laquelle ladite cellule hôte est une cellule eucaryote, par exemple une cellule COS.

43. Une méthode in vitro pour identifier un composé qui se lie à un polypeptide Mrg comprenant les étapes de :
1) contacter un polypeptide Mrg selon l'une quelconque des revendications 14 à 20, ou un fragment de celui-ci avec un composé test et un ligand connu dans des conditions où la liaison peut se produire ; et
2) déterminer la capacité du composé test d'interférer avec la liaison du ligand connu.

44. La méthode de la revendication 43, dans laquelle ledit polypeptide Mrg est contacté avec le ligand connu avant d'être contacté avec le composé test.

45. La méthode de la revendication 43, dans laquelle ledit ligand connu est un amido-peptide RF.

46. Une méthode in vitro pour identifier un composé qui module l'expression d'un acide nucléique codant un récepteur Mrg comprenant les étapes de :
1) exposer une cellule hôte transformée avec un acide nucléique codant un polypeptide chimère comprenant un polypeptide Mrg selon l'une quelconque des revendications 14 à 20, et une protéine rapporteur à un composé test; et
2) déterminer s'il y a différentiel d'expression du gêne rapporteur dans des cellules exposées au composé test comparées à des cellules de contrôle qui n'ont pas été exposées au composé test.

47. Une méthode in vitro pour identifier un agoniste de polypeptide Mrg comprenant les étapes de :
1) contacter une cellule hôte connue pour être capable de produire une seconde réponse messager et d'exprimer un polypeptide Mrg selon l'une quelconque des revendications 14 à 20 avec un agoniste potentiel ; et
2) mesurer une seconde réponse messager.

48. La méthode de la revendication 47, dans laquelle ladite cellule hôte est une cellule eucaryote, par exemple une cellule embryonnaire de rein de hamster (HEK).

49. La méthode de la revendication 48, dans laquelle ladite cellule HEK exprime G 15.

50. La méthode de la revendication 47, dans laquelle mesurer une seconde réponse messager comprend mesurer un changement dans la concentration de calcium intercellulaire.

51. La méthode de la revendication 50, dans laquelle ledit changement dans la concentration de calcium intercellulaire est mesuré avec le colorant indicateur de calcium FURA-2.

52. La méthode de la revendication 47, dans laquelle mesurer une seconde réponse messager comprend mesurer le flux de courant au travers de la membrane de la cellule.

53. Une méthode in vitro pour identifier un antagoniste de polypeptide Mrg comprenant les étapes de :
1) contacter une cellule hôte connue pour être capable de produire une seconde réponse messager et d'exprimer un polypeptide Mrg selon l'une quelconque des revendications 14 à 20 avec un agoniste de polypeptide Mrg connu et un candidat antagoniste ;
2) mesurer une seconde réponse messager.

54. La méthode de la revendication 53, dans laquelle ladite cellule hôte est une cellule eucaryote, par exemple une cellule embryonnaire de rein de hamster (HEK).

55. La méthode de la revendication 53, dans laquelle ledit agoniste de polypeptide Mrg connu est un amido-peptide RF.

56. La méthode de la revendication 53, dans laquelle la seconde réponse messager est un changement dans la concentration de calcium intercellulaire.

57. La méthode de la revendication 53, dans laquelle ladite seconde réponse messager est un changement dans le flux de courant au travers de la membrane de la cellule.

58. Une méthode in vitro pour identifier un anticorps agoniste de polypeptide Mrg comprenant les étapes de :
1) préparer un candidat anticorps agoniste qui se lie spécifiquement à un polypeptide Mrg selon l'une quelconque des revendications 14 à 20 ;
2) contacter une cellule hôte connue pour être capable de produire une seconde réponse messager et d'exprimer le polypeptide Mrg avec le candidat anticorps agoniste; et
3) mesurer une seconde réponse messager.

59. Une méthode in vitro pour identifier un anticorps neutralisant de polypeptide Mrg comprenant les étapes de :
1) préparer un candidat anticorps neutralisant qui se lie spécifiquement à un polypeptide Mrg selon l'une quelconque des revendications 14 à 20 ;
2) contacter une cellule hôte connue pour être capable de produire une seconde réponse messager et d'exprimer le polypeptide Mrg avec le candidat anticorps neutralisant; et
3) mesurer une seconde réponse messager.

60. Un mammifère transgénique non humain avec des niveaux d'expression d'un polypeptide Mrg augmentés ou réduits, dans lequel ledit mammifère transgénique a intégré durablement dans son génome une molécule d'acide nucléique codant un polypeptide Mrg de l'une quelconque des revendications 14 à 20.

61. Utilisation d'une composition de la revendication 25 en mélange avec un support pharmaceutiquement acceptable, pour la préparation d'un médicament.

62. L'utilisation de la revendication 61 pour traiter la perception sensorielle détériorée, en particulier la douleur.


### SEQUENCE LISTING

<110> California Institute of Technology
   Anderson, David J.
   Dong, Xinzhong
   Zylka, Mark
   Simon, Melvin
   Han, Sang-kyou
<120> PAIN SIGNALING MOLECULES
<130> CALTE.004C1
<150> US 60/222,344
   <151> 2000-08-01
<150> US 60/202,027
   <151> 2000-05-04
<150> US 09/704,707
   <151> 2000-11-03
<150> US unknown
   <151> 2001-04-19
<160> 109
<170> FastSEQ for Windows Version 4.0
<210> 1
   <211> 1088
   <212> DNA
   <213> Mus Musculus
<220>
   <221> CDS
   <222> (115)...(1026)
<400> 1
   acagaagcca gagagctaca tccagcaaga ggaatggggg aaagcagcac ctgtgcaggg 60
   tttctagccc taaacacatc ggcctcgcca acagcaccca caacaactaa tcca atg   117
                                                               Met
                                                                1
   gac aat acc atc cct gga ggt atc aac atc acg att ctg atc cca aac   165
   Asp Asn Thr Ile Pro Gly Gly Ile Asn Ile Thr Ile Leu Ile Pro Asn
               5                   10                  15
   ttg atg atc atc atc ttc gga ctg gtc ggg ctg aca gga aat ggc att   213
   Leu Met Ile Ile Ile Phe Gly Leu Val Gly Leu Thr Gly Asn Gly Ile
           20                  25                  30
   gtg ttc tgg ctc ctg ggc ttc tgt ttg cac agg aac gcc ttc tca gtc   261
   Val Phe Trp Leu Leu Gly Phe Cys Leu His Arg Asn Ala Phe Ser Val
       35                  40                  45
   tac atc cta aac tta gct cta gct gac ttc ttc ttc ctc cta ggt cac   309
   Tyr Ile Leu Asn Leu Ala Leu Ala Asp Phe Phe Phe Leu Leu Gly His
    50                  55                  60                  65
   atc ata gat tcc ata ctg ctt ctt ctc aat gtt ttc tac cca att acc   357
   Ile Ile Asp Ser Ile Leu Leu Leu Leu Asn Val Phe Tyr Pro Ile Thr
                   70                  75                  80
   ttt ctc ttg tgc ttt tac acg atc atg atg gtt ctc tat atc gca ggc     405
   Phe Leu Leu Cys Phe Tyr Thr Ile Met Met Val Leu Tyr Ile Ala Gly
               85                  90                  95
   ctg agc atg ctc agt gcc atc agc act gag cgc tgc ctg tct gta ctg     453
   Leu Ser Met Leu Ser Ala Ile Ser Thr Glu Arg Cys Leu Ser Val Leu
           100                 105                 110
   tgc ccc atc tgg tat cac tgt cac cgc cca gaa cac aca tca act gtc     501
   Cys Pro Ile Trp Tyr His Cys His Arg Pro Glu His Thr Ser Thr Val
       115                 120                 125
   atg tgt gct gtc atc tgg gtc ctg tcc ctg ttg atc tgc att ctg aat     549
   Met Cys Ala Val Ile Trp Val Leu Ser Leu Leu Ile Cys Ile Leu Asn
   130                 135                 140                 145
   agt tat ttc tgc ggt ttc tta aat acc caa tat aaa aat gaa aat ggg     597
   Ser Tyr Phe Cys Gly Phe Leu Asn Thr Gln Tyr Lys Asn Glu Asn Gly
                   150                 155                 160
   tgt ctg gca ttg aac ttc ttt act gct gca tac ctg atg ttt ttg ttt     645
   Cys Leu Ala Leu Asn Phe Phe Thr Ala Ala Tyr Leu Met Phe Leu Phe
               165                 170                 175
   gtg gtc ctc tgt ctg tcc agc ctg gct ctg gtg gcc agg ttg ttc tgt     693
   Val Val Leu Cys Leu Ser Ser Leu Ala Leu Val Ala Arg Leu Phe Cys
           180                 185                 190
   ggt act ggg cag ata aag ctt acc aga ttg tat gta acc att att ctg     741
   Gly Thr Gly Gln Ile Lys Leu Thr Arg Leu Tyr Val Thr Ile Ile Leu
       195                 200                 205
   agc att ttg gtt ttt ctc ctt tgc gga ttg ccc ttt ggc atc cac tgg     789
   Ser Ile Leu Val Phe Leu Leu Cys Gly Leu Pro Phe Gly Ile His Trp
   210                 215                 220                 225
   ttt ctg tta ttc aag att aag gat gat ttt cat gta ttt gat ctt gga     837
   Phe Leu Leu Phe Lys Ile Lys Asp Asp Phe His Val Phe Asp Leu Gly
                   230                 235                 240
   ttt tat ctg gca tca gtt gtc ctg act gct att aat agc tgt gcc aac     885
   Phe Tyr Leu Ala Ser Val Val Leu Thr Ala Ile Asn Ser Cys Ala Asn
               245                 250                 255
   ccc atc att tac ttc ttc gtg gga tcc ttc agg cat cgg ttg aag cac     933
   Pro Ile Ile Tyr Phe Phe Val Gly Ser Phe Arg His Arg Leu Lys His
           260                 265                 270
   cag acc ctc aaa atg gtt ctc cag aat gca ctg caa gac act cct gag     981
   Gln Thr Leu Lys Met Val Leu Gln Asn Ala Leu Gln Asp Thr Pro Glu
       275                 280                 285
   aca gcc aaa atc atg gtg gag atg tca aga agc aaa tca gag cca
   1026
   Thr Ala Lys Ile Met Val Glu Met Ser Arg Ser Lys Ser Glu Pro
   290                 295                 300
   tgatgaagag cctttgcctg gcccttagaa gtggctttgg ggtgagcatt gccctgctgc
   1086
   ac
   1088
<210> 2
   <211> 304
   <212> PRT
   <213> Mus Musculus
<400> 2
   Met Asp Asn Thr Ile Pro Gly Gly Ile Asn Ile Thr Ile Leu Ile Pro
    1               5                  10                  15
   Asn Leu Met Ile Ile Ile Phe Gly Leu Val Gly Leu Thr Gly Asn Gly
               20                  25                  30
   Ile Val Phe Trp Leu Leu Gly Phe Cys Leu His Arg Asn Ala Phe Ser
           35                  40                  45
   Val Tyr Ile Leu Asn Leu Ala Leu Ala Asp Phe Phe Phe Leu Leu Gly
       50                  55                  60
   His Ile Ile Asp Ser Ile Leu Leu Leu Leu Asn Val Phe Tyr Pro Ile
   65                  70                  75                  80
   Thr Phe Leu Leu Cys Phe Tyr Thr Ile Met Met Val Leu Tyr Ile Ala
                   85                  90                  95
   Gly Leu Ser Met Leu Ser Ala Ile Ser Thr Glu Arg Cys Leu Ser Val
               100                 105                 110
   Leu Cys Pro Ile Trp Tyr His Cys His Arg Pro Glu His Thr Ser Thr
           115                 120                 125
   Val Met Cys Ala Val Ile Trp Val Leu Ser Leu Leu Ile Cys Ile Leu
       130                 135                 140
   Asn Ser Tyr Phe Cys Gly Phe Leu Asn Thr Gln Tyr Lys Asn Glu Asn
   145                 150                 155                 160
   Gly Cys Leu Ala Leu Asn Phe Phe Thr Ala Ala Tyr Leu Met Phe Leu
                   165                 170                 175
   Phe Val Val Leu Cys Leu Ser Ser Leu Ala Leu Val Ala Arg Leu Phe
               180                 185                 190
   Cys Gly Thr Gly Gln Ile Lys Leu Thr Arg Leu Tyr Val Thr Ile Ile
           195                 200                 205
   Leu Ser Ile Leu Val Phe Leu Leu Cys Gly Leu Pro Phe Gly Ile His
       210                 215                 220
   Trp Phe Leu Leu Phe Lys Ile Lys Asp Asp Phe His Val Phe Asp Leu
   225                 230                 235                 240
   Gly Phe Tyr Leu Ala Ser Val Val Leu Thr Ala Ile Asn Ser Cys Ala
                   245                 250                 255
   Asn Pro Ile Ile Tyr Phe Phe Val Gly Ser Phe Arg His Arg Leu Lys
               260                 265                 270
   His Gln Thr Leu Lys Met Val Leu Gln Asn Ala Leu Gln Asp Thr Pro
           275                 280                 285
   Glu Thr Ala Lys Ile Met Val Glu Met Ser Arg Ser Lys Ser Glu Pro
       290                 295                 300
<210> 3
   <211> 1234
   <212> DNA
   <213> Mus musculus
<220>
   <221> CDS
   <222> (137)...(1051)
<400> 3
   tctgtagtga ctgtatcttt ccttctacac aagccagtga gctacatcca acaagaggat 60
   tggggaaagc aatggtgaag catttcttgc ctttaagacc tcagcctcac caacagcacc 120
   agtgacaaca aatcca atg gac gaa acc ctc cct gga agt atc aac att agg 172
                     Met Asp Glu Thr Leu Pro Gly Ser Ile Asn Ile Arg
                      1               5                  10
   att ctg atc cca aaa ttg atg atc atc atc ttc gga ctg gtc gga ctg    220
   Ile Leu Ile Pro Lys Leu Met Ile Ile Ile Phe Gly Leu Val Gly Leu
           15                  20                  25
   atg gga aac gcc att gtg ttc tgg ctc ctg ggc ttc cac ttg cgc aag    268
   Met Gly Asn Ala Ile Val Phe Trp Leu Leu Gly Phe His Leu Arg Lys
       30                  35                  40
   aat gac ttc tca ctc tac atc cta aac ttg gcc cgg gct gac ttc ctt     316
   Asn Asp Phe Ser Leu Tyr Ile Leu Asn Leu Ala Arg Ala Asp Phe Leu
    45                 50                  55                  60
   ttc ctc ctc agt agt atc ata gct tcc acc ctg ttt ctt ctc aaa gtt     364
   Phe Leu Leu Ser Ser Ile Ile Ala Ser Thr Leu Phe Leu Leu Lys Val
                   65                  70                  75
   tcc tac ctc agc atc atc ttt cac ttg tgc ttt aac acc att atg atg     412
   Ser Tyr Leu Ser Ile Ile Phe His Leu Cys Phe Asn Thr Ile Met Met
               80                  85                  90
   gtt gtc tac atc aca ggg ata agc atg ctc agt gcc atc agc act gag     460
   Val Val Tyr Ile Thr Gly Ile Ser Met Leu Ser Ala Ile Ser Thr Glu
           95                 100                 105
   tgc tgc ctg tct gtc ctg tgc ccc acc tgg tat cgc tgc cac cgt cca     508
   Cys Cys Leu Ser Val Leu Cys Pro Thr Trp Tyr Arg Cys His Arg Pro
       110                 115                 120
   gta cat aca tca act gtc atg tgt gct gtg atc tgg gtc cta tcc ctg     556
   Val His Thr Ser Thr Val Met Cys Ala Val Ile Trp Val Leu Ser Leu
   125                 130                 135                 140
   ttg atc tgc att ctg aat agc tat ttc tgt gct gtc tta cat acc aga     604
   Leu Ile Cys Ile Leu Asn Ser Tyr Phe Cys Ala Val Leu His Thr Arg
                   145                 150                 155
   tat gat aat gac aat gag tgt ctg gca act aac atc ttt acc gcc tcg     652
   Tyr Asp Asn Asp Asn Glu Cys Leu Ala Thr Asn Ile Phe Thr Ala Ser
               160                 165                 170
   tac atg ata ttt ttg ctt gtg gtc ctc tgt ctg tcc agc ctg gct ctg     700
   Tyr Met Ile Phe Leu Leu Val Val Leu Cys Leu Ser Ser Leu Ala Leu
           175                 180                 185
   ctg gcc agg ttg ttc tgt ggc gct ggg cag atg aag ctt acc aga ttt     748
   Leu Ala Arg Leu Phe Cys Gly Ala Gly Gln Met Lys Leu Thr Arg Phe
       190                 195                 200
   cat gtg acc atc ttg ctg acc ctt ttg gtt ttt ctc ctc tgc ggg ttg     796
   His Val Thr Ile Leu Leu Thr Leu Leu Val Phe Leu Leu Cys Gly Leu
   205                 210                 215                 220
   ccc ttt gtc atc tac tgc atc ctg tta ttc aag att aag gat gat ttc     844
   Pro Phe Val Ile Tyr Cys Ile Leu Leu Phe Lys Ile Lys Asp Asp Phe
                   225                 230                 235
   cat gta tta gat gtt aat ttt tat cta gca tta gaa gtc ctg act gct     892
   His Val Leu Asp Val Asn Phe Tyr Leu Ala Leu Glu Val Leu Thr Ala
               240                 245                 250
   att aac agc tgt gcc aac ccc atc atc tac ttc ttc gtg ggc tct ttc     940
   Ile Asn Ser Cys Ala Asn Pro Ile Ile Tyr Phe Phe Val Gly Ser Phe
           255                 260                 265
   aga cat cag ttg aag cac cag acc ctc aaa atg gtt ctc cag agt gca     988
   Arg His Gln Leu Lys His Gln Thr Leu Lys Met Val Leu Gln Ser Ala
       270                 275                 280
   ctg cag gac act cct gag aca gct gaa aac atg gta gag atg tca agt
   1036
   Leu Gln Asp Thr Pro Glu Thr Ala Glu Asn Met Val Glu Met Ser Ser
   285                 290                 295                 300
   aac aaa gca gag cct tgatgaagag cctctacctg gacctcagag gtggctttgg
   1091
   Asn Lys Ala Glu Pro
   305
   agtgagcact gccctgctgc acttgaccac tgtccactct tctctcagct tactgatttg
   1151
   acatgcctca gtggtccacc aacaacttca acatctctcc actaacttag tttttctacc
   1211
   cctcctgaat aaaagcatta atc
   1234
<210> 4
   <211> 305
   <212> PRT
   <213> Mus musculus
<400> 4
   Met Asp Glu Thr Leu Pro Gly Ser Ile Asn Ile Arg Ile Leu Ile Pro
    1               5                  10                  15
   Lys Leu Met Ile Ile Ile Phe Gly Leu Val Gly Leu Met Gly Asn Ala
               20                  25                  30
   Ile Val Phe Trp Leu Leu Gly Phe His Leu Arg Lys Asn Asp Phe Ser
           35                  40                  45
   Leu Tyr Ile Leu Asn Leu Ala Arg Ala Asp Phe Leu Phe Leu Leu Ser
       50                  55                  60
   Ser Ile Ile Ala Ser Thr Leu Phe Leu Leu Lys Val Ser Tyr Leu Ser
   65                  70                  75                  80
   Ile Ile Phe His Leu Cys Phe Asn Thr Ile Met Met Val Val Tyr Ile
                   85                  90                  95
   Thr Gly Ile Ser Met Leu Ser Ala Ile Ser Thr Glu Cys Cys Leu Ser
               100                 105                 110
   Val Leu Cys Pro Thr Trp Tyr Arg Cys His Arg Pro Val His Thr Ser
           115                 120                 125
   Thr Val Met Cys Ala Val Ile Trp Val Leu Ser Leu Leu Ile Cys Ile
       130                 135                 140
   Leu Asn Ser Tyr Phe Cys Ala Val Leu His Thr Arg Tyr Asp Asn Asp
   145                 150                 155                 160
   Asn Glu Cys Leu Ala Thr Asn Ile Phe Thr Ala Ser Tyr Met Ile Phe
                   165                 170                 175
   Leu Leu Val Val Leu Cys Leu Ser Ser Leu Ala Leu Leu Ala Arg Leu
               180                 185                 190
   Phe Cys Gly Ala Gly Gln Met Lys Leu Thr Arg Phe His Val Thr Ile
           195                 200                 205
   Leu Leu Thr Leu Leu Val Phe Leu Leu Cys Gly Leu Pro Phe Val Ile
       210                 215                 220
   Tyr Cys Ile Leu Leu Phe Lys Ile Lys Asp Asp Phe His Val Leu Asp
   225                 230                 235                 240
   Val Asn Phe Tyr Leu Ala Leu Glu Val Leu Thr Ala Ile Asn Ser Cys
                   245                 250                 255
   Ala Asn Pro Ile Ile Tyr Phe Phe Val Gly Ser Phe Arg His Gln Leu
               260                 265                 270
   Lys His Gln Thr Leu Lys Met Val Leu Gln Ser Ala Leu Gln Asp Thr
           275                 280                 285
   Pro Glu Thr Ala Glu Asn Met Val Glu Met Ser Ser Asn Lys Ala Glu
       290                 295                 300
   Pro
   305
<210> 5
   <211> 1312
   <212> DNA
   <213> Mus musculus
<220>
   <221> CDS
   <222> (165)...(1070)
<400> 5
   cgcggccgcg tcgacaagaa atattctgta gtgactgtat ccttccttct acacaagcca 60
   gcaagctaca tccagcaaga ggaatgggag aaagcaacac cagtgcaggg tttctggccc 120
   gaaacacctc agcctcgaca atgacaccca caacaacaaa ttca atg aac gaa acc  176
                                                    Met Asn Glu Thr
                                                    1
   atc cct gga agt att gac atc gag acc ctg atc cca gac ttg atg atc   224
   Ile Pro Gly Ser Ile Asp Ile Glu Thr Leu Ile Pro Asp Leu Met Ile
   5                   10                  15                  20
   atc atc ttc gga ctg gtc ggg ctg aca gga aat gcg att gtg ttc tgg   272
   Ile Ile Phe Gly Leu Val Gly Leu Thr Gly Asn Ala Ile Val Phe Trp
                   25                  30                  35
   ctc ctt ggc ttc cgc atg cac agg act gcc ttc tta gtc tac atc cta   320
   Leu Leu Gly Phe Arg Met His Arg Thr Ala Phe Leu Val Tyr Ile Leu
               40                  45                  50
   aac ttg gcc ctg gct gac ttc ctc ttc ctt ctc tgt cac atc ata aat   368
   Asn Leu Ala Leu Ala Asp Phe Leu Phe Leu Leu Cys His Ile Ile Asn
           55                  60                  65
   tcc aca gtg gat ctt ctc aag ttt acc cta ccc aaa gga att ttt gcc   416
   Ser Thr Val Asp Leu Leu Lys Phe Thr Leu Pro Lys Gly Ile Phe Ala
       70                  75                  80
   ttt tgt ttt cac act atc aaa agg gtt ctc tat atc aca ggc ctg agc   464
   Phe Cys Phe His Thr Ile Lys Arg Val Leu Tyr Ile Thr Gly Leu Ser
   85                  90                  95                  100
   atg ctc agt gcc atc agc act gag cgc tgc ctg tct gtc ctg tgc ccc   512
   Met Leu Ser Ala Ile Ser Thr Glu Arg Cys Leu Ser Val Leu Cys Pro
                   105                 110                 115
   atc tgg tat cac tgc cgc cgc cca gaa cac aca tca act gtc atg tgt   560
   Ile Trp Tyr His Cys Arg Arg Pro Glu His Thr Ser Thr Val Met Cys
               120                 125                 130
   gct gtg atc tgg gtc ctg tcc ctg ttg atc tgc att ctg gat ggt tat   608
   Ala Val Ile Trp Val Leu Ser Leu Leu Ile Cys Ile Leu Asp Gly Tyr
           135                 140                 145
   ttc tgc ggt tac tta gat aac cat tat ttc aat tac tct gtg tgt cag   656
   Phe Cys Gly Tyr Leu Asp Asn His Tyr Phe Asn Tyr Ser Val Cys Gln
       150                 155                 160
   gca tgg gac atc ttt atc gga gca tac ctg atg ttt ttg ttt gta gtc   704
   Ala Trp Asp Ile Phe Ile Gly Ala Tyr Leu Met Phe Leu Phe Val Val
   165                 170                 175                 180
   ctc tgt ctg tcc acc ctg gct cta ctg gcc agg ttg ttc tgt ggt gct   752
   Leu Cys Leu Ser Thr Leu Ala Leu Leu Ala Arg Leu Phe Cys Gly Ala
                   185                 190                 195
   agg aat atg aaa ttt acc aga tta ttc gtg acc atc atg ctg acc gtt   800
   Arg Asn Met Lys Phe Thr Arg Leu Phe Val Thr Ile Met Leu Thr Val
               200                 205                 210
   ttg gtt ttt ctt ctc tgt ggg ttg ccc tgg ggc atc acc tgg ttc ctg   848
   Leu Val Phe Leu Leu Cys Gly Leu Pro Trp Gly Ile Thr Trp Phe Leu
           215                 220                 225
   tta ttc tgg att gca cct ggt gtg ttt gta cta gat tat agc cct ctt   896
   Leu Phe Trp Ile Ala Pro Gly Val Phe Val Leu Asp Tyr Ser Pro Leu
       230                 235                 240
   ctg gtc cta act gct att aac agc tgt gcc aac ccc att att tac ttc    944
   Leu Val Leu Thr Ala Ile Asn Ser Cys Ala Asn Pro Ile Ile Tyr Phe
   245                 250                 255                 260
   ttc gtg ggc tcc ttc agg caa cgg ttg aat aaa cag acc ctc aaa atg    992
   Phe Val Gly Ser Phe Arg Gln Arg Leu Asn Lys Gln Thr Leu Lys Met
                   265                 270                 275
   gtt ctc cag aaa gcc ctg cag gac act cct gag aca cct gaa aac atg
   1040
   Val Leu Gln Lys Ala Leu Gln Asp Thr Pro Glu Thr Pro Glu Asn Met
               280                 285                 290
   gtg gag atg tca aga aac aaa gca gag ccg tgatgaagag cctctgccta
   1090
   Val Glu Met Ser Arg Asn Lys Ala Glu Pro
           295                 300
   gacttcagag gtggatttgg agtgagcact gccctgctgc acttgaccac tgtccactct
   1150
   cctctcagct tactgacttg acatgcctca ctggtccacc aacaccttcc aaagctctcc
   1210
   actgacttag tatttatacc tctcccaaac aatagcatta ttcaaaaact ataatttctg
   1270
   catccttctt tacattaata aaattcccat actaagttca aa
   1312
<210> 6
   <211> 302
   <212> PRT
   <213> Mus musculus
<400> 6
   Met Asn Glu Thr Ile Pro Gly Ser Ile Asp Ile Glu Thr Leu Ile Pro
    1               5                  10                  15
   Asp Leu Met Ile Ile Ile Phe Gly Leu Val Gly Leu Thr Gly Asn Ala
               20                  25                  30
   Ile Val Phe Trp Leu Leu Gly Phe Arg Met His Arg Thr Ala Phe Leu
           35                  40                  45
   Val Tyr Ile Leu Asn Leu Ala Leu Ala Asp Phe Leu Phe Leu Leu Cys
       50                  55                  60
   His Ile Ile Asn Ser Thr Val Asp Leu Leu Lys Phe Thr Leu Pro Lys
   65                  70                  75                  80
   Gly Ile Phe Ala Phe Cys Phe His Thr Ile Lys Arg Val Leu Tyr Ile
                   85                  90                  95
   Thr Gly Leu Ser Met Leu Ser Ala Ile Ser Thr Glu Arg Cys Leu Ser
               100                 105                 110
   Val Leu Cys Pro Ile Trp Tyr His Cys Arg Arg Pro Glu His Thr Ser
           115                 120                 125
   Thr Val Met Cys Ala Val Ile Trp Val Leu Ser Leu Leu Ile Cys Ile
       130                 135                 140
   Leu Asp Gly Tyr Phe Cys Gly Tyr Leu Asp Asn His Tyr Phe Asn Tyr
   145                 150                 155                 160
   Ser Val Cys Gln Ala Trp Asp Ile Phe Ile Gly Ala Tyr Leu Met Phe
                   165                 170                 175
   Leu Phe Val Val Leu Cys Leu Ser Thr Leu Ala Leu Leu Ala Arg Leu
               180                 185                 190
   Phe Cys Gly Ala Arg Asn Met Lys Phe Thr Arg Leu Phe Val Thr Ile
           195                 200                 205
   Met Leu Thr Val Leu Val Phe Leu Leu Cys Gly Leu Pro Trp Gly Ile
       210                 215                 220
   Thr Trp Phe Leu Leu Phe Trp Ile Ala Pro Gly Val Phe Val Leu Asp
   225                 230                 235                 240
   Tyr Ser Pro Leu Leu Val Leu Thr Ala Ile Asn Ser Cys Ala Asn Pro
                   245                 250                 255
   Ile Ile Tyr Phe Phe Val Gly Ser Phe Arg Gln Arg Leu Asn Lys Gln
               260                 265                 270
   Thr Leu Lys Met Val Leu Gln Lys Ala Leu Gln Asp Thr Pro Glu Thr
           275                 280                 285
   Pro Glu Asn Met Val Glu Met Ser Arg Asn Lys Ala Glu Pro
       290                 295                 300
<210> 7
   <211> 450
   <212> DNA
   <213> Mus musculus
<220>
   <221> CDS
   <222> (1)...(450)
<400> 7
   ctg tgc cgg atc tgg tat cac tgc cgc cgc cca gaa cac aca tca act     48
   Leu Cys Arg Ile Trp Tyr His Cys Arg Arg Pro Glu His Thr Ser Thr
    1               5                  10                  15
   gtc atg tgt gct gtc atc tgg gtc ctg tcc ctg ttg atc tgc att ctg     96
   Val Met Cys Ala Val Ile Trp Val Leu Ser Leu Leu Ile Cys Ile Leu
               20                  25                  30
   aat agt tat ttc tgc ggt ttc tta aat acc caa tat aaa aat gaa aat     144
   Asn Ser Tyr Phe Cys Gly Phe Leu Asn Thr Gln Tyr Lys Asn Glu Asn
           35                  40                  45
   ggg tgt ctg gca ttg agc ttc ttt act gct gca tac ctg atg ttt ttg     192
   Gly Cys Leu Ala Leu Ser Phe Phe Thr Ala Ala Tyr Leu Met Phe Leu
       50                  55                  60
   ttt gtg gtc ctc tgt ctg tcc agc ctg gct ctg gtg gcc agg ttg ttc     240
   Phe Val Val Leu Cys Leu Ser Ser Leu Ala Leu Val Ala Arg Leu Phe
   65                  70                  75                  80
   tgt ggt gct agg aat atg aaa ttt acc aga tta ttc gtg acc atc atg     288
   Cys Gly Ala Arg Asn Met Lys Phe Thr Arg Leu Phe Val Thr Ile Met
                   85                  90                  95
   ctg acc gtt ttg gtt ttt ctt ctc tgt ggg ttg ccc tgg ggc atc acc     336
   Leu Thr Val Leu Val Phe Leu Leu Cys Gly Leu Pro Trp Gly Ile Thr
               100                 105                 110
   tgg ttc ctg tta ttc tgg att gca cct ggt gtg ttt gta cta gat tat     384
   Trp Phe Leu Leu Phe Trp Ile Ala Pro Gly Val Phe Val Leu Asp Tyr
           115                 120                 125
   agc cct ctt ctg gtc cta act gct att aac agc tgt gcc aac ccc att     432
   Ser Pro Leu Leu Val Leu Thr Ala Ile Asn Ser Cys Ala Asn Pro Ile
       130                 135                 140
   att tac ttc ttc gtc ggc                                             450
   Ile Tyr Phe Phe Val Gly
   145                 150
<210> 8
   <211> 150
   <212> PRT
   <213> Mus musculus
<400> 8
   Leu Cys Arg Ile Trp Tyr His Cys Arg Arg Pro Glu His Thr Ser Thr
    1               5                  10                  15
   Val Met Cys Ala Val Ile Trp Val Leu Ser Leu Leu Ile Cys Ile Leu
               20                  25                  30
   Asn Ser Tyr Phe Cys Gly Phe Leu Asn Thr Gln Tyr Lys Asn Glu Asn
           35                  40                  45
   Gly Cys Leu Ala Leu Ser Phe Phe Thr Ala Ala Tyr Leu Met Phe Leu
       50                  55                  60
   Phe Val Val Leu Cys Leu Ser Ser Leu Ala Leu Val Ala Arg Leu Phe
   65                  70                  75                  80
   Cys Gly Ala Arg Asn Met Lys Phe Thr Arg Leu Phe Val Thr Ile Met
                   85                  90                  95
   Leu Thr Val Leu Val Phe Leu Leu Cys Gly Leu Pro Trp Gly Ile Thr
               100                 105                 110
   Trp Phe Leu Leu Phe Trp Ile Ala Pro Gly Val Phe Val Leu Asp Tyr
           115                 120                 125
   Ser Pro Leu Leu Val Leu Thr Ala Ile Asn Ser Cys Ala Asn Pro Ile
       130                 135                 140
   Ile Tyr Phe Phe Val Gly
   145                 150
<210> 9
   <211> 459
   <212> DNA
   <213> Mus musculus
<220>
   <221> CDS
   <222> (1)...(459)
<400> 9
   ctg tgc ccg acg tgg tat cgc tgc cac cgt cca gta cat aca tca act      48
   Leu Cys Pro Thr Trp Tyr Arg Cys His Arg Pro Val His Thr Ser Thr
    1               5                  10                  15
   gtc atg tgt gct gtg atc tgg gtc cta tcc ctg ttg atc tgc att ctg      96
   Val Met Cys Ala Val Ile Trp Val Leu Ser Leu Leu Ile Cys Ile Leu
               20                  25                  30
   aat agc tat ttc tgt gct gtc tta cat acc aga tat gat aat gac aat      144
   Asn Ser Tyr Phe Cys Ala Val Leu His Thr Arg Tyr Asp Asn Asp Asn
           35                  40                  45
   gag tgt ctg gca act aac atc ttt acc gcc tcg tac atg ata ttt ttg      192
   Glu Cys Leu Ala Thr Asn Ile Phe Thr Ala Ser Tyr Met Ile Phe Leu
       50                  55                  60
   ctt gtg gtc ctc tgt ctg tcc agc ctg gct ctg ctg gcc agg ttg ttc      240
   Leu Val Val Leu Cys Leu Ser Ser Leu Ala Leu Leu Ala Arg Leu Phe
   65                  70                  75                  80
   tgt ggc gct ggg cag atg aag ctt acc aga ttt cat gtg acc atc ttg      288
   Cys Gly Ala Gly Gln Met Lys Leu Thr Arg Phe His Val Thr Ile Leu
                   85                  90                  95
   ctg acc ctt ttg gtt ttt ctc ctc tgc ggg ttg ccc ttt gtc atc tac      336
   Leu Thr Leu Leu Val Phe Leu Leu Cys Gly Leu Pro Phe Val Ile Tyr
               100                 105                 110
   tgc atc ctg tta ttc aag att aag gat gat ttc cat gta tta gat gtt      384
   Cys Ile Leu Leu Phe Lys Ile Lys Asp Asp Phe His Val Leu Asp Val
           115                 120                 125
   aat ctt tat cta gca tta gaa gtc ctg act gct att aac agc tgt gcc      432
   Asn Leu Tyr Leu Ala Leu Glu Val Leu Thr Ala Ile Asn Ser Cys Ala
       130                 135                 140
   aac ccc atc atc tac ttc ttc gtc gga                                  459
   Asn Pro Ile Ile Tyr Phe Phe Val Gly
   145                 150
<210> 10
   <211> 153
   <212> PRT
   <213> Mus musculus
<400> 10
   Leu Cys Pro Thr Trp Tyr Arg Cys His Arg Pro Val His Thr Ser Thr
    1               5                  10                  15
   Val Met Cys Ala Val Ile Trp Val Leu Ser Leu Leu Ile Cys Ile Leu
               20                  25                  30
   Asn Ser Tyr Phe Cys Ala Val Leu His Thr Arg Tyr Asp Asn Asp Asn
           35                  40                  45
   Glu Cys Leu Ala Thr Asn Ile Phe Thr Ala Ser Tyr Met Ile Phe Leu
       50                  55                  60
   Leu Val Val Leu Cys Leu Ser Ser Leu Ala Leu Leu Ala Arg Leu Phe
   65                  70                  75                  80
   Cys Gly Ala Gly Gln Met Lys Leu Thr Arg Phe His Val Thr Ile Leu
                   85                  90                  95
   Leu Thr Leu Leu Val Phe Leu Leu Cys Gly Leu Pro Phe Val Ile Tyr
               100                 105                 110
   Cys Ile Leu Leu Phe Lys Ile Lys Asp Asp Phe His Val Leu Asp Val
           115                 120                 125
   Asn Leu Tyr Leu Ala Leu Glu Val Leu Thr Ala Ile Asn Ser Cys Ala
       130                 135                 140
   Asn Pro Ile Ile Tyr Phe Phe Val Gly
   145                 150
<210> 11
   <211> 2853
   <212> DNA
   <213> Mus musculus
<220>
   <221> CDS
   <222> (1820)...(2734)
<400> 11
   caaggattct acaaacccaa gtatgcaagt caacaatcta aatataattt gttccttttg 60
   aagttagtgg ttcaatataa cagacaaata catcatgccc tgaaattagc tttgaacaat 120
   gctaagccca taatgggaag taaaagattt gcttggttcc cactttcttc cttttctatt 180
   ccgtttggac catagtggct agtgtctctt acaagatcac aagaaggagg ctctgcattt 240
   atttctgagt gcctgtctgc atcctccttt ggcctggagg tcctctatga aatcctgaag 300
   taagaaagaa atgttccaga ctctgatttt tcttcctaga ccaatgctat tcccttccat 360
   gttgccaaca acttctcatc actctttctg tactttcttt tagctgggtg gtttcttaat 420
   ctacagtatt gactgtcatg tcaaagttgg gtattttttg gctttagata tttcttctct 480
   ggcttttctc ccatccacac ataatcaaaa cactgaggtg atgacactaa gggactgctc 540
   aaaggaaaag ggtgggttcc tgggctttgg ggttattaat aatttgcctg tcctctgcca 600
   gcctctatca actcccctaa aacacaaaaa taattgttcc tagcaggcaa gcacgacctg 660
   acaattaatt aatgatcata aaaagtgcat tataaacatc tgaaaacctc ataataaaac 720
   tcaacacctt atacagtgag tatgttgtgg ggtctgcata aatccaacaa aactccaatg 780
   gagtggtact cagctattaa aaatgaggaa ttcacgaaat tcttagccaa atgattagaa 840
   gtagaaaata tgatcctgag tgagaaaaga acaggcttgg tatgtactca ctgataagtg 900
   gatactagcc caaaagctgc aaataatcag gataaaattc acagaccaca tgaacctcaa 960
   taagaaggaa gaccaaagta tgggcgtttc ggtccttctt agaaggagaa caaaatactc
   1020
   ccaagagcaa atatggagat aaagtgtaga acaggcacta aaggaaaagt cacccagaga
   1080
   atgttccacc tggggattca tcccatatac agttaccaaa cccagacact cttatggatg 1140
   ccaaggagtg aatgctgaca tagctgtttc ctaagaggcc atgccagaca cttacaaata 1200
   cagaggccca agttagcaac caaccattag actgagcaca gggttcctaa tagaggagtc 1260
   agagaaagga ctgagggagt tgaaggggtt tgcatcccca taagaaaaac aacaacatga 1320
   accaacaaga cactctcccc accaaccccc tgaactccta gggactaagc catcaacaaa 1380
   agagtacaca tggctccaga tgcatatgtt gcagaggatg gccatatcat gcattgatgg 1440
   aagaggtcct tgaacctatg aaggttctat tgatgcccca gtgtaaggga atcgagggca 1500
   gagaggtgga agtgggtgtg tgggttgagc aacaccctca cagaagcagg gggagggagg 1560
   atgagatggg ggtttccagg aaggggggaa gcaggaaagg ggataacatt ttaaatttaa 1620
   atatagaaaa tatccaatac aaaacatttt gaacaaacaa caaaaaactc acaaaaacaa 1680
   caacaacaaa aaaaagaaat taaaagttgt gttcatagtg aaggcctcat ttcttctttg 1740
   tgttcccagc aacaccagtg cagggtttct ggccctaaac acctcagcct cggcaatggc 1800
   acccacaaca acaaatcca atg aac gaa acc atc cct gga agt att gac atc 1852
   Met Asn Glu Thr Ile Pro Gly Ser Ile Asp Ile
   1 5 10
   gag acc ctg atc cca aac ttg atg atc atc atc ttc gga ctg gtc ggg 1900
   Glu Thr Leu Ile Pro Asn Leu Met Ile Ile Ile Phe Gly Leu Val Gly
   15 20 25
   ctg aca gga aat gtc att ttg ttt tgg ctc ctg ggc ttc cac ttg cac 1948
   Leu Thr Gly Asn Val Ile Leu Phe Trp Leu Leu Gly Phe His Leu His
   30 35 40
   agg aat gcc ttc tta gtc tac atc cta aac ttg gcc ctg gct gac ttc 1996
   Arg Asn Ala Phe Leu Val Tyr Ile Leu Asn Leu Ala Leu Ala Asp Phe
   45 50 55
   ctc ttc ctt ctc tgt cac atc ata aat tcc aca atg ctt ctt ctc aag 2044
   Leu Phe Leu Leu Cys His Ile Ile Asn Ser Thr Met Leu Leu Leu Lys
   60 65 70 75
   gtt cac cta ccc aac aat att ttg aac cat tgc ttt gac atc atc atg 2092
   Val His Leu Pro Asn Asn Ile Leu Asn His Cys Phe Asp Ile Ile Met
   80 85 90
   aca gtt ctc tac atc aca ggc ctg agc atg ctc agt gcc atc agc act 2140
   Thr Val Leu Tyr Ile Thr Gly Leu Ser Met Leu Ser Ala Ile Ser Thr
   95 100 105
   gag cgc tgc ctg tct gtc ctg tgc ccc atc tgg tat cgg tgc cgc cgc 2188
   Glu Arg Cys Leu Ser Val Leu Cys Pro Ile Trp Tyr Arg Cys Arg Arg
   110 115 120
   cca gaa cac aca tca act gtc ctg tgt gct gtg atc tgg ttc ctg ccc 2236
   Pro Glu His Thr Ser Thr Val Leu Cys Ala Val Ile Trp Phe Leu Pro
       125                 130                 135
   ctg ttg atc tgc att ctg aat gga tat ttc tgt cat ttc ttt ggt ccc
   2284
   Leu Leu Ile Cys Ile Leu Asn Gly Tyr Phe Cys His Phe Phe Gly Pro
   140                 145                 150                 155
   aaa tat gta att gac tct gtg tgt ctg gca acg aac ttc ttt atc aga
   2332
   Lys Tyr Val Ile Asp Ser Val Cys Leu Ala Thr Asn Phe Phe Ile Arg
                   160                 165                 170
   aca tac ccg atg ttt ttg ttt ata gtc ctc tgt ctg tcc acc ctg gct
   2380
   Thr Tyr Pro Met Phe Leu Phe Ile Val Leu Cys Leu Ser Thr Leu Ala
               175                 180                 185
   ctg ctg gcc agg ttg ttc tgt ggt ggt ggg aag acg aaa ttt acc aga
   2428
   Leu Leu Ala Arg Leu Phe Cys Gly Gly Gly Lys Thr Lys Phe Thr Arg
           190                 195                 200
   tta ttc gtg acc atc atg ctg acc gtt ttg gtt ttt ctt ctc tgt ggg
   2476
   Leu Phe Val Thr Ile Met Leu Thr Val Leu Val Phe Leu Leu Cys Gly
       205                 210                 215
   ttg ccc ctg ggc ttc ttc tgg ttt ctg gtg ccg tgg att aac cgt gat
   2524
   Leu Pro Leu Gly Phe Phe Trp Phe Leu Val Pro Trp Ile Asn Arg Asp
   220                 225                 230                 235
   ttc agt gta cta gat tat ata ctt ttt cag aca tca ctt gtc cta act
   2572
   Phe Ser Val Leu Asp Tyr Ile Leu Phe Gln Thr Ser Leu Val Leu Thr
                   240                 245                 250
   tct gtt aac agc tgt gcc aac ccc atc att tac ttc ttt gtg ggc tcc
   2620
   Ser Val Asn Ser Cys Ala Asn Pro Ile Ile Tyr Phe Phe Val Gly Ser
               255                 260                 265
   ttc agg cat cgg ttg aag cac aag acc ctc aaa atg gtt ctc cag agt
   2668
   Phe Arg His Arg Leu Lys His Lys Thr Leu Lys Met Val Leu Gln Ser
           270                 275                 280
   gca ttg cag gac act cct gag aca cct gaa aac atg gtg gag atg tca
   2716
   Ala Leu Gln Asp Thr Pro Glu Thr Pro Glu Asn Met Val Glu Met Ser
       285                 290                 295
   aga agc aaa gca gag ccg tgatgaagag cctctacctg gacctcagag
   2764
   Arg Ser Lys Ala Glu Pro
   300                 305
   gtggctttgg attgagcact gccctgctgc acttgaccac tgtccactct cctctcagct
   2824
   tactgacttt ggatgcctca gtggtccaa
   2853
<210> 12
   <211> 305
   <212> PRT
   <213> Mus musculus
<400> 12
   Met Asn Glu Thr Ile Pro Gly Ser Ile Asp Ile Glu Thr Leu Ile Pro
    1               5                  10                  15
   Asn Leu Met Ile Ile Ile Phe Gly Leu Val Gly Leu Thr Gly Asn Val
               20                  25                  30
   Ile Leu Phe Trp Leu Leu Gly Phe His Leu His Arg Asn Ala Phe Leu
           35                  40                  45
   Val Tyr Ile Leu Asn Leu Ala Leu Ala Asp Phe Leu Phe Leu Leu Cys
       50                  55                  60
   His Ile Ile Asn Ser Thr Met Leu Leu Leu Lys Val His Leu Pro Asn
   65                  70                  75                  80
   Asn Ile Leu Asn His Cys Phe Asp Ile Ile Met Thr Val Leu Tyr Ile
                   85                  90                  95
   Thr Gly Leu Ser Met Leu Ser Ala Ile Ser Thr Glu Arg Cys Leu Ser
               100                 105                 110
   Val Leu Cys Pro Ile Trp Tyr Arg Cys Arg Arg Pro Glu His Thr Ser
           115                 120                 125
   Thr Val Leu Cys Ala Val Ile Trp Phe Leu Pro Leu Leu Ile Cys Ile
       130                 135                 140
   Leu Asn Gly Tyr Phe Cys His Phe Phe Gly Pro Lys Tyr Val Ile Asp
   145                 150                 155                 160
   Ser Val Cys Leu Ala Thr Asn Phe Phe Ile Arg Thr Tyr Pro Met Phe
                   165                 170                 175
   Leu Phe Ile Val Leu Cys Leu Ser Thr Leu Ala Leu Leu Ala Arg Leu
               180                 185                 190
   Phe Cys Gly Gly Gly Lys Thr Lys Phe Thr Arg Leu Phe Val Thr Ile
           195                 200                 205
   Met Leu Thr Val Leu Val Phe Leu Leu Cys Gly Leu Pro Leu Gly Phe
       210                 215                 220
   Phe Trp Phe Leu Val Pro Trp Ile Asn Arg Asp Phe Ser Val Leu Asp
   225                 230                 235                 240
   Tyr Ile Leu Phe Gln Thr Ser Leu Val Leu Thr Ser Val Asn Ser Cys
                   245                 250                 255
   Ala Asn Pro Ile Ile Tyr Phe Phe Val Gly Ser Phe Arg His Arg Leu
               260                 265                 270
   Lys His Lys Thr Leu Lys Met Val Leu Gln Ser Ala Leu Gln Asp Thr
           275                 280                 285
   Pro Glu Thr Pro Glu Asn Met Val Glu Met Ser Arg Ser Lys Ala Glu
       290                 295                 300
   Pro
   305
<210> 13
   <211> 3391
   <212> DNA
   <213> Mus musculus
<220>
   <221> CDS
   <222> (170)...(574)
<400> 13
   ccgaaaacca acaaaataga accgcgggtg cctttctcca gctgggatga aggacttgag 60
   cagaaactca ttgccagctt cctccctacg cgagagccga ctgagtccca ggtccccagt 120
   cttcccccgg gacgttgtgc acggtgccca ttcttgagca gccacaaca atg gag gtg 178
                                                         Met Glu Val
                                                          1
   ctc ccc aag gcc ctg gag gta gac gag agg tct cca gag tcc aag gac   226
   Leu Pro Lys Ala Leu Glu Val Asp Glu Arg Ser Pro Glu Ser Lys Asp
       5                   10                  15
   ctg ctg ccc agc cag aca gcc agc tcc ctg tgc atc agt tcc aga agt   274
   Leu Leu Pro Ser Gln Thr Ala Ser Ser Leu Cys Ile Ser Ser Arg Ser
    20                 25                  30                  35
   gag tct gtc tgg acc acc aca ccc aaa agc aac tgg gaa atc tac cac    322
   Glu Ser Val Trp Thr Thr Thr Pro Lys Ser Asn Trp Glu Ile Tyr His
                   40                  45                  50
   aag ccc atc atc atc atg tca gtg gga gct gcc att ctg ctc ttt ggc    370
   Lys Pro Ile Ile Ile Met Ser Val Gly Ala Ala Ile Leu Leu Phe Gly
               55                  60                  65
   gtg gcc atc acc tgt gtg gcc tac atc ttg gaa gag aag cat aaa gtt    418
   Val Ala Ile Thr Cys Val Ala Tyr Ile Leu Glu Glu Lys His Lys Val
           70                  75                  80
   gtg caa gtg ctc agg atg ata ggg cct gcc ttc ctg tcc ctg gga ctc    466
   Val Gln Val Leu Arg Met Ile Gly Pro Ala Phe Leu Ser Leu Gly Leu
       85                  90                  95
   atg atg ctg gtg tgt ggg ctg gtg tgg gtc ccc ata atc aaa aag aag    514
   Met Met Leu Val Cys Gly Leu Val Trp Val Pro Ile Ile Lys Lys Lys
   100                 105                 110                 115
   cag aag caa agg cag aag tcc aac ttc ttc caa agc ctc aag ttc ttc    562
   Gln Lys Gln Arg Gln Lys Ser Asn Phe Phe Gln Ser Leu Lys Phe Phe
                   120                 125                 130
   ctc ctg aac cgc tgatgactgg ttgtccagaa gatctgctaa ccaataagca        614
   Leu Leu Asn Arg
               135
   gcctcctacc ttctcttcgg gtaccacaaa gttgatccag gcaaaccctc ctcttggccc 674
   tgtggacagg atagagctca gggcttcacc ctcatacaac ctagcagcat tgctgactga 734
   gtctcacctg gtttccatag ctgtggatgc tgtgcccttg gatactttca ttaccctcat 794
   ccctggcacc tgcattcagc catcagccat cccattctct ctgccaaggg caatgtgtgc 854
   atgctaggaa attctttggg ggttgactac attcccaagg agaacttgta tgttacggtt 914
   gtgtgcctga tcttagattc ccatctacat ccttctggaa ccaaaagtga ccaagcagat 974
   aaggctgact tcagtcccat tgggtttgac agccttggct ccctccttgg atgggacatt
   1034
   gactaacatt acaagagaaa ggatatgtct catgtatcac acattccaaa atctggacag
   1094
   tgatggggct gggggtgagg gaaacactgt ctagagtaaa ccattcctct gggagtaatc
   1154
   tggaacttat acagtgaagg aagttagctc ctaaatatat gatattggca caagaggcaa
   1214
   tatgcaggct aagaggtatc aacacttccc cttgatcctc caatgcgctt cttgcagaat
   1274
   gcctttatat tagcaattag ccaagaacaa atgctctttg ttctaacttc cttccccacc
   1334
   acatctctgc gtctacacag ctccagaaca gaaggacggg aggccacaga tgtgacctgt
   1394
   aagatcatct ccttctcctg tcaatcaaga cctaacctga aattgaatgc catgtccgac
   1454
   tcacgctgca tggggtttta gagataggtt cactggaaaa aaggaaatct cagcctccct
   1514
   cctccctgtt cctccctacc aaacaagcaa gtatttattg agtttccttc tctaggccta
   1574
   cgttgggaac agccagaccc agtctctgat gtcatcttat ttccaaaagt gaaagaggga
   1634
   aaaacatggc caagccaact ggcaatactc catactgagt tcttagggtg gccatgggaa
   1694
   cacatggatc taacaaatgt acaggaagat agatttctgg agaccatgtt caccccttct
   1754
   gaatatgaag gggaaggaag tgtttggaat gagcaagatg tgcaaggtag tcagcaactg
   1814
   ccttgcatgt ggagaagcta aggggaaaga gacagggtgg ggttaggatt ccgcatagct
   1874
   cccggatgct attccatcct ctcttgccta cttcccccct gcttccccag gtaccttaca
   1934
   tccagctact ccttggtaca ctgcaggctt ctggggtcaa tagggactgg gaggggcatc
   1994
   tccagagggc ctaacaagta gatataaccc aagaggtaag taccctcaaa acttcattat
   2054
   agtcaccaag acacctttag gcaaaagacc gggcacctat aagaaatttc caaagctgtt
   2114
   ccaggcaagg ccaggccaga gagcagagga aggtacctag tagcaaagtg aatgacaaga
   2174
   gctgcattgg ttcaggttga ctcttcatcc ttaacctttg ggcatttggg aacactatgg
   2234
   caaacaacct ccaacaggtc tccagatatc tcaaccattc acagtacttc tataggcagt
   2294
   tagaatccac cacctttgtt cctgttgcat tgtgggacat tcctcggagg aagtatttgt
   2354
   tttgtggaat caacacacac acacacacgc acagagagag agagagagag agagagagag
   2414
   agagagagag agagagagag agagaaagaa agagaaagaa agaaagaaag agaaagagac
   2474
   tgactcccta actaaaaagt cagagtttgg gaagcctgtg gcctttcaaa gctcacttaa
   2534
   gaatatcatg ttcctcatta agactcacat catcgagccc aggccctgca gtccacccat
   2594
   tccctgaata caggcagctc aggaccaacc ctggggttgt tgaaatactg cctagtgctt
   2654
   ccacgaatgt ctaatgcctc catgacaggg ctttcagacc actcctttct cctgacatgg
   2714
   aaggacagcc ctggggtgga gcctctcaat cttctgtgcc ttcatgaaag ggaacacaca
   2774
   gatgagctca cagccagctc acttggaatc cgcaccccat gcacctcatt gtcctgagag
   2834
   ctcattgtct gggcacagct gtgggaagac ctttgcagat ctcactttca agtatgtctc
   2894
   aacagaaggg agtttgggga taatcacgat gccaggaaat cttcaagttc tagacatctt
   2954
   tcatagccac atcagtacct gttccccaac ccctgcccct caaggtaagt acttagcaaa
   3014
   caaaatcaaa gagcctttga gaaaatatcc caaatactgg ttaactcccc cggccttgca
   3074
   ccaaactccc cacaaaagtg atagtcagga agtgagcaga gtcacaccca acatcttgga
   3134
   aaattttgcc aaagaccatt gcctcatgaa aactggggtg gggataacct gtgagtgcag
   3194
   ccgggttgga tgccgtgtct ctgcaacaaa gcattctggg tagtgatttc agtcatctca
   3254
   gaagacaaga gcaacatcca cagcaccatc ccaccggact gtattacggg cttctgtcgc
   3314
   tcttctgttt tggagaattt aatctaaccc aacgcctaat ggaatcaatg tcgtattgaa
   3374
   ctgtattctg tttaaaa
   3391
<210> 14
   <211> 135
   <212> PRT
   <213> Mus musculus
<400> 14
   Met Glu Val Leu Pro Lys Ala Leu Glu Val Asp Glu Arg Ser Pro Glu
    1               5                  10                  15
   Ser Lys Asp Leu Leu Pro Ser Gln Thr Ala Ser Ser Leu Cys Ile Ser
               20                  25                  30
   Ser Arg Ser Glu Ser Val Trp Thr Thr Thr Pro Lys Ser Asn Trp Glu
           35                  40                  45
   Ile Tyr His Lys Pro Ile Ile Ile Met Ser Val Gly Ala Ala Ile Leu
       50                  55                  60
   Leu Phe Gly Val Ala Ile Thr Cys Val Ala Tyr Ile Leu Glu Glu Lys
   65                  70                  75                  80
   His Lys Val Val Gln Val Leu Arg Met Ile Gly Pro Ala Phe Leu Ser
                   85                  90                  95
   Leu Gly Leu Met Met Leu Val Cys Gly Leu Val Trp Val Pro Ile Ile
               100                 105                 110
   Lys Lys Lys Gln Lys Gln Arg Gln Lys Ser Asn Phe Phe Gln Ser Leu
           115                 120                 125
   Lys Phe Phe Leu Leu Asn Arg
       130                 135
<210> 15
   <211> 2040
   <212> DNA
   <213> Homo sapiens
<220>
   <221> CDS
   <222> (328)...(1293)
<400> 15
   gcccaggata gagtaatcat cgggtccaca gccctggcta gatgagtggg ggtgttttga 60
   tcctaatgtt attcccatgt tagcacagaa cttgtgtggc agtagagaga ggtcaggctt 120
   cagagtcagc aagaactgga tttcaaactg gatttgagga cccccacctt ttgataggtg 180
   acttattctc tgtgagtctc tgatctgccc tctttaaatg aggaagtaaa tcccacatgg 240
   cagggtggtg gggagaatca gagatcatac agctggtgat cacaactggt ttctgtttcc 300
   agggtcacca gactagggtt tctgagc atg gat cca acc atc tca acc ttg gac 354
                                 Met Asp Pro Thr Ile Ser Thr Leu Asp
                                  1               5
   aca gaa ctg aca cca atc aac gga act gag gag act ctt tgc tac aag   402
   Thr Glu Leu Thr Pro Ile Asn Gly Thr Glu Glu Thr Leu Cys Tyr Lys
    10                 15                  20                  25
   cag acc ttg agc ctc acg gtg ctg acg tgc atc gtt tcc ctt gtc ggg   450
   Gln Thr Leu Ser Leu Thr Val Leu Thr Cys Ile Val Ser Leu Val Gly
                       30                  35                  40
   ctg aca gga aac gca gtt gtg ctc tgg ctc ctg ggc tgc cgc atg cgc   498
   Leu Thr Gly Asn Ala Val Val Leu Trp Leu Leu Gly Cys Arg Met Arg
               45                  50                  55
   agg aac gcc ttc tcc atc tac atc ctc aac ttg gcc gca gca gac ttc   546
   Arg Asn Ala Phe Ser Ile Tyr Ile Leu Asn Leu Ala Ala Ala Asp Phe
           60                  65                  70
   ctc ttc ctc agc ggc cgc ctt ata tat tcc ctg tta agc ttc atc agt   594
   Leu Phe Leu Ser Gly Arg Leu Ile Tyr Ser Leu Leu Ser Phe Ile Ser
       75                  80                  85
   atc ccc cat acc atc tct aaa atc ctc tat cct gtg atg atg ttt tcc   642
   Ile Pro His Thr Ile Ser Lys Ile Leu Tyr Pro Val Met Met Phe Ser
    90                 95                 100                 105
   tac ttt gca ggc ctg agc ttt ctg agt gcc gtg agc acc gag cgc tgc   690
   Tyr Phe Ala Gly Leu Ser Phe Leu Ser Ala Val Ser Thr Glu Arg Cys
                   110                 115                 120
   ctg tcc gtc ctg tgg ccc atc tgg tac cgc tgc cac cgc ccc aca cac   738
   Leu Ser Val Leu Trp Pro Ile Trp Tyr Arg Cys His Arg Pro Thr His
               125                 130                 135
   ctg tca gcg gtg gtg tgt gtc ctg ctc tgg gcc ctg tcc ctg ctg cgg   786
   Leu Ser Ala Val Val Cys Val Leu Leu Trp Ala Leu Ser Leu Leu Arg
           140                 145                 150
   agc atc ctg gag tgg atg tta tgt ggc ttc ctg ttc agt ggt gct gat     834
   Ser Ile Leu Glu Trp Met Leu Cys Gly Phe Leu Phe Ser Gly Ala Asp
       155                 160                 165
   tct gct tgg tgt caa aca tca gat ttc atc aca gtc gcg tgg ctg att     882
   Ser Ala Trp Cys Gln Thr Ser Asp Phe Ile Thr Val Ala Trp Leu Ile
   170                 175                 180                 185
   ttt tta tgt gtg gtt ctc tgt ggg tcc agc ctg gtc ctg ctg atc agg     930
   Phe Leu Cys Val Val Leu Cys Gly Ser Ser Leu Val Leu Leu Ile Arg
                   190                 195                 200
   att ctc tgt gga tcc cgg aag ata ccg ctg acc agg ctg tac gtg acc     978
   Ile Leu Cys Gly Ser Arg Lys Ile Pro Leu Thr Arg Leu Tyr Val Thr
               205                 210                 215
   atc ctg ctc aca gta ctg gtc ttc ctc ctc tgt ggc ctg ccc ttt ggc
   1026
   Ile Leu Leu Thr Val Leu Val Phe Leu Leu Cys Gly Leu Pro Phe Gly
           220                 225                 230
   att cag ttt ttc cta ttt tta tgg atc cac gtg gac agg gaa gtc tta
   1074
   Ile Gln Phe Phe Leu Phe Leu Trp Ile His Val Asp Arg Glu Val Leu
       235                 240                 245
   ttt tgt cat gtt cat cta gtt tct att ttc ctg tcc gct ctt aac agc
   1122
   Phe Cys His Val His Leu Val Ser Ile Phe Leu Ser Ala Leu Asn Ser
   250                 255                 260                 265
   agt gcc aac ccc atc att tac ttc ttc gtg ggc tcc ttt agg cag cgt
   1170
   Ser Ala Asn Pro Ile Ile Tyr Phe Phe Val Gly Ser Phe Arg Gln Arg
                   270                 275                 280
   caa aat agg cag aac ctg aag ctg gtt ctc cag agg gct ctg cag gac
   1218
   Gln Asn Arg Gln Asn Leu Lys Leu Val Leu Gln Arg Ala Leu Gln Asp
               285                 290                 295
   gcg tct gag gtg gat gaa ggt gga ggg cag ctt cct gag gaa atc ctg
   1266
   Ala Ser Glu Val Asp Glu Gly Gly Gly Gln Leu Pro Glu Glu Ile Leu
           300                 305                 310
   gag ctg tcg gga agc aga ttg gag cag tgaggaagag cctctgccct
   1313
   Glu Leu Ser Gly Ser Arg Leu Glu Gln
       315                 320
   gtcagacagg actttgagag caacactgcc ctgccaccct tgacaattat atgcgttttt
   1373
   cttagccttc tgcctcagaa atgtctcagt ggttcctcaa ggtcttcaaa tagatgttta
   1433
   tctaacctga cagttgcggt tttcacccat ggaaagcatt agtctgacag tacaatgttt
   1493
   agattctcct tgatattacc aacacatttt ccctgttatc tcacactgaa tctttcctac
   1553
   agaacacttt ttctgcaatt ttctttgtaa taaaaggagt tcctgtacaa aaccctaaaa
   1613
   cactctttat acttctttcc tacctgatag catcaaaaag gaagattcct tattaatctc
   1673
   tcagactatg ttcccctgaa aatcatgttc ccttctatga ctggaggcat tactgcagtt 1733
   agaagctcga ttcttaataa gtgagttctg ctatctctac attccattga attctcagat 1793
   acagagcaaa ataatgtcct tagagacaga ctctctcttc ataaaaacac tctcacctat 1853
   tggttttata aaaagtcttc ccctgtcatt tgttcacagc atggtgatat gttggccttg 1913
   gtttctagta aagacaactg tggccccttc cccttgagaa cttttaagtg cttatttagc 1973
   tcttcctgga ctaatggacc agtgaggagc ccataaatgt gccccagttc tattttggcc 2033
   attggaa 2040
<210> 16
   <211> 322
   <212> PRT
   <213> Homo sapiens
<400> 16
   Met Asp Pro Thr Ile Ser Thr Leu Asp Thr Glu Leu Thr Pro Ile Asn
    1               5                  10                  15
   Gly Thr Glu Glu Thr Leu Cys Tyr Lys Gln Thr Leu Ser Leu Thr Val
               20                  25                  30
   Leu Thr Cys Ile Val Ser Leu Val Gly Leu Thr Gly Asn Ala Val Val
           35                  40                  45
   Leu Trp Leu Leu Gly Cys Arg Met Arg Arg Asn Ala Phe Ser Ile Tyr
       50                  55                  60
   Ile Leu Asn Leu Ala Ala Ala Asp Phe Leu Phe Leu Ser Gly Arg Leu
   65                  70                  75                  80
   Ile Tyr Ser Leu Leu Ser Phe Ile Ser Ile Pro His Thr Ile Ser Lys
                   85                  90                  95
   Ile Leu Tyr Pro Val Met Met Phe Ser Tyr Phe Ala Gly Leu Ser Phe
               100                 105                 110
   Leu Ser Ala Val Ser Thr Glu Arg Cys Leu Ser Val Leu Trp Pro Ile
           115                 120                 125
   Trp Tyr Arg Cys His Arg Pro Thr His Leu Ser Ala Val Val Cys Val
       130                 135                 140
   Leu Leu Trp Ala Leu Ser Leu Leu Arg Ser Ile Leu Glu Trp Met Leu
   145                 150                 155                 160
   Cys Gly Phe Leu Phe Ser Gly Ala Asp Ser Ala Trp Cys Gln Thr Ser
                   165                 170                 175
   Asp Phe Ile Thr Val Ala Trp Leu Ile Phe Leu Cys Val Val Leu Cys
               180                 185                 190
   Gly Ser Ser Leu Val Leu Leu Ile Arg Ile Leu Cys Gly Ser Arg Lys
           195                 200                 205
   Ile Pro Leu Thr Arg Leu Tyr Val Thr Ile Leu Leu Thr Val Leu Val
       210                 215                 220
   Phe Leu Leu Cys Gly Leu Pro Phe Gly Ile Gln Phe Phe Leu Phe Leu
   225                 230                 235                 240
   Trp Ile His Val Asp Arg Glu Val Leu Phe Cys His Val His Leu Val
                   245                 250                 255
   Ser Ile Phe Leu Ser Ala Leu Asn Ser Ser Ala Asn Pro Ile Ile Tyr
               260                 265                 270
   Phe Phe Val Gly Ser Phe Arg Gln Arg Gln Asn Arg Gln Asn Leu Lys
           275                 280                 285
   Leu Val Leu Gln Arg Ala Leu Gln Asp Ala Ser Glu Val Asp Glu Gly
       290                 295                 300
   Gly Gly Gln Leu Pro Glu Glu Ile Leu Glu Leu Ser Gly Ser Arg Leu
   305                 310                 315                 320
   Glu Gln
<210> 17
   <211> 1300
   <212> DNA
   <213> Homo sapiens
<220>
   <221> CDS
   <222> (171)...(1160)
<400> 17
   tccctggccc ttaataaatg acttaatctc ttcaagcctc tgatttcctc tcctgtaaaa 60
   caggggcggt aattaccaca taacaggctg gtcatgaaaa tcagtgaaca tgcagcaggt 120
   gctcaagtct tgtttttgtt tccaggggca ccagtggagg ttttctgagc atg gat    176
                                                         Met Asp
                                                          1
   cca acc acc ccg gcc tgg gga aca gaa agt aca aca gtg aat gga aat   224
   Pro Thr Thr Pro Ala Trp Gly Thr Glu Ser Thr Thr Val Asn Gly Asn
           5                   10                  15
   gac caa gcc ctt ctt ctg ctt tgt ggc aag gag acc ctg atc ccg gtc   272
   Asp Gln Ala Leu Leu Leu Leu Cys Gly Lys Glu Thr Leu Ile Pro Val
       20                  25                  30
   ttc ctg atc ctt ttc att gcc ctg gtc ggg ctg gta gga aac ggg ttt   320
   Phe Leu Ile Leu Phe Ile Ala Leu Val Gly Leu Val Gly Asn Gly Phe
   35                  40                  45                  50
   gtg ctc tgg ctc ctg ggc ttc cgc atg cgc agg aac gcc ttc tct gtc   368
   Val Leu Trp Leu Leu Gly Phe Arg Met Arg Arg Asn Ala Phe Ser Val
                   55                  60                  65
   tac gtc ctc agc ctg gcc ggg gcc gac ttc ctc ttc ctc tgc ttc cag   416
   Tyr Val Leu Ser Leu Ala Gly Ala Asp Phe Leu Phe Leu Cys Phe Gln
               70                  75                  80
   att ata aat tgc ctg gtg tac ctc agt aac ttc ttc tgt tcc atc tcc   464
   Ile Ile Asn Cys Leu Val Tyr Leu Ser Asn Phe Phe Cys Ser Ile Ser
           85                  90                  95
   atc aat ttc cct agc ttc ttc acc act gtg atg acc tgt gcc tac ctt   512
   Ile Asn Phe Pro Ser Phe Phe Thr Thr Val Met Thr Cys Ala Tyr Leu
       100                 105                 110
   gca ggc ctg agc atg ctg agc acc gtc agc acc gag cgc tgc ctg tcc   560
   Ala Gly Leu Ser Met Leu Ser Thr Val Ser Thr Glu Arg Cys Leu Ser
   115                 120                 125                 130
   gtc ctg tgg ccc atc tgg tat cgc tgc cgc cgc ccc aga cac ctg tca   608
   Val Leu Trp Pro Ile Trp Tyr Arg Cys Arg Arg Pro Arg His Leu Ser
                   135                 140                 145
   gcg gtc gtg tgt gtc ctg ctc tgg gcc ctg tcc cta ctg ctg agc atc   656
   Ala Val Val Cys Val Leu Leu Trp Ala Leu Ser Leu Leu Leu Ser Ile
               150                 155                 160
   ttg gaa ggg aag ttc tgt ggc ttc tta ttt agt gat ggt gac tct ggt   704
   Leu Glu Gly Lys Phe Cys Gly Phe Leu Phe Ser Asp Gly Asp Ser Gly
           165                 170                 175
   tgg tgt cag aca ttt gat ttc atc act gca gcg tgg ctg att ttt tta   752
   Trp Cys Gln Thr Phe Asp Phe Ile Thr Ala Ala Trp Leu Ile Phe Leu
       180                 185                 190
   ttc atg gtt ctc tgt ggg tcc agt ctg gcc ctg ctg gtc agg atc ctc   800
   Phe Met Val Leu Cys Gly Ser Ser Leu Ala Leu Leu Val Arg Ile Leu
   195                 200                 205                 210
   tgt ggc tcc agg ggt ctg cca ctg acc agg ctg tac ctg acc atc ctg      848
   Cys Gly Ser Arg Gly Leu Pro Leu Thr Arg Leu Tyr Leu Thr Ile Leu
                   215                 220                 225
   ctc aca gtg ctg gtg ttc ctc ctc tgc ggc ctg ccc ttt ggc att cag      896
   Leu Thr Val Leu Val Phe Leu Leu Cys Gly Leu Pro Phe Gly Ile Gln
               230                 235                 240
   tgg ttc cta ata tta tgg atc tgg aag gat tct gat gtc tta ttt tgt      944
   Trp Phe Leu Ile Leu Trp Ile Trp Lys Asp Ser Asp Val Leu Phe Cys
           245                 250                 255
   cat att cat cca gtt tca gtt gtc ctg tca tct ctt aac agc agt gcc      992
   His Ile His Pro Val Ser Val Val Leu Ser Ser Leu Asn Ser Ser Ala
       260                 265                 270
   aac ccc atc att tac ttc ttc gtg ggc tct ttt agg aag cag tgg cgg
   1040
   Asn Pro Ile Ile Tyr Phe Phe Val Gly Ser Phe Arg Lys Gln Trp Arg
   275                 280                 285                 290
   ctg cag cag ccg atc ctc aag ctg gct ctc cag agg gct ctg cag gac
   1088
   Leu Gln Gln Pro Ile Leu Lys Leu Ala Leu Gln Arg Ala Leu Gln Asp
                   295                 300                 305
   att gct gag gtg gat cac agt gaa gga tgc ttc cgt cag ggc acc ccg
   1136
   Ile Ala Glu Val Asp His Ser Glu Gly Cys Phe Arg Gln Gly Thr Pro
               310                 315                 320
   gag atg tcg aga agc agt ctg gtg tagagatgga cagcctctac ttccatcaga
   1190
   Glu Met Ser Arg Ser Ser Leu Val
           325                 330
   tatatgtggc tttgagaggc aactttgccc ctgtctgtct gatttgctga actttctcag
   1250
   tcctgatttt aaaacagtta agagagtcct tgtgaggatt aagtgagaca
   1300
<210> 18
   <211> 330
   <212> PRT
   <213> Homo sapiens
<400> 18
   Met Asp Pro Thr Thr Pro Ala Trp Gly Thr Glu Ser Thr Thr Val Asn
    1               5                  10                  15
   Gly Asn Asp Gln Ala Leu Leu Leu Leu Cys Gly Lys Glu Thr Leu Ile
               20                  25                  30
   Pro Val Phe Leu Ile Leu Phe Ile Ala Leu Val Gly Leu Val Gly Asn
           35                  40                  45
   Gly Phe Val Leu Trp Leu Leu Gly Phe Arg Met Arg Arg Asn Ala Phe
       50                  55                  60
   Ser Val Tyr Val Leu Ser Leu Ala Gly Ala Asp Phe Leu Phe Leu Cys
   65                  70                  75                  80
   Phe Gln Ile Ile Asn Cys Leu Val Tyr Leu Ser Asn Phe Phe Cys Ser
                   85                  90                  95
   Ile Ser Ile Asn Phe Pro Ser Phe Phe Thr Thr Val Met Thr Cys Ala
               100                 105                 110
   Tyr Leu Ala Gly Leu Ser Met Leu Ser Thr Val Ser Thr Glu Arg Cys
           115                 120                 125
   Leu Ser Val Leu Trp Pro Ile Trp Tyr Arg Cys Arg Arg Pro Arg His
       130                 135                 140
   Leu Ser Ala Val Val Cys Val Leu Leu Trp Ala Leu Ser Leu Leu Leu
   145                 150                 155                 160
   Ser Ile Leu Glu Gly Lys Phe Cys Gly Phe Leu Phe Ser Asp Gly Asp
                   165                 170                 175
   Ser Gly Trp Cys Gln Thr Phe Asp Phe Ile Thr Ala Ala Trp Leu Ile
               180                 185                 190
   Phe Leu Phe Met Val Leu Cys Gly Ser Ser Leu Ala Leu Leu Val Arg
           195                 200                 205
   Ile Leu Cys Gly Ser Arg Gly Leu Pro Leu Thr Arg Leu Tyr Leu Thr
       210                 215                 220
   Ile Leu Leu Thr Val Leu Val Phe Leu Leu Cys Gly Leu Pro Phe Gly
   225                 230                 235                 240
   Ile Gln Trp Phe Leu Ile Leu Trp Ile Trp Lys Asp Ser Asp Val Leu
                   245                 250                 255
   Phe Cys His Ile His Pro Val Ser Val Val Leu Ser Ser Leu Asn Ser
               260                 265                 270
   Ser Ala Asn Pro Ile Ile Tyr Phe Phe Val Gly Ser Phe Arg Lys Gln
           275                 280                 285
   Trp Arg Leu Gln Gln Pro Ile Leu Lys Leu Ala Leu Gln Arg Ala Leu
       290                 295                 300
   Gln Asp Ile Ala Glu Val Asp His Ser Glu Gly Cys Phe Arg Gln Gly
   305                 310                 315                 320
   Thr Pro Glu Met Ser Arg Ser Ser Leu Val
                   325                 330
<210> 19
   <211> 135
   <212> PRT
   <213> Homo sapiens
<400> 19
<210> 20
   <211> 970
   <212> DNA
   <213> Mus musculus
<220>
   <221> CDS
   <222> (83)...(943)
<400> 20
   gtgtcaccaa cagcacccac aacaaatcca atggacaaac ctctttggaa gtatggacat 60
   ctggattctg acccgaaact ag atg atc atc ata ttc aga ctg gtt ggg atg  112
                            Met Ile Ile Ile Phe Arg Leu Val Gly Met
                             1               5                  10
   aca gga aat gcc att gtg ttc tgg ctc ctg ggc ttc agc ttg cac agg      160
   Thr Gly Asn Ala Ile Val Phe Trp Leu Leu Gly Phe Ser Leu His Arg
                    15                  20                  25
   aat gcc ttc tca gtc tac att tta aac ttg gcc ctt gct gac ttc gtc      208
   Asn Ala Phe Ser Val Tyr Ile Leu Asn Leu Ala Leu Ala Asp Phe Val
               30                  35                  40
   ttc ctc ctc tgt cac atc ata gat tcc atg ctg ctt ctt ctc act gtt      256
   Phe Leu Leu Cys His Ile Ile Asp Ser Met Leu Leu Leu Leu Thr Val
           45                  50                  55
   ttc tac ccc aac aat atc ttt tct ggg tac ttt tac acc atc atg acg      304
   Phe Tyr Pro Asn Asn Ile Phe Ser Gly Tyr Phe Tyr Thr Ile Met Thr
       60                  65                  70
   gtt ccc tac atc gca ggc ctg agc atg ctc agt gcc atc agc act gag      352
   Val Pro Tyr Ile Ala Gly Leu Ser Met Leu Ser Ala Ile Ser Thr Glu
   75                  80                  85                  90
   ctc tgc ctg tct gtc ctg tgc ccc atc tgg tat cgc tgc cac cac cca      400
   Leu Cys Leu Ser Val Leu Cys Pro Ile Trp Tyr Arg Cys His His Pro
                   95                  100                 105
   gaa cac aca tca act gtc atg tgt gct gcg ata tgg gtc ctg ccc ctg      448
   Glu His Thr Ser Thr Val Met Cys Ala Ala Ile Trp Val Leu Pro Leu
               110                 115                 120
   ttg gtc tgc att ctg aat agg tat ttc tgc agt ttc tta gat atc aat      496
   Leu Val Cys Ile Leu Asn Arg Tyr Phe Cys Ser Phe Leu Asp Ile Asn
           125                 130                 135
   tat aac aat gac aaa cag tgt ctg gca tca aac ttc ttt act aga gca      544
   Tyr Asn Asn Asp Lys Gln Cys Leu Ala Ser Asn Phe Phe Thr Arg Ala
       140                 145                 150
   tac ctg atg ttt ttg ttt gtg gtc ctt tgt ctg tcc agc atg gct ctg      592
   Tyr Leu Met Phe Leu Phe Val Val Leu Cys Leu Ser Ser Met Ala Leu
   155                 160                 165                 170
   ctg gcc agg ttg ttc tgt ggc act ggg cag atg aag ctt acc aga ttg      640
   Leu Ala Arg Leu Phe Cys Gly Thr Gly Gln Met Lys Leu Thr Arg Leu
                   175                 180                 185
   tac gtg acc atc atg ctg act gtt ttg ggt ttt ctc ctc tgt ggg ttg      688
   Tyr Val Thr Ile Met Leu Thr Val Leu Gly Phe Leu Leu Cys Gly Leu
               190                 195                 200
   ccc ttt gtc atc tac tac ttc ctg tta ttc aat att aag gat ggt ttt      736
   Pro Phe Val Ile Tyr Tyr Phe Leu Leu Phe Asn Ile Lys Asp Gly Phe
           205                 210                 215
   tgt tta ttt gat ttt aga ttt tat atg tca aca cat gtc ctg act gct      784
   Cys Leu Phe Asp Phe Arg Phe Tyr Met Ser Thr His Val Leu Thr Ala
       220                 225                 230
   att aac aac tgt gcc aac ccc ata att tac ttt ttc gag ggc tcc ttc      832
   Ile Asn Asn Cys Ala Asn Pro Ile Ile Tyr Phe Phe Glu Gly Ser Phe
   235                 240                 245                 250
   agg cat cag ttg aag cac cag acc ctc aaa atg gtt ctc cag agt gta      880
   Arg His Gln Leu Lys His Gln Thr Leu Lys Met Val Leu Gln Ser Val
                   255                 260                 265
   ctg cag gac act cct gag ata gct gaa aat atg gtg gag atg tca aga      928
   Leu Gln Asp Thr Pro Glu Ile Ala Glu Asn Met Val Glu Met Ser Arg
               270                 275                     280
   aac ata cca aag cca tgatgaaaag cctttgcctg gacctca                   970
   Asn Ile Pro Lys Pro
           285
<210> 21
   <211> 287
   <212> PRT
   <213> Mus musculus
<400> 21
   Met Ile Ile Ile Phe Arg Leu Val Gly Met Thr Gly Asn Ala Ile Val
    1               5                  10                  15
   Phe Trp Leu Leu Gly Phe Ser Leu His Arg Asn Ala Phe Ser Val Tyr
               20                  25                  30
   Ile Leu Asn Leu Ala Leu Ala Asp Phe Val Phe Leu Leu Cys His Ile
           35                  40                  45
   Ile Asp Ser Met Leu Leu Leu Leu Thr Val Phe Tyr Pro Asn Asn Ile
       50                  55                  60
   Phe Ser Gly Tyr Phe Tyr Thr Ile Met Thr Val Pro Tyr Ile Ala Gly
   65                  70                  75                  80
   Leu Ser Met Leu Ser Ala Ile Ser Thr Glu Leu Cys Leu Ser Val Leu
                   85                  90                  95
   Cys Pro Ile Trp Tyr Arg Cys His His Pro Glu His Thr Ser Thr Val
               100                 105                 110
   Met Cys Ala Ala Ile Trp Val Leu Pro Leu Leu Val Cys Ile Leu Asn
           115                 120                 125
   Arg Tyr Phe Cys Ser Phe Leu Asp Ile Asn Tyr Asn Asn Asp Lys Gln
       130                 135                 140
   Cys Leu Ala Ser Asn Phe Phe Thr Arg Ala Tyr Leu Met Phe Leu Phe
   145                 150                 155                 160
   Val Val Leu Cys Leu Ser Ser Met Ala Leu Leu Ala Arg Leu Phe Cys
                   165                 170                 175
   Gly Thr Gly Gln Met Lys Leu Thr Arg Leu Tyr Val Thr Ile Met Leu
               180                 185                 190
   Thr Val Leu Gly Phe Leu Leu Cys Gly Leu Pro Phe Val Ile Tyr Tyr
           195                 200                 205
   Phe Leu Leu Phe Asn Ile Lys Asp Gly Phe Cys Leu Phe Asp Phe Arg
       210                 215                 220
   Phe Tyr Met Ser Thr His Val Leu Thr Ala Ile Asn Asn Cys Ala Asn
   225                 230                 235                 240
   Pro Ile Ile Tyr Phe Phe Glu Gly Ser Phe Arg His Gln Leu Lys His
                   245                 250                 255
   Gln Thr Leu Lys Met Val Leu Gln Ser Val Leu Gln Asp Thr Pro Glu
               260                 265                 270
   Ile Ala Glu Asn Met Val Glu Met Ser Arg Asn Ile Pro Lys Pro
           275                 280                 285
<210> 22
   <211> 1024
   <212> DNA
   <213> Mus musculus
<220>
   <221> CDS
   <222> (16)...(918)
<400> 22
   ccagtgcacg aaacc atg cat aga agt atc agc atc agg att ctg ata aca   51
                    Met His Arg Ser Ile Ser Ile Arg Ile Leu Ile Thr
                    1               5                   10
   aac ttg atg atc gtc atc ctc gga cta gtc ggg ctg aca gga aac gcc     99
   Asn Leu Met Ile Val Ile Leu Gly Leu Val Gly Leu Thr Gly Asn Ala
           15                  20                  25
   att gtg ttc tgg ctc ctg ctc ttc cgc ttg cgc agg aac gcc ttc tca    147
   Ile Val Phe Trp Leu Leu Leu Phe Arg Leu Arg Arg Asn Ala Phe Ser
       30                  35                  40
   atc tac atc cta aac ttg gcc ctg gct gac ttc ctc ttc ctc ctc tgc    195
   Ile Tyr Ile Leu Asn Leu Ala Leu Ala Asp Phe Leu Phe Leu Leu Cys
   45                  50                  55                  60
   cac atc ata gct tcc aca gag cat att ctc acg ttt tcc tcc ccc aac    243
   His Ile Ile Ala Ser Thr Glu His Ile Leu Thr Phe Ser Ser Pro Asn
                   65                  70                  75
   agt atc ttt atc aat tgc ctt tac acc ttc agg gtg ctt ctc tac atc    291
   Ser Ile Phe Ile Asn Cys Leu Tyr Thr Phe Arg Val Leu Leu Tyr Ile
               80                  85                  90
   gca ggc ctg agc atg ctc agt gcc atc agc att gag cgc tgc ctg tct    339
   Ala Gly Leu Ser Met Leu Ser Ala Ile Ser Ile Glu Arg Cys Leu Ser
           95                  100                 105
   gtc atg tgc ccc atc tgg tat cgc tgc cac agc cca gaa cac aca tca    387
   Val Met Cys Pro Ile Trp Tyr Arg Cys His Ser Pro Glu His Thr Ser
       110                 115                 120
   act gtc atg tgt gct atg atc tgg gtc ctg tct cta ttg ctc tgc att    435
   Thr Val Met Cys Ala Met Ile Trp Val Leu Ser Leu Leu Leu Cys Ile
   125                 130                 135                 140
   ctg tat agg tat ttc tgc ggc ttc ttg gat acc aaa tat gaa gat gac    483
   Leu Tyr Arg Tyr Phe Cys Gly Phe Leu Asp Thr Lys Tyr Glu Asp Asp
                   145                 150                 155
   tat ggg tgt cta gca atg aac ttc ctt act acc gca tac ctg atg ttt    531
   Tyr Gly Cys Leu Ala Met Asn Phe Leu Thr Thr Ala Tyr Leu Met Phe
               160                 165                 170
   ttg ttt gta gtc ctc tgt gtg tcc agc ctg gct ctg ctg gcc agg ttg    579
   Leu Phe Val Val Leu Cys Val Ser Ser Leu Ala Leu Leu Ala Arg Leu
           175                 180                 185
   ttc tgt ggc gct gga cgg atg aag ctt acc aga tta tac gtg acc atc    627
   Phe Cys Gly Ala Gly Arg Met Lys Leu Thr Arg Leu Tyr Val Thr Ile
       190                 195                 200
   acg ctg acc ctt ttg gtt ttt ctc ctc tgc ggg ttg ccc tgt ggc ttc    675
   Thr Leu Thr Leu Leu Val Phe Leu Leu Cys Gly Leu Pro Cys Gly Phe
   205                 210                 215                 220
   tac tgg ttc ctg tta tcc aaa att aag aat gtt ttt act gta ttt gaa    723
   Tyr Trp Phe Leu Leu Ser Lys Ile Lys Asn Val Phe Thr Val Phe Glu
                   225                 230                 235
   ttt agt ctt tat ctg gca tca gtt gtc ctg act gct att aac agc tgt    771
   Phe Ser Leu Tyr Leu Ala Ser Val Val Leu Thr Ala Ile Asn Ser Cys
               240                 245                 250
   gcc aac ccc atc att tac ttc ttt gtg ggc tca ttc agg cat cgg ttg    819
   Ala Asn Pro Ile Ile Tyr Phe Phe Val Gly Ser Phe Arg His Arg Leu
           255                 260                 265
   aag cac cag acc ctc aaa atg gtt ctc cag agt gca ctg cag gac act    867
   Lys His Gln Thr Leu Lys Met Val Leu Gln Ser Ala Leu Gln Asp Thr
       270                 275                 280
   cct gag aca cct gaa aac atg gtg gag atg tca aga aac aaa gca gag    915
   Pro Glu Thr Pro Glu Asn Met Val Glu Met Ser Arg Asn Lys Ala Glu
   285                 290                 295                 300
   ctg tgatgaagag cctctgcccg gacctcagag gtggctttgg agtgagcact         968
   Leu
   gccctgctgc acttggccac tgtccactct cctctcagct tactcacttg gcatgc
   1024
<210> 23
   <211> 301
   <212> PRT
   <213> Mus musculus
<400> 23
   Met His Arg Ser Ile Ser Ile Arg Ile Leu Ile Thr Asn Leu Met Ile
    1               5                  10                  15
   Val Ile Leu Gly Leu Val Gly Leu Thr Gly Asn Ala Ile Val Phe Trp
               20                  25                  30
   Leu Leu Leu Phe Arg Leu Arg Arg Asn Ala Phe Ser Ile Tyr Ile Leu
           35                  40                  45
   Asn Leu Ala Leu Ala Asp Phe Leu Phe Leu Leu Cys His Ile Ile Ala
       50                  55                  60
   Ser Thr Glu His Ile Leu Thr Phe Ser Ser Pro Asn Ser Ile Phe Ile
   65                  70                  75                  80
   Asn Cys Leu Tyr Thr Phe Arg Val Leu Leu Tyr Ile Ala Gly Leu Ser
                   85                  90                  95
   Met Leu Ser Ala Ile Ser Ile Glu Arg Cys Leu Ser Val Met Cys Pro
               100                 105                 110
   Ile Trp Tyr Arg Cys His Ser Pro Glu His Thr Ser Thr Val Met Cys
           115                 120                 125
   Ala Met Ile Trp Val Leu Ser Leu Leu Leu Cys Ile Leu Tyr Arg Tyr
       130                 135                 140
   Phe Cys Gly Phe Leu Asp Thr Lys Tyr Glu Asp Asp Tyr Gly Cys Leu
   145                 150                 155                 160
   Ala Met Asn Phe Leu Thr Thr Ala Tyr Leu Met Phe Leu Phe Val Val
                   165                 170                 175
   Leu Cys Val Ser Ser Leu Ala Leu Leu Ala Arg Leu Phe Cys Gly Ala
               180                 185                 190
   Gly Arg Met Lys Leu Thr Arg Leu Tyr Val Thr Ile Thr Leu Thr Leu
           195                 200                 205
   Leu Val Phe Leu Leu Cys Gly Leu Pro Cys Gly Phe Tyr Trp Phe Leu
       210                 215                 220
   Leu Ser Lys Ile Lys Asn Val Phe Thr Val Phe Glu Phe Ser Leu Tyr
   225                 230                 235                 240
   Leu Ala Ser Val Val Leu Thr Ala Ile Asn Ser Cys Ala Asn Pro Ile
                   245                 250                 255
   Ile Tyr Phe Phe Val Gly Ser Phe Arg His Arg Leu Lys His Gln Thr
               260                 265                 270
   Leu Lys Met Val Leu Gln Ser Ala Leu Gln Asp Thr Pro Glu Thr Pro
           275                 280                 285
   Glu Asn Met Val Glu Met Ser Arg Asn Lys Ala Glu Leu
       290                 295                 300
<210> 24
   <211> 1045
   <212> DNA
   <213> Mus musculus
<220>
   <221> CDS
   <222> (106)...(1020)
<400> 24
<210> 25
   <211> 305
   <212> PRT
   <213> Mus musculus
<400> 25
   Met Asp Glu Thr Ser Pro Arg Ser Ile Asp Ile Glu Ser Leu Ile Pro
   1               5                   10                  15
   Asn Leu Met Ile Ile Ile Phe Gly Leu Val Gly Leu Thr Gly Asn Ala
               20                  25                  30
   Ile Val Leu Trp Leu Leu Gly Phe Cys Leu His Arg Asn Ala Phe Leu
           35                  40                  45
   Val Tyr Ile Leu Asn Leu Ala Leu Ala Asp Phe Leu Phe Leu Leu Cys
       50                  55                  60
   His Phe Ile Asn Ser Ala Met Phe Leu Leu Lys Val Pro Ile Pro Asn
   65                  70                  75                  80
   Gly Ile Phe Val Tyr Cys Phe Tyr Thr Ile Lys Met Val Leu Tyr Ile
                   85                  90                  95
   Thr Gly Leu Ser Met Leu Ser Ala Ile Ser Thr Glu Arg Cys Leu Ser
               100                 105                 110
   Val Leu Cys Pro Ile Trp Tyr His Cys Arg Arg Pro Glu His Thr Ser
           115                 120                 125
   Thr Val Met Cys Ala Val Ile Trp Ile Phe Ser Val Leu Ile Cys Ile
       130                 135                 140
   Leu Lys Glu Tyr Phe Cys Asp Phe Phe Gly Thr Lys Leu Gly Asn Tyr
   145                 150                 155                 160
   Tyr Val Cys Gln Ala Ser Asn Phe Phe Met Gly Ala Tyr Leu Met Phe
                   165                 170                 175
   Leu Phe Val Val Leu Cys Leu Ser Thr Leu Ala Leu Leu Ala Arg Leu
               180                 185                 190
   Phe Cys Gly Ala Glu Lys Met Lys Phe Thr Arg Leu Phe Val Thr Ile
           195                 200                 205
   Met Leu Thr Ile Leu Val Phe Leu Leu Cys Gly Leu Pro Trp Gly Phe
       210                 215                 220
   Phe Trp Phe Leu Leu Ile Trp Ile Lys Gly Gly Phe Ser Val Leu Asp
   225                 230                 235                 240
   Tyr Arg Leu Tyr Leu Ala Ser Ile Val Leu Thr Val Val Asn Ser Cys
                   245                 250                 255
   Ala Asn Pro Ile Ile Tyr Phe Phe Val Gly Ser Phe Arg His Arg Leu
               260                 265                 270
   Lys His Gln Thr Leu Lys Met Val Leu Gln Ser Ala Leu Gln Asp Thr
           275                 280                 285
   Pro Glu Thr His Glu Asn Met Val Glu Met Ser Arg Ile Lys Ala Glu
       290                 295                 300
   Gln
   305
<210> 26
   <211> 980
   <212> DNA
   <213> Mus musculus
<220>
   <221> CDS
   <222> (45)...(959)
<400> 26
   tagacacctc agcatatgca atggcaccca cgaccacaaa tcca atg gac aaa acc   56
                                                    Met Asp Lys Thr
                                                     1
   atc ctt gga agt att gac atc gag acc ctg atc cga cat ttg atg atc   104
   Ile Leu Gly Ser Ile Asp Ile Glu Thr Leu Ile Arg His Leu Met Ile
    5                  10                  15                  20
   atc atc ttc gga ctg gtc ggg ctg aca gga aat gcc att gtg ttc tgg   152
   Ile Ile Phe Gly Leu Val Gly Leu Thr Gly Asn Ala Ile Val Phe Trp
                   25                  30                  35
   ctc ctg ggc ttc cac ttg cac agg aat gcc ttc tta gtc tac atc ata   200
   Leu Leu Gly Phe His Leu His Arg Asn Ala Phe Leu Val Tyr Ile Ile
               40                  45                  50
   aac ttg gcc ctg gct gac ttc ttc tat ctg ctc tgt cac atc ata aat   248
   Asn Leu Ala Leu Ala Asp Phe Phe Tyr Leu Leu Cys His Ile Ile Asn
           55                  60                  65
   tcc ata atg ttt ctt ctc aag gtt ccc tca ccc aac att atc ttg gac   296
   Ser Ile Met Phe Leu Leu Lys Val Pro Ser Pro Asn Ile Ile Leu Asp
       70                  75                  80
   cat tgc ttt tac acc atc atg ata gtt ctc tac atc aca ggc ctg agc   344
   His Cys Phe Tyr Thr Ile Met Ile Val Leu Tyr Ile Thr Gly Leu Ser
   85                  90                  95                  100
   atg ctc agc gcc atc agc act gag cgc tgc ctg tct gtc ctg tgc ccc   392
   Met Leu Ser Ala Ile Ser Thr Glu Arg Cys Leu Ser Val Leu Cys Pro
                   105                 110                 115
   atc tgg tat cgc tgc cac cgt cca gaa cac aca tca act gtc atg tgt   440
   Ile Trp Tyr Arg Cys His Arg Pro Glu His Thr Ser Thr Val Met Cys
               120                 125                 130
   gct gtg atc tgg gta atg tcc ctg ttg atc tct att ctc aat gga tat   488
   Ala Val Ile Trp Val Met Ser Leu Leu Ile Ser Ile Leu Asn Gly Tyr
           135                 140                 145
   ttc tgt aat ttc tct agt ccc aaa tat gta aat aac tct gtg tgt cag   536
   Phe Cys Asn Phe Ser Ser Pro Lys Tyr Val Asn Asn Ser Val Cys Gln
       150                 155                 160
   gca tca cac atc ttt atc aga aca tac cca ata ttt ttg ttt gta ctc   584
   Ala Ser His Ile Phe Ile Arg Thr Tyr Pro Ile Phe Leu Phe Val Leu
   165                 170                 175                 180
   ctc tgt ctg tcc acc ctt gct ctg ctg gcc agg ttg ttc tct ggt gct   632
   Leu Cys Leu Ser Thr Leu Ala Leu Leu Ala Arg Leu Phe Ser Gly Ala
                   185                 190                 195
   ggg aag agg aaa ttt acc aga tta ttc gtg acc atc atg ctg gcc att     680
   Gly Lys Arg Lys Phe Thr Arg Leu Phe Val Thr Ile Met Leu Ala Ile
               200                 205                 210
   ttg gtt ttt ctt ctc tgt ggg tta ccc ctg ggc ttc ttc tgg ttt ctg     728
   Leu Val Phe Leu Leu Cys Gly Leu Pro Leu Gly Phe Phe Trp Phe Leu
           215                 220                 225
   tca ccc tgg att gag gat cgt ttc att gta cta gat tat aga ctt ttt     776
   Ser Pro Trp Ile Glu Asp Arg Phe Ile Val Leu Asp Tyr Arg Leu Phe
       230                 235                 240
   ttt gca tca gtt gtc cta act gtt gtt aac agc tgt gcc aac ccc atc     824
   Phe Ala Ser Val Val Leu Thr Val Val Asn Ser Cys Ala Asn Pro Ile
   245                 250                 255                 260
   att tac ttc ttt gtg ggc tcc ttc agg cat cgg ttg aag caa cag acc     872
   Ile Tyr Phe Phe Val Gly Ser Phe Arg His Arg Leu Lys Gln Gln Thr
                   265                 270                 275
   ctc aaa atg ttt ctc cag aga gca ctg cag gac acc cct gag aca cct     920
   Leu Lys Met Phe Leu Gln Arg Ala Leu Gln Asp Thr Pro Glu Thr Pro
               280                 285                 290
   gaa aac atg gtg gag atg tca aga agc aaa gca gag ccg tgatgaagag      969
   Glu Asn Met Val Glu Met Ser Arg Ser Lys Ala Glu Pro
           295                 300                 305
   cctcttccag g                                                        980
<210> 27
   <211> 305
   <212> PRT
   <213> Mus musculus
<400> 27
   Met Asp Lys Thr Ile Leu Gly Ser Ile Asp Ile Glu Thr Leu Ile Arg
    1               5                  10                  15
   His Leu Met Ile Ile Ile Phe Gly Leu Val Gly Leu Thr Gly Asn Ala
               20                  25                  30
   Ile Val Phe Trp Leu Leu Gly Phe His Leu His Arg Asn Ala Phe Leu
           35                  40                  45
   Val Tyr Ile Ile Asn Leu Ala Leu Ala Asp Phe Phe Tyr Leu Leu Cys
       50                  55                  60
   His Ile Ile Asn Ser Ile Met Phe Leu Leu Lys Val Pro Ser Pro Asn
   65                  70                  75                  80
   Ile Ile Leu Asp His Cys Phe Tyr Thr Ile Met Ile Val Leu Tyr Ile
                   85                  90                  95
   Thr Gly Leu Ser Met Leu Ser Ala Ile Ser Thr Glu Arg Cys Leu Ser
               100                 105                 110
   Val Leu Cys Pro Ile Trp Tyr Arg Cys His Arg Pro Glu His Thr Ser
           115                 120                 125
   Thr Val Met Cys Ala Val Ile Trp Val Met Ser Leu Leu Ile Ser Ile
       130                 135                 140
   Leu Asn Gly Tyr Phe Cys Asn Phe Ser Ser Pro Lys Tyr Val Asn Asn
   145                 150                 155                 160
   Ser Val Cys Gln Ala Ser His Ile Phe Ile Arg Thr Tyr Pro Ile Phe
                   165                 170                 175
   Leu Phe Val Leu Leu Cys Leu Ser Thr Leu Ala Leu Leu Ala Arg Leu
               180                 185                 190
   Phe Ser Gly Ala Gly Lys Arg Lys Phe Thr Arg Leu Phe Val Thr Ile
           195                 200                 205
   Met Leu Ala Ile Leu Val Phe Leu Leu Cys Gly Leu Pro Leu Gly Phe
       210                 215                 220
   Phe Trp Phe Leu Ser Pro Trp Ile Glu Asp Arg Phe Ile Val Leu Asp
   225                 230                 235                 240
   Tyr Arg Leu Phe Phe Ala Ser Val Val Leu Thr Val Val Asn Ser Cys
                   245                 250                 255
   Ala Asn Pro Ile Ile Tyr Phe Phe Val Gly Ser Phe Arg His Arg Leu
               260                 265                 270
   Lys Gln Gln Thr Leu Lys Met Phe Leu Gln Arg Ala Leu Gln Asp Thr
           275                 280                 285
   Pro Glu Thr Pro Glu Asn Met Val Glu Met Ser Arg Ser Lys Ala Glu
       290                 295                 300
   Pro
   305
<210> 28
   <211> 408
   <212> DNA
   <213> Homo sapiens
<220>
   <221> CDS
   <222> (1)...(405)
<400> 28
   atg gag act ctc ccc aag gtt cta gag gtc gat gag aag tct cca gaa      48
   gcc aag gac ctg ctg ccc agc cag acc gcc agc tcc ctg tgc atc agc      96
   tcc agg agc gag tct gtc tgg acc acc acc ccc agg agt aac tgg gaa     144
   atc tac cgc aag ccc atc gtt atc atg tca gtg ggc ggt gcc atc ctg     192
   ctt ttc ggc gtg gtc atc acc tgc ttg gcc tac acc ttg aag ctg agt     240
   gac aag agt ctc tcc atc ctc aaa atg gta ggg cct ggc ttc ctg tcc     288
   ctg gga ctc atg atg ctg gtg tgc ggg ctg gtg tgg gtg ccc atc atc     336
   aaa aag aaa cag aag cac aga cag aag tcg aat ttc tta cgc agc ctc     384
   aag tcc ttc ttc ctg act cgc tga                                     408
<210> 29
   <211> 135
   <212> PRT
   <213> Homo sapiens
<400> 29
<210> 30
   <211> 1400
   <212> DNA
   <213> Homo sapiens
<220>
   <221> CDS
   <222> (332)...(1297)
<400> 30
   tcaggcccag gatagagtaa tcatcgggtc cacagcactg gctagatgag tgggggtgtt 60
   ttgatcctaa tgttattccc atgttagcac agaacttgtg tggcagtaga gagaggtcag 120
   gcttcagagt cagcaagaac tggatttcaa actggatttg aggaccccca ccttttgata 180
   ggtgacttat tctctgtgag tctctgatct gccctcttta aatgaggaag taaatcccac 240
   atggcagggt ggtggggaga atcagagatc atacagctgg tgatcacaac tggtttctgt 300
   ttccagggtc accagactgg ggtttctgag c atg gat tca acc atc cca gtc    352
                                     Met Asp Ser Thr Ile Pro Val
                                     1               5
   ttg ggt aca gaa ctg aca cca atc aac gga cgt gag gag act cct tgc   400
   Leu Gly Thr Glu Leu Thr Pro Ile Asn Gly Arg Glu Glu Thr Pro Cys
           10                  15                  20
   tac aag cag acc ctg agc ttc acg ggg ctg acg tgc atc gtt tcc ctt   448
   Tyr Lys Gln Thr Leu Ser Phe Thr Gly Leu Thr Cys Ile Val Ser Leu
       25                  30                  35
   gtc gcg ctg aca gga aac gcg gtt gtg ctc tgg ctc ctg ggc tgc cgc   496
   Val Ala Leu Thr Gly Asn Ala Val Val Leu Trp Leu Leu Gly Cys Arg
   40                  45                  50                  55
   atg cgc agg aac gct gtc tcc atc tac atc ctc aac ctg gtc gcg gcc   544
   Met Arg Arg Asn Ala Val Ser Ile Tyr Ile Leu Asn Leu Val Ala Ala
                   60                  65                  70
   gac ttc ctc ttc ctt agc ggc cac att ata tgt tcg ccg tta cgc ctc   592
   Asp Phe Leu Phe Leu Ser Gly His Ile Ile Cys Ser Pro Leu Arg Leu
               75                  80                  85
   atc aat atc cgc cat ccc atc tcc aaa atc ctc agt cct gtg atg acc   640
   Ile Asn Ile Arg His Pro Ile Ser Lys Ile Leu Ser Pro Val Met Thr
           90                  95                  100
   ttt ccc tac ttt ata ggc cta agc atg ctg agc gcc atc agc acc gag   688
   Phe Pro Tyr Phe Ile Gly Leu Ser Met Leu Ser Ala Ile Ser Thr Glu
       105                 110                 115
   cgc tgc ctg tcc atc ctg tgg ccc atc tgg tac cac tgc cgc cgc ccc     736
   Arg Cys Leu Ser Ile Leu Trp Pro Ile Trp Tyr His Cys Arg Arg Pro
   120                 125                 130                 135
   aga tac ctg tca tcg gtc atg tgt gtc ctg ctc tgg gcc ctg tcc ctg     784
   Arg Tyr Leu Ser Ser Val Met Cys Val Leu Leu Trp Ala Leu Ser Leu
                   140                 145                 150
   ctg cgg agt atc ctg gag tgg atg ttc tgt gac ttc ctg ttt agt ggt     832
   Leu Arg Ser Ile Leu Glu Trp Met Phe Cys Asp Phe Leu Phe Ser Gly
               155                 160                 165
   gct gat tct gtt tgg tgt gaa acg tca gat ttc att aca atc gcg tgg     880
   Ala Asp Ser Val Trp Cys Glu Thr Ser Asp Phe Ile Thr Ile Ala Trp
           170                 175                 180
   ctg gtt ttt tta tgt gtg gtt ctc tgt ggg tcc agc ctg gtc ctg ctg     928
   Leu Val Phe Leu Cys Val Val Leu Cys Gly Ser Ser Leu Val Leu Leu
       185                 190                 195
   gtc agg att ctc tgt gga tcc cgg aag atg ccg ctg acc agg ctg tac     976
   Val Arg Ile Leu Cys Gly Ser Arg Lys Met Pro Leu Thr Arg Leu Tyr
   200                 205                 210                 215
   gtg acc atc ctc ctc aca gtg ctg gtc ttc ctc ctc tgt ggc ctg ccc
   1024
   Val Thr Ile Leu Leu Thr Val Leu Val Phe Leu Leu Cys Gly Leu Pro
                   220                 225                 230
   ttt ggc att cag tgg gcc ctg ttt tcc agg atc cac ctg gat tgg aaa
   1072
   Phe Gly Ile Gln Trp Ala Leu Phe Ser Arg Ile His Leu Asp Trp Lys
               235                 240                 245
   gtc tta ttt tgt cat gtg cat cta gtt tcc att ttc ctg tcc gct ctt
   1120
   Val Leu Phe Cys His Val His Leu Val Ser Ile Phe Leu Ser Ala Leu
           250                 255                 260
   aac agc agt gcc aac ccc atc att tac ttc ttc gtg ggc tcc ttt agg
   1168
   Asn Ser Ser Ala Asn Pro Ile Ile Tyr Phe Phe Val Gly Ser Phe Arg
       265                 270                 275
   cag cgt caa aat agg cag aac ctg aag ctg gtt ctc cag agg gct ctg
   1216
   Gln Arg Gln Asn Arg Gln Asn Leu Lys Leu Val Leu Gln Arg Ala Leu
   280                 285                 290                 295
   cag gac acg cct gag gtg gat gaa ggt gga ggg tgg ctt cct cag gaa
   1264
   Gln Asp Thr Pro Glu Val Asp Glu Gly Gly Gly Trp Leu Pro Gln Glu
                   300                 305                 310
   acc ctg gag ctg tcg gga agc aga ttg gag cag tgaggaagaa cctctgccct
   1317
   Thr Leu Glu Leu Ser Gly Ser Arg Leu Glu Gln
               315                 320
   gtcagacagg actttgagag caatgctgcc ctgccaccct tgacaattat atgcattttt
   1377
   cttagccttc tgcctcagaa atg
   1400
<210> 31
   <211> 322
   <212> PRT
   <213> Homo sapiens
<400> 31
   Met Asp Ser Thr Ile Pro Val Leu Gly Thr Glu Leu Thr Pro Ile Asn
    1               5                  10                  15
   Gly Arg Glu Glu Thr Pro Cys Tyr Lys Gln Thr Leu Ser Phe Thr Gly
               20                  25                  30
   Leu Thr Cys Ile Val Ser Leu Val Ala Leu Thr Gly Asn Ala Val Val
           35                  40                  45
   Leu Trp Leu Leu Gly Cys Arg Met Arg Arg Asn Ala Val Ser Ile Tyr
       50                  55                  60
   Ile Leu Asn Leu Val Ala Ala Asp Phe Leu Phe Leu Ser Gly His Ile
   65                  70                  75                  80
   Ile Cys Ser Pro Leu Arg Leu Ile Asn Ile Arg His Pro Ile Ser Lys
                   85                  90                  95
   Ile Leu Ser Pro Val Met Thr Phe Pro Tyr Phe Ile Gly Leu Ser Met
               100                 105                 110
   Leu Ser Ala Ile Ser Thr Glu Arg Cys Leu Ser Ile Leu Trp Pro Ile
           115                 120                 125
   Trp Tyr His Cys Arg Arg Pro Arg Tyr Leu Ser Ser Val Met Cys Val
       130                 135                 140
   Leu Leu Trp Ala Leu Ser Leu Leu Arg Ser Ile Leu Glu Trp Met Phe
   145                 150                 155                 160
   Cys Asp Phe Leu Phe Ser Gly Ala Asp Ser Val Trp Cys Glu Thr Ser
                   165                 170                 175
   Asp Phe Ile Thr Ile Ala Trp Leu Val Phe Leu Cys Val Val Leu Cys
               180                 185                 190
   Gly Ser Ser Leu Val Leu Leu Val Arg Ile Leu Cys Gly Ser Arg Lys
           195                 200                 205
   Met Pro Leu Thr Arg Leu Tyr Val Thr Ile Leu Leu Thr Val Leu Val
       210                 215                 220
   Phe Leu Leu Cys Gly Leu Pro Phe Gly Ile Gln Trp Ala Leu Phe Ser
   225                 230                 235                 240
   Arg Ile His Leu Asp Trp Lys Val Leu Phe Cys His Val His Leu Val
                   245                 250                 255
   Ser Ile Phe Leu Ser Ala Leu Asn Ser Ser Ala Asn Pro Ile Ile Tyr
               260                 265                 270
   Phe Phe Val Gly Ser Phe Arg Gln Arg Gln Asn Arg Gln Asn Leu Lys
           275                 280                 285
   Leu Val Leu Gln Arg Ala Leu Gln Asp Thr Pro Glu Val Asp Glu Gly
       290                 295                 300
   Gly Gly Trp Leu Pro Gln Glu Thr Leu Glu Leu Ser Gly Ser Arg Leu
   305                 310                 315                 320
   Glu Gln
<210> 32
   <211> 1604
   <212> DNA
   <213> Homo sapiens
<220>
   <221> CDS
   <222> (433)...(1398)
<400> 32
   tgcatggtct tccttcctgt ccatggatga ccagtcctag tcacgagtgt gtcacaacca 60
   cctctttgtg tatctgaatt cctccacctg aaagaaaatt tcagacccag gatagattaa 120
   tcatcgggtc caaagccctg gccggatgag tgggggtgtt ttgatcctaa tgttattccc 180
   atgtcagcac agaacttgtg tggcagtaga gagatgtcag gcttcagagt caacaagaac 240
   tggatttcaa actggatttg aggaccccca cctttggtaa gtgacttatt atctgcgagc 300
   ctctgtttct ctcttcttta aatgaggaca gtaaatccca tacggcaggg tggtggggag 360
   aatcagagat gatacagctg gtgatcacat ctggtttgtg ttcccagggg caccagacta 420
   gagtttctga gc atg gat cca acc gtc cca gtc ttc ggt aca aaa ctg aca 471
                       Met Asp Pro Thr Val Pro Val Phe Gly Thr Lys Leu Thr
                        1               5                   10
   cca atc aac gga cgt gag gag act cct tgc tac aat cag acc ctg agc    519
   Pro Ile Asn Gly Arg Glu Glu Thr Pro Cys Tyr Asn Gln Thr Leu Ser
        15                  20                  25
   ttc acg gtg ctg acg tgc atc att tcc ctt gtc gga ctg aca gga aac    567
   Phe Thr Val Leu Thr Cys Ile Ile Ser Leu Val Gly Leu Thr Gly Asn
    30                  35                  40                  45
   gcg gta gtg ctc tgg ctc ctg ggc tac cgc atg cgc agg aac gct gtc    615
   Ala Val Val Leu Trp Leu Leu Gly Tyr Arg Met Arg Arg Asn Ala Val
                    50                  55                  60
   tcc atc tac atc ctc aac ctg gcc gca gca gac ttc ctc ttc ctc agc    663
   Ser Ile Tyr Ile Leu Asn Leu Ala Ala Ala Asp Phe Leu Phe Leu Ser
                65                  70                  75
   ttc cag att ata cgt tcg cca tta cgc ctc atc aat atc agc cat ctc    711
   Phe Gln Ile Ile Arg Ser Pro Leu Arg Leu Ile Asn Ile Ser His Leu
            80                  85                  90
   atc cgc aaa atc ctc gtt tct gtg atg acc ttt ccc tac ttt aca ggc    759
   Ile Arg Lys Ile Leu Val Ser Val Met Thr Phe Pro Tyr Phe Thr Gly
        95                 100                 105
   ctg agt atg ctg agc gcc atc agc acc gag cgc tgc ctg tct gtt ctg    807
   Leu Ser Met Leu Ser Ala Ile Ser Thr Glu Arg Cys Leu Ser Val Leu
   110                 115                 120                 125
   tgg ccc atc tgg tac cgc tgc cgc cgc ccc aca cac ctg tca gcg gtc    855
   Trp Pro Ile Trp Tyr Arg Cys Arg Arg Pro Thr His Leu Ser Ala Val
                   130                 135                 140
   gtg tgt gtc ctg ctc tgg ggc ctg tcc ctg ctg ttt agt atg ctg gag    903
   Val Cys Val Leu Leu Trp Gly Leu Ser Leu Leu Phe Ser Met Leu Glu
               145                 150                 155
   tgg agg ttc tgt gac ttc ctg ttt agt ggt gct gat tct agt tgg tgt    951
   Trp Arg Phe Cys Asp Phe Leu Phe Ser Gly Ala Asp Ser Ser Trp Cys
           160                 165                 170
   gaa acg tca gat ttc atc cca gtc gcg tgg ctg att ttt tta tgt gtg    999
   Glu Thr Ser Asp Phe Ile Pro Val Ala Trp Leu Ile Phe Leu Cys Val
       175                 180                 185
   gtt ctc tgt gtt tcc agc ctg gtc ctg ctg gtc agg atc ctc tgt gga
   1047
   Val Leu Cys Val Ser Ser Leu Val Leu Leu Val Arg Ile Leu Cys Gly
   190                 195                 200                 205
   tcc cgg aag atg ccg ctg acc agg ctg tac gtg acc atc ctg ctc aca
   1095
   Ser Arg Lys Met Pro Leu Thr Arg Leu Tyr Val Thr Ile Leu Leu Thr
                   210                 215                 220
   gtg ctg gtc ttc ctc ctc tgc ggc ctg ccc ttc ggc att ctg ggg gcc
   1143
   Val Leu Val Phe Leu Leu Cys Gly Leu Pro Phe Gly Ile Leu Gly Ala
               225                 230                 235
   cta att tac agg atg cac ctg aat ttg gaa gtc tta tat tgt cat gtt
   1191
   Leu Ile Tyr Arg Met His Leu Asn Leu Glu Val Leu Tyr Cys His Val
           240                 245                 250
   tat ctg gtt tgc atg tcc ctg tcc tct cta aac agt agt gcc aac ccc
   1239
   Tyr Leu Val Cys Met Ser Leu Ser Ser Leu Asn Ser Ser Ala Asn Pro
       255                 260                 265
   atc att tac ttc ttc gtg ggc tcc ttt agg cag cgt caa aat agg cag
   1287
   Ile Ile Tyr Phe Phe Val Gly Ser Phe Arg Gln Arg Gln Asn Arg Gln
   270                 275                 280                 285
   aac ctg aag ctg gtt ctc cag agg gct ctg cag gac aag cct gag gtg
   1335
   Asn Leu Lys Leu Val Leu Gln Arg Ala Leu Gln Asp Lys Pro Glu Val
                   290                 295                 300
   gat aaa ggt gaa ggg cag ctt cct gag gaa agc ctg gag ctg tcg gga
   1383
   Asp Lys Gly Glu Gly Gln Leu Pro Glu Glu Ser Leu Glu Leu Ser Gly
               305                 310                 315
   agc aga ttg ggg cca tgagggagag cctctgccct gtcagtcaga cgggactttg
   1438
   Ser Arg Leu Gly Pro
           320
   agagcaacac tgtcctgcca cccttgacaa ttacatgcgt ttttcttagc gtttcgcctc
   1498
   agaaatgtct cagtggtaac tcaaggtctt caaataaatg tttatctaac ctgacagttg
   1558
   cagttttcac ccatggaaag cattagtctg acagtacaat gtttgg
   1604
<210> 33
   <211> 322
   <212> PRT
   <213> Homo sapiens
<400> 33
   Met Asp Pro Thr Val Pro Val Phe Gly Thr Lys Leu Thr Pro Ile Asn
    1               5                  10                  15
   Gly Arg Glu Glu Thr Pro Cys Tyr Asn Gln Thr Leu Ser Phe Thr Val
               20                  25                  30
   Leu Thr Cys Ile Ile Ser Leu Val Gly Leu Thr Gly Asn Ala Val Val
           35                  40                  45
   Leu Trp Leu Leu Gly Tyr Arg Met Arg Arg Asn Ala Val Ser Ile Tyr
       50                  55                  60
   Ile Leu Asn Leu Ala Ala Ala Asp Phe Leu Phe Leu Ser Phe Gln Ile
   65                  70                  75                  80
   Ile Arg Ser Pro Leu Arg Leu Ile Asn Ile Ser His Leu Ile Arg Lys
                   85                  90                  95
   Ile Leu Val Ser Val Met Thr Phe Pro Tyr Phe Thr Gly Leu Ser Met
               100                 105                 110
   Leu Ser Ala Ile Ser Thr Glu Arg Cys Leu Ser Val Leu Trp Pro Ile
           115                 120                 125
   Trp Tyr Arg Cys Arg Arg Pro Thr His Leu Ser Ala Val Val Cys Val
       130                 135                 140
   Leu Leu Trp Gly Leu Ser Leu Leu Phe Ser Met Leu Glu Trp Arg Phe
   145                 150                 155                 160
   Cys Asp Phe Leu Phe Ser Gly Ala Asp Ser Ser Trp Cys Glu Thr Ser
                   165                 170                 175
   Asp Phe Ile Pro Val Ala Trp Leu Ile Phe Leu Cys Val Val Leu Cys
               180                 185                 190
   Val Ser Ser Leu Val Leu Leu Val Arg Ile Leu Cys Gly Ser Arg Lys
           195                 200                 205
   Met Pro Leu Thr Arg Leu Tyr Val Thr Ile Leu Leu Thr Val Leu Val
       210                 215                 220
   Phe Leu Leu Cys Gly Leu Pro Phe Gly Ile Leu Gly Ala Leu Ile Tyr
   225                 230                 235                 240
   Arg Met His Leu Asn Leu Glu Val Leu Tyr Cys His Val Tyr Leu Val
                   245                 250                 255
   Cys Met Ser Leu Ser Ser Leu Asn Ser Ser Ala Asn Pro Ile Ile Tyr
               260                 265                 270
   Phe Phe Val Gly Ser Phe Arg Gln Arg Gln Asn Arg Gln Asn Leu Lys
           275                 280                 285
   Leu Val Leu Gln Arg Ala Leu Gln Asp Lys Pro Glu Val Asp Lys Gly
       290                 295                 300
   Glu Gly Gln Leu Pro Glu Glu Ser Leu Glu Leu Ser Gly Ser Arg Leu
   305                 310                 315                 320
   Gly Pro
<210> 34
   <211> 1540
   <212> DNA
   <213> Homo sapiens
<400> 34
   aggcacacct ggggaaaggt gcacgggggc accaccttgg tggccagttg atgccaccca 60
   aggaccagca tagggccaaa gatcacccga ggtcacctgc ctcctccaca aagatgccgt 120
   cttaggcaga gaaggtggtt gggagaaagc tttcatattc aaatgagatt cctgttatcc 180
   acccatagat aaccagctta aagcagggta gggctaaaag ctaatatttt cccccaacca 240
   gataatctgc tataaacaaa taaattgcat cttccagcgg ggttgcattg tgagatccag 300
   gacacaggtg ttgtggggag ttttgacatg cagggaagtg acccccacat gcagctgcaa 360
   agtccttggg gctcccccaa gaaggcgggc cagacacttg gcagggacga ggtgggaggc 420
   agctcacggc tcgggaatct ccagggcatg ggctcgcaca ggtgggaagc acctgtgggc 480
   ggctctcaag cccccatctc attggtgccc acggtgggcg tctccccacc ttccagctcg 540
   ggctcctcgc gaagcgcctg ttggagcaca gtccccaggg acctggtggg cagcctgtgg 600
   ctctccggct gcccaccagg aagtagatga cggggttggc gctgctgctt acggacgagg 660
   agaggcgtga caagctgaag cacaggacct gcatctcggg cggcaggctc aaccagtaga 720
   gcacaaacca gtagatgctc agaggcaggg aacagatgag gaacaccagg acagaggcca 780
   ggaccaccac gaacagccgt gtgggctgcc gccgccactg ctgggagctc ctccgcaccc 840
   agacaaagag ggtcaggctg gacagagtca tcactggggt taagaccccc atgatgaggg 900
   cggcctggac catgtccacc ctgaagcacc gatcttcatt gaatttcaag aacttgctgc 960
   agaaggaaga ggtcaacccg ttcatcagga gacagagtgt ccacagcagg ccacacaccc
   1020
   aggctgacag gtgcctgggc cggtgacact tgaaccagat agggaagagg acagagagac
   1080
   agcgctgggt gctgatggcc gtcagcaggc tcaggcccac tgtgtaggca aagtacatca
   1140
   gtctcttcat cagctcgtgg accttgtcag tggtattgac caggggctgg gtttccaggc
   1200
   tgagcgtgga agccatgctg aagaggaaga ggaggtcggc tgccgccagg ttgaggatat
   1260
   agatgcagaa ggggttcctg tgcattcgaa agcccagcag ccagatcacc atgctgttgc
   1320
   ctgccatccc gcacaggcag gtgaacatgg ccagggagct cagcaccagg taggccgtgt
   1380
   gcactgtgct ccctctggaa tagtttaggg ctgactccac ggtcccactg ctattcaaag
   1440
   tctggttcat ccctacgaga ggaagatgta ccaatgtgaa attctgtgtt gctgggacca
   1500
   cgggggaccc ctgggtgccc ctcgaatttc cagcttcaga
   1540
<210> 35
   <211> 409
   <212> PRT
   <213> Homo sapiens
<400> 35
   Met Asn Gln Thr Leu Asn Ser Ser Gly Thr Val Glu Ser Ala Leu Asn
    1               5                  10                  15
   Tyr Ser Arg Gly Ser Thr Val His Thr Ala Tyr Leu Val Leu Ser Ser
               20                  25                  30
   Leu Ala Met Phe Thr Cys Leu Cys Gly Met Ala Gly Asn Ser Met Val
           35                  40                  45
   Ile Trp Leu Leu Gly Phe Arg Met His Arg Asn Pro Phe Cys Ile Tyr
       50                  55                  60
   Ile Leu Asn Leu Ala Ala Ala Asp Leu Leu Phe Leu Phe Ser Met Ala
   65                  70                  75                  80
   Ser Thr Leu Ser Leu Glu Thr Gln Pro Leu Val Asn Thr Thr Asp Lys
                   85                  90                  95
   Val His Glu Leu Met Lys Arg Leu Met Tyr Phe Ala Tyr Thr Val Gly
               100                 105                 110
   Leu Ser Leu Leu Thr Ala Ile Ser Thr Gln Arg Cys Leu Ser Val Leu
           115                 120                 125
   Phe Pro Ile Trp Phe Lys Cys His Arg Pro Arg His Leu Ser Ala Trp
       130                 135                 140
   Val Cys Gly Leu Leu Trp Thr Leu Cys Leu Leu Met Asn Gly Leu Thr
   145                 150                 155                 160
   Ser Ser Phe Cys Ser Lys Phe Leu Lys Phe Asn Glu Asp Arg Cys Phe
                   165                 170                 175
   Arg Val Asp Met Val Gln Ala Ala Leu Ile Met Gly Val Leu Thr Pro
               180                 185                 190
   Val Met Thr Leu Ser Ser Leu Thr Leu Phe Val Trp Val Arg Arg Ser
           195                 200                 205
   Ser Gln Gln Trp Arg Arg Gln Pro Thr Arg Leu Phe Val Val Val Leu
       210                 215                 220
   Ala Ser Val Leu Val Phe Leu Ile Cys Ser Leu Pro Leu Ser Ile Tyr
   225                 230                 235                 240
   Trp Phe Val Leu Tyr Trp Leu Ser Leu Pro Pro Glu Met Gln Val Leu
                   245                 250                 255
   Cys Phe Ser Leu Ser Arg Leu Ser Ser Ser Val Ser Ser Ser Ala Asn
               260                 265                 270
   Pro Val Ile Tyr Phe Leu Val Gly Ser Arg Arg Ala Thr Gly Cys Pro
           275                 280                 285
   Pro Gly Pro Trp Gly Leu Cys Ser Asn Arg Arg Phe Ala Arg Ser Pro
       290                 295                 300
   Ser Trp Lys Val Gly Arg Arg Pro Pro Trp Ala Pro Met Arg Trp Gly
   305                 310                 315                 320
   Leu Glu Ser Arg Pro Gln Val Leu Pro Thr Cys Ala Ser Pro Cys Pro
                   325                 330                 335
   Gly Asp Ser Arg Ala Val Ser Cys Leu Pro Pro Arg Pro Cys Gln Val
               340                 345                 350
   Ser Gly Pro Pro Ser Trp Gly Ser Pro Lys Asp Phe Ala Ala Ala Cys
           355                 360                 365
   Gly Gly His Phe Pro Ala Cys Gln Asn Ser Pro Gln His Leu Cys Pro
       370                 375                 380
   Gly Ser His Asn Ala Thr Pro Leu Glu Asp Ala Ile Tyr Leu Phe Ile
   385                 390                 395                 400
   Ala Asp Tyr Leu Val Gly Gly Lys Tyr
                   405
<210> 36
   <211> 767
   <212> DNA
   <213> Homo sapiens
<220>
   <221> CDS
   <222> (2)...(716)
<400> 36
   c cac atg gtg gcc atc gtc ccc gac ttg ctg caa ggc cgg ctg gac ttc 49
     His Met Val Ala Ile Val Pro Asp Leu Leu Gln Gly Arg Leu Asp Phe
      1               5                   10                  15
   ccg ggc ttc gtg cag acc agc ctg gca acg ctg cgc ttc ttc tgc tac    97
   Pro Gly Phe Val Gln Thr Ser Leu Ala Thr Leu Arg Phe Phe Cys Tyr
               20                  25                  30
   atc gtg ggc ctg agt ctc ctg gcg gcc gtc agc gtg gag cag tgc ctg    145
   Ile Val Gly Leu Ser Leu Leu Ala Ala Val Ser Val Glu Gln Cys Leu
           35                  40                  45
   gcc gcc ctc ttc cca gcc tgg tac tcg tgc cgc cgc cca cgc cac ctg    193
   Ala Ala Leu Phe Pro Ala Trp Tyr Ser Cys Arg Arg Pro Arg His Leu
       50                  55                  60
   acc acc tgt gtg tgc gcc ctc acc tgg gcc ctc tgc ctg ctg ctg cac    241
   Thr Thr Cys Val Cys Ala Leu Thr Trp Ala Leu Cys Leu Leu Leu His
   65                  70                  75                  80
   ctg ctg ctc agc agc gcc tgc acc cag ttc ttc ggg gag ccc agc cgc    289
   Leu Leu Leu Ser Ser Ala Cys Thr Gln Phe Phe Gly Glu Pro Ser Arg
                   85                  90                  95
   cac ttg tgc cgg acg ctg tgg ctg gtg gca gcg gtg ctg ctg gct ctg    337
   His Leu Cys Arg Thr Leu Trp Leu Val Ala Ala Val Leu Leu Ala Leu
               100                 105                 110
   ctg tgt tgc acc atg tgt ggg gcc agc ctt atg ctg ctg ctg cgg gtg    385
   Leu Cys Cys Thr Met Cys Gly Ala Ser Leu Met Leu Leu Leu Arg Val
           115                 120                 125
   gag cga ggc ccc cag cgg ccc cca ccc cgg ggc ttc cct ggg ctc atc    433
   Glu Arg Gly Pro Gln Arg Pro Pro Pro Arg Gly Phe Pro Gly Leu Ile
       130                 135                 140
   ctc ctc acc gtc ctc ctc ttc ctc ttc tgc ggc ctg ccc ttc ggc atc    481
   Leu Leu Thr Val Leu Leu Phe Leu Phe Cys Gly Leu Pro Phe Gly Ile
   145                 150                 155                 160
   tac tgg ctg tcc cgg aac ctg ctc tgg tac atc ccc cac tac ttc tac    529
   Tyr Trp Leu Ser Arg Asn Leu Leu Trp Tyr Ile Pro His Tyr Phe Tyr
                   165                 170                 175
   cac ttc agc ttc ctc atg gcc gcc gtg cac tgc gcg gcc aag ccc gtc    577
   His Phe Ser Phe Leu Met Ala Ala Val His Cys Ala Ala Lys Pro Val
               180                 185                 190
   gtc tac ttc tgc ctg ggc agt gcc cag ggc cgc agg ctg ccc ctc cgg    625
   Val Tyr Phe Cys Leu Gly Ser Ala Gln Gly Arg Arg Leu Pro Leu Arg
           195                 200                 205
   ctg gtc ctc cag cga gcg ctg gga gac gag gct gag ctg ggg gcc gtc    673
   Leu Val Leu Gln Arg Ala Leu Gly Asp Glu Ala Glu Leu Gly Ala Val
       210                 215                 220
   agg gag acc tcc cgc cgg ggc ctg gtg gac ata gca gcc tga g          716
   Arg Glu Thr Ser Arg Arg Gly Leu Val Asp Ile Ala Ala  *
   225                 230                 235
   ccctggggcc cccgacccca gctgcagccc ccgtgaggca agagggtgac t           767
<210> 37
   <211> 237
   <212> PRT
   <213> Homo sapiens
<400> 37
   His Met Val Ala Ile Val Pro Asp Leu Leu Gln Gly Arg Leu Asp Phe
    1               5                  10                  15
   Pro Gly Phe Val Gln Thr Ser Leu Ala Thr Leu Arg Phe Phe Cys Tyr
               20                  25                  30
   Ile Val Gly Leu Ser Leu Leu Ala Ala Val Ser Val Glu Gln Cys Leu
           35                  40                  45
   Ala Ala Leu Phe Pro Ala Trp Tyr Ser Cys Arg Arg Pro Arg His Leu
       50                  55                  60
   Thr Thr Cys Val Cys Ala Leu Thr Trp Ala Leu Cys Leu Leu Leu His
   65                  70                  75                  80
   Leu Leu Leu Ser Ser Ala Cys Thr Gln Phe Phe Gly Glu Pro Ser Arg
                   85                  90                  95
   His Leu Cys Arg Thr Leu Trp Leu Val Ala Ala Val Leu Leu Ala Leu
               100                 105                 110
   Leu Cys Cys Thr Met Cys Gly Ala Ser Leu Met Leu Leu Leu Arg Val
           115                 120                 125
   Glu Arg Gly Pro Gln Arg Pro Pro Pro Arg Gly Phe Pro Gly Leu Ile
       130                 135                 140
   Leu Leu Thr Val Leu Leu Phe Leu Phe Cys Gly Leu Pro Phe Gly Ile
   145                 150                 155                 160
   Tyr Trp Leu Ser Arg Asn Leu Leu Trp Tyr Ile Pro His Tyr Phe Tyr
                   165                 170                 175
   His Phe Ser Phe Leu Met Ala Ala Val His Cys Ala Ala Lys Pro Val
               180                 185                 190
   Val Tyr Phe Cys Leu Gly Ser Ala Gln Gly Arg Arg Leu Pro Leu Arg
           195                 200                 205
   Leu Val Leu Gln Arg Ala Leu Gly Asp Glu Ala Glu Leu Gly Ala Val
       210                 215                 220
   Arg Glu Thr Ser Arg Arg Gly Leu Val Asp Ile Ala Ala
   225                 230                 235
<210> 38
   <211> 1361
   <212> DNA
   <213> Mus musculus
<220>
   <221> CDS
   <222> (48)...(1064)
<400> 38
   tctttttttt ttttcattgc agaactgaga ttgcaccact cctgaaa atg gac tta      56
                                                       Met Asp Leu
                                                       1
   gtc atc caa gac tgg acc att aat att aca gca ctg aaa gaa agc aat   104
   Val Ile Gln Asp Trp Thr Ile Asn Ile Thr Ala Leu Lys Glu Ser Asn
        5                  10                  15
   gac aat gga ata tca ttt tgt gaa gtt gtg tct cgt acc atg act ttt   152
   Asp Asn Gly Ile Ser Phe Cys Glu Val Val Ser Arg Thr Met Thr Phe
    20                  25                  30                  35
   ctt tcc ctc atc att gcc tta gtt ggg ctg gtt gga aat gcc aca gtg   200
   Leu Ser Leu Ile Ile Ala Leu Val Gly Leu Val Gly Asn Ala Thr Val
                    40                  45                  50
   tta tgg ttt ctg ggc ttc cag atg agc agg aat gcc ttc tct gtc tac   248
   Leu Trp Phe Leu Gly Phe Gln Met Ser Arg Asn Ala Phe Ser Val Tyr
                55                  60                  65
   atc ctc aac ctt gct ggt gct gac ttt gtc ttc atg tgc ttt caa att   296
   Ile Leu Asn Leu Ala Gly Ala Asp Phe Val Phe Met Cys Phe Gln Ile
            70                  75                  80
   gta cat tgt ttt tat att atc tta gac atc tac ttc atc ccc act aat     344
   Val His Cys Phe Tyr Ile Ile Leu Asp Ile Tyr Phe Ile Pro Thr Asn
       85                  90                  95
   ttt ttt tca tct tac act atg gtg tta aac att gct tac ctt agt ggt     392
   Phe Phe Ser Ser Tyr Thr Met Val Leu Asn Ile Ala Tyr Leu Ser Gly
   100                 105                 110                 115
   ctg agc atc ctc act gtc att agc act gaa cgc ttc cta tct gtc atg     440
   Leu Ser Ile Leu Thr Val Ile Ser Thr Glu Arg Phe Leu Ser Val Met
                   120                 125                 130
   tgg ccc atc tgg tac cgc tgc caa cgc cca agg cac aca tca gct gtc     488
   Trp Pro Ile Trp Tyr Arg Cys Gln Arg Pro Arg His Thr Ser Ala Val
               135                 140                 145
   ata tgt act gtg ctt tgg gtc ttg tcc ctg gtg ttg agc ctc ctg gaa     536
   Ile Cys Thr Val Leu Trp Val Leu Ser Leu Val Leu Ser Leu Leu Glu
           150                 155                 160
   gga aag gaa tgt ggc ttc cta tat tac act agt ggc cct ggt ttg tgt     584
   Gly Lys Glu Cys Gly Phe Leu Tyr Tyr Thr Ser Gly Pro Gly Leu Cys
       165                 170                 175
   aag aca ttt gat tta atc act act gca tgg tta att gtt tta ttt gtg     632
   Lys Thr Phe Asp Leu Ile Thr Thr Ala Trp Leu Ile Val Leu Phe Val
   180                 185                 190                 195
   gtt ctc ttg gga tcc agt ctg gcc ttg gtg ctt acc atc ttc tgt ggc     680
   Val Leu Leu Gly Ser Ser Leu Ala Leu Val Leu Thr Ile Phe Cys Gly
                   200                 205                 210
   tta cac aag gtt cct gtg acc agg ttg tat gtg acc att gtg ttt aca     728
   Leu His Lys Val Pro Val Thr Arg Leu Tyr Val Thr Ile Val Phe Thr
               215                 220                 225
   gtg ctt gtc ttc ctg atc ttt ggt ctg ccc tat ggg atc tac tgg ttc     776
   Val Leu Val Phe Leu Ile Phe Gly Leu Pro Tyr Gly Ile Tyr Trp Phe
           230                 235                 240
   ctc tta gag tgg att agg gaa ttt cat gat aat aaa cct tgt ggt ttt     824
   Leu Leu Glu Trp Ile Arg Glu Phe His Asp Asn Lys Pro Cys Gly Phe
       245                 250                 255
   cgt aac gtg aca ata ttt ctg tcc tgt att aac agc tgt gcc aac ccc     872
   Arg Asn Val Thr Ile Phe Leu Ser Cys Ile Asn Ser Cys Ala Asn Pro
   260                 265                 270                 275
   atc att tac ttc ctt gtt ggc tcc att agg cac cat cgg ttt caa cgg     920
   Ile Ile Tyr Phe Leu Val Gly Ser Ile Arg His His Arg Phe Gln Arg
                   280                 285                 290
   aag act ctc aag ctt ctt ctg cag aga gcc atg caa gac tct cct gag     968
   Lys Thr Leu Lys Leu Leu Leu Gln Arg Ala Met Gln Asp Ser Pro Glu
               295                 300                 305
   gag gaa gaa tgt gga gag atg ggt tcc tca aga aga cct aga gaa ata
   1016
   Glu Glu Glu Cys Gly Glu Met Gly Ser Ser Arg Arg Pro Arg Glu Ile
           310                 315                 320
   aaa act gtc tgg aag gga ctg aga gct gct ttg atc agg cat aaa tag
   1064
   Lys Thr Val Trp Lys Gly Leu Arg Ala Ala Leu Ile Arg His Lys  *
       325                 330                 335
   ctttgaagag aactatgttt ttatcacttt gtggcatttt cataatgttg tttagttgat 1124
   gacccaaggt taactcagtt ggggaagtag tcaatgttgt agaagttgat tgatattgaa 1184
   cttgttataa atactgagta cagtattttt gcagctatct tgctcagagc tttaccaact 1244
   ccatttgatg ggactcctta taagctctat ggggtccagg agaggtgttg accacaattg 1304
   acaaatccct cttcagaaga aaactcaaga aagtgcaatg aaaagttata tttcttt 1361
<210> 39
   <211> 338
   <212> PRT
   <213> Mus musculus
<400> 39
   Met Asp Leu Val Ile Gln Asp Trp Thr Ile Asn Ile Thr Ala Leu Lys
    1               5                  10                  15
   Glu Ser Asn Asp Asn Gly Ile Ser Phe Cys Glu Val Val Ser Arg Thr
               20                  25                  30
   Met Thr Phe Leu Ser Leu Ile Ile Ala Leu Val Gly Leu Val Gly Asn
           35                  40                  45
   Ala Thr Val Leu Trp Phe Leu Gly Phe Gln Met Ser Arg Asn Ala Phe
       50                  55                  60
   Ser Val Tyr Ile Leu Asn Leu Ala Gly Ala Asp Phe Val Phe Met Cys
   65                  70                  75                  80
   Phe Gln Ile Val His Cys Phe Tyr Ile Ile Leu Asp Ile Tyr Phe Ile
                   85                  90                  95
   Pro Thr Asn Phe Phe Ser Ser Tyr Thr Met Val Leu Asn Ile Ala Tyr
               100                 105                 110
   Leu Ser Gly Leu Ser Ile Leu Thr Val Ile Ser Thr Glu Arg Phe Leu
           115                 120                 125
   Ser Val Met Trp Pro Ile Trp Tyr Arg Cys Gln Arg Pro Arg His Thr
       130                 135                 140
   Ser Ala Val Ile Cys Thr Val Leu Trp Val Leu Ser Leu Val Leu Ser
   145                 150                 155                 160
   Leu Leu Glu Gly Lys Glu Cys Gly Phe Leu Tyr Tyr Thr Ser Gly Pro
                   165                 170                 175
   Gly Leu Cys Lys Thr Phe Asp Leu Ile Thr Thr Ala Trp Leu Ile Val
               180                 185                 190
   Leu Phe Val Val Leu Leu Gly Ser Ser Leu Ala Leu Val Leu Thr Ile
           195                 200                 205
   Phe Cys Gly Leu His Lys Val Pro Val Thr Arg Leu Tyr Val Thr Ile
       210                 215                 220
   Val Phe Thr Val Leu Val Phe Leu Ile Phe Gly Leu Pro Tyr Gly Ile
   225                 230                 235                 240
   Tyr Trp Phe Leu Leu Glu Trp Ile Arg Glu Phe His Asp Asn Lys Pro
                   245                 250                 255
   Cys Gly Phe Arg Asn Val Thr Ile Phe Leu Ser Cys Ile Asn Ser Cys
               260                 265                 270
   Ala Asn Pro Ile Ile Tyr Phe Leu Val Gly Ser Ile Arg His His Arg
           275                 280                 285
   Phe Gln Arg Lys Thr Leu Lys Leu Leu Leu Gln Arg Ala Met Gln Asp
       290                 295                 300
   Ser Pro Glu Glu Glu Glu Cys Gly Glu Met Gly Ser Ser Arg Arg Pro
   305                 310                 315                 320
   Arg Glu Ile Lys Thr Val Trp Lys Gly Leu Arg Ala Ala Leu Ile Arg
                   325                 330                 335
   His Lys
<210> 40
   <211> 1278
   <212> DNA
   <213> Mus musculus
<400> 40
   atttcctaat caagaatcta agcacctcag cctggaaaac gaacatcaca gtgctgaatg 60
   gaagctacta catcgatact tcagtttgtg tcaccaggaa ccaagccatg attttgcttt 120
   ccatcatcat ttccctggtt gggatgggac taaatgccat agtgctgtgg ttcctgggca 180
   tccgtatgca cacgaatgcc ttcactgtct acattctcaa cctggctatg gctgactttc 240
   tttacctgtg ctctcagttt gtaatttgtc ttcttattgc cttttatatc ttctactcaa 300
   ttgacatcaa catccctttg gttctttatg ttgtgccaat atttgcttat ctttcaggtc 360
   tgagcattct cagcaccatt agcattgagc gctgcttgtc tgtaatatgg cccatttggt 420
   atcgctgtaa acgtccaaga cacacatcag ctatcacatg ttttgtgctt tgggttatgt 480
   ccttattgtt gggtctcctg gaagggaagg catgtggctt actgtttaat agctttgact 540
   cttattggtg tgaaacattt gatgttatca ctaatatatg gtcagttgtt ttttttggtg 600
   ttctctgtgg gtctagcctc accctgcttg tcaggatctt ctgtggctca cagcgaattc 660
   ctatgaccag gctgtatgtg actattacac tcacagtctt ggtcttcctg atctttggtc 720
   ttccctttgg gatctattgg atactctatc agtggattag caatttttat tatgttgaaa 780
   tttgtaattt ttatcttgag atactattcc tatcctgtgt taacagctgt atgaacccca 840
   tcatttattt ccttgttggc tccattaggc accgaaggtt caggcggaag actctcaagc 900
   tacttctgca gagagccatg caagacaccc ctgaggagga acaaagtgga aataagagtt 960
   cttcagaaca ccctgaagaa ctggaaactg ttcagagctg cagctgacaa ctgcttgatc
   1020
   agacaaaaat ggttttgatg gaaatacttt ttcttatccg tgtggaccat ttttacaacc
   1080
   tttattcagt ttgttatctc atcttcaatt gtttaattag gacaataatt tttgtaaaag
   1140
   ttgagagaaa tgggtcttgt catactaata ctgaatgtag catttctgaa gctgtgttac
   1200
   ttagggattt accatctcct tttcatggga ctccttgtaa gtattctgtg gtagagaact
   1260
   tctcctattg ttgacaaa
   1278
<210> 41
   <211> 338
   <212> PRT
   <213> Mus musculus
<400> 41
   Met Ser Gly Asp Phe Leu Ile Lys Asn Leu Ser Thr Ser Ala Trp Lys
    1               5                  10                  15
   Thr Asn Ile Thr Val Leu Asn Gly Ser Tyr Tyr Ile Asp Thr Ser Val
               20                  25                  30
   Cys Val Thr Arg Asn Gln Ala Met Ile Leu Leu Ser Ile Ile Ile Ser
           35                  40                  45
   Leu Val Gly Met Gly Leu Asn Ala Ile Val Leu Trp Phe Leu Gly Ile
       50                  55                  60
   Arg Met His Thr Asn Ala Phe Thr Val Tyr Ile Leu Asn Leu Ala Met
   65                  70                  75                  80
   Ala Asp Phe Leu Tyr Leu Cys Ser Gln Phe Val Ile Cys Leu Leu Ile
                   85                  90                  95
   Ala Phe Tyr Ile Phe Tyr Ser Ile Asp Ile Asn Ile Pro Leu Val Leu
               100                 105                 110
   Tyr Val Val Pro Ile Phe Ala Tyr Leu Ser Gly Leu Ser Ile Leu Ser
           115                 120                 125
   Thr Ile Ser Ile Glu Arg Cys Leu Ser Val Ile Trp Pro Ile Trp Tyr
       130                 135                 140
   Arg Cys Lys Arg Pro Arg His Thr Ser Ala Ile Thr Cys Phe Val Leu
   145                 150                 155                 160
   Trp Val Met Ser Leu Leu Leu Gly Leu Leu Glu Gly Lys Ala Cys Gly
                   165                 170                 175
   Leu Leu Phe Asn Ser Phe Asp Ser Tyr Trp Cys Glu Thr Phe Asp Val
               180                 185                 190
   Ile Thr Asn Ile Trp Ser Val Val Phe Phe Gly Val Leu Cys Gly Ser
           195                 200                 205
   Ser Leu Thr Leu Leu Val Arg Ile Phe Cys Gly Ser Gln Arg Ile Pro
       210                 215                 220
   Met Thr Arg Leu Tyr Val Thr Ile Thr Leu Thr Val Leu Val Phe Leu
   225                 230                 235                 240
   Ile Phe Gly Leu Pro Phe Gly Ile Tyr Trp Ile Leu Tyr Gln Trp Ile
                   245                 250                 255
   Ser Asn Phe Tyr Tyr Val Glu Ile Cys Asn Phe Tyr Leu Glu Ile Leu
               260                 265                 270
   Phe Leu Ser Cys Val Asn Ser Cys Met Asn Pro Ile Ile Tyr Phe Leu
           275                 280                 285
   Val Gly Ser Ile Arg His Arg Arg Phe Arg Arg Lys Thr Leu Lys Leu
       290                 295                 300
   Leu Leu Gln Arg Ala Met Gln Asp Thr Pro Glu Glu Glu Gln Ser Gly
   305                 310                 315                 320
   Asn Lys Ser Ser Ser Glu His Pro Glu Glu Leu Glu Thr Val Gln Ser
                   325                 330                 335
   Cys Ser
<210> 42
   <211> 1009
   <212> DNA
   <213> Mus musculus
<400> 42
   ttttctaagc atggctctaa gaacctcact aataaccacc acagcaccgg ataaaaccag 60
   ccttccaatt tcaatttgta tcatcaagtt ccaagtcatg aatttgcttt ccatcaccat 120
   ttcccctgtt gggatggtac tgaatatcat agtgctgtgg ttcctgggct tccagatatg 180
   caggaatgcc ttctctgcct acatcctcaa cctggctgtg gctgattttc tcttcctgtg 240
   ttctcattct atattttctt ttcttattgt ctgcaaactg cactattttt tattctacat 300
   tagacagctt ttggatactg tgacaatgtt tgcttatgtt tttggcctga gcattaccac 360
   catcattagc attgagtgct gcctgtctat catgtggccc atctggtatc actgccaacg 420
   tccaagacac acatcagctg tcatttgtgt cttgctttgg gctctatctc tgctgtttcc 480
   tgctctgcag atggaaaaat gtagcgtcct gtttaatact tttgaatatt cttggtgtgg 540
   gataatcaat ataatctctg gtgcatggtt agttgtttta tttgtggttc tctgtgggtt 600
   cagcctcatc ctgctcctca ggatctcctg tggatcacag cagattcctg tgaccaggct 660
   gaatgtaact attgcactca gagtgctact cctcctgatc tttggtattc cctttgggat 720
   cttctggata gttgacaaat ggaatgaaga aaattttttc gttagagctt gtggtttttc 780
   acatcatata ctatacgtat actgtattaa catctgtgtc aatgctacca tatacttcct 840
   tgttggctcc attaggcatg gcaagtttca gaagatgact ctgaagctga ttctgcagag 900
   agctatacag ggcacccccg aggaagaagg tggagagagg ggtccttaag gaaatactga 960
   agaactggga acagtctagt gcagcaaccg agagctgctt taataataa
   1009
<210> 43
   <211> 312
   <212> PRT
   <213> Mus musculus
<400> 43
   Met Ala Leu Arg Thr Ser Leu Ile Thr Thr Thr Ala Pro Asp Lys Thr
    1               5                  10                  15
   Ser Leu Pro Ile Ser Ile Cys Ile Ile Lys Phe Gln Val Met Asn Leu
               20                  25                  30
   Leu Ser Ile Thr Ile Ser Pro Val Gly Met Val Leu Asn Ile Ile Val
           35                  40                  45
   Leu Trp Phe Leu Gly Phe Gln Ile Cys Arg Asn Ala Phe Ser Ala Tyr
       50                  55                  60
   Ile Leu Asn Leu Ala Val Ala Asp Phe Leu Phe Leu Cys Ser His Ser
   65                  70                  75                  80
   Ile Phe Ser Phe Leu Ile Val Cys Lys Leu His Tyr Phe Leu Phe Tyr
                   85                  90                  95
   Ile Arg Gln Leu Leu Asp Thr Val Thr Met Phe Ala Tyr Val Phe Gly
               100                 105                 110
   Leu Ser Ile Thr Thr Ile Ile Ser Ile Glu Cys Cys Leu Ser Ile Met
           115                 120                 125
   Trp Pro Ile Trp Tyr His Cys Gln Arg Pro Arg His Thr Ser Ala Val
       130                 135                 140
   Ile Cys Val Leu Leu Trp Ala Leu Ser Leu Leu Phe Pro Ala Leu Gln
   145                 150                 155                 160
   Met Glu Lys Cys Ser Val Leu Phe Asn Thr Phe Glu Tyr Ser Trp Cys
                   165                 170                 175
   Gly Ile Ile Asn Ile Ile Ser Gly Ala Trp Leu Val Val Leu Phe Val
               180                 185                 190
   Val Leu Cys Gly Phe Ser Leu Ile Leu Leu Leu Arg Ile Ser Cys Gly
           195                 200                 205
   Ser Gln Gln Ile Pro Val Thr Arg Leu Asn Val Thr Ile Ala Leu Arg
       210                 215                 220
   Val Leu Leu Leu Leu Ile Phe Gly Ile Pro Phe Gly Ile Phe Trp Ile
   225                 230                 235                 240
   Val Asp Lys Trp Asn Glu Glu Asn Phe Phe Val Arg Ala Cys Gly Phe
                   245                 250                 255
   Ser His His Ile Leu Tyr Val Tyr Cys Ile Asn Ile Cys Val Asn Ala
               260                 265                 270
   Thr Ile Tyr Phe Leu Val Gly Ser Ile Arg His Gly Lys Phe Gln Lys
           275                 280                 285
   Met Thr Leu Lys Leu Ile Leu Gln Arg Ala Ile Gln Gly Thr Pro Glu
       290                 295                 300
   Glu Glu Gly Gly Glu Arg Gly Pro
   305                 310
<210> 44
   <211> 1219
   <212> DNA
   <213> Mus musculus
<400> 44
   tttatggacc tgtgccagat attcctacat aatcacatgg tcctgactga gactatcttg 60
   tgttcatatc tcgatttctt tgcaggaatg ccagtggaaa attcctaagc atgggtacaa 120
   ccaccctggc ctggaacatt aacaacaccg ctgaaaatgg aagttacact gaaatgttct 180
   cctgtatcac caagttcaat accctgaatt ttcttactgt catcatagct gtggttggcc 240
   tggcaggaaa cggcatagtg ctatggcttc tagccttcca cctgcatagg aatgccttct 300
   ctgtctatgt cctcaatctg gctggtgctg atttcttgta ccttttcact caagttgtgc 360
   attccctgga atgtgtcctt cagttagata ataactcctt ttatattctc ctcattgtaa 420
   caatgtttgc ttaccttgca ggtttgtgta tgattgcagc catcagtgct gaacgctgcc 480
   tatctgttat gtggcctatc tggtatcact gccaaagacc aagacacaca tcagccatca 540
   tgtgtgctct ggtctgggtt tcctctctat tgttgagcct cgtggtaggg ctaggctgtg 600
   gttttctgtt cagttattat gattattatt tctgtattac tttgaatttt atcactgctg 660
   catttttaat agtgttatct gtggttcttt ctgtatctag cctggccctg ttggtgaaga 720
   ttgtgtgggg gtcacacagg attcctgtga ccaggttctt tgtgaccatt gctctcacag 780
   tggtggtctt catatacttt ggcatgccct ttggtatctg ctggttcctc ttatcaagga 840
   ttatggagtt tgatagcatt ttctttaaca atgtttatga aataatagaa ttcctgtcct 900
   gtgttaacag ctgtgccaat cccatcattt acttccttgt tggctccatt agacaacaca 960
   ggttgcgatg gcagtctctg aagctacttc ttcagagagc catgcaggac actcctgagg
   1020
   aagagagtgg agagaggggt ccttcgcaaa ggtctgggga actggaaaca gtctagtaca
   1080
   gtagttgagt gagtccctgg tcaaacatag tttctgtgag agtcaatttt gcctttatct
   1140
   atataagcaa ttttcataat ttgtttaatc agtagagaat atagtcattt tatagaaatt
   1200
   aggagaaatg agcttgtta
   1219
<210> 45
   <211> 321
   <212> PRT
   <213> Mus musculus
<400> 45
   Met Gly Thr Thr Thr Leu Ala Trp Asn Ile Asn Asn Thr Ala Glu Asn
    1               5                  10                  15
   Gly Ser Tyr Thr Glu Met Phe Ser Cys Ile Thr Lys Phe Asn Thr Leu
               20                  25                  30
   Asn Phe Leu Thr Val Ile Ile Ala Val Val Gly Leu Ala Gly Asn Gly
           35                  40                  45
   Ile Val Leu Trp Leu Leu Ala Phe His Leu His Arg Asn Ala Phe Ser
       50                  55                  60
   Val Tyr Val Leu Asn Leu Ala Gly Ala Asp Phe Leu Tyr Leu Phe Thr
   65                  70                  75                  80
   Gln Val Val His Ser Leu Glu Cys Val Leu Gln Leu Asp Asn Asn Ser
                   85                  90                  95
   Phe Tyr Ile Leu Leu Ile Val Thr Met Phe Ala Tyr Leu Ala Gly Leu
               100                 105                 110
   Cys Met Ile Ala Ala Ile Ser Ala Glu Arg Cys Leu Ser Val Met Trp
           115                 120                 125
   Pro Ile Trp Tyr His Cys Gln Arg Pro Arg His Thr Ser Ala Ile Met
       130                 135                 140
   Cys Ala Leu Val Trp Val Ser Ser Leu Leu Leu Ser Leu Val Val Gly
   145                 150                 155                 160
   Leu Gly Cys Gly Phe Leu Phe Ser Tyr Tyr Asp Tyr Tyr Phe Cys Ile
                   165                 170                 175
   Thr Leu Asn Phe Ile Thr Ala Ala Phe Leu Ile Val Leu Ser Val Val
               180                 185                 190
   Leu Ser Val Ser Ser Leu Ala Leu Leu Val Lys Ile Val Trp Gly Ser
           195                 200                 205
   His Arg Ile Pro Val Thr Arg Phe Phe Val Thr Ile Ala Leu Thr Val
       210                 215                 220
   Val Val Phe Ile Tyr Phe Gly Met Pro Phe Gly Ile Cys Trp Phe Leu
   225                 230                 235                 240
   Leu Ser Arg Ile Met Glu Phe Asp Ser Ile Phe Phe Asn Asn Val Tyr
                   245                 250                 255
   Glu Ile Ile Glu Phe Leu Ser Cys Val Asn Ser Cys Ala Asn Pro Ile
               260                 265                 270
   Ile Tyr Phe Leu Val Gly Ser Ile Arg Gln His Arg Leu Arg Trp Gln
           275                 280                 285
   Ser Leu Lys Leu Leu Leu Gln Arg Ala Met Gln Asp Thr Pro Glu Glu
       290                 295                 300
   Glu Ser Gly Glu Arg Gly Pro Ser Gln Arg Ser Gly Glu Leu Glu Thr
   305                 310                 315                 320
   Val
<210> 46
   <211> 1281
   <212> DNA
   <213> Mus musculus
<400> 46
   atggtcctga cagagagtat catgtgttca tatctctatt tttttgcggg aacaccactg 60
   gaaacttcct aaacatgggt ctaaccactc cagcctggaa cattaacaac acagtagtga 120
   atggaagtaa caatactgaa catttctcct gtgtcagcaa gttcaatacc ctgaactttc 180
   ttactgtcat cattgccatg tttggcctgg caggaaatgc catagtccta tggcttctag 240
   ccttccacct gcctaggaat gccttctctg tctatgtctg caacttggct tgtgctgatt 300
   tcttgcaact ttgcactcag attttaggtt ccctggaatg tttccttcag ttaaatagga 360
   gacacacttt ttttctcacc gttgtattta tgtttgctta ccttgcaggt ttgtgtatga 420
   ttgcagccat cagtgttgag cgctctctat ctgttatgtg gcccatctgg tatcactgcc 480
   aaagaccaag acatacatca tccatcatgt gtgctctgct ctgggctttc tgtctactgt 540
   tgaatttcct attaggggaa ggctgtggcc ttctgttcag tgatcctaaa tattatttct 600
   gtattacttg tgccttaatc actactgcac ttataatatt attaactgtg gttccttctg 660
   tgtccagcct ggccctgttg gtcaagatga tctgtggatc acacaggatt cctgtgacca 720
   ggttctatgt gaccattgct ctcacattgg tggtcttcat attcttgggt ctgccctttg 780
   ggatttactc atctttcttg ataatgttta aggagtttca aagcattttc tcttaccatg 840
   tccttgaagt gacaatattc ctgtcctgtg ttaacagctg tgccaatccc atcatttact 900
   ttcttgttgg ctccattagg cagcacaggt tgcaatggca gtctctgaag ctacttcttc 960
   agagagccat gcaggacact cctgaggaag atagtggaga gagggttccc tcacaaaggt
   1020
   ctggggaact ggaaagtgtt tagtgcagta gttgagtgag tctttgatca gacatggtta
   1080
   ctctgagagt cagttttgcc tttgtttatg taagcaattt tcacaatctt gtacaatttg
   1140
   taaagaaata gtcattttat agaaattggg agaaaggggc ttgttacaca gaaactgagt
   1200
   gcaacaccat aaagctgtct tatgtgggtc tcattacatt ctcttgtgat ataagccttg
   1260
   taatcacttg ggaacaaaac t
   1281
<210> 47
   <211> 322
   <212> PRT
   <213> Mus musculus
<400> 47
   Met Gly Leu Thr Thr Pro Ala Trp Asn Ile Asn Asn Thr Val Val Asn
    1               5                  10                  15
   Gly Ser Asn Asn Thr Glu His Phe Ser Cys Val Ser Lys Phe Asn Thr
               20                  25                  30
   Leu Asn Phe Leu Thr Val Ile Ile Ala Met Phe Gly Leu Ala Gly Asn
           35                  40                  45
   Ala Ile Val Leu Trp Leu Leu Ala Phe His Leu Pro Arg Asn Ala Phe
       50                  55                  60
   Ser Val Tyr Val Cys Asn Leu Ala Cys Ala Asp Phe Leu Gln Leu Cys
   65                  70                  75                  80
   Thr Gln Ile Leu Gly Ser Leu Glu Cys Phe Leu Gln Leu Asn Arg Arg
                   85                  90                  95
   His Thr Phe Phe Leu Thr Val Val Phe Met Phe Ala Tyr Leu Ala Gly
               100                 105                 110
   Leu Cys Met Ile Ala Ala Ile Ser Val Glu Arg Ser Leu Ser Val Met
           115                 120                 125
   Trp Pro Ile Trp Tyr His Cys Gln Arg Pro Arg His Thr Ser Ser Ile
       130                 135                 140
   Met Cys Ala Leu Leu Trp Ala Phe Cys Leu Leu Leu Asn Phe Leu Leu
   145                 150                 155                 160
   Gly Glu Gly Cys Gly Leu Leu Phe Ser Asp Pro Lys Tyr Tyr Phe Cys
                   165                 170                 175
   Ile Thr Cys Ala Leu Ile Thr Thr Ala Leu Ile Ile Leu Leu Thr Val
               180                 185                 190
   Val Pro Ser Val Ser Ser Leu Ala Leu Leu Val Lys Met Ile Cys Gly
           195                 200                 205
   Ser His Arg Ile Pro Val Thr Arg Phe Tyr Val Thr Ile Ala Leu Thr
       210                 215                 220
   Leu Val Val Phe Ile Phe Leu Gly Leu Pro Phe Gly Ile Tyr Ser Ser
   225                 230                 235                 240
   Phe Leu Ile Met Phe Lys Glu Phe Gln Ser Ile Phe Ser Tyr His Val
                   245                 250                 255
   Leu Glu Val Thr Ile Phe Leu Ser Cys Val Asn Ser Cys Ala Asn Pro
               260                 265                 270
   Ile Ile Tyr Phe Leu Val Gly Ser Ile Arg Gln His Arg Leu Gln Trp
           275                 280                 285
   Gln Ser Leu Lys Leu Leu Leu Gln Arg Ala Met Gln Asp Thr Pro Glu
       290                 295                 300
   Glu Asp Ser Gly Glu Arg Val Pro Ser Gln Arg Ser Gly Glu Leu Glu
   305                 310                 315                 320
   Ser Val
<210> 48
   <211> 1280
   <212> DNA
   <213> Mus musculus
<400> 48
   ccccactagt tcataacaca gaatttaaca tgggttcttc ttccacccat aggaatgaac 60
   tccactcttg acagcagccc agctccaggt ctgaccatca gtcccaccat ggaccttgtg 120
   acctggatct acttttcagt gacattcctc gccatggcca cgtgtgtggg gggggatggc 180
   aggcaactca ttggtgattt ggctcctgag ctgcaatggc atgcagaggt ctcccttctg 240
   tgtctatgtg ctcaacctgg cggtggctga cttcctcttc ttattctgca tggcctccat 300
   gctcagcctg gaaacagggc ccctgctcat agtcaacatt tctgccaaaa tctatgaagg 360
   gatgaggaga atcaagtact ttgcctatac agcaggcctg agcctgctga cagccatcag 420
   cacccagcgc tgcctctccg tgcttttccc catctggtat aagtgccacc ggccccggca 480
   cctgtcatca gtggtatctg gtgcactctg ggcactggcc ttcctgatga acttcctggc 540
   ttctttcttc tgcgtccaat tctggcatcc caacaaacac cagtgcttca aggtggacat 600
   tgttttcaac agtcttatcc tggggatctt catgccggtc atgatcctga ccagcaccat 660
   cctcttcatc cgggtgcgga agaacagcct gatgcagaga cggcggcccc ggcggctgta 720
   cgtggtcatc ctgacttcca tccttgtctt cctcacctgt tctctgccct tgggcatcaa 780
   ctggttctta ctctactggg tggatgtgaa acgggatgtg aggctacttt atagctgcgt 840
   atcacgcttc tcttcgtctt tgagcagcag tgccaacccg gtcatttact tcctcgtggg 900
   cagccagaag agccaccggc tgcaggagtc cctgggtgct gtgctggggc gggcactgcg 960
   ggatgagcct gagccagagg gcagagagac gccatccacg tgtactaatg atggggtctg
   1020
   aagggagccc aaccaggaac tcctccaaag ccccacccag cccttcccta aaagtaccca
   1080
   gcaagcctgc aatgcaaagg ccttgcacct caaaatgttt gggtcacgtt cctctctgcc
   1140
   agggagggtt caccactatc accttgtgtt cctaatctaa actaagaggt gaggcaatat
   1200
   atctttctgt tttacctgtt tagacacaga tcctaacttt gggtcccatc atgggcaagg
   1260
   ctgtctggga aatggagttt
   1280
<210> 49
   <211> 281
   <212> PRT
   <213> Mus musculus
<400> 49
   Met Ala Gly Asn Ser Leu Val Ile Trp Leu Leu Ser Cys Asn Gly Met
   1               5                   10                  15
   Gln Arg Ser Pro Phe Cys Val Tyr Val Leu Asn Leu Ala Val Ala Asp
               20                  25                  30
   Phe Leu Phe Leu Phe Cys Met Ala Ser Met Leu Ser Leu Glu Thr Gly
           35                  40                  45
   Pro Leu Leu Ile Val Asn Ile Ser Ala Lys Ile Tyr Glu Gly Met Arg
       50                  55                  60
   Arg Ile Lys Tyr Phe Ala Tyr Thr Ala Gly Leu Ser Leu Leu Thr Ala
   65                  70                  75                  80
   Ile Ser Thr Gln Arg Cys Leu Ser Val Leu Phe Pro Ile Trp Tyr Lys
                   85                  90                  95
   Cys His Arg Pro Arg His Leu Ser Ser Val Val Ser Gly Ala Leu Trp
               100                 105                 110
   Ala Leu Ala Phe Leu Met Asn Phe Leu Ala Ser Phe Phe Cys Val Gln
           115                 120                 125
   Phe Trp His Pro Asn Lys His Gln Cys Phe Lys Val Asp Ile Val Phe
       130                 135                 140
   Asn Ser Leu Ile Leu Gly Ile Phe Met Pro Val Met Ile Leu Thr Ser
   145                 150                 155                 160
   Thr Ile Leu Phe Ile Arg Val Arg Lys Asn Ser Leu Met Gln Arg Arg
                   165                 170                 175
   Arg Pro Arg Arg Leu Tyr Val Val Ile Leu Thr Ser Ile Leu Val Phe
               180                 185                 190
   Leu Thr Cys Ser Leu Pro Leu Gly Ile Asn Trp Phe Leu Leu Tyr Trp
           195                 200                 205
   Val Asp Val Lys Arg Asp Val Arg Leu Leu Tyr Ser Cys Val Ser Arg
       210                 215                 220
   Phe Ser Ser Ser Leu Ser Ser Ser Ala Asn Pro Val Ile Tyr Phe Leu
   225                 230                 235                 240
   Val Gly Ser Gln Lys Ser His Arg Leu Gln Glu Ser Leu Gly Ala Val
                   245                 250                 255
   Leu Gly Arg Ala Leu Arg Asp Glu Pro Glu Pro Glu Gly Arg Glu Thr
               260                 265                 270
   Pro Ser Thr Cys Thr Asn Asp Gly Val
           275                 280
<210> 50
   <211> 1170
   <212> DNA
   <213> Mus musculus
<400> 50
   gacttctgca gacatcagcc atgacgtccc tgagcgtgca cacagattct cccagcaccc 60
   agggagaaat ggctttcaac ctgaccatcc tgtccctcac agagctcctc agcctgggcg 120
   ggctgctggg caatggagtg gccctctggc tgctcaacca aaatgtctac aggaacccct 180
   tctccatcta tctcttggat gtggcctgcg ccgacctcat cttcctctgc tgccacatgg 240
   tggccatcat ccctgagctg ctgcaggacc agctgaactt ccctgaattt gtacatatca 300
   gcctgaccat gctgcggttc ttctgctaca ttgtgggcct gagcctcctg gcggccatca 360
   gcacggagca gtgcctggcc actctcttcc ctgcctggta cctgtgccgc cgcccacgct 420
   acctgaccac ctgtgtgtgt gcgctcatct gggtgctctg cctgctactg gacctgctgc 480
   tgagcggcgc ctgcacccag ttctttggag cacccagcta ccacctgtgt gacatgctgt 540
   ggctggtggt ggcagttctc ctggctgccc tgtgctgcac catgtgtgtg accagcctgc 600
   tcctgctgct gcgggtggag cgtggtccag agagacacca gcctcggggc ttccccaccc 660
   tggtcctgct ggccgtcctg ctcttcctct tctgcggcct gccctttggc atcttctggc 720
   tgtccaagaa cctgtcctgg cacatccccc tctacttcta tcatttcagc ttcttcatgg 780
   ccagtgtgca cagtgcagcc aagcctgcca tctacttttt cttgggcagc acacctggcc 840
   agaggtttcg ggaacccctc cggctggtgc tccagcgggc acttggagat gaggctgagc 900
   tgggagctgg gagagaggct tcccaagggg gacttgtgga catgactgtc taagcacagt 960
   gggtcacaac tgcagcttca gcccatgggg gtccagggga gctgcctgat gtaggtaaag 1020
   ctgggatcag agctccatca gtaagactct tgagggacat ctttgctgat gacccagtgc 1080
   tgtgtcccct gggaggattc tgggaagggg caagcagaga gtgatgcttc tgtggagggc 1140
   ctggggttgt gtgtgttagg cagagctcct 1170
<210> 51
   <211> 310
   <212> PRT
   <213> Mus musculus
<400> 51
   Met Thr Ser Leu Ser Val His Thr Asp Ser Pro Ser Thr Gln Gly Glu
   1               5                   10                  15
   Met Ala Phe Asn Leu Thr Ile Leu Ser Leu Thr Glu Leu Leu Ser Leu
               20                  25                  30
   Gly Gly Leu Leu Gly Asn Gly Val Ala Leu Trp Leu Leu Asn Gln Asn
           35                  40                  45
   Val Tyr Arg Asn Pro Phe Ser Ile Tyr Leu Leu Asp Val Ala Cys Ala
       50                  55                  60
   Asp Leu Ile Phe Leu Cys Cys His Met Val Ala Ile Ile Pro Glu Leu
   65                  70                  75                  80
   Leu Gln Asp Gln Leu Asn Phe Pro Glu Phe Val His Ile Ser Leu Thr
                   85                  90                  95
   Met Leu Arg Phe Phe Cys Tyr Ile Val Gly Leu Ser Leu Leu Ala Ala
               100                 105                 110
   Ile Ser Thr Glu Gln Cys Leu Ala Thr Leu Phe Pro Ala Trp Tyr Leu
           115                 120                 125
   Cys Arg Arg Pro Arg Tyr Leu Thr Thr Cys Val Cys Ala Leu Ile Trp
       130                 135                 140
   Val Leu Cys Leu Leu Leu Asp Leu Leu Leu Ser Gly Ala Cys Thr Gln
   145                 150                 155                 160
   Phe Phe Gly Ala Pro Ser Tyr His Leu Cys Asp Met Leu Trp Leu Val
                       165                     170                     175
   Val Ala Val Leu Leu Ala Ala Leu Cys Cys Thr Met Cys Val Thr Ser
               180                     185                     190
   Leu Leu Leu Leu Leu Arg Val Glu Arg Gly Pro Glu Arg His Gln Pro
           195                     200                     205
   Arg Gly Phe Pro Thr Leu Val Leu Leu Ala Val Leu Leu Phe Leu Phe
       210                     215                     220
   Cys Gly Leu Pro Phe Gly Ile Phe Trp Leu Ser Lys Asn Leu Ser Trp
   225                     230                     235                     240
   His Ile Pro Leu Tyr Phe Tyr His Phe Ser Phe Phe Met Ala Ser Val
                   245                     250                     255
   His Ser Ala Ala Lys Pro Ala Ile Tyr Phe Phe Leu Gly Ser Thr Pro
               260                     265                     270
   Gly Gln Arg Phe Arg Glu Pro Leu Arg Leu Val Leu Gln Arg Ala Leu
           275                     280                     285
   Gly Asp Glu Ala Glu Leu Gly Ala Gly Arg Glu Ala Ser Gln Gly Gly
       290                     295                     300
   Leu Val Asp Met Thr Val
   305                 310
<210> 52
   <211> 1519
   <212> DNA
   <213> Mus musculus
<400> 52
   tgtgttccca gcagcaccca gtgcagggtt tctggcccta aacatytyma gcctccacaa 60
   tggcactcac aacaacaaaa tccaatggac gaaacccatc ccctggaagt accagcatca 120
   agattctgat cccaaacttg atgatcatca tctttggact ggtcgggctg acaggaaacg 180
   ccattgtgtt ctggctcctg ggcttccact tgcgcaggaa tgccttctca gtctacatcc 240
   taaacttggc cctggctgac ttcctcttcc tcctctgtcg catcatagct tccacgcaga 300
   aacttctcac gttctcctca cccaacatta cctttctcat ttgcctttac accttcaggg 360
   tgattctcta catcgcaggc ctgagcatgc tcactgccat cagcattgag cgctgcctgt 420
   ctgtcctgtg ccccatctgg tatcgctgcc accgcccaga acacacatca actgtcatgt 480
   gtgctgcaat ctgggtcctg tccctgttga tctgcattct gaataggtat ttctgcggtt 540
   tcttagatac caaatatgta aatgactatg ggtgtatggc atcaaatttc tttaatgctg 600
   catacctgat gtttttgttt gtagtcctct gtgtgtccag cctggctctg ctggccaggt 660
   tgttctgtgg cactgggcgg atgaagctta ccagattgta cgtgaccatc atgctgacca 720
   ttttggtttt tctcctctgc gggttgccct gtggcttata ctggttcctg ttattctgga 780
   ttaagaatgg ttttgctgta tttgatttta acttttatct agcatcaact gtcctgagtg 840
   ctattaatag ctctgccaac cccatcattt acttcttcgt gggctcattc aggcatcggt 900
   tgaagcacca gaccctcaaa atggttctcc agagtgcact gcaggatact cctgagacag 960
   ctgaaaacat ggtggagatg tcaagaagca aagcagagcc gtgatgaaga gcctctgcct
   1020
   ggacctcgga ggtagctttg gagtgagcac ttccctgctg caattgacca ctgtccactc
   1080
   tcctctcagc ttactgactc aacatgcctc agtggtccac caacatcttc aacagctctc
   1140
   cattgattta gtttttctaa ctctcccaag taatagcatt aatcagaaag tatcatgtct
   1200
   gcatccttct tgacattaat caaattctca aactaacttc ctctgaagct ttcttgctga
   1260
   ttctttggaa cttttgttgc catggaacta gcccaggtcc agaaccatga ctctcgtatc
   1320
   tgtgatggtt ctgtacctga atataaagac aaaggagcct agagatgatc ctgtccattc
   1380
   ccaaatacca cctagagagc tggtctccca ggattgcaga caagcctgtg agcacaggta
   1440
   agaccaccac ttctgctcaa agggacatgc ctggaactac tcaggacaca ggtacagagg
   1500
   agcattttgg gacaagata
   1519
<210> 53
   <211> 303
   <212> PRT
   <213> Mus musculus
<400> 53
   Asn Pro Ser Pro Gly Ser Thr Ser Ile Lys Ile Leu Ile Pro Asn Leu
    1               5                  10                  15
   Met Ile Ile Ile Phe Gly Leu Val Gly Leu Thr Gly Asn Ala Ile Val
               20                  25                  30
   Phe Trp Leu Leu Gly Phe His Leu Arg Arg Asn Ala Phe Ser Val Tyr
           35                  40                  45
   Ile Leu Asn Leu Ala Leu Ala Asp Phe Leu Phe Leu Leu Cys Arg Ile
       50                  55                  60
   Ile Ala Ser Thr Gln Lys Leu Leu Thr Phe Ser Ser Pro Asn Ile Thr
   65                  70                  75                  80
   Phe Leu Ile Cys Leu Tyr Thr Phe Arg Val Ile Leu Tyr Ile Ala Gly
                   85                  90                  95
   Leu Ser Met Leu Thr Ala Ile Ser Ile Glu Arg Cys Leu Ser Val Leu
               100                 105                 110
   Cys Pro Ile Trp Tyr Arg Cys His Arg Pro Glu His Thr Ser Thr Val
           115                 120                 125
   Met Cys Ala Ala Ile Trp Val Leu Ser Leu Leu Ile Cys Ile Leu Asn
       130                 135                 140
   Arg Tyr Phe Cys Gly Phe Leu Asp Thr Lys Tyr Val Asn Asp Tyr Gly
   145                 150                 155                 160
   Cys Met Ala Ser Asn Phe Phe Asn Ala Ala Tyr Leu Met Phe Leu Phe
                   165                 170                 175
   Val Val Leu Cys Val Ser Ser Leu Ala Leu Leu Ala Arg Leu Phe Cys
               180                 185                 190
   Gly Thr Gly Arg Met Lys Leu Thr Arg Leu Tyr Val Thr Ile Met Leu
           195                 200                 205
   Thr Ile Leu Val Phe Leu Leu Cys Gly Leu Pro Cys Gly Leu Tyr Trp
       210                 215                 220
   Phe Leu Leu Phe Trp Ile Lys Asn Gly Phe Ala Val Phe Asp Phe Asn
   225                 230                 235                 240
   Phe Tyr Leu Ala Ser Thr Val Leu Ser Ala Ile Asn Ser Ser Ala Asn
                   245                 250                 255
   Pro Ile Ile Tyr Phe Phe Val Gly Ser Phe Arg His Arg Leu Lys His
               260                 265                 270
   Gln Thr Leu Lys Met Val Leu Gln Ser Ala Leu Gln Asp Thr Pro Glu
           275                 280                 285
   Thr Ala Glu Asn Met Val Glu Met Ser Arg Ser Lys Ala Glu Pro
       290                 295                 300
<210> 54
   <211> 2093
   <212> DNA
   <213> Mus musculus
<400> 54
   tggtatgcac tcactgataa gcggatatag cccaaaagct gcaaacaacc aggataaaat 60
   tcacagacca catgaagctc aataagaagg aagaacaaag tgtaggtgtt tcagtccttc 120
   ttagaaggag aacaaaatac tcacaggagc aaatatggag atacagtata gagcagagac 180
   taaaggaaag gtcattcaga gactgtccca actggggatt cattccatat agagatacca 240
   aacccagact ctaaattgga tgcaaacaag tgcatgccaa aaggagctag ataaggtaac 300
   cctgtctcaa aaaaaaaaaa aaggctgtca cctgaaaggc cctgtcaaag gcttacaaat 360
   acagaagcag atgttagtag tcaacaattg gacagagcat ggggttccta atagaggagt 420
   tagaggaagg aattagggag ttgaagggat ttgcagcccc ataagaacaa caatatcaac 480
   caaccggaca ctcccccaga tatcacaggg tctaagccat caacaaagga gtacacatgg 540
   ctccagatgc acatatagca gaggacggcc atgtcatgca tcaatggaag aagagatcct 600
   tgtacctatg aaggatcgat agatgaccca gtgtagggga atcaaggaca gaaaggttgg 660
   agtggatgtg tggactggcc ggactgacag gaaatgccat tgtgttctgg ctcctgctct 720
   tccacttgca caggaatgct ttctcaatct acatcttaaa tttggtcata gctgacttcc 780
   ttttcctcct tggtcacatc atagcttcca caatgcaact tctcaaggtt tcctacctca 840
   acattatttt tctttaccgt ttttacacaa tcatgatggt gctctacaac acaggcctga 900
   ccatgctcag tgccatcaac actaagcact gcctgtctgt cctgtgtccc atctggtatc 960
   gctcccactg cacaaaacac acatcaactg tcatatgtgc tgctatacgg gacctgtccc 1020
   tgttgatctg ctttctgaat acgtatttct gtggtctctt agataccaaa tataaaaatg 1080
   acaatgggtg tctggcatcg aatttcttta ttaatgcata ccctgatgtt tttgtttgta 1140
   gtcctactgt ctgtccactc tggctctgct ggccaggttg ttctgtggtg ctgggaagat 1200
   gaaatttaca agattattcg tgaccatcat gctgacagtt ttagtttttc tcctctgtgg 1260
   gttgccctct gccatctact ggttcctgtt aatctggatt aagattgatt atggtgtatt 1320
   tgcttatgat gtttttctgg catcactcgt cctgagtgct gttaacagct gtgccaaccc 1380
   catcatttac ttcttcgtgg gctctttcag gcatcggttg aagcaccaaa ccctcaaaat 1440
   ggttctccag aatgtactgc aggacactcc tgagacagct gaaaacatgg tagagatgtc 1500
   aagaggcaaa gcagagccat gatgaagagc ctctgcctgg agctcagagg tggctttgga 1560
   gtgagcactg ccctgatgta cttgaccact gtccactctc ctctcagctt actgactaga 1620
   catgcctcag tggtccacca tctccaagag ctctccactg actttgtttt ctacctctcc 1680
   tgaataatag cattaatcag aaagtatcat gtctacatcc ttcttgacat taatcaaatt 1740
   ctcatgctat cttcccctga agctttcttg ctgtttcttt gggacttttt gttgccatgg 1800
   aaataacaaa ggtccagaac catgactctc ttgcctgtga ttgttctgta cctgaatgta 1860
   aagataaagg agccaggaga tgatcctgta tcacggtgct ccataccaaa ataccaccaa 1920
   gagagctggt ctcccaggag tgcagacaag cctgtgagca caggtaagac caccatttct 1980
   gctcaaaggg acatgcctgg aaccctcagt acacaggaac agaggagcct ggaactggat 2040
   atttccagtt tccatctgca ccccagagct gactctgtac cacagctctc cat 2093
<210> 55
   <211> 282
   <212> PRT
   <213> Mus musculus
<400> 55
   Gly Leu Ala Gly Leu Thr Gly Asn Ala Ile Val Phe Trp Leu Leu Leu
    1               5                  10                  15
   Phe His Leu His Arg Asn Ala Phe Ser Ile Tyr Ile Leu Asn Leu Val
               20                  25                  30
   Ile Ala Asp Phe Leu Phe Leu Leu Gly His Ile Ile Ala Ser Thr Met
           35                  40                  45
   Gln Leu Leu Lys Val Ser Tyr Leu Asn Ile Ile Phe Leu Tyr Arg Phe
       50                  55                  60
   Tyr Thr Ile Met Met Val Leu Tyr Asn Thr Gly Leu Thr Met Leu Ser
   65                  70                  75                  80
   Ala Ile Asn Thr Lys His Cys Leu Ser Val Leu Cys Pro Ile Trp Tyr
                   85                  90                  95
   Arg Ser His Cys Thr Lys His Thr Ser Thr Val Ile Cys Ala Ala Ile
               100                 105                 110
   Arg Asp Leu Ser Leu Leu Ile Cys Phe Leu Asn Thr Tyr Phe Cys Gly
           115                 120                 125
   Leu Leu Asp Thr Lys Tyr Lys Asn Asp Asn Gly Cys Leu Ala Ser Asn
       130                 135                 140
   Phe Phe Ile Asn Ala Tyr Leu Met Phe Leu Phe Val Val Leu Cys Leu
   145                 150                 155                 160
   Ser Thr Leu Ala Leu Leu Ala Arg Leu Phe Cys Gly Ala Gly Lys Met
                   165                 170                 175
   Lys Phe Thr Arg Leu Phe Val Thr Ile Met Leu Thr Val Leu Val Phe
               180                 185                 190
   Leu Leu Cys Gly Leu Pro Ser Ala Ile Tyr Trp Phe Leu Leu Ile Trp
           195                 200                 205
   Ile Lys Ile Asp Tyr Gly Val Phe Ala Tyr Asp Val Phe Leu Ala Ser
       210                 215                 220
   Leu Val Leu Ser Ala Val Asn Ser Cys Ala Asn Pro Ile Ile Tyr Phe
   225                 230                 235                 240
   Phe Val Gly Ser Phe Arg His Arg Leu Lys His Gln Thr Leu Lys Met
                   245                 250                 255
   Val Leu Gln Asn Val Leu Gln Asp Thr Pro Glu Thr Ala Glu Asn Met
               260                 265                 270
   Val Glu Met Ser Arg Gly Lys Ala Glu Pro
           275                 280
<210> 56
   <211> 2401
   <212> DNA
   <213> Mus musculus
<400> 56
   acttgctaac ttctgtaatt gatggccccc aaacaggaaa catcattata tctcacatga 60
   ctataattaa tcacccactg tgttcatatc tttgactcaa aatctttccc ttgtagttaa 120
   cttcagacga gcactcgata gattatagta agatctgaga cttctcagag ttatgaccat 180
   gttgggaatt tggttttccc aagctcagga atctgtccaa atggattgcc acaactacac 240
   agagatggaa ggaaaggtag agaactttcc cagtgccatt acattctaca ggctacagga 300
   gccttggctg gtcagaatgc aactttggtt ggcactcaga acaatgttaa ttttcctttt 360
   caattctctc ctatctcttt ccactctgct catttgttct gttgcagcac atctgtgact 420
   tccatgtatg aaagtagttt ctttttctac tctactctct caattatctt tttaattcta 480
   ctatttctac tcacacatta aaatgtgtgt atgtgtgttt gtgttcatac gtgtgtgttg 540
   aggctgattt tttccttatt tgctgtatat gaaactctac attctgttgt acaccccaga 600
   tgtcatgtgt taaattgtat ttcatgttct gctctctaaa acctacattc aggtacagaa 660
   caatcacaga caagagagtc atggttttgg acctgggcta tttccatgrc aacaaaagtt 720
   tcaaagaaac agcaagaaag cttcagagga agttagcacg acaatttgat taatgtcaag 780
   aaggatgcag acatgatact ttctgattaa tgcttttact caggagatgg agaaaaacta 840
   agttatggaa gagctgttga aggtgttggt agaccactga ggcatgccaa gtaggtcagc 900
   tgaaaggaga gtggacagtg tggtcaagtg cagcagggca gtgctcactc caaaactacc 960
   tctgaaatcc aggcagaggc tcttcatcat ggctctgctt tgctttttga catctccact
   1020
   atgttttcag gtgtctcagg aatgtcctgc agtgcactct ggataaccat tttgagggtc
   1080
   tggtgctgca atcgatgcct gaaggagccc acgaagaagt aaatgatggg gttggcacag
   1140
   ctgttaagag cagtcaggac acttgatgcc ataaaaagac taaaatcaaa tacaataaaa
   1200
   acattcttaa tcttggataa caggaaccag tagatgccac agggcaaccc gcagaggaga
   1260
   aaaaccaaaa tggtcagcat gatggtcacg tacaatctgg taagtttcat acgcccagcg
   1320
   ccacagaaca acctggccag cagagccagg ctggatagac agaggaccac aaacaaaaac
   1380
   atcaggtatg cagcagtaaa gaagtttgat gccatacatc catagtcatt tacatatttg
   1440
   gtatctaaga aaacgcagaa atacttattc agaatgctga tcaacaggga caggacccag
   1500
   atcatagcac acgtgacagt tgatgtgtgt tctgggcggt ggcagcgata ccagatgggg
   1560
   cacagtacag acagacaccg ttcagtgccg atggcactga gtatgctcag gcctgcaatg
   1620
   tagagaacca gcatgatgct gaagaagcac ctgcgaaaga taatgttagg gtaggaaacc
   1680
   ttgagaagaa acagagtgga agctatgatg tgacagagga ggaagaggaa gtcagccaga
   1740
   gccaagttta ggatgtagac tgagaaggca ttcttgcgca agcggaagcc caggagccag
   1800
   aacacaatgg catttcctgt catcccaacc agtccgaaga tgatgatcat caagtgtggg
   1860
   atcagggtgc tgatgtcaat acttccaggg atggtttcgt ccattagatt tgttgtcgac
   1920
   ggtgccattg atgaggcaga ggtgtttagg gccagaaacc ctgcaccggt gctgctggga
   1980
   acacaaagaa gaaatgaggc tttccctatg aacacacctt ttgtttttct tttccctttt
   2040
   ttgtttttgt tgttgttttt aaaaattttt ttctattgga tattttcttt atttaaattt
   2100
   caaatgttat cccctttcct gcttttccct ctccaggaaa tccccatctc atcctccctc
   2160
   cttctgcttc tatgatggtg ttcctcaacc cacacaccca cttccacctc tctgccctcg
   2220
   attcccatac actggagcat ctattgagcc ttcaaaggtc ctaggacctt tttttccatt
   2280
   gatgcatgac acagcaattc tctcatacat atacagctgg agccatgttt acttwctttg
   2340
   ttgatggctt attccatgga ggctggggcc agggggkgtg tctgatttgt tgatattggt
   2400
   t 2401
<210> 57
   <211> 305
   <212> PRT
   <213> Mus musculus
<400> 57
   Met Asp Glu Thr Ile Pro Gly Ser Ile Asp Ile Ser Thr Leu Ile Pro
   1               5                   10                  15
   His Leu Met Ile Ile Ile Phe Gly Leu Val Gly Met Thr Gly Asn Ala
               20                  25                  30
   Ile Val Phe Trp Leu Leu Gly Phe Arg Leu Arg Lys Asn Ala Phe Ser
           35                  40                  45
   Val Tyr Ile Leu Asn Leu Ala Leu Ala Asp Phe Leu Phe Leu Leu Cys
       50                  55                  60
   His Ile Ile Ala Ser Thr Leu Phe Leu Leu Lys Val Ser Tyr Pro Asn
   65                  70                  75                  80
   Ile Ile Phe Arg Arg Cys Phe Phe Ser Ile Met Leu Val Leu Tyr Ile
                   85                  90                  95
   Ala Gly Leu Ser Ile Leu Ser Ala Ile Gly Thr Glu Arg Cys Leu Ser
               100                 105                 110
   Val Leu Cys Pro Ile Trp Tyr Arg Cys His Arg Pro Glu His Thr Ser
           115                 120                 125
   Thr Val Thr Cys Ala Met Ile Trp Val Leu Ser Leu Leu Ile Ser Ile
       130                 135                 140
   Leu Asn Lys Tyr Phe Cys Val Phe Leu Asp Thr Lys Tyr Val Asn Asp
   145                 150                 155                 160
   Tyr Gly Cys Met Ala Ser Asn Phe Phe Thr Ala Ala Tyr Leu Met Phe
                   165                 170                 175
   Leu Phe Val Val Leu Cys Leu Ser Ser Leu Ala Leu Leu Ala Arg Leu
               180                 185                 190
   Phe Cys Gly Ala Gly Arg Met Lys Leu Thr Arg Leu Tyr Val Thr Ile
           195                 200                 205
   Met Leu Thr Ile Leu Val Phe Leu Leu Cys Gly Leu Pro Cys Gly Ile
       210                 215                 220
   Tyr Trp Phe Leu Leu Ser Lys Ile Lys Asn Val Phe Ile Val Phe Asp
   225                 230                 235                 240
   Phe Ser Leu Phe Met Ala Ser Ser Val Leu Thr Ala Leu Asn Ser Cys
                   245                 250                 255
   Ala Asn Pro Ile Ile Tyr Phe Phe Val Gly Ser Phe Arg His Arg Leu
               260                 265                 270
   Gln His Gln Thr Leu Lys Met Val Ile Gln Ser Ala Leu Gln Asp Ile
           275                 280                 285
   Pro Glu Thr Pro Glu Asn Ile Val Glu Met Ser Lys Ser Lys Ala Glu
       290                 295                 300
   Pro
   305
<210> 58
   <211> 2110
   <212> DNA
   <213> Mus musculus
<400> 58
   agaggtgtaa gtgggtatgt gggttgagga acacccttca tagaagcagg gggagggagg 60
   atgagatggg gttttctggg aaggggcaaa agcaggaaag tggataacat ttgtaattta 120
   aataaagaaa atatccaata caaaaaattt aaaaaaaaaa acacaaaacc acacaaaaaa 180
   aagacaaaaa aaaagaaatt aaaagttgtg ttcatagtta atgcctcatt tttctttgtg 240
   ttcccagcaa aaccagtgca gggtttctgg ccctaaacac cttcagcctt ttcaatggca 300
   cccaacgaca accaatacaa tggacgaaac catccctgga cgtattgaca tcgagaccct 360
   gatcccaaac ttgatgatca tcatcttcgg actggtcggg ctgacaggaa atggcattgt 420
   gttctggctc ctgggcttcc gcatgcacag gaatgccttc ttagtctaca tcctaaactt 480
   ggccctggct gactttctct tccttctctg tcacatcatt aattccacaa tgcttcttct 540
   caaggttctc ccactcaact ggatscttgt tccattgctt taacaccatc agaacggttc 600
   tatacatcac aggcctgagc atgctcagcg ccatcagcac tgagcgctgc ctgtctgtcc 660
   tgtgccccat ctggtatcga tgccgtcgcc gagaaaacac atcagctgtc atgtgtgctg 720
   tgatctgggt cctgtccctg ttgatctgta ttctgaatag ttatttctgt tattactctg 780
   gtcccaaaga tgtaaataac tctgtgtgtc tggtatcgaa attcttcatc agtacatacc 840
   caatgttttt gtttgtagtc ctctgtctgt ccaccctgac tctgctggcc aggttgttct 900
   gtggtgctgg gaagaggaaa tttaccagat tattcgtgac catcatactg accattttgg 960
   tttttcttct gtgtgggttg cccctgggct tctactggtt cctgttacac tgtattaagg 1020
   gtagtttcag tgtactacat aatagacttt ttcaggcatc acttgtccta acttctgtta 1080
   acagctgtgc caaccccatc atttacttct tcgtgggctc cttcagggat cgggtgaagc 1140
   accagaccct caaaatggta ctccagaatg cactgcagga cactcctgag acacctgaaa 1200
   acaaggtgga gatgtcaaga agtaaagcag agccatgatg aagagactcg gccaggacct 1260
   cagaggtagc tttggagtsa gwactgccct gctrcacttg accactgtcc actctcctct 1320
   cagcttacts acttyggatg cctcagtggt ccaacaacam cttcaaawgc tctccactga 1380
   cttagtattt atacctctcc caagtaatag cattaatcag aaagtatcat gtctgcatcc 1440
   ttcttgacat taatccaatt ctcatactaa cttcatctga aactttcttg atgttccttt 1500
   ggaacttttg ttgccatggt aatagccyag gtccagcacc atgactctct tgtctgtgat 1560
   tkttctgtac ctgaatgtaa agtcaaagga gccaggagat gatcctgtgt cacagtgctc 1620
   attacccaaa caccaccaac agagcttgtc tcccaggagt gcagacacgc ctgtgaacac 1680
   aggtaagacc accacttctg cttaaaggga catgcctgga accctcagaa cacaggaaga 1740
   aaagagcagc cttggacagg atacttccag tttccaactg caccccggag ctgaccctgt 1800
   gccacagctc tccataccca aattcctccc agaaagaacy ggtcwaccaa gagtactgac 1860
   acayaggctt gcaggaggga caagccacmg tcagagatag caaggaccag ctaacaccag 1920
   agataaccag atggcaagag gcaagggcaa aaatataagc aatgggaacc aagactattt 1980
   ggcatcatca gaacctagtt ctctcaacat ggtgagccat ggctactcca acagacaaga 2040
   aaagcatgac tctgatttaa tgtcacagat gatgatgatg atgatgatga tgatgatgat 2100
   gatgatgatg 2110
<210> 59
   <211> 305
   <212> PRT
   <213> Mus musculus
<400> 59
   Met Asp Glu Thr Ile Pro Gly Arg Ile Asp Ile Glu Thr Leu Ile Pro
    1               5                  10                  15
   Asn Leu Met Ile Ile Ile Phe Gly Leu Val Gly Leu Thr Gly Asn Gly
               20                  25                  30
   Ile Val Phe Trp Leu Leu Gly Phe Arg Met His Arg Asn Ala Phe Leu
           35                  40                  45
   Val Tyr Ile Leu Asn Leu Ala Leu Ala Asp Phe Leu Phe Leu Leu Cys
       50                  55                  60
   His Ile Ile Asn Ser Thr Met Leu Leu Leu Lys Val Leu Pro Pro Thr
   65                  70                  75                  80
   Gly Ser Leu Phe His Cys Phe Asn Thr Ile Arg Thr Val Leu Tyr Ile
                   85                  90                  95
   Thr Gly Leu Ser Met Leu Ser Ala Ile Ser Thr Glu Arg Cys Leu Ser
               100                 105                 110
   Val Leu Cys Pro Ile Trp Tyr Arg Cys Arg Arg Arg Glu Asn Thr Ser
           115                 120                 125
   Ala Val Met Cys Ala Val Ile Trp Val Leu Ser Leu Leu Ile Cys Ile
       130                 135                 140
   Leu Asn Ser Tyr Phe Cys Tyr Tyr Ser Gly Pro Lys Asp Val Asn Asn
   145                 150                 155                 160
   Ser Val Cys Leu Val Ser Lys Phe Phe Ile Ser Thr Tyr Pro Met Phe
                   165                 170                 175
   Leu Phe Val Val Leu Cys Leu Ser Thr Leu Thr Leu Leu Ala Arg Leu
               180                 185                 190
   Phe Cys Gly Ala Gly Lys Arg Lys Phe Thr Arg Leu Phe Val Thr Ile
           195                 200                 205
   Ile Leu Thr Ile Leu Val Phe Leu Leu Cys Gly Leu Pro Leu Gly Phe
       210                 215                 220
   Tyr Trp Phe Leu Leu His Cys Ile Lys Gly Ser Phe Ser Val Leu His
   225                 230                 235                 240
   Asn Arg Leu Phe Gln Ala Ser Leu Val Leu Thr Ser Val Asn Ser Cys
                   245                 250                 255
   Ala Asn Pro Ile Ile Tyr Phe Phe Val Gly Ser Phe Arg Asp Arg Val
               260                 265                 270
   Lys His Gln Thr Leu Lys Met Val Leu Gln Asn Ala Leu Gln Asp Thr
           275                 280                 285
   Pro Glu Thr Pro Glu Asn Lys Val Glu Met Ser Arg Ser Lys Ala Glu
       290                 295                 300
   Pro
   305
<210> 60
   <211> 740
   <212> DNA
   <213> Mus musculus
<400> 60
   cagggtttct ggccctaaac acctcagcct cggcaatgac acccacgaca aacaattcaa 60
   tggacgaaac catccctgga agtattggca ctgagaccct gattcaaaac ttgatgatca 120
   tcatcttcgg actggtcggg ctgacaggaa atgccattgt gttctggctc ctgggcttcc 180
   acttgcacag gaatgccttt ttagtctaca tcctaaactt ggccctggct gatttcctct 240
   tccttctctg tcacatcata gattccacag tgtttcttct caaggttccc ccacccaacc 300
   ggatcttggt ccattgcttt aacatcatca gaattgtact ctacatcaca ggcttgagca 360
   tgctcagtgc catcagcatg gagcgctgcc tgtctgtcct gtgccccatc tggtatcgct 420
   gccgccgccc agaaaacaca tcaactgtca tttgtgctgt gatctggatc ctgtccctgt 480
   tgttctgcat tctgaatgga tatttctgtt atttctctgg tcccaactat gtaaatgact 540
   atgtgtgttt tgcatcggac atctttatca gaacataccc aatgtttttg tttgtagtcc 600
   tctgtctgtc cactctggct ctgctggcca ggttgttctg tggtgctggg aagacgaaat 660
   ttaccagatt attcgtcacc atcatactga ccgttttggt ttttcttctc tgtgggttgc 720
   ccctgggctt cttctggttc                                             740
<210> 61
   <211> 227
   <212> PRT
   <213> Mus musculus
<400> 61
   Met Asp Glu Thr Ile Pro Gly Ser Ile Gly Thr Glu Thr Leu Ile Gln
    1               5                  10                  15
   Asn Leu Met Ile Ile Ile Phe Gly Leu Val Gly Leu Thr Gly Asn Ala
               20                  25                  30
   Ile Val Phe Trp Leu Leu Gly Phe His Leu His Arg Asn Ala Phe Leu
           35                  40                  45
   Val Tyr Ile Leu Asn Leu Ala Leu Ala Asp Phe Leu Phe Leu Leu Cys
       50                  55                  60
   His Ile Ile Asp Ser Thr Val Phe Leu Leu Lys Val Pro Pro Pro Asn
   65                  70                  75                  80
   Arg Ile Leu Val His Cys Phe Asn Ile Ile Arg Ile Val Leu Tyr Ile
                   85                  90                  95
   Thr Gly Leu Ser Met Leu Ser Ala Ile Ser Met Glu Arg Cys Leu Ser
               100                 105                 110
   Val Leu Cys Pro Ile Trp Tyr Arg Cys Arg Arg Pro Glu Asn Thr Ser
           115                 120                 125
   Thr Val Ile Cys Ala Val Ile Trp Ile Leu Ser Leu Leu Phe Cys Ile
       130                 135                 140
   Leu Asn Gly Tyr Phe Cys Tyr Phe Ser Gly Pro Asn Tyr Val Asn Asp
   145                 150                 155                 160
   Tyr Val Cys Phe Ala Ser Asp Ile Phe Ile Arg Thr Tyr Pro Met Phe
                   165                 170                 175
   Leu Phe Val Val Leu Cys Leu Ser Thr Leu Ala Leu Leu Ala Arg Leu
               180                 185                 190
   Phe Cys Gly Ala Gly Lys Thr Lys Phe Thr Arg Leu Phe Val Thr Ile
           195                 200                 205
   Ile Leu Thr Val Leu Val Phe Leu Leu Cys Gly Leu Pro Leu Gly Phe
       210                 215                 220
   Phe Trp Phe
   225
<210> 62
   <211> 1979
   <212> DNA
   <213> Mus musculus
<400> 62
   aatacacaaa attaaaaaca acaacaacaa caacacgccc cacaaaaaaa gaaaacaaaa 60
   acaaaaaaga aattaaaagt tgtggtcata gtaaaggcct cacttcttct ttgtgttccc 120
   agcaacacca gtgcagggtt tctggcccga aacacctcag cctcgacaat gacacccaca 180
   acaacaaatc caatgaacga aaccatccct ggaagtattg acatcgagac cctgatacca 240
   aacttgatga tcatcatctt cggactggtc gggctgacag gaaatgccat tgtgttctgg 300
   ctcctgggct tccgcatgca caggactgcc ttctcagtct acatcctaaa cttggccctg 360
   gctgacttcc tcttccttct ctgtcacatc ataaattcca cagtgcttct tctccaggtt 420
   tccccaccca acagtacctt ggtccattgc tttgacacca tcagaatggt tctctacatc 480
   gcaggcctga gcatgctcag tgccattagc actgagcact gcctgtctgt cctgtgcccc 540
   atctggtatc gctgccgccg cccagaacat acttcaactg tcatgtgtgc tgtgatctgg 600
   gtcctgtccc tgttgatctg cattctaagt ggatatttct gtaatttttt tcttcacaaa 660
   tatgtatatt actctgtgtg tcgggcattg gaattctgta tcggaacata ccccgatgtt 720
   tttgttttgt agtcctctgt ctgtccaccc tggctctgct ggtcaggttg ttctgtggta 780
   ctgggaaggc aaaatttacc agattattcg tgaccatcat gctgactgtt ttggtttttc 840
   ttctctgtgg gttgcccctg tgtttcttct ggttcctggt agtctggatt aagcgtcctc 900
   tcagtgtact aaatattaca ttttattttg catccattgt cctaactgtt gttaacagct 960
   gtgccaaccc catcatttac ttcttcgtgg gctccttcag gcatcggttg aagcaacaga
   1020
   acctcaaaat ggttctccag aatgcactgc aggacactgc tgagacacct gaaaacgtgg
   1080
   cagagatttc aagaagcaaa gcagagccct gatgaggagc ctctgcctgg acctcagagg
   1140
   tggctttggc actgagcact gccctgctgc acttgcccac tgtccactct cctctcagct
   1200
   tactgactgg caataactca gtggtacaac aacaccttca aaagctcacc actgacttag
   1260
   tatttctacc tatcccaagt aatagcatta atcagaaagt atcatgtctg catccttcta
   1320
   gacattattc aaattctcat ccaacttcat ctgaaacttt cttgctattt ctttggaaca
   1380
   ttttttgcca tggtaatagc ccaggtccag catcatgcct ctcttacctt tgattgttct
   1440
   gtacctgaat gtaaagaaaa aggagagaga agatgatcct ctgtcacagt gctcattacc
   1500
   caagcaccac taagagagct tgtctcccag gagtgcagac aaacctgtga gcacaggtaa
   1560
   gactaccact tctgcttaaa ggggcatgcc tggaacccac aggacacagg taaagaggag
   1620
   cagcctgaga aaggatactt tccagtttcc aactgcaccc tggagctgac cctgtgccac
   1680
   agctctcccc accttaattc ttcccagaaa gaactggtct mccaggaagt actgacacat
   1740
   agccttgcag gaggtacaag acactgtcac agatagcaag accagctaac accagagata
   1800
   accagatggc aagaggcaag ggcaaaaaca taagcaatgg gaaccaaggc tacttggcat
   1860
   catcagaacc tagttctctc aacaaagtga gccctggata ctccaacaca caagaaaagt
   1920
   atgactgtga ttaaaagtca ccgatgatga tgatgatgat gatgatgatg atgatgatg
   1979
<210> 63
   <211> 305
   <212> PRT
   <213> Mus musculus
<400> 63
   Met Asn Glu Thr Ile Pro Gly Ser Ile Asp Ile Glu Thr Leu Ile Pro
   1               5                   10                  15
   Asn Leu Met Ile Ile Ile Phe Gly Leu Val Gly Leu Thr Gly Asn Ala
               20                  25                  30
   Ile Val Phe Trp Leu Leu Gly Phe Arg Met His Arg Thr Ala Phe Ser
           35                  40                  45
   Val Tyr Ile Leu Asn Leu Ala Leu Ala Asp Phe Leu Phe Leu Leu Cys
       50                  55                  60
   His Ile Ile Asn Ser Thr Val Leu Leu Leu Gln Val Ser Pro Pro Asn
   65                  70                  75                  80
   Ser Thr Leu Val His Cys Phe Asp Thr Ile Arg Met Val Leu Tyr Ile
                   85                  90                  95
   Ala Gly Leu Ser Met Leu Ser Ala Ile Ser Thr Glu His Cys Leu Ser
               100                 105                 110
   Val Leu Cys Pro Ile Trp Tyr Arg Cys Arg Arg Pro Glu His Thr Ser
           115                 120                 125
   Thr Val Met Cys Ala Val Ile Trp Val Leu Ser Leu Leu Ile Cys Ile
       130                 135                 140
   Leu Ser Gly Tyr Phe Cys Asn Phe Phe Leu His Lys Tyr Val Tyr Tyr
   145                 150                 155                 160
   Ser Val Cys Arg Ala Leu Glu Phe Cys Ile Gly Thr Tyr Pro Met Phe
                   165                 170                 175
   Leu Phe Val Val Leu Cys Leu Ser Thr Leu Ala Leu Leu Val Arg Leu
               180                 185                 190
   Phe Cys Gly Thr Gly Lys Ala Lys Phe Thr Arg Leu Phe Val Thr Ile
           195                 200                 205
   Met Leu Thr Val Leu Val Phe Leu Leu Cys Gly Leu Pro Leu Cys Phe
       210                 215                 220
   Phe Trp Phe Leu Val Val Trp Ile Lys Arg Pro Leu Ser Val Leu Asn
   225                 230                 235                 240
   Ile Thr Phe Tyr Phe Ala Ser Ile Val Leu Thr Val Val Asn Ser Cys
                   245                 250                 255
   Ala Asn Pro Ile Ile Tyr Phe Phe Val Gly Ser Phe Arg His Arg Leu
               260                 265                 270
   Lys Gln Gln Asn Leu Lys Met Val Leu Gln Asn Ala Leu Gln Asp Thr
           275                 280                 285
   Ala Glu Thr Pro Glu Asn Val Ala Glu Ile Ser Arg Ser Lys Ala Glu
       290                 295                 300
   Pro
   305
<210> 64
   <211> 1485
   <212> DNA
   <213> Mus musculus
<400> 64
   aacaacacaa aaccctgaaa aaaaaaaaga aattaaaagt tttgttcata gtaaaggcct 60
   catttcttct ttgtgttcac agcaacatca gtgcacggtt aatggcaata aacacctcag 120
   cctcggcaat ggcacccacg acaacaaatc caaagggaag caaacaatcc ctgggaagta 180
   ttgacatcga gaccctgatc tcaaacttga tgatcatcat tttcgggctg gtagggctgc 240
   caggaaatgc cattgtgttc tggctcctgg gcttctgctt gcacaggaat gccttcttag 300
   tctacatcct aaacttggcc ctggctgacg tcctcttcct tctctgtcac atcataaatt 360
   ccacagtgct tcttctcaag gttcccccac ccaacggtaa tattggtcca ttgcttcaac 420
   atcatcagaa ttgttctcta catcacaggc ctgagcatgc tcagtgccat catcactgag 480
   cgctgcctgt ctatcctgtg ccccatctgg tatcgctgcc accgcccaga acacacatca 540
   actgccatgt gtgctgtgat ctgggtcctg tctctgttga tctgcattct tggaagaata 600
   tttctgtaat tttttccttc acaaatatgt aaattactct gtgtgtctgg cattggactc 660
   ctttatcgga acatacccaa tgtttttgct tgtagtcctc tgtctgtcca ccatggctct 720
   gctggccagg ttgttctgtg gttctgggaa gacgaaattt accagattat ttgtgaccat 780
   catgcttacc gttttggttt ttcttctctg cttggtttgc ccctgggctt cttctggttc 840
   ctgttactct ggattaaggg tgcttacagt gtactaggtt atagatttta ttttgcatca 900
   attgtcctaa ctgctgttaa cagctgtgcc aaccccatca tttacttctt catgggctca 960
   ttcaggcaac gattgcagca caagaccctc aaaatcgttc tccagagtgc actgcacgac
   1020
   actcctgaga cacctgaaaa catggtggag atgtcaagaa gcaaagcaga gccataatga
   1080
   agagcctctg cctggacctc agaggtggat ttggagtgag aactgcccta cgcttgacca
   1140
   ctgtccactc tcctctcagc ttactgactt tggatgccta agtggtccaa caacaacttc
   1200
   aaaatctctc cactgactta gtatttatac ctctcccaag taatagcatt aatcagaaag
   1260
   tatcatgtct gcatccttct tgacattaat ccaattctca tactaacttc atctgaaact
   1320
   ttcttgctgt ttctttggaa cttttgttgc catagtaata gcccagatcc agcaccatga
   1380
   ctcacttgtc tgtgattatt ctgtacctga atgtaaagaa aaaggcagga gatgatcctg
   1440
   tatcacagtg ctcattacac aaacaccacc aagaaagctc gtctc
   1485
<210> 65
   <211> 300
   <212> PRT
   <213> Mus musculus
<400> 65
   Gly Ser Ile Asp Ile Glu Thr Leu Ile Ser Asn Leu Met Ile Ile Ile
    1               5                  10                  15
   Phe Gly Leu Val Gly Leu Pro Gly Asn Ala Ile Val Phe Trp Leu Leu
                   20                  25                  30
   Gly Phe Cys Leu His Arg Asn Ala Phe Leu Val Tyr Ile Leu Asn Leu
           35                  40                  45
   Ala Leu Ala Asp Val Leu Phe Leu Leu Cys His Ile Ile Asn Ser Thr
       50                  55                  60
   Val Leu Leu Leu Lys Val Pro His Pro Thr Val Ile Leu Val His Cys
   65                  70                  75                  80
   Phe Asn Ile Ile Arg Ile Val Leu Tyr Ile Thr Gly Leu Ser Met Leu
                   85                  90                  95
   Ser Ala Ile Ile Thr Glu Arg Cys Leu Ser Ile Leu Cys Pro Ile Trp
               100                 105                 110
   Tyr Arg Cys His Arg Pro Glu His Thr Ser Thr Ala Met Cys Ala Val
           115                 120                 125
   Ile Trp Val Leu Ser Leu Leu Ile Cys Ile Leu Gly Lys Tyr Phe Cys
       130                 135                 140
   Asn Phe Phe Leu His Lys Tyr Val Asn Tyr Ser Val Cys Leu Ala Leu
   145                 150                 155                 160
   Asp Ser Phe Ile Gly Thr Tyr Pro Met Phe Leu Leu Val Val Leu Cys
                   165                 170                 175
   Leu Ser Thr Met Ala Leu Leu Ala Arg Leu Phe Cys Gly Ser Gly Lys
               180                 185                 190
   Thr Lys Phe Thr Arg Leu Phe Val Thr Ile Met Leu Thr Val Leu Val
           195                 200                 205
   Phe Leu Leu Cys Leu Gly Leu Pro Leu Gly Phe Phe Trp Phe Leu Leu
       210                 215                 220
   Leu Trp Ile Lys Gly Ala Tyr Ser Val Leu Gly Tyr Arg Phe Tyr Phe
   225                 230                 235                 240
   Ala Ser Ile Val Leu Thr Ala Val Asn Ser Cys Ala Asn Pro Ile Ile
                   245                 250                 255
   Tyr Phe Phe Met Gly Ser Phe Arg Gln Arg Leu Gln His Lys Thr Leu
               260                 265                 270
   Lys Ile Val Leu Gln Ser Ala Leu His Asp Thr Pro Glu Thr Pro Glu
           275                 280                 285
   Asn Met Val Glu Met Ser Arg Ser Lys Ala Glu Pro
       290                 295                 300
<210> 66
   <211> 1518
   <212> DNA
   <213> Mus musculus
<400> 66
   aacaacaaaa aaaaaaaaca gaaaaagaaa ttaaaagttg tgtccatagt gaaggcctca 60
   tttcttcttt gtgtttccag caacaccagt gcagggtttc tggacctaaa cacctcagcc 120
   tcggcaatag cacccacaac aaccaaacca atggacgaaa ccatccctgg aagtattgac 180
   actgagaccc tgtatccaac acttgatgat catcatcttc ggactggtcg ggctgacagg 240
   aaatggcatt gtgttgtggc tcctgggctt ccacttgcaa aggaatgcct ttttagtcta 300
   catcctaaac ttggccctag ctgacttcct ctaccttctc tgtcacatca tagattccac 360
   aatgcttctt ctcaaggttc ccccacccaa ctggatcttg gtccattgct ttaggaccat 420
   ccaaattttt ctctacatca caggcctgag catgctcagt gccatcagca cagagcgctg 480
   cctgtctgtc ctgtgcccca tctggtatcg ctgccgccgc ccagaaaaca catcaactgt 540
   gatgtgtgct gtgatctggg tcctgtcctt gttgatctgc attctgcatg gatatttttc 600
   tgttatttct ctggtctcag ttatgaaaat tactctgtgt gttttgcatc agcgatcatt 660
   atcagttcat acccaacgtt tttgcttgta gtcctctgtc tgtccaccct ggctctgctg 720
   gccaggttgt tctgtggtgc tgggaagagg aaattttcca gattattcgt gaccatcata 780
   cttaccgttt tggtttttct tctctgtggg ttgccctggg gagccctctg gttcccatta 840
   ctctggattc agggtggttt ctggaaaaga ctttttcagg catcaattgt cctatcttct 900
   gttaacagct gtgccaaccc catcatttat ttcttcgtgg gctcattcag gcatcgattg 960
   aagcaccaga cccttaaaat ggttctccag aatgcactgc aggacactcc tgagacaact 1020
   gaaaacatgg tggagatgtc aagaagtaaa gcagagccat gatgaagagc ctctgcctgg 1080
   acctcagagg tggatttgga gtgagcactg ccctgctgca cttgaccact gtccactctc 1140
   ctctcagctt actgacttgg aatgcctcag tggtccaaaa acaccttcaa aagctctcca 1200
   ctgactaagt atttctacct atcccaagta atagcattaa tcagaaagta ccatgtctgc 1260
   atccttcttg acattaatca aattctctta ctatcttcat ctgaaacttt cttgttgttt 1320
   ctttggaact tttgttgcca tggtaatagc ccaagtccag caccatgact ttcttatctg 1380
   tgattgttct atacctgaat gtaaaggcaa aggagccagg agatgatcct gtgttacagt 1440
   gctcattacc caaacaccac caagagagct tgtctcccag gagtgcagac acgcctgtga 1500
   acacaggtaa gaccacca 1518
<210> 67
   <211> 303
   <212> PRT
   <213> Mus musculus
<400> 67
   Met Asp Glu Thr Ile Pro Gly Ser Ile Asp Thr Glu Thr Leu Tyr Pro
    1               5                  10                  15
   Asn Leu Met Ile Ile Ile Phe Gly Leu Val Gly Leu Thr Gly Asn Gly
               20                  25                  30
   Ile Val Leu Trp Leu Leu Gly Phe His Leu Gln Arg Asn Ala Phe Leu
           35                  40                  45
   Val Tyr Ile Leu Asn Leu Ala Leu Ala Asp Phe Leu Tyr Leu Leu Cys
       50                  55                  60
   His Ile Ile Asp Ser Thr Met Leu Leu Leu Lys Val Pro Pro Pro Asn
   65                  70                  75                  80
   Trp Ile Leu Val His Cys Phe Arg Thr Ile Gln Ile Phe Leu Tyr Ile
                   85                  90                  95
   Thr Gly Leu Ser Met Leu Ser Ala Ile Ser Thr Glu Arg Cys Leu Ser
               100                 105                 110
   Val Leu Cys Pro Ile Trp Tyr Arg Cys Arg Arg Pro Glu Asn Thr Ser
           115                 120                 125
   Thr Val Met Cys Ala Val Ile Trp Val Leu Ser Leu Leu Ile Cys Ile
       130                 135                 140
   Leu His Gly Tyr Phe Cys Cys Tyr Phe Ser Gly Leu Ser Tyr Glu Asn
   145                 150                 155                 160
   Tyr Ser Val Cys Phe Ala Ser Ala Ile Ile Ile Ser Ser Tyr Pro Thr
                   165                 170                 175
   Phe Leu Leu Val Val Leu Cys Leu Ser Thr Leu Ala Leu Leu Ala Arg
               180                 185                 190
   Leu Phe Cys Gly Ala Gly Lys Arg Lys Phe Ser Arg Leu Phe Val Thr
           195                 200                 205
   Ile Ile Leu Thr Val Leu Val Phe Leu Leu Cys Gly Leu Pro Trp Gly
       210                 215                 220
   Ala Leu Trp Phe Pro Leu Leu Trp Ile Gln Gly Gly Phe Trp Lys Arg
   225                 230                 235                 240
   Leu Phe Gln Ala Ser Ile Val Leu Ser Ser Val Asn Ser Cys Ala Asn
                   245                 250                 255
   Pro Ile Ile Tyr Phe Phe Val Gly Ser Phe Arg His Arg Leu Lys His
               260                 265                 270
   Gln Thr Leu Lys Met Val Leu Gln Asn Ala Leu Gln Asp Thr Pro Glu
           275                 280                 285
   Thr Thr Glu Asn Met Val Glu Met Ser Arg Ser Lys Ala Glu Pro
       290                 295                 300
<210> 68
   <211> 1500
   <212> DNA
   <213> Mus musculus
<400> 68
   cattttcgga ctggtcgggc tgacaggaaa taccattgtg ttctggctcc tgggcttctg 60
   cttgcacagg aatgcctttt tagtctacat cctaaacttg gccctggctg acttcctctt 120
   ccttctctgc cacatcataa attccacagt acttcttctc aaggttcccc tacccaactg 180
   gatcttgttc cattgcttta acaccatcag aattgttctt tacatcacag gcctgaacat 240
   gctcagtgcc atcaacatgg agcactgcct gtctgtcctg tgccccatct ggtatcactg 300
   ctgccgccca gaacacacat caactgtcat gtgtgctgtg atctgggtcc tgtccctgtt 360
   gatctgcatt ctgaatgaat atttctgtga tttctttggt accaaattgg taaattacta 420
   tgtgtgtctg gcatcgaact tctttatggg agcatacctg ttgtttttgt ttgtagtcct 480
   ctgtctgtcc accctggctc tgctggccag gttgttctgt ggtgctggga atacgaaatt 540
   taccagattt cacatgacca tcttgctgac ccctttgttc tttctcctct gcgggttgcc 600
   ctttgccatc taatgcttcc tgttattcaa gattaaggat gatttccatg tattttatat 660
   taaccttttt ctagcattag aagtcctgac ttctattaac agctgtgaca accccatcat 720
   ctatttcttc ctggactcct tcagacatca ggagaagcac cagaccctca aaatggttct 780
   ccagagtgca ctgcaggata ctcytgagac acctgaaaac atggcagaga tgtcaagaag 840
   caaagcagag ccgtgatgaa gagcctctgc ctggatgtca gaggtggctt tggagtgagc 900
   actgccctgc tgcacttgac cactgtcaac tctactctca gcttactgac ttgtcatgcc 960
   tcagtggttc aacaacacct tcaaaagctc tccactgact tagtattttt acctctccca 1020
   agtagtagca ttaatcagaa agtatcatgt ctgcatcctt cttgacatta ttcaaattct 1080
   catctaactt catctgaaac tttctcccta tttctttgga acttttgttg ccatggkaat 1140
   agcccagatc cagcaccatg actctcttgt ctgtgattgt tctgaacctg aatgtaaaga 1200
   caaaggagag agaagatgat cctgtgtcac agtgctcatt acccaagcac cgccaagaga 1260
   tcttgtctcc caggagtgca gacaagcctg tgcgcactgg taagaccacc acttttgctt 1320
   aaaggacatg gcctggaact ttcaagacag agtaacagag gagcaccctg gaacaggata 1380
   cttccagttt ccaactgcac accggagctg accctatgca acagctctcc atacccaact 1440
   tcttcccaca aagaactggt gctaccagga gtactgacac acaggttttc aggaaggaca 1500
<210> 69
   <211> 283
   <212> PRT
   <213> Mus musculus
<400> 69
   Ile Phe Gly Leu Val Gly Leu Thr Gly Asn Thr Ile Val Phe Trp Leu
   1               5                   10                  15
   Leu Gly Phe Cys Leu His Arg Asn Ala Phe Leu Val Tyr Ile Leu Asn
               20                  25                  30
   Leu Ala Leu Ala Asp Phe Leu Phe Leu Leu Cys His Ile Ile Asn Ser
           35                  40                  45
   Thr Val Leu Leu Leu Lys Val Pro Leu Pro Asn Trp Ile Leu Phe His
       50                  55                  60
   Cys Phe Asn Thr Ile Arg Ile Val Leu Tyr Ile Thr Gly Leu Asn Met
   65                  70                  75                  80
   Leu Ser Ala Ile Asn Met Glu His Cys Leu Ser Val Leu Cys Pro Ile
                   85                  90                  95
   Trp Tyr His Cys Cys Arg Pro Glu His Thr Ser Thr Val Met Cys Ala
               100                 105                 110
   Val Ile Trp Val Leu Ser Leu Leu Ile Cys Ile Leu Asn Glu Tyr Phe
           115                 120                 125
   Cys Asp Phe Phe Gly Thr Lys Leu Val Asn Tyr Tyr Val Cys Leu Ala
       130                 135                 140
   Ser Asn Phe Phe Met Gly Ala Tyr Leu Leu Phe Leu Phe Val Val Leu
   145                 150                 155                 160
   Cys Leu Ser Thr Leu Ala Leu Leu Ala Arg Leu Phe Cys Gly Ala Gly
                   165                 170                 175
   Asn Thr Lys Phe Thr Arg Phe His Met Thr Ile Leu Leu Thr Pro Leu
               180                 185                 190
   Phe Phe Leu Leu Cys Gly Leu Pro Phe Ala Ile Cys Phe Leu Leu Phe
           195                 200                 205
   Lys Ile Lys Asp Asp Phe His Val Phe Tyr Ile Asn Leu Phe Leu Ala
       210                 215                 220
   Leu Glu Val Leu Thr Ser Ile Asn Ser Cys Asp Asn Pro Ile Ile Tyr
   225                 230                 235                 240
   Phe Phe Leu Asp Ser Phe Arg His Gln Glu Lys His Gln Thr Leu Lys
                   245                 250                 255
   Met Val Leu Gln Ser Ala Leu Gln Asp Thr Pro Glu Thr Pro Glu Asn
               260                 265                 270
   Met Ala Glu Met Ser Arg Ser Lys Ala Glu Pro
           275                 280
<210> 70
   <211> 2504
   <212> DNA
   <213> Mus musculus
<400> 70
<210> 71
   <211> 301
   <212> PRT
   <213> Mus musculus
<400> 71
   Met Asp Lys Thr Ile Pro Gly Gly Ile Asn Ile Arg Ile Leu Ile Pro
    1               5                  10                  15
   Asn Leu Ile Thr Ile Ile Phe Gly Leu Val Gly Leu Thr Gly Asn Ser
               20                  25                  30
   Ile Val Phe Trp Ile Leu His Phe Pro Leu Arg Arg Asn Ala Phe Lys
           35                  40                  45
   Val Tyr Ile Leu Asn Leu Asp Leu Ala Asp Phe Phe Phe Leu Leu Gly
       50                  55                  60
   His Thr Ile Asp Ser Ile Leu Leu Leu Leu Asn Val Phe Tyr Pro Ile
   65                  70                  75                  80
   Ile Phe Ile Leu Cys Phe Tyr Ile Ile Met Met Val Leu Tyr Ile Ala
                   85                  90                  95
   Gly Leu Ser Met Leu Thr Ala Ile Ser Thr Glu His Gly Leu Ser Val
               100                 105                 110
   Leu Cys Pro Ile Trp Asp Cys Cys His His Pro Glu His Thr Ser Ala
           115                 120                 125
   Ala Met Cys Ala Val Ile Trp Val Leu Ser Leu Leu Ile Cys Ile Leu
       130                 135                 140
   Asn Ser Tyr Phe Gly Phe Leu His Ser Lys Tyr Glu Asn Asp Asn Gly
   145                 150                 155                 160
   Cys Leu Ala Leu Asn Phe Phe Thr Ser Ala Tyr Leu Met Phe Leu Phe
                   165                 170                 175
   Val Asp Leu Cys Leu Ser Ser Leu Ala Leu Leu Ala Arg Leu Phe Cys
               180                 185                 190
   Asp Val Gly Gln Met Lys Leu Thr Arg Tyr Val Thr Ile Leu Leu Thr
           195                 200                 205
   Ala Leu Val Phe Leu Leu Cys Arg Leu Asn Phe Gly Ile Tyr Trp Phe
       210                 215                 220
   Leu Leu Cys Lys Ile Lys Asp Ala Phe His Val Phe Thr Leu Val Phe
   225                 230                 235                 240
   Tyr Leu Glu Ser Leu Val Met Thr Ala Ile Asn Ser Cys Ala Asn Ser
                   245                 250                 255
   Ile Ile Tyr Phe Phe Thr Gly Ser Phe Arg Leu Arg Leu Gln His Gln
               260                 265                 270
   Thr Leu Lys Met Val Leu Gln Arg Thr Met Asp Thr Pro Glu Thr Pro
           275                 280                 285
   Glu Asn Met Val Gly Met Ser Arg Ser Lys Ala Glu Pro
       290                 295                 300
<210> 72
   <211> 2758
   <212> DNA
   <213> Mus musculus
<400> 72
   aatttttgtg tttcctcttt aagggcttct accaatttat ctgtgttctc ctgtattatt 60
   ttaagggagt tatttatgtc tttcttaatg tcctctatca tcatcatcat catccttatc 120
   attttcatca tcatcaccag aggtgacttt aaatcagagt catgcttttc tggtgtgttg 180
   gagtatccag ggctcaccat gttgagagaa ctaggttctg atgatgccaa gtagccttgg 240
   ttcccattgc ttatgttttt gcccttgcct cttgccatct gattatctct ggagtaagct 300
   ggtcttgctc tctctaactg tggcttgtcc ctcctgcaag cctatgtgtc agtactcctg 360
   gtagaccagt tctttctggg agaaatttgg gtatggagag ctgtggcaca gggtcagctc 420
   cggggtacag ttggaaactg gaagtatcct gtcccaggct gctcctctgt tcctgtgtcc 480
   tgaggattcc aggcatgtcc atttaagcag aagtggtggt cttacctatg ttcacaggca 540
   tatctgcact cctgggagac aagctttctt ggtggtgttt gggtaatgag cactgggaca 600
   caggaacatc tcctggctcc tttgtcttta catttgggta cagaacaatc acagacaaga 660
   gagtaattgt gctgaaccta agctattacc atggcaacaa aagttccaaa gaaacagcaa 720
   gaatgtttca gatgaagtta gtatgagaat tggattaatg tcaggaagga tgcagacatg 780
   gtactttctg attaatgcta ttacttggga gaggtagaaa tactaagtca gtggagagct 840
   tttgaaggtg ttgttggacc actgaggaat gccaagtcag taagctgaga ggaaagtgga 900
   cagtggtcta gtgcagcatg gcagtgctca ctccaaagcc acctctgagg tccaggcaga 960
   ggctcttcat catggctctg ctttgcttct tgatatatcc accatgtttt caggtgtctc
   1020
   aggagtgtcc tgcaatgcac tctggagaac cattttgagg gtcttgtgct tcaacggatg
   1080
   cctgtatgag cccacgaaga agtaaatgat ggggttggca cagctgttaa cagcagttag
   1140
   gacaagtgat gccagaaaga atctatagtc tagtatactg aaaccaccct caatccaggg
   1200
   taacaggaac cagaggaagc ccaggggcaa cccacagaga agaaaaacca aaatggtcac
   1260
   catgatggtc atgaataatc tggtaaattt cttctttcca gcaccacaga acaacctggc
   1320
   cagcagagtc agggtagaaa aacagaggac tacaaacaaa aaaatagggt atattctgat
   1380
   gaagaattct gatgcctgac acacagagtt aatttcatat ttgggaccaa ataaatcaca
   1440
   gaaatatctg ttcagaaggc agatcaacag gggacaggac ccagatcacg acacacatga
   1500
   tggttgatgt gtgttmtggg cggtggcagc gataccagat ggggcacagg acagacaggc
   1560
   agcgmtcagt gctgatggca ctgagcatgc tcaggcctgt gatgtagaga accgttctga
   1620
   tggtgtcaaa gcaatggatg aagatactgt tgtgtgggcg aaccttgaaa agatgcattg
   1680
   tggaatttat gatgtgacag agaagaaaga aggaagtcag ccagggccaa gtttaggatg
   1740
   tagactaaga tggcattcct gtgaaatcgg aagcccagga tccagaatac aatggcattt
   1800
   ccagtcagcc caaccagtcc gaagatgatg atcatcaagt gtgggataag ggtctcgatt
   1860
   tcaatacttc cagagatggt ttcatccatt ggatttgttg tcgtgggtgc cattgctgag
   1920
   gctgaggtgt ttagggccag aaaccctgca ctggtattgc tggaaacaca aacaagaaat
   1980
   gaggccttca ctgtgaacac aacttttaat ttctttcttt ttgtttgttt gtttgtttgt
   2040
   ttgtggggtt ttgttttttt ttttaatttt tttttgtatt agatattttc ttcatttaat
   2100
   tttcaaatgt tatccctttt cctggctttc ccccctccca gaaaccccct tctgatcctc
   2160
   ccaccctctt caacccacac acccacttcc acctctctgc ccctgattcc cttacactgg
   2220
   agcatctata gaaccttcat aggttcaagg acctcttctt ccatccatgc aagacatggc
   2280
   catcatctgc tacatatgca tctggagcca cacgtactcc tttgttgatg gcttagtccc
   2340
   tgggagttca gggggtgggg gtgggggtgg gggcagtggt ctcttggttc atactgttgc
   2400
   tcttcttatg gagcttcaaa ccacttcaac tccctcaggc ctttctctaa ctcctctatt
   2460
   agggaccctg tgctcagttt aattgttggc tgctaacatc agactctgca tttgaaaggc
   2520
   cctgacatgg cctcttagga aacagctata tcaggttcct gtcagcattc actccttgac
   2580
   atccacaata gtgtctgcat ttggtaactg tgtatgagat gaatccccag gtggaacatt
   2640
   ctctgggtga cttttccttt agtgtctgtt ctacacatta tctccatatt tgctcttgtg
   2700
   agtattttgt tcttcttcta agaaggtctg aaacacccac actttcgtct tccttgtt
   2758
<210> 73
   <211> 304
   <212> PRT
   <213> Mus musculus
<400> 73
   Met Asp Glu Thr Ile Ser Gly Ser Ile Glu Ile Glu Thr Leu Ile Pro
    1               5                  10                  15
   His Leu Met Ile Ile Ile Phe Gly Leu Val Gly Leu Thr Gly Asn Ala
               20                  25                  30
   Ile Val Phe Trp Ile Leu Gly Phe Arg Phe His Arg Asn Ala Ile Leu
           35                  40                  45
   Val Tyr Ile Leu Asn Leu Ala Leu Ala Asp Phe Phe Phe Leu Leu Cys
       50                  55                  60
   His Ile Ile Asn Ser Thr Met His Leu Phe Lys Val Arg Pro His Asn
   65                  70                  75                  80
   Ser Ile Phe Ile His Cys Phe Asp Thr Ile Arg Thr Val Leu Tyr Ile
                   85                  90                  95
   Thr Gly Leu Ser Met Leu Ser Ala Ile Ser Thr Asp Arg Cys Leu Ser
               100                 105                 110
   Val Leu Cys Pro Ile Trp Tyr Arg Cys His Arg Pro His Thr Ser Thr
           115                 120                 125
   Ile Met Cys Val Val Ile Trp Val Leu Ser Leu Leu Ile Cys Leu Leu
       130                 135                 140
   Asn Arg Tyr Phe Cys Asp Leu Phe Gly Pro Lys Tyr Glu Ile Asn Ser
   145                 150                 155                 160
   Val Cys Gln Ala Ser Glu Phe Phe Ile Arg Ile Tyr Pro Ile Phe Leu
                   165                 170                 175
   Phe Val Val Leu Cys Phe Ser Thr Leu Thr Leu Leu Ala Arg Leu Phe
               180                 185                 190
   Cys Gly Ala Gly Lys Lys Lys Phe Thr Arg Leu Phe Met Thr Ile Met
           195                 200                 205
   Val Thr Ile Leu Val Phe Leu Leu Cys Gly Leu Pro Leu Gly Phe Leu
       210                 215                 220
   Trp Phe Leu Leu Pro Trp Ile Glu Gly Gly Phe Ser Ile Leu Asp Tyr
   225                 230                 235                 240
   Arg Phe Phe Leu Ala Ser Leu Val Leu Thr Ala Val Asn Ser Cys Ala
                   245                 250                 255
   Asn Pro Ile Ile Tyr Phe Phe Val Gly Ser Tyr Arg His Pro Leu Lys
               260                 265                 270
   His Lys Thr Leu Lys Met Val Leu Gln Ser Ala Leu Gln Asp Thr Pro
           275                 280                 285
   Glu Thr Pro Glu Asn Met Val Asp Ile Ser Arg Ser Lys Ala Glu Pro
       290                 295                 300
<210> 74
   <211> 1738
   <212> DNA
   <213> Mus musculus
<400> 74
   cacccacaac aaccaaatcc aatggacgaa accatcccct ggaagtattg acatcaagac 60
   cctgatcgca aatttgatga tcatcatctt cggactggtc gggctgacag aaactgcctt 120
   tgtgttctga ctcctgggct tccacttgca caggaacgcc ttcttagtct acatcctaaa 180
   cttggccctg actgacttcc tcttccttct ctgtcacatc ataaattcca cagtgattct 240
   tctcaatgtt cccctaccta acatgatctt ggtccattgc tttagcacca tcagaatatt 300
   tctcaacatc acaggcctaa gcattctcag tgccatcagc actgagcgct gcctgtctgt 360
   cctgtgcccc atctggtatc gctgccacca cccagaacac acatcaactg tcatgtgtgc 420
   tgtgatctga gtcctgtccc tgttgatttg cactctgtat agatatttct gttttttctt 480
   tggtcccaaa tatgtatttg actctgtgtg tctggcaacg acctacttta tcagaacata 540
   cccaatgttt ttgtttatgg tcctctgtct gtccactctg gctctgctgg ccaggttgtt 600
   ctgtggtgct gggaagamra aatttaccag gattattcgt gaccatcatg ctgacygttt 660
   tggtttttct tctctgtggg atgcccctag gcttcttctg gttcgtgttc ccatggatta 720
   actgtgattt cagtgtacta gattatagac tttttctggc atcaattgta ctaactgctg 780
   ttaacagtta tggcaacccc atcatttact tcttcgtggg ctccttcagg aatcggttga 840
   agcaccagac cctccaaaag gttctccaga gtgcactgca cgacactcct gagacacctg 900
   aaaacatggt agagatgtca agaagcaaag cagagccatg atgaagagtc tctgacagga 960
   cttcagaggt ggctttggag tgagcactgc cctgctgcac ttaaccacac tccactctcc
   1020
   tctcagctta ctgactatgg atgcctcagt ggtccaacaa tgccttcaaa agctctccac
   1080
   tgacttagta tttctacctc tcccaagtaa tagcattaat cagaaagtac catgtctgca
   1140
   tccttcttga cattaatcca attctcatac taacttcatc tgtaactttc ttgctgtttc
   1200
   tttggaactt ttgttaccat agtaatagcc taggtccagc accatgattc ccttgtctgt
   1260
   gattgttctg tacctacctg aatgtaaagc aaagtagcca ggagatgttc ctgtgtycca
   1320
   gtgctcatta cccaaacacc accaagaaag cttgtctccc aggagtgcag acaagcctgt
   1380
   gaacacaggt aagaccacca cttctgctta aaggggcatg cctggaaccc tcaggacaca
   1440
   ggaacagagg agcagcctgg gacaggatac ttccagtttc caactgcact ccagagctga
   1500
   ccctgtgcca cagctctcca tacccaaatt cctcccagaa agaattggtg taccaggagt
   1560
   actgacacac aggcttgcag aaggaacaag ccacagtcaa agttagcaag acctgctaac
   1620
   accagagata accagatggc aagacacaag ggcaaaaaca taagcaatgg gaaccaagac
   1680
   tacttggcat catcagaaac tagttctctc aacatggtga gccatggata cttcaaca
   1738
<210> 75
   <211> 303
   <212> PRT
   <213> Mus musculus
<400> 75
   Met Asp Glu Thr Ile Pro Gly Ser Ile Asp Ile Lys Thr Leu Ile Ala
   1 5 10 15
   Asn Leu Met Ile Ile Ile Phe Gly Leu Val Gly Leu Thr Glu Thr Ala
   20 25 30
   Phe Val Phe Leu Leu Gly Phe His Leu His Arg Asn Ala Phe Leu Val
   35 40 45
   Tyr Ile Leu Asn Leu Ala Leu Thr Asp Phe Leu Phe Leu Leu Cys His
   50 55 60
   Ile Ile Asn Ser Thr Val Ile Leu Leu Asn Val Pro Leu Pro Asn Met
   65 70 75 80
   Ile Leu Val His Cys Phe Ser Thr Ile Arg Ile Phe Leu Asn Ile Thr
                               85                  90                  95
   Gly Leu Ser Ile Leu Ser Ala Ile Ser Thr Glu Arg Cys Leu Ser Val
                   100                 105                 110
   Leu Cys Pro Ile Trp Tyr Arg Cys His His Pro Glu His Thr Ser Thr
               115                 120                 125
   Val Met Cys Ala Val Ile Val Leu Ser Leu Leu Ile Cys Thr Leu Tyr
       130                 135                 140
   Arg Tyr Phe Cys Phe Phe Phe Gly Pro Lys Tyr Val Phe Asp Ser Val
   145                 150                 155                 160
   Cys Leu Ala Thr Thr Tyr Phe Ile Arg Thr Tyr Pro Met Phe Leu Phe
                   165                 170                 175
   Met Val Leu Cys Leu Ser Thr Leu Ala Leu Leu Ala Arg Leu Phe Cys
               180                 185                 190
   Gly Ala Gly Lys Lys Lys Phe Thr Arg Leu Phe Val Thr Ile Met Leu
           195                 200                 205
   Thr Val Leu Val Phe Leu Leu Cys Gly Met Pro Leu Gly Phe Phe Trp
       210                 215                 220
   Phe Val Phe Pro Trp Ile Asn Cys Asp Phe Ser Val Leu Asp Tyr Arg
   225                 230                 235                 240
   Leu Phe Leu Ala Ser Ile Val Leu Thr Ala Val Asn Ser Tyr Gly Asn
                   245                 250                 255
   Pro Ile Ile Tyr Phe Phe Val Gly Ser Phe Arg Asn Arg Leu Lys His
               260                 265                 270
   Gln Thr Leu Gln Lys Val Leu Gln Ser Ala Leu His Asp Thr Pro Glu
           275                 280                 285
   Thr Pro Glu Asn Met Val Glu Met Ser Arg Ser Lys Ala Glu Pro
       290                 295                 300
<210> 76
   <211> 1011
   <212> DNA
   <213> Mus musculus
<400> 76
   aagaggaaac acatatattt gggatgttaa ccaaggtttt ctatagggaa caatggaaaa 60
   ctgttcactt caagattaca gtttagctgc atgattaaac tttaaattga cattaacatt 120
   taattactgg gttttataaa ggtcctgaga tatttaaggt tggattgtct tttatattat 180
   gatattaata tgcttagaac aaagaaagaa aagtttattg ttcaatggtg aagtgtcttt 240
   taaatagaag tgggcagagt gtcctggcaa acctcaattt ttaccttgac acagattaaa 300
   gtcgtatgag aggagaaatc acaacagcag aaatgacaac tgaggaattg tctagattat 360
   cttggcctgt gggcatgatt atgaggaatt atctttaaca taaattaatg taagcaaaca 420
   tggtctatgg taggttgcac caataagcta cttaagcagg acctgtaatc atccagaatt 480
   ggagcttgga aggagtgttt cttgtagata ctgttccttg tgttccttga gttcctgaca 540
   tgacttccct cactgatgga gtctgtacta agagtataag ccagataacc cattttattt 600
   tctaggatgt ttgtggtcaa aatgttttcc catgaaacag aaaaggaaac tagaacatgc 660
   acaaattacc taacagatat ttattaagtt agagaatatt ctaagttata caaatactaa 720
   aggaaactac aaatgtggat ctattaaatt cttatttaaa caaaatctgt agagatgata 780
   aattgttaaa aatgtcataa attttcaatc actatcaagt tcagttacca atgaaattca 840
   gttattaact gaaaactcct gatctttgga tgaagaaggg gcttgtcaaa aatgggagca 900
   gtcttggacc tataattatt acagtgggtc tcatctcaag gggatccagt gaagtgtcat 960
   taagaggaga gtaggaaagt tcaacatagt atttctatta aaagtggtgt a
   1011
<210> 77
   <211> 274
   <212> PRT
   <213> Mus musculus
<400> 77
   Leu Leu Ser Ile Ile Ile Ala Phe Ile Gly Leu Ala Glu Asn Ala Ile
    1               5                  10                  15
   Val Leu Trp Leu Leu Gly Phe His Met His Arg Asn Ala Phe Ser Val
               20                  25                  30
   Tyr Ile Leu Asn Ala Gly Ala Asn Phe Leu Phe Leu Cys Pro Tyr Ile
           35                  40                  45
   Val Phe Ser Leu Val Thr Ile Thr Val Asn Phe His Ser Ile Asn Ser
       50                  55                  60
   His Ile Ile Leu Phe Leu Asn Thr Val Phe Thr Leu Ala Tyr Leu Ala
   65                  70                  75                  80
   Gly Val Ser Met Ile Thr Ala Ile Ser Val Glu Tyr Trp Leu Ser Val
                   85                  90                  95
   Ile Trp Ser Asn Trp Tyr His Gly Arg His Pro Lys His Thr Ser Ala
               100                 105                 110
   Phe Ile Cys Thr Leu Leu Trp Ala Val Ser Leu Leu Leu Ser Leu Pro
           115                 120                 125
   His Glu Ile Ile Cys Gly Leu Leu Asp His Ile Tyr Asn Trp Asp Met
       130                 135                 140
   Cys Trp Lys Cys Lys Leu Ile Ile Val Val Trp Leu Leu Ile Glu Phe
   145                 150                 155                 160
   Val Val Leu Ser Gln Ser Asn Gln Ala Met Met Phe Arg Ile Phe Cys
                   165                 170                 175
   Gly Ser Gln Gln Thr Pro Met Thr Arg Leu Phe Val Thr Ile Val Leu
               180                 185                 190
   Thr Ala Leu Val Val Leu Ile Cys Gly Phe Pro Leu Gly Ile Tyr Ile
           195                 200                 205
   Tyr Phe Leu Tyr Trp Thr Thr Asp Val Tyr Phe Ile Met Pro Cys Asn
       210                 215                 220
   Ser Phe His Glu Thr Ile Leu Leu Leu Ser Ala Val Asn Ser Cys Ala
   225                 230                 235                 240
   Asn Pro Ile Ile Cys Leu Leu Val Gly Ser Ile Lys His Cys Gln Phe
                   245                 250                 255
   Gln Cys Gly Thr Leu Arg Leu Ile Leu Gln Arg Ala Ile Gln Asp Thr
               260                 265                 270
   Pro Glu
<210> 78
   <211> 1358
   <212> DNA
   <213> Mus musculus
<400> 78
   taaattactg aatctctgtg atcctgattc cctctcttta tggacctgtg cctgacatac 60
   ccacatagtc acatggtcct gacagaaact atcatgtgtt catatctcta tgtcttttca 120
   ggaatgtcag tggaaaattc ctaagcatgg gtacaactag cctggcctgg aacattaaca 180
   acacagctga aaatggaagc tacactgaaa tgttctcctg tatcaccacg ttcaataccc 240
   tgaattttct tactgtcatc attgctgtgg ttgtcctggc aggaaattcc atagtgctat 300
   ggcttctagc cttccacctg cacaggaatg ccttcttcgt ctatgtcctc aatctggctg 360
   gtgctgattt cttgtacctt tgcactcaga ttgtgtattc cctggagtgt gtcattcagt 420
   ttgataaaag ctccttttat attctcctca ttttatcaat gtttgcttac cttgcaggat 480
   tgagtatgat tgcaaccatc agtactgagc gctgcctatc tgttatgtgg cccatctggt 540
   atcactgcca aagaccaaga cacacatcag ccatcatgtc tgttctgctc tgggttttct 600
   ctatactgtt gagcctcctg gtaggactag gctgtggttt tctgttcaga tattctgaat 660
   attatttctg tattactttg aactttatca ctgctgcatt tatcataggg ttatctgtgg 720
   ttctttctgt atctagcctg accctgttgg tcaagatcat ctgtggatca cacaggatac 780
   ctgtgaccag gttgtttgtt accatttgct ctcacagtgg tggtcttcat aatctttggc 840
   atgccccttg gaatctgctg gttcctcttt ccaagtatta ttgagtttca taaaattttc 900
   tctaacaatt tttatgaaat gatagcattc ctgtcatgta ttaatagttg tgccaatccc 960
   atcatttact tccttgttgg ctctattagg caccacaggt tgaaatggca gtctcttaag
   1020
   ctacttcttc agagagccat gcaggacact cctgaggaag tgagtggaga gaggggtcct
   1080
   tcagaaaggt ctggggaact ggaaagagtc tagtgcagta gtggagtgag tccttgatca
   1140
   gatatagttt ctctgagagt caattttgcc tttatctatt taggcaattt tcacagtctt
   1200
   gttcaatcag tagagaaaat agtcatttta tagaaattag gaggaacagg cttgttacac
   1260
   agaaactgac ttgcagcacc ataaagctgc cttatgtggt gctcagtgca tcccctcgtg
   1320
   atataagcct tgtaatcact tggggccaga acagctcc
   1358
<210> 79
   <211> 268
   <212> PRT
   <213> Mus musculus
<400> 79
   Phe Leu Thr Val Ile Ile Ala Val Val Val Leu Ala Gly Asn Ser Ile
   1               5                   10                  15
   Val Leu Trp Leu Leu Ala Phe His Leu His Arg Asn Ala Phe Phe Val
               20                  25                  30
   Tyr Val Leu Asn Leu Ala Gly Ala Asp Phe Leu Tyr Leu Cys Thr Gln
           35                  40                  45
   Ile Val Tyr Ser Leu Glu Cys Val Ile Gln Phe Asp Lys Ser Ser Phe
       50                  55                  60
   Tyr Ile Leu Leu Ile Leu Ser Met Phe Ala Tyr Leu Ala Gly Leu Ser
   65                  70                  75                  80
   Met Ile Ala Thr Ile Ser Thr Glu Arg Cys Leu Ser Val Met Trp Pro
                   85                  90                  95
   Ile Trp Tyr His Cys Gln Arg Pro Arg His Thr Ser Ala Ile Met Ser
               100                 105                 110
   Val Leu Leu Trp Val Phe Ser Ile Leu Leu Ser Leu Leu Val Gly Leu
           115                 120                 125
   Gly Cys Gly Phe Leu Phe Arg Tyr Ser Glu Tyr Tyr Phe Cys Ile Thr
       130                 135                 140
   Leu Asn Phe Ile Thr Ala Ala Phe Ile Ile Gly Leu Ser Val Val Leu
   145                 150                 155                 160
   Ser Val Ser Ser Leu Thr Leu Leu Val Lys Ile Ile Cys Gly Ser His
                   165                 170                 175
   Arg Ile Pro Val Thr Arg Leu Phe Val Thr Ile Cys Phe Thr Val Val
               180                 185                 190
   Val Phe Ile Ile Phe Gly Met Pro Leu Gly Ile Cys Trp Phe Leu Phe
           195                 200                 205
   Pro Ser Ile Ile Glu Phe His Lys Ile Phe Ser Asn Asn Phe Tyr Glu
       210                 215                 220
   Met Ile Ala Phe Leu Ser Cys Ile Asn Ser Cys Ala Asn Pro Ile Ile
   225                 230                 235                 240
   Tyr Phe Leu Val Gly Ser Ile Arg His His Arg Leu Lys Trp Gln Ser
                   245                 250                 255
   Leu Lys Leu Leu Leu Gln Arg Ala Met Gln Asp Thr
               260                 265
<210> 80
   <211> 2387
   <212> DNA
   <213> Mus musculus
<400> 80
<210> 81
   <211> 273
   <212> PRT
   <213> Mus musculus
<400> 81
   Leu Leu Ser Ile Ile Ile Ala Ile Ile Gly Leu Thr Gly Asn Val Ile
    1               5                  10                  15
   Val Leu Gln Leu Leu Gly Phe His Met His Arg Asn Ala Phe Ser Val
               20                  25                  30
   Tyr Ile Phe Asn Leu Ser Gly Ala Asn Phe Leu Phe Leu Cys Thr His
           35                  40                  45
   Ile Val Phe Ser Leu Glu Ile Ser Leu Gly Ser Phe Thr Thr Ser Thr
       50                  55                  60
   Phe Thr Trp Ala Leu Phe Ser Val Asn Val Thr Ile Leu Ala Tyr Leu
   65                  70                  75                  80
   Ala Gly Val Ser Met Ile Thr Ala Ile Ser Val Glu Tyr Trp Leu Ser
                   85                  90                  95
   Val Leu Trp Pro Thr Trp Tyr His Ala Gln Arg Pro Lys His Thr Ser
               100                 105                 110
   Thr Val Ile Cys Thr Leu Leu Trp Val Phe Ser Leu Leu Leu Thr Leu
           115                 120                 125
   Trp Asn Trp Ile Ile Cys Lys Val Leu Asp Tyr Ile Tyr Asn Trp Asp
       130                 135                 140
   Met Cys Trp Lys Leu Ala Leu Ile Ile Val Val Trp Leu Leu Val Leu
   145                 150                 155                 160
   Phe Val Val Leu Ser Arg Ser Asn Gln Ala Leu Leu Phe Arg Val Phe
                   165                 170                 175
   Cys Gly Ser Gln Gln Thr Pro Val Thr Arg Leu Leu Val Thr Ile Met
               180                 185                 190
   Leu Thr Ala Leu Val Val Leu Ile Cys Gly Phe Gly Ile Gly Ile Cys
           195                 200                 205
   Phe Phe Tyr Trp Lys Lys Glu Glu Asn Ser Ile Met Pro Cys Gly Tyr
       210                 215                 220
   Phe Tyr Glu Thr Ile Leu Leu Leu Ser Gly Val Asn Ser Cys Ala Asn
   225                 230                 235                 240
   Pro Ile Ile Cys Leu Phe Val Gly Ser Ile Lys His Cys Gln Phe Gln
                   245                 250                 255
   Cys Gly Thr Leu Arg Leu Ile Leu Gln Arg Ala Ile Gln Glu Ser Pro
               260                 265                 270
   Glu
<210> 82
   <211> 1319
   <212> DNA
   <213> Mus musculus
<400> 82
   tttataaacc aggtcagtaa ttaccacata gcaggatgtt cctgaatcag aaagaacata 60
   gcatgtgctc attgttttgt ttattttgtt ccagaaatag tactggagac ttcctaaaca 120
   aggatctaag catctcaacc ttggaagcta actccagaac atctactgaa cccaatgata 180
   cttcaggttg tggcatcaag ttccaaacca agatgttgct ttccctcatt tccctgtttg 240
   ggatggtact aaatcccata gtgctgtgat tgctgagctt ccaggtgcac aggaatgcct 300
   tgtttgtcta catcctcaac cttgctgtgg ttgacatttt cttccggttt gatcagtttg 360
   cattttgtgt ttttgttatc atttacacta tcaagtccat ttccaatgat atcctatcat 420
   tttttatttt tgtgccagca tttctgtatc ttttaagcct gagcattctc ataaccatta 480
   gcattgaacg atgcctgtat gtcatgtggc ccatctggta tcactgtcaa tgtccaagac 540
   acacatcagc tgtcatttgt gtcttgcttt gggctctgtc ccttgtgttt atgtttctgg 600
   atgggaaggc atatttttta ctgttttctg accctaactc tttttggtat cagacatttg 660
   atatcatcat tactgtatag acaattgttt tatttgtggt tctctgtggg tccagcttaa 720
   tcctacttgt cagaatcttc tgtggctccc agcagatccc tgtaaccagg ctggatgtga 780
   tcattgcact cagagtgctt ttcttcctga tatttagttt tcccttttgg atctactggc 840
   tccttgacca acggattggg agacgttgta attttttgat gaaatgattt tcttatcctg 900
   tattaagagc tgtgtcaact ccatcattta ctttcttgtt gcctccatta tgcacagtag 960
   tggattcaag gtgaagagtc tcaaactatt tccagagaga gccatgcagg acattcctga
   1020
   agaaggagaa ggtgttgaga atagttctta aggaaatcat gaagaactgg agaaatctag
   1080
   tgcagcagac gacagctact ttgattagac agagtggtcg tttttcttat ctttgtggac
   1140
   taatttaatg accttattca gtttgttact taatcttcaa tcagttaaaa atgacaatca
   1200
   tttttgtaat agttgaaaga tacagtactt gtcacacaaa tattaactgt gccatttctc
   1260
   ttgctgtgtt tttgaggcct ttaccatttc cttttgatgg gagtacttgc aagtattct
   1319
<210> 83
   <211> 264
   <212> PRT
   <213> Mus musculus
<400> 83
   Leu Ile Ser Leu Phe Gly Met Val Leu Asn Pro Ile Val Leu Leu Leu
    1               5                  10                  15
   Ser Phe Gln Val His Arg Asn Ala Leu Phe Val Tyr Ile Leu Asn Leu
               20                  25                  30
   Ala Val Val Asp Ile Phe Phe Arg Phe Asp Gln Phe Ala Phe Cys Val
           35                  40                  45
   Phe Val Ile Ile Tyr Thr Ile Lys Ser Ile Ser Asn Asp Ile Leu Ser
       50                  55                  60
   Phe Phe Ile Phe Val Pro Ala Phe Leu Tyr Leu Leu Ser Leu Ser Ile
   65                  70                  75                  80
   Leu Ile Thr Ile Ser Ile Glu Arg Cys Leu Tyr Val Met Trp Pro Ile
                   85                  90                  95
   Trp Tyr His Cys Gln Cys Pro Arg His Thr Ser Ala Val Ile Cys Val
               100                 105                 110
   Leu Leu Trp Ala Leu Ser Leu Val Phe Met Phe Leu Asp Gly Lys Ala
           115                 120                 125
   Tyr Phe Leu Leu Phe Ser Asp Pro Asn Ser Phe Trp Tyr Gln Thr Phe
       130                 135                 140
   Asp Ile Ile Ile Thr Val Thr Ile Val Leu Phe Val Val Leu Cys Gly
   145                 150                 155                 160
   Ser Ser Leu Ile Leu Leu Phe Arg Ile Phe Cys Gly Ser Gln Gln Ile
                   165                 170                 175
   Pro Val Thr Arg Leu Asp Val Ile Ile Ala Leu Arg Val Leu Phe Phe
               180                 185                 190
   Leu Ile Phe Ser Phe Pro Phe Trp Ile Tyr Trp Leu Leu Asp Gln Arg
           195                 200                 205
   Ile Gly Arg Arg Cys Asn Phe Leu Asn Glu Met Ile Phe Leu Ser Cys
       210                 215                 220
   Ile Lys Ser Cys Val Asn Ser Ile Ile Tyr Phe Leu Val Ala Ser Ile
   225                 230                 235                 240
   Met His Ser Ser Gly Phe Lys Val Lys Ser Leu Lys Leu Phe Pro Glu
                   245                 250                 255
   Arg Ala Met Gln Asp Thr Pro Glu
               260
<210> 84
   <211> 2349
   <212> DNA
   <213> Mus musculus
<400> 84
   tttctttctg agaaatagtt tgttttaaaa taggaatttt aaaacagctt gagacactga 60
   gagtttatac tggaaccatc aactactcta atgtcaatac aggatatggg ttgtagataa 120
   cccaaatata tatgaatgat atatttaaat taaggctcca gaaatattga ttttgataaa 180
   ttgcttcatg tctaccaccc tgtttcacca ttttaagaac taggtaaacc gttaacatct 240
   ataatggtga tcctaagaat cagagaacaa aaagcatgtg ttcatgtctt gtttttcttt 300
   ccagaaacat cagtggaagg gatctaagag tggattcaaa cataacatac tggggaacaa 360
   acatcacagc tgtgaatgaa agcaaccaya ctggaatgtc attttgtgaa gtcgtgtctt 420
   gtaccatgkt ttttctttcc ctcattgttg ccctagttgg gctggttgga aatgccacag 480
   tgctgtggtt cctgggcttc cagatgcgca ggaatgcatt ctctgtttac atcctcaacc 540
   tcgctggtgc tgactttctc ttcatttgct ttcaaattgg atattgtttt cacatgatct 600
   tggacattga ttccatcccc attgaaattg atctgtttta ccttgttgtg ttaaactttc 660
   cttatttttg tggcctgagt atcctcagtg ctattagcat tgaacgttgc ctgtctgtca 720
   tgtggcccat ttggtatcac tgccaacgcc caaggcacac atcagctgtc atatgtaccc 780
   tgctttgggt cttgtcccta gtgtgtagcc tcctggaagg gaaggaatgt ggcttcctat 840
   attacactag tgaccctggt tggtgtaaga catttgattt aatcactgct acatggttaa 900
   ttgttttatt tgtagctctc ttgggatcca gtctggcctt agtgattacc atcttctggg 960
   gcttacacaa gattcctgtg accaggctgt atgtggccat tgtgttcaca gtgcttgttt
   1020
   tcctgctctt tggtctgccc tatgggatct actggttcct cttagtgtgg attgagaaat
   1080
   tttattatgt tttaccttgt agtatatatc cggtcacagt atttctctcc tgtgttaaca
   1140
   gctctgcaaa acccatcatt tattgccttg taggctccat taggcatcat cgatttcaac
   1200
   ggaagactct caagctattt ctgcagagag ccatgcaaga cactcctgag gaggaagaat
   1260
   gtggagagat gggttcctca ggaagatcta gagaaataaa aacaatctgg aaaggactga
   1320
   gagctgcttt gatcaggcat aaagagctct gaagagaact atgtttttat cactttgttg
   1380
   cattttcata acgttgttta gttgatgacc caaggttaac tcagttggga aagtagtcaa
   1440
   tgttgtagaa gttgattgat attggacttg ttacaaatac tgggtacaac atttctgcag
   1500
   ctatcttgct cagggtttta ccaacttctt ttgatgttac tccttgcaag ctctgtgggg
   1560
   tccaggaaag ctgttgacca caattgataa atcccttctt cagaagaaag cttaagaaag
   1620
   tacaggaaag ggttgcattt cttaactcac ttaacttgat agtggataaa ttcatgttat
   1680
   atttgcaaaa aaaattattc tgtttcaagg caaacttttc ttcagtgttg aagggttaaa
   1740
   tagatacatt atataatccc agactttatt aatttctgta tgttttaaag aatatgtgga
   1800
   gcaatagttt ttcttataca catttcttaa taaagaagta aacattctca agagaagtgt
   1860
   taaacatcca tgtacatagg aaggtgcagt gtcctctgtg gttctattca cagtttcctt
   1920
   tttagcatcc catagttgag tattgtcttt gatatgatcc tcatgctctc tgactgtgta
   1980
   atccctcatg aaaagtttcc aatgaggtcc tctataaaga ctcccttgaa atacaactta
   2040
   ttttaaattt ataccatttc aaggagccca cagcatctat taacttagct atatgcacag
   2100
   tttagtaaaa ttttctataa aataatattc cttttataaa gctgcagtaa taatttcaat
   2160
   ttttctacaa ttaagagaat aaaatatcaa caaattaaat aaaactaatc agtaggtttt
   2220
   cttaagttaa tgtagctgca tgactctgta cctaatcaag acacaaaata ctacactata
   2280
   tcttttaatt ttcatttctt ctcctgtcat aattttatat cacagataaa tatgatatcc
   2340
   atacttctg
   2349
<210> 85
   <211> 273
   <212> PRT
   <213> Mus musculus
<400> 85
   Phe Leu Ser Leu Ile Val Ala Leu Val Gly Leu Val Gly Asn Ala Thr
   1               5                   10                  15
   Val Leu Trp Phe Leu Gly Phe Gln Met Arg Arg Asn Ala Phe Ser Val
               20                  25                  30
   Tyr Ile Leu Asn Leu Ala Gly Ala Asp Phe Leu Phe Ile Cys Phe Gln
           35                  40                  45
   Ile Gly Tyr Cys Phe His Met Ile Leu Asp Ile Asp Ser Ile Pro Ile
       50                  55                  60
   Glu Ile Asp Leu Phe Tyr Leu Val Val Leu Asn Phe Pro Tyr Phe Cys
   65                  70                  75                  80
   Gly Leu Ser Ile Leu Ser Ala Ile Ser Ile Glu Arg Cys Leu Ser Val
                   85                  90                  95
   Met Trp Pro Ile Trp Tyr His Cys Gln Arg Pro Arg His Thr Ser Ala
               100                 105                 110
   Val Ile Cys Thr Leu Leu Trp Val Leu Ser Leu Val Cys Ser Leu Leu
           115                 120                 125
   Glu Gly Lys Glu Cys Gly Phe Leu Tyr Tyr Thr Ser Asp Pro Gly Trp
       130                 135                 140
   Cys Lys Thr Phe Asp Leu Ile Thr Ala Thr Trp Leu Ile Val Leu Phe
   145                 150                 155                 160
   Val Ala Leu Leu Gly Ser Ser Leu Ala Leu Val Ile Thr Ile Phe Trp
                   165                 170                 175
   Gly Leu His Lys Ile Pro Val Thr Arg Leu Tyr Val Ala Ile Val Phe
               180                 185                 190
   Thr Val Leu Val Phe Leu Leu Phe Gly Leu Pro Tyr Gly Ile Tyr Trp
           195                 200                 205
   Phe Leu Leu Val Trp Ile Glu Lys Phe Tyr Tyr Val Leu Pro Cys Ser
       210                 215                 220
   Ile Tyr Pro Val Thr Val Phe Leu Ser Cys Val Asn Ser Ser Ala Lys
   225                 230                 235                 240
   Pro Ile Ile Tyr Cys Leu Val Gly Ser Ile Arg His His Arg Phe Gln
                   245                 250                 255
   Arg Lys Thr Leu Lys Leu Phe Leu Gln Arg Ala Met Gln Asp Thr Pro
               260                 265                 270
   Glu
<210> 86
   <211> 1313
   <212> DNA
   <213> Mus musculus
<400> 86
   tttatttaat tattttgtta ttgttgtttc aggtagcaag tatttcctaa gcatgggata 60
   tagacatttc gagcctgggc atttacatca tagcaccgaa tggaagcagc tacactaata 120
   gtgttgattg tttcttcaaa atccaagtca tgggttttct ttccctcatc atttcccctg 180
   ttgggatggt attaaattcc acagtgctgt ggtttctggg cttccagata cgtaggaatg 240
   ccttctctgt ctacatcctc aacctggccg gggctgactt tctcttcctg cactctcagt 300
   ttttatttta ccttcttgct atttttccct ccattcctat ccagatccct ctcttttttg 360
   atatgttgac aaaatttgca tatctttctg ggctgagcat tctcagcacc attagcattg 420
   agcgctgcct gtgtgtcatg tggcccatct ggtaccgctg tcaaagacca agacacacat 480
   catctgtaac ctgttccttg ctttgggctt tgtccctgtt gtttgctctt ctggatggga 540
   tgggatgtgg cttactgttt aatagttttg accagtcttg gtgtttgaaa tttgatttaa 600
   tcatttgtgc gtggtcaatt gttttatttg tggttctctg tgggtccagt ctcatcctac 660
   ttgttaggat cttctgtggc tcccagcaga tccctgtgac caggctgtat gtgaccattg 720
   cactcacagt gttattcttc ctaatctgct gtcttccctt tggaatctcc tggatcatcc 780
   aatggagtga aactttgata tatgttggat tttgtgatta ttttcacgag gaactattcc 840
   tatcctgtat taacagctgt gccaacccta tcatttactt ccttgttggt tttattcgtc 900
   agcgaaagtt ccaacagaag tctctgaagg tgcttcttca aagagcgatg gaggacactc 960
   ctgaagaaga aaatgaagac atgggtcctt caagaaatcc agaagaattt gaaacagtct
   1020
   gtagcaactg agaggttctt tgatcagaca gaaatggttt tttagagaaa aaaatttttt
   1080
   ctcatttctg tgggccattt tcacagtttt gyacagtttg tttcctgata ttcaatcagt
   1140
   taaaaaataa gcatttttgt gaaagtggat agatacaaga cttgtcatac aaatactgac
   1200
   tgtagtattt ttggagctgt tactcagact ttcatcatct ccttttgatg ggattccatg
   1260
   taagtgtctg gagttgagga gatgtgttga ccactattga caaagccctc att
   1313
<210> 87
   <211> 270
   <212> PRT
   <213> Mus musculus
<400> 87
   Phe Leu Ser Leu Ile Ile Ser Pro Val Gly Met Val Leu Asn Ser Thr
   1 5 10 15
   Val Leu Trp Phe Leu Gly Phe Gln Ile Arg Arg Asn Ala Phe Ser Val
                           20                      25                      30
   Tyr Ile Leu Asn Leu Ala Gly Ala Asp Phe Leu Phe Leu His Ser Gln
               35                      40                      45
   Phe Leu Phe Tyr Leu Leu Ala Ile Phe Pro Ser Ile Pro Ile Gln Ile
           50                      55                      60
   Pro Leu Phe Phe Asp Met Leu Thr Lys Phe Ala Tyr Leu Ser Gly Leu
   65                      70                      75                      80
   Ser Ile Leu Ser Thr Ile Ser Ile Glu Arg Cys Leu Cys Val Met Trp
                       85                      90                      95
   Pro Ile Trp Tyr Arg Cys Gln Arg Pro Arg His Thr Ser Ser Val Thr
               100                     105                     110
   Cys Ser Leu Leu Trp Ala Leu Ser Leu Leu Phe Ala Leu Leu Asp Gly
           115                     120                     125
   Met Gly Cys Gly Leu Leu Phe Asn Ser Phe Asp Gln Ser Trp Cys Leu
       130                     135                     140
   Lys Phe Asp Leu Ile Ile Cys Ala Trp Ser Ile Val Leu Phe Val Val
   145                     150                     155                     160
   Leu Cys Gly Ser Ser Leu Ile Leu Leu Val Arg Ile Phe Cys Gly Ser
                       165                     170                     175
   Gln Gln Ile Pro Val Thr Arg Leu Tyr Val Thr Ile Ala Leu Thr Val
               180                     185                     190
   Leu Phe Phe Leu Ile Cys Cys Leu Pro Phe Gly Ile Ser Trp Ile Ile
           195                     200                     205
   Gln Trp Ser Glu Thr Leu Ile Tyr Val Gly Phe Cys Asp Tyr Phe His
       210                     215                     220
   Glu Glu Leu Phe Leu Ser Cys Ile Asn Ser Cys Ala Asn Pro Ile Ile
   225                     230                     235                     240
   Tyr Phe Leu Val Gly Phe Ile Arg Gln Arg Lys Phe Gln Gln Lys Ser
                       245                     250                     255
   Leu Lys Val Leu Leu Gln Arg Ala Met Glu Asp Thr Pro Glu
               260                     265                     270
<210> 88
   <211> 1883
   <212> DNA
   <213> Mus musculus
<400> 88
   cgtgtgccac caccaccaac aggtgggaca tttcttaaag tatactattc atttaatctt 60
   tatcaagttt aattaccaaa gcaattctga cacttcttgc actaccttga tccttttcct 120
   gagggaggca tttgttccca gtgagagctg ttctgacccc aagagattac aagggttaca 180
   tcacaagggg gtgcagtaag gcatacataa ggcagtttga tggtgctgca gtgaatttct 240
   gagtaacaag ctccatttct cctaatttga ataaaatgac tattttctct accaattaaa 300
   caagattgtg aaaactgcct acatagataa aagcaaaatt gactctcaga gaaactatgt 360
   ctcatcaagt actctttcaa agcctgcact agactctttc cagttcccta gcctttgtga 420
   aggacccctc tctcctctct tttcctcact actgtcctac atggttctct gcagaagttg 480
   cttcaaactc tgacattgca acctacggtg cctaacagag ccaaggagag agtaaataat 540
   gggattggca cagctgttaa cacaggaatg ctatcacttc aaaaacattg tatgagaaca 600
   tgctatgtaa gtccataaac attgtcaaga ggaatgtgca gattccaatg ggcataccaa 660
   agaatatgaa gaccatcaat gtgagggcaa tggacacata gaacatggtc acaggaatcc 720
   tgagtgatac acagaacatt tgacaaacag ggccaggcta gacacaaaak aaaccacaga 780
   taatactatt atcaatgcag tagygatata gtggcatrta atacagaaat tgtgttcwta 840
   ataacttaac agaaagccac agccttgtrc aaasrgaagg atcarcagta tagagaaaac 900
   ccagagcaga gcacacatga cagctgatgt gtgtcttggt cttcagcagt gataccagat 960
   gggacacata acagataggc agtgctcagc actgattgtt gmaatcatac acaaacctgc
   1020
   aagttaagca atcataaatc ctgtgaggat aaaatgatag tagatcataa gtatcttaag
   1080
   gaaacactgc aggggaatgt acaaactgtg tgcaaatttg caagaaatca gcacaagaca
   1140
   ggtttaagac atagacagag aaggcattcc tatgcaggtg gaaggctaga agccatagca
   1200
   ctatggcatt tcctgccagg ccaagcacag caatgatgac aataagaaaa ttgaatgtgg
   1260
   tgaaacagga taaatttttc agtgcattaa cttccattga cttctgtgtt tttaaatttc
   1320
   cattccaggg tggttggatc catgcttagg aattttccac tggcattcct gcaaagaaat
   1380
   agagatatga atctagggta ctctttgtag ggactatgtg actatgtagg aatgtatggc
   1440
   acaggtacat aaggagggag aaacaggatc acagagatta agtaatttac caacattcca
   1500
   aaagtgctac acatttttga aatccatttt gtactattca gtctaactgc agaccagtat
   1560
   gatgtaaggt agttgatggt cccagtacag ttgctaggca tttatttcag gttatgtgag
   1620
   gaagagacag aactctgaaa ccaacattct ttttgttcta gggctgagat ttcttctctg
   1680
   gtgtaggaaa atggaagttc ttggtgcaag ccatatcttc cctcagtcac tgggaggaat
   1740
   ctatcaaaca ggcaaaatag aatcatgaat gagagtcatg aatgagattc acgaagggaa
   1800
   tggtacttgc tatgaagacc tgtaggggaa tagccatgct tcttatgctt gaaagggtag
   1860
   ttgctcattt aacaatttta aaa
   1883
<210> 89
   <211> 263
   <212> PRT
   <213> Mus musculus
<400> 89
   Phe Leu Ile Val Ile Ile Ala Val Leu Gly Leu Ala Gly Asn Ala Ile
    1               5                  10                  15
   Val Leu Trp Leu Leu Ala Phe His Leu His Arg Asn Ala Phe Ser Val
               20                  25                  30
   Tyr Val Leu Asn Leu Ser Cys Ala Asp Phe Leu Gln Ile Cys Thr Gln
           35                  40                  45
   Phe Val His Ser Pro Ala Val Phe Leu Lys Ile Leu Met Ile Tyr Tyr
       50                  55                  60
   His Phe Ile Leu Thr Gly Phe Met Ile Ala Leu Ala Gly Leu Cys Met
   65                  70                  75                  80
   Ile Ser Thr Ile Ser Ala Glu His Cys Leu Ser Val Met Trp Pro Ile
                   85                  90                  95
   Trp Tyr His Cys Arg Pro Arg His Thr Ser Ala Val Met Cys Ala Leu
               100                 105                 110
   Leu Trp Val Phe Ser Ile Leu Leu Ile Leu Leu Phe Val Gln Gly Cys
           115                 120                 125
   Gly Phe Leu Leu Ser Tyr Tyr Glu His Asn Phe Cys Ile Ile Cys His
       130                 135                 140
   Tyr Ile Ala Thr Ala Leu Ile Ile Val Leu Ser Val Val Ser Phe Val
   145                 150                 155                 160
   Ser Ser Leu Ala Leu Phe Val Thr Met Phe Cys Val Ser Leu Arg Ile
                   165                 170                 175
   Pro Val Thr Met Phe Tyr Val Ser Ile Ala Leu Thr Leu Met Val Phe
               180                 185                 190
   Ile Phe Phe Gly Met Pro Ile Gly Ile Cys Thr Phe Leu Leu Thr Met
           195                 200                 205
   Phe Met Asp Leu His Ser Ser Ser His Thr Met Phe Leu Lys His Ser
       210                 215                 220
   Cys Val Asn Ser Cys Ala Asn Pro Ile Ile Tyr Ser Leu Leu Gly Ser
   225                 230                 235                 240
   Val Arg His Arg Arg Leu Gln Cys Gln Ser Leu Lys Gln Leu Leu Gln
                   245                 250                 255
   Arg Thr Met Asp Ser Ser Glu
               260
<210> 90
   <211> 1219
   <212> DNA
   <213> Mus musculus
<400> 90
   ttataaatga ttttattaag ccatattgac aataatatct atattatatg atgattgcca 60
   gaagaagggt aaatgttaag gtgatcaaat atggtctgtg ttctcagaga caccactgga 120
   agatttgtga gcatggatcc aaccatctca tcccacaaca cagaatctac accactgaat 180
   gaaactggtc attccaaatg cagtccaatc ctgactctgt cctttctggt cctcatcact 240
   gtcctggtgg aactaggagg aagcaccatt gtactctggc tcctggaatt cagcatgccc 300
   aggaaagcca tctcagtcta tgtcctcaat ctggctctgg cagactcctt cttcctcggc 360
   tgcgatttca ttgaatttct gctacggatc attgacttca tctatgccca taaattaagc 420
   aaagatatct taggcaatac agcaatcatt ccttatatcg caggacagaa cgttctcagt 480
   gctattagca tggagcactg cctgtctgta ttgtggccaa tctggtacca ctaccaccac 540
   ccaagaaaca tgtcagctat catatgtgcc ctaatctggg ttctgtactt tctcatgggc 600
   atcctccatt ggttcttctc agtattcctg ggtgaggctc atcatcattt gaggaaaaag 660
   gttgacttta ctataactgc atttctgaat ttttatttat gcttcactct gtgtccagtc 720
   tggccctact gctgaggatc ctctgtggct ccaggaggaa acccctgtcc aggctgtatg 780
   ttaccatcgc tctcacagtg atggtcacct catctctggc ctgcctcttg ggctttactt 840
   gttcctgtta tactggtttg gggttcattt gcatcatccc tcttgtcaca attaccaagt 900
   tacttcagtc ctgccctgtg taaacagcta taacaacccc atcatttact tcattgtagg 960
   ctcctttagg cctcttagaa agcattaatc cctccaaact attcttaaga gggctctgga
   1020
   ggacactcct gaggagcatg aatatacagc cagccatctt cagaaaacca ctgagatgtc
   1080
   agaaagcatt tttgagagtc aaaacaacat taacttaatc ttctctcaga aacccctcag
   1140
   tgattgcact gctttcaatt gattattttt tatccaattt tcttatactt ctcaaagtag
   1200
   tcataaataa gaatttctc
   1219
<210> 91
   <211> 270
   <212> PRT
   <213> Mus musculus
<400> 91
   Phe Leu Val Leu Ile Thr Val Leu Val Glu Leu Gly Gly Ser Thr Ile
    1               5                  10                  15
   Val Leu Trp Leu Leu Glu Phe Ser Met Pro Arg Lys Ala Ile Ser Val
               20                  25                  30
   Tyr Val Leu Asn Leu Ala Leu Ala Asp Ser Phe Phe Leu Gly Cys Asp
           35                  40                  45
   Phe Ile Glu Phe Leu Leu Arg Ile Ile Asp Phe Ile Tyr Ala His Lys
       50                  55                  60
   Leu Ser Lys Asp Ile Leu Gly Asn Thr Ala Ile Ile Pro Tyr Ile Ala
   65                  70                  75                  80
   Gly Gln Asn Val Leu Ser Ala Ile Ser Met Glu His Cys Leu Ser Val
                   85                  90                  95
   Leu Trp Pro Ile Trp Tyr His Tyr His His Pro Arg Asn Met Ser Ala
               100                 105                 110
   Ile Ile Cys Ala Leu Ile Trp Val Leu Tyr Phe Leu Met Gly Ile Leu
           115                 120                 125
   His Trp Phe Phe Ser Val Phe Leu Gly Glu Ala His His His Leu Arg
       130                 135                 140
   Lys Lys Val Asp Phe Thr Ile Thr Ala Phe Leu Ile Phe Leu Phe Met
   145                 150                 155                 160
   Leu His Ser Val Ser Ser Leu Ala Leu Leu Leu Arg Ile Leu Cys Gly
                   165                 170                 175
   Ser Arg Arg Lys Pro Leu Ser Arg Leu Tyr Val Thr Ile Ala Leu Thr
               180                 185                 190
   Val Met Val Tyr Leu Ile Ser Gly Leu Pro Leu Gly Leu Tyr Leu Phe
           195                 200                 205
   Leu Leu Tyr Trp Phe Gly Val His Leu His His Pro Ser Cys His Asn
       210                 215                 220
   Tyr Gln Val Thr Ser Val Leu Pro Cys Val Asn Ser Tyr Asn Asn Pro
   225                 230                 235                 240
   Ile Ile Tyr Phe Ile Val Gly Ser Phe Arg Pro Leu Arg Lys His Ser
                   245                 250                 255
   Leu Gln Thr Ile Leu Lys Arg Ala Leu Glu Asp Thr Pro Glu
               260                 265                 270
<210> 92
   <211> 1178
   <212> DNA
   <213> Mus musculus
<400> 92
   ttaaggtgat gaaatatggt ctgtgttctc agggacacca ctggaagatt tgtgagcatg 60
   gatccaatca tctcatccca caacagagaa tcacaccact gaatgaaact gcaatcattc 120
   caactgcagt ccaatcctga ctctgtcctt tctggtcctc atcactatcc tggtggaact 180
   ggcaggaaac accattgtcc tctggctctt gggattccgc atgcacagga aagccatctc 240
   agtttatgtc ctcaatctgg ctctggcaga ctccgtattc ctctgctgtc atttcattga 300
   ctctctgcta tgcatcattg acttcatcta tgcccataaa ttaagcagat accttaggca 360
   atgcagaaat cattccctat atcacagggc tgagcatcct cagtgctatt agcatggagg 420
   actacctgtc tgtattgtgg ccaatctggt accactgcca tcacccaagg aacatgtcaa 480
   ctatcctatg tgccctaatc tgggttctat cctttctcat gggcatcctc gattggttct 540
   tctcaggatt cctgggtgag actcatcatt atttgtgaaa aaatgttgac tttattataa 600
   ctgcatttct gatttttttt tttatttatg cttctctctg ggtccagtct ggccctactg 660
   ctgaggatcc tctgtggctc caggaggaaa ccactgtcca ggttgtatgc taccatctca 720
   ctcacagtga tggtctacct catctgtggc ctacctcttg ggctttactt gtttctgtta 780
   cactcctttg gggttaattt gcatcatccc ttttgtcacc tttacaaagt tactgcagtc 840
   ctgtcctgtg taaacatctc taccaacccc atcaatcatt taattcattg gcatttcttt 900
   tttttttaat taggtatttt cctcgtttac attttcaatg ctatcccaaa ggtcccccat 960
   acccaccccc cccaatccct acccacccac tgcccctttt tggcactggc gttcccctgt
   1020
   actggggcat ataaagtttg caagtccaat gggcctctct ttgcagtgat gaccgactag
   1080
   gccatctttt gatacatatg cagctaaaga catgagctcc cgggtactgg ttagttcata
   1140
   ttgttgttcc acctataggg ttgcagttcc ctttagct
   1178
<210> 93
   <211> 243
   <212> PRT
   <213> Mus musculus
<400> 93
   Phe Leu Val Leu Ile Thr Ile Leu Val Glu Leu Ala Gly Asn Thr Ile
    1               5                  10                  15
   Val Leu Trp Leu Leu Gly Phe Arg Met His Arg Lys Ala Ile Ser Val
               20                  25                  30
   Tyr Val Leu Asn Leu Ala Leu Ala Asp Ser Val Phe Leu Cys Cys His
           35                  40                  45
   Phe Ile Asp Ser Leu Leu Cys Ile Ile Asp Phe Tyr Leu Cys Pro Asp
       50                  55                  60
   Ala Asp Thr Leu Gly Asn Ala Glu Ile Ile Pro Tyr Ile Thr Gly Leu
   65                  70                  75                  80
   Ser Ile Leu Ser Ala Ile Ser Met Glu Asp Tyr Leu Ser Val Leu Trp
                   85                  90                  95
   Pro Ile Trp Tyr His Cys His His Pro Arg Asn Met Ser Thr Ile Leu
               100                 105                 110
   Cys Ala Leu Ile Trp Val Leu Ser Phe Leu Met Gly Ile Leu Asp Trp
           115                 120                 125
   Phe Phe Ser Gly Phe Leu Gly Glu Thr His His Tyr Leu Lys Asn Val
       130                 135                 140
   Asp Phe Ile Ile Thr Ala Phe Leu Ile Phe Phe Phe Ile Leu Leu Leu
   145                 150                 155                 160
   Ser Gly Ser Ser Leu Ala Leu Leu Leu Arg Ile Leu Cys Gly Ser Arg
                   165                 170                 175
   Arg Lys Pro Leu Ser Arg Leu Tyr Ala Thr Ile Ser Leu Thr Val Met
               180                 185                 190
   Val Tyr Leu Ile Cys Gly Leu Pro Leu Gly Leu Tyr Leu Phe Leu Leu
           195                 200                 205
   His Ser Phe Gly Val Asn Leu His His Pro Phe Cys His Leu Tyr Lys
       210                 215                 220
   Val Thr Ala Val Leu Ser Cys Val Asn Ile Ser Thr Asn Pro Ile Asn
   225                 230                 235                 240
   His Leu Ile
<210> 94
   <211> 2416
   <212> DNA
   <213> Mus musculus
<400> 94
   atggagggac ccatggctcc agttgcatgt gtagcagagg atggccttgt agctcatcaa 60
   tgggaggaga gacttttggt cctgtgaagg ccctataccc cagtgttggg ggttgccagg 120
   gagaagaagt gggagtgggt gggttggtgt acagagggag ggcgataatg ggttttcaaa 180
   ggaaaaatca ggaaaaggga taacatttga aatgtaaata aagaaaatat ttaataaaaa 240
   gcaaaaatga aaaaaaagtg caaaaacatg ttctattatg ggagtgggtg tgttgaggag 300
   cagtggggga gggttaaata gagaggggac tgttggaggg gaaactagga aaggggataa 360
   cattggaaat gtaaataaag aaaatatcta ataaaaaata aaataaaaaa ttttggaaga 420
   tatttgaaaa attcattgac aagggcaaga atgttggaga aattcttatt tttgactact 480
   ttgagaagta taagaaaatt agattaaaaa taatcaattg aaagcactgc aatcactgag 540
   gcgtttctga gagaagagta agttaatgtt gtcttgactc tcaacatatg ctttctgaca 600
   tctcagtggt tttctgaaga tggctgtctg tatattcatc ctcttcagga gtgtctttca 660
   gagccctatt aagaatagtt tggaaggaac aacactttct acaatgccta aaggagccta 720
   caatgaagta aatgatggga ttggcagagc tgtttacaca ggacaggact gcagttactt 780
   ggtaaatgtg acaagaggga taatgcaaat gaaccccaaa ccagtgtagc aggaaaaagt 840
   aaagcccaag aggcaggcca cagatgagat agaccatcac tgtgagagag atggtaactt 900
   acagcctgga caggggtttc ttcctaggac cacagaggat cctcagcagt agggccagac 960
   tggacacaga gagaagcata aataaaaaaa tcagaaatgc agttataata aaggcaacat 1020
   tttccacaaa tgatgattag tctcacccag gaatcctaag aagaaccaat ccaggatgcc 1080
   tatgagaatg gacagaaccc agattagggc atataggata gctgacatgt tactttggtg 1140
   gtggaagtca taccagattg gccacaatac agacaggcag tgctccatgc taatagcact 1200
   gagcaggctg tgccctgcca tatagggaat gattgctgca ttgcctaaga tatctttgtt 1260
   taatttatgg gcatagatga agtcaatgat ccatagcaga gagtcaatga aatggcagca 1320
   gaggaagaag gagtcgccca gagccagatt gaggacatag cctgagatgg gtttcctgtg 1380
   cattcagaat cccaggagcc agagaacaat cgtgtttcct gccagttcca ccaggacagt 1440
   gatgaggacc agaaaggacg gagtcaggat tggactgcag ttgggatgac cagtttcatt 1500
   cagtggtatg attcctgtgt tgtgtgatga gatgattgga tccatgctca caaatctttc 1560
   agtggtgtta ctgagaacac agaccacatt taatcacctt aaaattgacc cttcttctgg 1620
   aaatcataat ataatataga tatttttgtc aatatgcctt aataaaatca tttataaata 1680
   aaaggaaagt aacatgacca tatggatcaa gaattctggg ctgtgaattc aaattcagag 1740
   cttgtgtata ctctatagtg tgggtcatac ttcctgtgta taactcagga ctttttaatc 1800
   gcgtggaaat ggttccattc tctcatggac aaggttggat ccatttcctg ctctcctgta 1860
   accccagaaa gggaagcacc agatttgcct ccccagggct taaaataaca caggaaagat 1920
   gaagatatca gggtattgtc gaggtacatt aagggaaata tccttctgca tggtcaaaag
   1980
   aatgtattct gagttatgca cctaactctc ggtcgagaca tgacactggt ctgtgcaaca
   2040
   gattacagat cacatgcatt tacctcctcc cttgagatga ccaagctgca cctatcagtc
   2100
   acttcaccag gggattgctg aggtggcaga aggaatgaca actcactcat ctttcacagg
   2160
   agttatacct tctctgcagc catctctgac cttccctcag ctggtacagt taagcctgtc
   2220
   tgcttttctg aaagcactta aggttccttt ttctttcttt agatctcctt ttcttttgaa
   2280
   catgggtcaa aagaccaagc aacattttcc tgagagtctg gactctctca atcatttctg
   2340
   aaacccacat ctctttccac catgaaagtt ttttcccaac ttccattgct ggacatacca
   2400
   gctttcttgg ggatgt
   2416
<210> 95
   <211> 269
   <212> PRT
   <213> Mus musculus
<400> 95
   Phe Leu Val Leu Ile Thr Val Leu Val Glu Leu Ala Gly Asn Thr Ile
    1               5                  10                  15
   Val Leu Trp Leu Leu Gly Phe Met His Arg Lys Pro Ile Ser Gly Tyr
               20                  25                  30
   Val Leu Asn Leu Ala Leu Gly Asp Ser Phe Phe Leu Cys Cys His Phe
           35                  40                  45
   Ile Asp Ser Leu Leu Trp Ile Ile Asp Phe Ile Tyr Ala His Lys Leu
       50                  55                  60
   Asn Lys Asp Ile Leu Gly Asn Ala Ala Ile Ile Pro Tyr Met Ala Gly
   65                  70                  75                  80
   His Ser Leu Leu Ser Ala Ile Ser Met Glu His Cys Leu Ser Val Leu
                   85                  90                  95
   Trp Pro Ile Trp Tyr Asp Phe His His Gln Ser Asn Met Ser Ala Ile
               100                 105                 110
   Leu Tyr Ala Leu Ile Trp Val Leu Ser Ile Leu Ile Gly Ile Leu Asp
           115                 120                 125
   Trp Phe Phe Leu Gly Phe Leu Gly Glu Thr Asn His His Leu Cys Glu
       130                 135                 140
   Asn Val Ala Phe Ile Ile Thr Ala Phe Leu Ile Phe Leu Phe Met Leu
   145                 150                 155                 160
   Leu Ser Val Ser Ser Leu Ala Leu Leu Leu Arg Ile Leu Cys Gly Pro
                   165                 170                 175
   Arg Lys Lys Pro Leu Ser Arg Leu Val Thr Ile Ser Leu Thr Val Met
               180                 185                 190
   Val Tyr Leu Ile Cys Gly Leu Pro Leu Gly Leu Tyr Phe Phe Leu Leu
           195                 200                 205
   His Trp Phe Gly Val His Leu His Tyr Pro Ser Cys His Ile Tyr Gln
       210                 215                 220
   Val Thr Ala Val Leu Ser Cys Val Asn Ser Ser Ala Asn Pro Ile Ile
   225                 230                 235                 240
   Tyr Phe Ile Val Gly Ser Phe Arg His Cys Arg Lys Cys Cys Ser Phe
                   245                 250                 255
   Gln Thr Ile Leu Asn Arg Ala Leu Lys Asp Thr Pro Glu
               260                 265
<210> 96
   <211> 1954
   <212> DNA
   <213> Mus musculus
<400> 96
   tggcattcgg tacctgcctc ctggcagaag atgaaggccc gaaatagggc atgtcccagt 60
   aagctgttag cttctgtatt ccaaactctc acctacacag actagtctca gagggatcgg 120
   ggaaccaaga tggcttcccc atggtactcc agcaaaacac tcccaggtga ggtggacacc 180
   tctcctctga cagggaaggt gcccggatat ctggagcctg aaacggggtc tgcctcagaa 240
   gctgttagct tctgtagtcc acactctcac atgtgtaggc tagtctcagc aggatccagg 300
   aaccaagatc agaagggtca atgttcaggt gatcaaatgt agtctgtgtt cacagggata 360
   ccactggaag atttgtgagc atggatccaa tcatctcatc ccacaacaca gaatcacacc 420
   actgaatgaa actggtcatc ccaactgcag tacaatcctg actccatcct ttctggtcct 480
   catcactgtc ctggtggaac tggcaggaaa taccattgta ctctggctcc tgagattcca 540
   catgcacagg atagcccatc tcagactatg tcctcaatct ggctctggca gattccttct 600
   tcctctcctg ccagttcatt gactctctgc tatggatcct tgacttcatc taggcccata 660
   aattaagcaa agatatctta tggaatgcag caatcattcc caataatgca gggctgagct 720
   acctcagtgc tattagcatg gagcactgcc tgcctgtatt gtggccaatc tggcaccact 780
   gccaccacac aagaaacatg tcagctatca tatgtgccct aatctgggtt ctgtcctttc 840
   tcatgggcat cctcgattag tacttctcag gattcctggg tgagactcat catcagttgt 900
   ggaaaaatgt tgattttatt ctaactgcat ttctgatttc tttttttttt tatttatgct 960
   tctctctggg tccagtctgg ccctacgact gaggatcctc tgtggctcca ggaggaaacc
   1020
   cctgtccttg ctgtatgtta tcatctctct cacagtgatg gtctacctca tctgtggcct
   1080
   acctgttggg ctttacttgt tcctgttaaa ctggtttggg gttcatttgc atcatcccat
   1140
   ttgtcacatt tatcaagtta ctgcactcct gccctttgta aacagctttg ccaaacccat
   1200
   catttccttc attgtaggct cctttaggca ttgtagaaag cattggtccc gccaaactat
   1260
   tattaagagg gctctggagg acactcctga ggaggatgaa tatacagata gccatcttca
   1320
   gaaaactact gagatgtcag aaagcagatg ttgagagtca agacaacatt aacttaatct
   1380
   tctctcagaa acacctcact ggttgcagtg ctttcaattg attatttttt aatccaattt
   1440
   tcttataagt ctcaaagtag tcataaataa gaatttctcc aacattcttg gccttgtcaa
   1500
   tgaatttctc aaatatcctc caaaacattt tgtatataat ttaatttttt tagatatttt
   1560
   ctatatttat atttccaatg ttatcccctt yccttagttt cccctccaaa agccccctct
   1620
   ccccttcccc cccccactgc tcctcaatat actcactccc ataattgaac acctttttgc
   1680
   acttttttct tttttttcac tttttgtttt ttattagata ttttctttat ttacatttca
   1740
   aatgttgtcc cttttcctga ttttccctct gaaaacccat tactgtcatc cccctgtaca
   1800
   ccatccctcc cacttctact tctatcctag gcattcccct acactggggt atagggcctt
   1860
   cacaggacca agagtctctc ctcccattga tgagctacaa ggccatcctc tgctacacat
   1920
   ggcaactgga gccatgggtc cctccatgtg tact
   1954
<210> 97
   <211> 272
   <212> PRT
   <213> Mus musculus
<400> 97
   Phe Leu Val Leu Ile Thr Val Leu Val Glu Leu Ala Gly Asn Thr Ile
    1               5                  10                  15
   Val Leu Trp Leu Leu Arg Phe His Met His Arg Ile Ala Leu Ser Asp
               20                  25                  30
   Tyr Val Leu Asn Leu Ala Leu Ala Asp Ser Phe Phe Leu Ser Cys Gln
           35                  40                  45
   Phe Ile Asp Ser Leu Leu Trp Ile Leu Asp Phe Ile Ala His Lys Leu
       50                  55                  60
   Ser Lys Asp Ile Leu Trp Asn Ala Ala Ile Ile Pro Asn Asn Ala Gly
   65                  70                  75                  80
   Leu Ser Tyr Leu Ser Ala Ile Ser Met Glu His Cys Leu Pro Val Leu
                   85                  90                  95
   Trp Pro Ile Trp His His Cys His His Thr Arg Asn Met Ser Ala Ile
               100                 105                 110
   Ile Cys Ala Leu Ile Trp Val Leu Ser Phe Leu Met Gly Ile Leu Asp
           115                 120                 125
   Tyr Phe Ser Gly Phe Leu Gly Glu Thr His His Gln Leu Trp Lys Asn
       130                 135                 140
   Val Asp Phe Ile Leu Thr Ala Phe Leu Ile Val Phe Phe Phe Leu Phe
   145                 150                 155                 160
   Met Leu Leu Ser Gly Ser Ser Leu Ala Leu Arg Leu Arg Ile Leu Cys
                   165                 170                 175
   Gly Ser Arg Arg Lys Pro Leu Ser Leu Leu Tyr Val Ile Ile Ser Leu
               180                 185                 190
   Thr Val Met Val Tyr Leu Ile Cys Gly Leu Pro Val Gly Leu Tyr Leu
           195                 200                 205
   Phe Leu Leu Asn Trp Phe Gly Val His Leu His His Pro Ile Cys His
       210                 215                 220
   Ile Tyr Gln Val Thr Ala Leu Leu Pro Phe Val Asn Ser Phe Ala Lys
   225                 230                 235                 240
   Pro Ile Ile Ser Phe Ile Val Gly Ser Phe Arg His Cys Arg Lys His
                   245                 250                 255
   Trp Ser Arg Gln Thr Ile Ile Lys Arg Ala Leu Glu Asp Thr Pro Glu
               260                 265                 270
<210> 98
   <211> 1893
   <212> DNA
   <213> Mus musculus
<400> 98
   ttagcaatcc cctggccagg tgactgacag gtgcagctta gtctttctca agggatgagg 60
   taattgcatg tgatctgtaa tctgttgcac agaccagtgt catgtctcaa cccagagtta 120
   ggtgtataac tcagaatcca tttttttgac catgcagaag catctttcct ttaatgtact 180
   tcaacaaaac cctgatatct tcatcttttc tgcgttattt taagccctgg ggaggcaaat 240
   atgatgcttc ccctttctag gggttacagg ggagcaggaa atggatgcag ccctgaccat 300
   gatagtaggg aatcatttcc atgtgattta aaggtcctga gttatacaca ggaagaatga 360
   cccagactag agtatgtaca agctctgaat ttgaatccaa atccagaatt cttgatccac 420
   atggtcatgt tattctcctt tttttataaa tgattttatt aagccatatt gacaacaata 480
   tctatattac attatgattg ccagaagaag ggtcaatgtt aaggtgatga aatatggtct 540
   gtgttcctca ggcacaacac tggaagattt ttgagcatgg atccaaccat ctcattccac 600
   aacacagaat ctacaccact gaatgaaact tgtcatccaa atacagtcca atcctgactc 660
   cgtcctttct ggtcctcatc actgtcctgg tggacctggc aggaaacacc attgttctct 720
   ggctcctggg attccgcatg cacaggaaac ccatctcagt ctatgtcctc aacctggctc 780
   tgggcgactc cttcttctgc tgccatttca ttgactctct gctatggatc attgacttca 840
   tctatgccca taaattaagc aaagatatct taggcaatgt agcaatcgtt ccctatatcg 900
   cagggctgag cgtcctcagt gctattagca tggagaactg actgtttata ttgtggccaa 960
   tctggtacca ctgccaccac ccaagaaaca tgtcagctat cctatgtgcc ctaatctggg 1020
   ttctgttctt tctcatgggc atcctcggtt ggttcttctt aagatttttg ggtgaaactc 1080
   atcattgact ttattatacc tgcatttctg attttttttt tatttatgct tctctctggg 1140
   tccattctgg ccctactgct gaggatcctc tatggttcca ggaggaaatc cctgtccagg 1200
   ttgtatgtta acatctctct cacagtgatg gtctacctca tctgtggcct gcctcttgga 1260
   ctttacttgg tcctgttata ctgctttggg gttcatttac atcatccctc tcctcacatt 1320
   taccaagtta ctgtggtctt gtcctatgtg gacagctctg ccaaccacat cttttatttc 1380
   cttgcaggtt cctttaggta ttgtagaaag cattggtccc tccaaactct tctaaagagg 1440
   actctagagg acactcctgg ggaggatgaa tatacagaca gccatcttca gaaaaccact 1500
   gagatgtcag aaagcagatg ttgagagtca acacattaac ttactcttct ctaagaaacg 1560
   cctcagtgat tgcaatgctt tcaattggtt tttcttttta atcaaatttt cttatacttc 1620
   tcaaagtagt cagaaatgag aatttctcga aaattcttgg cactgtcaat gaatttttca 1680
   aatatcttcc aaaactttct tattttattt tattttattt ttattagaca ttttctttat 1740
   ttacatttca aatgttatcc cctttactag tttcccctcc aaaaaagcac tatcccctca 1800
   cccctctacc tgctccccac attacccact cccataattg aacacttttt tcttttttta 1860
   acttattatt tttattagat attttcttta ttt 1893
<210> 99
   <211> 262
   <212> PRT
   <213> Mus musculus
<400> 99
   Phe Leu Val Leu Ile Thr Val Leu Val Asp Leu Ala Gly Asn Thr Ile
    1               5                  10                  15
   Val Leu Trp Leu Leu Gly Phe Arg Met His Arg Lys Pro Ile Ser Val
               20                  25                  30
   Tyr Val Leu Asn Leu Ala Leu Gly Asp Ser Phe Phe Cys Cys His Phe
           35                  40                  45
   Ile Asp Ser Leu Leu Trp Ile Ile Asp Phe Ile Tyr Ala His Lys Leu
       50                  55                  60
   Ser Lys Asp Ile Leu Gly Asn Val Ala Ile Val Pro Tyr Ile Ala Gly
   65                  70                  75                  80
   Leu Ser Val Leu Ser Ala Ile Ser Met Glu Asn Leu Phe Ile Leu Trp
                   85                  90                  95
   Pro Ile Trp Tyr His Cys His His Pro Arg Asn Met Ser Ala Ile Leu
               100                 105                 110
   Cys Ala Leu Ile Trp Val Leu Phe Phe Leu Met Gly Ile Leu Gly Gly
           115                 120                 125
   Ser Ser Asp Phe Trp Val Lys Leu Ile Ile Asp Phe Ile Ile Pro Ala
       130                 135                 140
   Phe Leu Ile Phe Phe Leu Phe Met Leu Leu Ser Gly Ser Ile Leu Ala
   145                 150                 155                 160
   Leu Leu Leu Arg Ile Leu Tyr Gly Ser Arg Arg Lys Ser Leu Ser Arg
                   165                 170                 175
   Leu Tyr Val Asn Ile Ser Leu Thr Val Met Val Tyr Leu Ile Cys Gly
               180                 185                 190
   Leu Pro Leu Gly Leu Tyr Leu Val Leu Leu Tyr Cys Phe Gly Val His
           195                 200                 205
   Leu His His Pro Ser Pro His Ile Tyr Gln Val Thr Val Val Leu Ser
       210                 215                 220
   Tyr Val Asp Ser Ser Ala Asn His Ile Phe Tyr Phe Leu Ala Gly Ser
   225                 230                 235                 240
   Phe Arg Tyr Cys Arg Lys His Trp Ser Leu Gln Thr Leu Leu Lys Arg
                   245                 250                 255
   Thr Leu Glu Asp Thr Pro
               260
<210> 100
   <211> 1290
   <212> DNA
   <213> Mus musculus
<400> 100
   cctctggcta ggtgactgac aggtgcagct tggtcatctc aagggaggag gttactgcat 60
   ttgatctata atctgttgca cagaccagtg tcttgtctcg acccagagtt aggtgtataa 120
   ctcagaatcc attcttttga ccgtgcaaaa gtatctttct cttgatgtac ctcaacaaaa 180
   ccctgatatc ttcatctttc ctgtgttatt ttaagccctg ggggagtaca aatctgatgc 240
   ttccctttct gtggttacag gtagagcagg aaatggatcc taccctgacc atgagagaag 300
   ggaatcattt ccatgtgatt aaaaggtcct gagttataca ctggaagtat gacccagact 360
   acagagtata cacaagctct gaatttgaat ccacagtcca gaattcttga tcaatgtagt 420
   catgttactc tccttttttt tataaatgat tttagcaagc catattgaca acaatatcta 480
   tattacatta tgatcgccag aagaaaggtc aatgttaagg tgatcaaaca tggtcttgtt 540
   ctcagggaca ccactggaag atttgtgcgc atggatccaa tcatcttatc ccacaacaca 600
   gaatcacact gctgaatgaa actggtcaac ccaacttcag tccaatcctg actctgtctc 660
   tctggtcctc atcactgtcc tgtttgaact ggcaggaaac accattgtac tctggctcct 720
   gggattccac atgcacaagg aaagtcatct cagtctatgt cctcaatctg gctcttgcag 780
   actccttctt cctcagctgc caattcattg actctctgct ttgaagcatt gacttcctct 840
   atgcatataa attaagcaaa gatatcttag gcaatgcagc aatcgttccc tatatcgcag 900
   ggctgagtat cctcagtgct attagcatgg agcactgcct gtctgtatag tggcaaatgc 960
   ggtaccactg ccactaccca agaaacatgt cagctatcct atgtgcccta atctgggttc
   1020
   tgtcttttct catggacatc ctggattggt tcttctcagg attcctgggt gagactcatc
   1080
   atcatttatg gaaaaatatt gacttcatta taactgcatt tctgattttt ttatttatgc
   1140
   ttctctctgg ctccagtctg gccctactgc tgaggattct ttatggcttc aagaggaaac
   1200
   ccctgtccag gctatatatt atcatctctc tcacagtgat ggtctacctc atctgggcct
   1260
   gccccttggg ctttcatttt tcctgttaca
   1290
<210> 101
   <211> 207
   <212> PRT
   <213> Mus musculus
<400> 101
   Leu Val Leu Ile Thr Val Leu Phe Glu Leu Ala Gly Asn Thr Ile Val
   1 5 10 15
   Leu Trp Leu Leu Gly Phe His Met Thr Arg Lys Val Ile Ser Val Tyr
   20 25 30
   Val Leu Asn Leu Ala Leu Ala Asp Ser Phe Phe Leu Ser Cys Gln Phe
   35 40 45
   Ile Asp Ser Leu Leu Ser Ile Asp Phe Leu Tyr Ala Tyr Lys Leu Ser
   50 55 60
   Lys Asp Ile Leu Gly Asn Ala Ala Ile Val Pro Tyr Ile Ala Gly Leu
   65 70 75 80
   Ser Ile Leu Ser Ala Ile Ser Met Glu His Cys Leu Ser Val Trp Gln
   85 90 95
   Met Arg Tyr His Cys His Tyr Pro Arg Asn Met Ser Ala Ile Leu Cys
   100 105 110
   Ala Leu Ile Trp Val Leu Ser Phe Leu Met Asp Ile Leu Asp Trp Phe
   115 120 125
   Phe Ser Gly Phe Leu Gly Glu Thr His His His Leu Trp Lys Asn Ile
   130 135 140
   Asp Phe Ile Ile Thr Ala Phe Leu Ile Phe Leu Phe Met Leu Leu Ser
   145 150 155 160
   Gly Ser Ser Leu Ala Leu Leu Leu Arg Ile Leu Tyr Gly Phe Lys Arg
   165 170 175
   Lys Pro Leu Ser Arg Leu Tyr Ile Ile Ile Ser Leu Thr Val Met Val
   180 185 190
   Tyr Leu Ile Leu Gly Leu Pro Leu Gly Leu Ser Phe Phe Leu Leu
   195 200 205
<210> 102
   <211> 1389
   <212> DNA
   <213> Mus musculus
<400> 102
   ttaaggtgat caaatatggc ctgttttctc agggacacca ctggaagatt tttaaacatg 60
   gatccaaaca tctcatccca caacacagaa tctactccac tgaatgaaac tggtcatcca 120
   aacttcagta caatactcac gctgtccttt ctggtcctcg tcactgtcct cgtggaactg 180
   gcaggaaaca ccattgtact ctggctcctg ggattccgca tgcacaggaa agccatctca 240
   gtctatgtcc tcaatctggc tctggcagac tccttcttct gctgccattt cattgactct 300
   ctgctatgga tcactgactt catctatacc cataaattaa gcaaagatat cttacgcaat 360
   gcagcaattg ttccctatat cgcaagactg agcgtcctca gtgctattag aatggagcac 420
   ttactgttta tattgtggcc aatctggtac cactgccacc acccaagaaa catatcagct 480
   atcctatgtg ccctaatctg ggttctgttc tttctcatgg gcatccttga ttggttcttc 540
   ttaggattcc tgggtgagac tcatcatcat ttgtggaaaa atattgactt tattatacct 600
   gcatttctga tttttttaat gctgctttct gggtccactc tggccctact gctgaggata 660
   ctttgtggtt ccaggaggaa actcctgtcc aggctgtatg ttaccatctc tctcacagtg 720
   atggtctacc tcatctgtgg catgcctctt gggctttact tgttcctgtt atactggttt 780
   gggattcatt tacactatcc ctcttgtcac atttaccaag ttactgcact cttgtcctat 840
   gtggacagct ctgccaacca catcttttat ttccttgtag gctcctttag gcattttaga 900
   aagcattggt ccctctaaac tattctaaag aggaccctgg agaacattcc tgaggaggat 960
   gaatatacag acagctatct tcagaatacc actgagatgt cagaaatcag atgttgagag
   1020
   tcaacacatt aacttactct tctctcagaa acgcctcagt gattgcaacg ctttcaattt
   1080
   ttttgtttgt ttggtttttt tttttttgga ttgttttaaa ttaggtattt tggtatttta
   1140
   catttccaaa tttatattta tacttccaaa agtcccccat accttcccct gccaatcccc
   1200
   tacccacttt ttggccctgg cgtttccctg tactggggca tataaagttt gcaagtccag
   1260
   tgggcctctc tttccagtga tggcctacta agccatcttt tgatacatat gcagctagag
   1320
   tcaagagctc cagggtactg attaattcat aatgttgttc cacctatagg gttgcagatc
   1380
   cctttagca
   1389
<210> 103
   <211> 206
   <212> PRT
   <213> Mus musculus
<400> 103
   Phe Phe Cys Cys His Phe Ile Asp Ser Leu Leu Trp Ile Thr Asp Phe
    1               5                  10                  15
   Ile Tyr Thr His Lys Leu Ser Lys Val Tyr Leu Thr Gln Cys Ser Asn
               20                  25                  30
   Phe Pro Tyr Ile Ala Arg Leu Ser Val Leu Ser Ala Ile Arg Met Glu
           35                  40                  45
   His Leu Leu Phe Ile Leu Trp Pro Ile Trp Tyr His Cys His His Pro
       50                  55                  60
   Arg Asn Ile Ser Ala Ile Leu Cys Ala Leu Ile Trp Val Leu Phe Phe
   65                  70                  75                  80
   Leu Met Gly Ile Leu Asp Trp Phe Phe Leu Gly Phe Leu Gly Glu Thr
                   85                  90                  95
   His His His Leu Trp Lys Asn Ile Asp Phe Ile Ile Pro Ala Phe Leu
               100                 105                 110
   Ile Phe Leu Met Leu Leu Ser Gly Ser Thr Leu Ala Leu Leu Leu Arg
           115                 120                 125
   Ile Leu Cys Gly Ser Arg Arg Lys Leu Leu Ser Arg Leu Tyr Val Thr
       130                 135                 140
   Ile Ser Leu Thr Val Met Val Tyr Leu Ile Cys Gly Met Pro Leu Gly
   145                 150                 155                 160
   Leu Tyr Leu Phe Leu Leu Tyr Trp Phe Gly Ile His Leu His Tyr Pro
                   165                 170                 175
   Ser Cys His Ile Tyr Gln Val Thr Ala Leu Leu Ser Tyr Val Asp Ser
               180                 185                 190
   Ser Ala Asn His Ile Phe Tyr Phe Leu Val Gly Ser Phe Arg
           195                 200                 205
<210> 104
   <211> 1420
   <212> DNA
   <213> Mus musculus
<400> 104
   aaaaaggaac cttacacttt tctgagttag tgtgcattca gagaatcaga cagtcttaac 60
   tgtaccccct gagggaaggt cagagatggc tgcatagagg gtgcaactcc tgtgaaggat 120
   gagtgaattg tcattccttc tgccatctta gcaatcccct ggccaggtga ctgacaggta 180
   caacattgtc aactcaaggg aggakrtaaa tgyrtgtgat ccttaatcta gagcacagac 240
   cagagtcaca tmtcaaccca gagttagggg tagaaytcag aatccattct tttgatgatg 300
   aggaagtatc tttcccttaa tatgcctcaa caaaaccctg atatcatcat cttttctgtg 360
   tcattttaag ccctggggag gtaaatgtga tgcttccctt tctggagtta ccaaggtggc 420
   aggaaatgga tccaaccctg accatgaaaa aaggaaatcg tttccatgtg aattaaagat 480
   cctgagttat acacaggaag aatgatgcag actatagagt aaacacaagc tctaaatttg 540
   aatccacagt ccagaattct taatcccatg tggtcatgtt actttccttt tatttataaa 600
   tcattttatt taataatgtt gacaagaata tctatattay rttatgattg ccagaagaag 660
   ggtcagtgtt aatgtgctca aatatggtct gtgttctcag ggacacaact ggaagatttg 720
   tgagcatgga ttcaaccatc tcatcccaca acacaawatc tacacaactg aatgaaactg 780
   stratcctaa ctgcagtcca atcctgacmc tgyccttcct ggccctcatc actgccctgg 840
   tttgactggc agaaaacact attatactct gactcctggg attccccatg cacaggaaag 900
   ccatctcagt ctatatcctc aaccaggctc tggcagactc cttcttcctc tgctgtcact 960
   tccttgactc tatgctacag atcattgact tctatggcat ctatggccat aaattaagca
   1020
   aagatatctt aggcaatgca gcaatcattc cctatatcac agggctgagc gtcctcagtg
   1080
   ctattagcac tgcctgtcta tattgtggcc aatctggtac cattgccacc acccaagaaa
   1140
   catgtcaggt atcatatgtg ccctaatctg ggttctgtcc tttctcatgg gcatccttga
   1200
   ttggttcttc tcaggattcc tgggtgagac tcattatcat ttgtgggaaa atgttgactt
   1260
   tattataact gcatttttta tttatgcttc tctctgggtc tactcatgag gatcctctgt
   1320
   ggaggaaacc cctgtccagg ctgtatgtta ccatctctct cacagtgatg ggctacctca
   1380
   tctgtggcct gcctcttggg ctttacttgt ctctgttaca
   1420
<210> 105
   <211> 200
   <212> PRT
   <213> Mus musculus
<400> 105
   Phe Leu Ala Leu Ile Thr Ala Leu Val Leu Ala Glu Asn Thr Ile Ile
    1               5                  10                  15
   Leu Leu Leu Gly Phe Pro Met His Arg Lys Ala Ile Ser Val Tyr Ile
               20                  25                  30
   Leu Asn Gln Ala Leu Ala Asp Ser Phe Phe Leu Cys Cys His Phe Leu
           35                  40                  45
   Asp Ser Met Leu Gln Ile Ile Asp Phe Tyr Gly Ile Tyr Gly His Lys
       50                  55                  60
   Leu Ser Lys Asp Ile Leu Gly Asn Ala Ala Ile Ile Pro Tyr Ile Thr
   65                  70                  75                  80
   Gly Leu Ser Val Leu Ser Ala Ile Ser Thr Asp Leu Ser Ile Leu Trp
                   85                  90                  95
   Pro Ile Trp Tyr His Cys His His Pro Arg Asn Met Ser Gly Ile Ile
               100                 105                 110
   Cys Ala Leu Ile Trp Val Leu Ser Phe Leu Met Gly Ile Leu Asp Trp
           115                 120                 125
   Phe Phe Ser Gly Phe Leu Gly Glu Thr His Tyr His Leu Trp Glu Asn
       130                 135                 140
   Val Asp Phe Ile Ile Thr Ala Phe Phe Ile Val Cys Phe Ser Leu Gly
   145                 150                 155                 160
   Leu Leu Met Arg Ile Leu Cys Gly Gly Ile Pro Leu Ser Arg Leu Tyr
                   165                 170                 175
   Val Thr Ile Ser Leu Thr Val Met Gly Tyr Leu Ile Cys Gly Leu Pro
               180                 185                 190
   Leu Gly Leu Tyr Leu Ser Leu Leu
           195                 200
<210> 106
   <211> 730
   <212> DNA
   <213> Mus musculus
<400> 106
   tgtgatctgt gttctcaggg acaccgctgg aagcatttgt gagcatggat ccaatcatct 60
   catcccacaa cacagaatca caccactgaa tgaaactggt catcccaact gcagtccaat 120
   cctgacacca ttctttctgg tcctcatcac tgtactggtg gaattggcag gggaacacca 180
   ttatactctg gctcctggga tttcgcatga acaggaaagc aatctcagtt tatgtcctca 240
   atctggctct ggcagactcc ttcttttcct ctgttgccat ttcattgact ctctgctaca 300
   gaacattgac ttcatcaatg cccataaatt aagcaaacat atcttaggaa atgcagcaat 360
   cattccctat attgcagggc tgagcctcct cagtgctatt agcatggagc actgcctgtt 420
   tatattatgg ccaatctggt accactgcca ccacatgtca gctatcatat gtgccctaat 480
   ctgggttccg tcctttctca agggcatcct caatttgttc ttctcaggat tcctgggtga 540
   gactcatcat catttgtggg aaaatattga ctttattata actgcatttc tgattttttt 600
   atttatgctt ctctgtgggt gcactttggc cctagagctg aggatactct gtggctccag 660
   gaagaaaccc ctgtccaggc tgtaagttac catctctctc acagcgatgg tctacctcat 720
   ctgtggcctg                                                        730
<210> 107
   <211> 198
   <212> PRT
   <213> Mus musculus
<400> 107
   Phe Leu Val Leu Ile Thr Val Leu Val Glu Leu Ala Gly Asn Thr Ile
   1               5                   10                  15
   Ile Leu Trp Leu Leu Gly Phe Arg Met Asn Arg Lys Ala Ile Ser Val
               20                  25                  30
   Tyr Val Leu Asn Leu Ala Leu Ala Asp Ser Phe Val Phe Leu Cys Cys
           35                  40                  45
   His Phe Ile Asp Ser Leu Leu Gln Asn Ile Asp Phe Ile Asn Ala His
       50                  55                  60
   Lys Leu Ser Lys His Ile Leu Gly Asn Ala Ala Ile Ile Pro Tyr Ile
   65                  70                  75                  80
   Ala Gly Leu Ser Leu Leu Ser Ala Ile Ser Met Glu His Cys Leu Phe
                   85                  90                  95
   Ile Leu Trp Pro Ile Trp Tyr His Cys His His Met Ser Ala Ile Ile
               100                 105                 110
   Cys Ala Leu Ile Trp Val Pro Ser Phe Leu Lys Gly Ile Leu Asn Leu
           115                 120                 125
   Phe Phe Ser Gly Phe Leu Gly Glu Thr His His His Leu Trp Glu Asn
       130                 135                 140
   Ile Asp Phe Ile Ile Thr Ala Phe Leu Ile Phe Leu Phe Met Leu Leu
   145                 150                 155                 160
   Cys Gly Cys Thr Leu Ala Leu Glu Leu Arg Ile Leu Cys Gly Ser Arg
                   165                 170                 175
   Lys Lys Pro Leu Ser Arg Leu Val Thr Ile Ser Leu Thr Ala Met Val
               180                 185                 190
   Tyr Leu Ile Cys Gly Leu
           195
<210> 108
   <211> 847
   <212> DNA
   <213> Mus musculus
<400> 108
   ttcagaattc ttgatccatg tggtcatgtt actccccttt tattaataaa tgagtacatt 60
   aagccatatt gaaaacaata tctatattat attatgattg cccgaagaag ggtcaatgtt 120
   aaggtgatca aatatggcct gttttcctca gggacaccaa tgggtgattt gtttagcatg 180
   gatccaacca tctcatccca caacacagaa tcacaccact gaatgaacct ggcccatccc 240
   gactgcaatc caatcctggt tctgtccttt ctggtcctca tcgctgtcct ggtggaactg 300
   gcaggaaaca ccattgttct ctggctcctg ggattccgca tgcacaggaa acccatctca 360
   gtctatgtcc tcaatctggc tctggcagac tccttcttcc tctgctgcca tttcattgac 420
   tctctgctac aaatcattga cttcacctat gcccataaat taagcaaaga tatcttagac 480
   aatgcagcaa ttgttccctt tatcacaggg ctgagggtcc tcagtgctat tagcatggag 540
   cactgcctgt ctgtattgtg gctaatctgg taccactgcc accacctgag aaatatgtca 600
   gctatcctat gtgccctaat ctgggttctg tcctttctca tgtccatcct ggactagttc 660
   ttctcagaat tcctgcatga gactcatcat catttgtggg aaaatgttga ctttattata 720
   actgcatttc tgattttttt atttatgctt ctctttaggt ccagtctggc cctactgcgg 780
   aggatcctcc tgtggctcca ggaggaaata cctgtccacg ctatatgtta tcatttctct 840
   cacagtg                                                           847
<210> 109
   <211> 192
   <212> PRT
   <213> Mus musculus
<400> 109
   Phe Leu Val Leu Ile Ala Val Leu Val Glu Leu Ala Gly Asn Thr Ile
    1              5                   10                  15
   Val Leu Trp Leu Leu Gly Phe Arg Met His Arg Lys Pro Ile Ser Val
               20                  25                  30
   Tyr Val Leu Asn Leu Ala Leu Ala Asp Ser Phe Phe Leu Cys Cys His
           35                  40                  45
   Phe Ile Asp Ser Leu Leu Gln Ile Ile Asp Phe Thr Tyr Ala His Lys
       50                  55                  60
   Leu Ser Lys Asp Ile Leu Asp Asn Ala Ala Ile Val Pro Phe Ile Thr
   65                  70                  75                  80
   Gly Leu Arg Val Leu Ser Ala Ile Ser Met Glu His Cys Leu Ser Val
                   85                  90                  95
   Leu Trp Leu Ile Trp Tyr His Cys His His Leu Arg Asn Met Ser Ala
               100                 105                 110
   Ile Leu Cys Ala Leu Ile Trp Val Leu Ser Phe Leu Met Ser Ile Leu
           115                 120                 125
   Asp Phe Phe Ser Glu Phe Leu His Glu Thr His His His Leu Trp Glu
       130                 135                 140
   Asn Val Asp Phe Ile Ile Thr Ala Phe Leu Ile Phe Leu Phe Met Leu
   145                 150                 155                 160
   Leu Phe Arg Ser Ser Leu Ala Leu Leu Arg Arg Ile Leu Cys Gly Ser
                   165                 170                 175
   Arg Arg Lys Tyr Leu Ser Thr Leu Tyr Val Ile Ile Ser Leu Thr Val
               180                 185                 190